(19) Europäisches Patentamt
European Patent Office
Office européen des brevets

(11) EP 1 670 770 B1

(12) **EUROPEAN PATENT SPECIFICATION**

(45) Date of publication and mention of the grant of the patent:
**20.08.2008 Bulletin 2008/34**

(21) Application number: **04768669.6**

(22) Date of filing: **24.09.2004**

(51) Int Cl.:
*C07D 235/08* (2006.01) *C07D 401/12* (2006.01)
*C07D 405/06* (2006.01) *C07F 7/18* (2006.01)
*A61K 31/4184* (2006.01) *A61P 25/00* (2006.01)

(86) International application number:
**PCT/GB2004/004126**

(87) International publication number:
**WO 2005/030733 (07.04.2005 Gazette 2005/14)**

(54) **BENZIMIDAZOLE DERIVATIVES, COMPOSITIONS CONTAINING THEM, PREPARATION THEROF AND USES THEREOF**

BENZIMIDAZOLDERIVATE, ZUSAMMENSETZUNGEN, DIE DIESE ENTHALTEN, ZUBEREITUNG DAVON UND DEREN VERWENDUNGEN

DERIVES DE BENZIMIDAZOLE, COMPOSITIONS CONTENANT CES DERIVES, PREPARATION ET UTILISATIONS DE CEUX-CI

(84) Designated Contracting States:
**AT BE BG CH CY CZ DE DK EE ES FI FR GB GR HU IE IT LI LU MC NL PL PT RO SE SI SK TR**

(30) Priority: **26.09.2003 SE 0302571**

(43) Date of publication of application:
**21.06.2006 Bulletin 2006/25**

(73) Proprietor: **AstraZeneca AB**
**151 85 Södertälje (SE)**

(72) Inventors:
- **PAGE, Daniel,**
  **AstraZeneca R & D Montréal**
  **St Laurent,**
  **Québec H4S 1Z9 (CA)**
- **LIU, Ziping,**
  **AstraZeneca R & D Montréal**
  **St Laurent, Québec H4S 1Z9 (CA)**
- **TREMBLAY, Maxime,**
  **AstraZeneca R & D Montréal**
  **St Laurent, Québec H4S 1Z9 (CA)**
- **WALPOLE, Christopher,**
  **AstraZeneca R & D Montréal**
  **St Laurent,**
  **Québec H4S 1Z9 (CA)**
- **YANG, Hua,**
  **AstraZeneca R & D Montréal**
  **St Laurent,**
  **Québec H4S 1Z9 (CA)**

(56) References cited:
**WO-A-02/085866**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 670 770 B1

## Description

## BACKGROUND OF THE INVENTION

### 1. Field of the invention

The invention is related to therapeutic compounds, pharmaceutical compositions containing these compounds, manufacturing processes thereof and uses thereof. Particularly, the present invention is related to compounds that may be effective in treating pain, cancer, multiple sclerosis, Parkinson's disease, Huntington's chorea, Alzheimer's disease, anxiety disorders, gastrointestinal disorders and/or cardiovascular disorders.

### 2. Discussion of Relevant Technology

Pain management has been studied for many years. It is known that cannabinoid receptor (e.g., $CB_1$ receptor, $CB_2$ receptor) ligands including agonists, antagonists and inverse agonists produce relief of pain in a variety of animal models by interacting with $CB_1$ and/or $CB_2$ receptors. Generally, $CB_1$ receptors are located predominately in the central nervous system, whereas $CB_2$ receptors are located primarily in the periphery and are primarily restricted to the cells and tissues derived from the immune system.

While $CB_1$ receptor agonists, such as $\Delta^9$-tetrahydrocannabinol ($\Delta^9$-THC) and anadamide, are useful in anti-nociception models in animals, they tend to exert undesired CNS side-effects, e.g., psychoactive side effects, the abuse potential, drug dependence and tolerance, etc. These undesired side effects are known to be mediated by the $CB_1$ receptors located in CNS. There are lines of evidence, however, suggesting that CB1 agonists acting at peripheral sites or with limited CNS exposure can manage pain in humans or animals with much improved overall in vivo profile.

Therefore, there is a need for new $CB_1$ receptor ligands such as agonists that may be useful in managing pain or treating other related symptoms or diseases with reduced or minimal undesirable CNS side-effects.

## DESCRIPTION OF THE EMBODIMENTS

The present invention provides $CB_1$ receptor ligands which may be useful in treating pain and/or other related symptoms or diseases.

Unless specified otherwise within this specification, the nomenclature used in this specification generally follows the examples and rules stated in Nomenclature of Organic Chemistry, Sections A, B, C, D, E, F, and H, Pergamon Press, Oxford, 1979, which is incorporated by references herein for its exemplary chemical structure names and rules on naming chemical structures.

"$CB_1$/$CB_2$ receptors" means $CB_1$ and/or $CB_2$ receptors.

The term "$C_{m-n}$" or "$C_{m-n}$ group" used alone or as a prefix, refers to any group having m to n carbon atoms.

The term "hydrocarbon" used alone or as a suffix or prefix, refers to any structure comprising only carbon and hydrogen atoms up to 14 carbon atoms. The term "hydrocarbon radical" or "hydrocarbyl" used alone or as a suffix or prefix, refers to any structure as a result of removing one or more hydrogens from a hydrocarbon.

The term "alkyl" used alone or as a suffix or prefix, refers to monovalent straight or branched chain hydrocarbon radicals comprising 1 to about 12 carbon atoms. Unless otherwise specified, "alkyl" general includes both saturated alkyl and unsaturated alkyl.

The term "alkylene" used alone or as suffix or prefix, refers to divalent straight or branched chain hydrocarbon radicals comprising 1 to about 12 carbon atoms, which serves to links two structures together.

The term "alkenyl" used alone or as suffix or prefix, refers to a monovalent straight or branched chain hydrocarbon radical having at least one carbon-carbon double bond and comprising at least 2 up to about 12 carbon atoms.

The term "alkynyl" used alone or as suffix or prefix, refers to a monovalent straight or branched chain hydrocarbon radical having at least one carbon-carbon triple bond and comprising at least 2 up to about 12 carbon atoms.

The term "cycloalkyl," used alone or as suffix or prefix, refers to a monovalent ring-containing hydrocarbon radical comprising at least 3 up to about 12 carbon atoms. "Cycloalkyl" includes both monocyclic and multicyclic hydrocarbon structures. Multicyclic hydrocarbon structure includes non-fused, fused and bridged rings.

The term "cycloalkenyl" used alone or as suffix or prefix, refers to a monovalent ring-containing hydrocarbon radical having at least one carbon-carbon double bond and comprising at least 3 up to about 12 carbon atoms. "Cycloalkenyl" includes both monocyclic and multicyclic hydrocarbon structures. Multicyclic hydrocarbon structure includes non-fused, fused and bridged rings.

The term "cycloalkynyl" used alone or as suffix or prefix, refers to a monovalent ring-containing hydrocarbon radical having at least one carbon-carbon triple bond and comprising about 7 up to about 12 carbon atoms. "Cycloalkenyl" includes both monocyclic and multicyclic hydrocarbon structures. Multicyclic hydrocarbon structure includes non-fused,

fused and bridged rings.

The term "aryl" used alone or as suffix or prefix, refers to a hydrocarbon radical having one or more polyunsaturated carbon rings having aromatic character, (*e.g.*, 4n + 2 delocalized electrons) and comprising 5 up to about 14 carbon atoms, wherein the radical is located on a carbon of the aromatic ring.

The term "non-aromatic group" or "non-aromatic" used alone, as suffix or as prefix, refers to a chemical group or radical that does not contain a ring having aromatic character (*e.g.*, 4n + 2 delocalized electrons).

The term "arylene" used alone or as suffix or prefix, refers to a divalent hydrocarbon radical having one or more polyunsaturated carbon rings having aromatic character, (*e.g.*, 4n + 2 delocalized electrons) and comprising 5 up to about 14 carbon atoms, which serves to link two structures together.

The term "heterocycle" used alone or as a suffix or prefix, refers to a ring-containing structure or molecule having one or more multivalent heteroatoms, independently selected from N, O, P and S, as a part of the ring structure and including at least 3 and up to about 20 atoms in the ring(s). Heterocycle may be saturated or unsaturated, containing one or more double bonds, and heterocycle may contain more than one ring. When a heterocycle contains more than one ring, the rings may be fused or unfused. Fused rings generally refer to at least two rings share two atoms therebetween. Heterocycle may have aromatic character or may not have aromatic character.

The term "heteroalkyl" used alone or as a suffix or prefix, refers to a radical formed as a result of replacing one or more carbon atom of an alkyl with one or more heteroatoms selected from N, O, P and S.

The term "heteroaromatic" used alone or as a suffix or prefix, refers to a ring-containing structure or molecule having one or more multivalent heteroatoms, independently selected from N, O, P and S, as a part of the ring structure and including at least 3 and up to about 20 atoms in the ring(s), wherein the ring-containing structure or molecule has an aromatic character (e.g., 4n + 2 delocalized electrons).

The term "heterocyclic group," "heterocyclic moiety," "heterocyclic," or "heterocyclo" used alone or as a suffix or prefix, refers to a radical derived from a heterocycle by removing one or more hydrogens therefrom.

The term "heterocyclyl" used alone or as a suffix or prefix, refers a radical derived from a heterocycle by removing one hydrogen from a carbon of a ring of the heterocycle.

The term "heterocyclylene" used alone or as a suffix or prefix, refers to a divalent radical derived from a heterocycle by removing two hydrogens therefrom, which serves to links two structures together.

The term "heteroaryl" used alone or as a suffix or prefix, refers to a heterocyclyl having aromatic character, wherein the radical of the heterocyclyl is located on a carbon of an aromatic ring of the heterocyclyl. A heteroaryl may contain both aromatic and non-aromatic rings therein. These rings may be fused or otherwised linked together.

The term "heterocylcoalkyl" used alone or as a suffix or prefix, refers to a heterocyclyl that does not have aromatic character.

The term "heteroarylene" used alone or as a suffix or prefix, refers to a heterocyclylene having aromatic character.

The term "heterocycloalkylene" used alone or as a suffix or prefix, refers to a heterocyclylene that does not have aromatic character.

The term "six-membered" used as prefix refers to a group having a ring that contains six ring atoms.

The term "five-membered" used as prefix refers to a group having a ring that contains five ring atoms.

A five-membered ring heteroaryl is a heteroaryl with a ring having five ring atoms wherein 1, 2 or 3 ring atoms are independently selected from N, O and S.

Exemplary five-membered ring heteroaryls are thienyl, furyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, and 1,3,4- oxadiazolyl.

A six-membered ring heteroaryl is a heteroaryl with a ring having six ring atoms wherein 1, 2 or 3 ring atoms are independently selected from N, O and S.

Exemplary six-membered ring heteroaryls are pyridyl, pyrazinyl, pyrimidinyl, triazinyl and pyridazinyl.

The term "substituted" used as a prefix refers to a structure, molecule or group, wherein one or more hydrogens are replaced with one or more $C_{1-12}$hydrocarbon groups, or one or more chemical groups containing one or more heteroatoms selected from N, O, S, F, Cl, Br, I, and P. Exemplary chemical groups containing one or more heteroatoms include heterocyclyl, $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$, -S(=O)R, - CN, -OH, -C(=O)OR, $-C(=O)NR_2$, -NRC(=O)R, oxo (=O), imino (=NR), thio (=S), and oximino (=N-OR), wherein each "R" is a $C_{1-12}$hydrocarbyl. For example, substituted phenyl may refer to nitrophenyl, pyridylphenyl, methoxyphenyl, chlorophenyl, aminophenyl, etc., wherein the nitro, pyridyl, methoxy, chloro, and amino groups may replace any suitable hydrogen on the phenyl ring.

The term "substituted" used as a suffix of a first structure, molecule or group, followed by one or more names of chemical groups refers to a second structure, molecule or group, which is a result of replacing one or more hydrogens of the first structure, molecule or group with the one or more named chemical groups. For example, a "phenyl substituted by nitro" refers to nitrophenyl.

The term "optionally substituted" refers to both groups, structures, or molecules that are substituted and those that are not substituted.

Heterocycle includes, for example, monocyclic heterocycles such as: aziridine, oxirane, thiirane, azetidine, oxetane, thietane, pyrrolidine, pyrroline, imidazolidine, pyrazolidine, pyrazoline, dioxolane, sulfolane 2,3-dihydrofuran, 2,5-dihydrofuran tetrahydrofuran, thiophane, piperidine, 1,2,3,6-tetrahydro-pyridine, piperazine, morpholine, thiomorpholine, pyran, thiopyran, 2,3-dihydropyran, tetrahydropyran, 1,4-dihydropyridine, 1,4-dioxane, 1,3-dioxane, dioxane, homopiperidine, 2,3,4,7-tetrahydro-1*H*-azepine homopiperazine, 1,3-dioxepane, 4,7-dihydro-1,3-dioxepin, and hexamethylene oxide.

In addition, heterocycle includes aromatic heterocycles, for example, pyridine, pyrazine, pyrimidine, pyridazine, thiophene, furan, furazan, pyrrole, imidazole, thiazole, oxazole, pyrazole, isothiazole, isoxazole, 1,2,3-triazole, tetrazole, 1,2,3-thiadiazole, 1,2,3-oxadiazole, 1,2,4-triazole, 1,2,4-thiadiazole, 1,2,4-oxadiazole, 1,3,4-triazole, 1,3,4-thiadiazole, and 1,3,4- oxadiazole.

Additionally, heterocycle encompass polycyclic heterocycles, for example, indole, indoline, isoindoline, quinoline, tetrahydroquinoline, isoquinoline, tetrahydroisoquinoline, 1,4-benzodioxan, coumarin, dihydrocoumarin, benzofuran, 2,3-dihydrobenzofuran, isobenzofuran, chromene, chroman, isochroman, xanthene, phenoxathiin, thianthrene, indolizine, isoindole, indazole, purine, phthalazine, naphthyridine, quinoxaline, quinazoline, cinnoline, pteridine, phenanthridine, perimidine, phenanthroline, phenazine, phenothiazine, phenoxazine, 1,2-benzisoxazole, benzothiophene, benzoxazole, benzthiazole, benzimidazole, benztriazole, thioxanthine, carbazole, carboline, acridine, pyrolizidine, and quinolizidine.

In addition to the polycyclic heterocycles described above, heterocycle includes polycyclic heterocycles wherein the ring fusion between two or more rings includes more than one bond common to both rings and more than two atoms common to both rings. Examples of such bridged heterocycles include quinuclidine, diazabicyclo[2.2.1]heptane and 7-oxabicyclo[2.2.1]heptane.

Heterocyclyl includes, for example, monocyclic heterocyclyls, such as: aziridinyl, oxiranyl, thiiranyl, azetidinyl, oxetanyl, thietanyl, pyrrolidinyl, pyrrolinyl, imidazolidinyl, pyrazolidinyl, pyrazolinyl, dioxolanyl, sulfolanyl, 2,3-dihydrofuranyl, 2,5-dihydrofuranyl, tetrahydrofuranyl, thiophanyl, piperidinyl, 1,2,3,6-tetrahydro-pyridinyl, piperazinyl, morpholinyl, thiomorpholinyl, pyranyl, thiopyranyl, 2,3-dihydropyranyl, tetrahydropyranyl, 1,4-dihydropyridinyl, 1,4-dioxanyl, 1,3-dioxanyl, dioxanyl, homopiperidinyl, 2,3,4,7-tetrahydro-1*H*-azepinyl, homopiperazinyl, 1,3-dioxepanyl, 4,7-dihydro-1,3-dioxepinyl, and hexamethylene oxidyl. In addition, heterocyclyl includes aromatic heterocyclyls or heteroaryl, for example, pyridinyl, pyrazinyl, pyrimidinyl, pyridazinyl, thienyl, furyl, furazanyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, and 1,3,4 oxadiazolyl.

Additionally, heterocyclyl encompasses polycyclic heterocyclyls (including both aromatic or non-aromatic), for example, indolyl, indolinyl, isoindolinyl, quinolinyl, tetrahydroquinolinyl, isoquinolinyl, tetrahydroisoquinolinyl, 1,4-benzodioxanyl, coumarinyl, dihydrocoumarinyl, benzofuranyl, 2,3-dihydrobenzofuranyl, isobenzofuranyl, chromenyl, chromanyl, isochromanyl, xanthenyl, phenoxathiinyl, thianthrenyl, indolizinyl, isoindolyl, indazolyl, purinyl, phthalazinyl, naphthyridinyl, quinoxalinyl, quinazolinyl, cinnolinyl, pteridinyl, phenanthridinyl, perimidinyl, phenanthrolinyl, phenazinyl, phenothiazinyl, phenoxazinyl, 1,2-benzisoxazolyl, benzothiophenyl, benzoxazolyl, benzthiazolyl, benzimidazolyl, benztriazolyl, thioxanthinyl, carbazolyl, carbolinyl, acridinyl, pyrolizidinyl, and quinolizidinyl.

In addition to the polycyclic heterocyclyls described above, heterocyclyl includes polycyclic heterocyclyls wherein the ring fusion between two or more rings includes more than one bond common to both rings and more than two atoms common to both rings. Examples of such bridged heterocyclyls include quinuclidinyl, diazabicyclo[2.2.1]heptyl; and 7-oxabicyclo[2.2.1]heptyl.

The term "alkoxy" used alone or as a suffix or prefix, refers to radicals of the general formula -O-R, wherein -R is selected from a hydrocarbon radical. Exemplary alkoxy includes methoxy, ethoxy, propoxy, isopropoxy, butoxy, t-butoxy, isobutoxy, cyclopropylmethoxy, allyloxy, and propargyloxy.

The term "aryloxy" used alone or as suffix or prefix, refers to radicals of the general formula -O-Ar, wherein -Ar is an aryl.

The term "heteroaryloxy" used alone or as suffix or prefix, refers to radicals of the general formula -O-Ar', wherein -Ar' is a heteroaryl.

The term "amine" or "amino" used alone or as a suffix or prefix, refers to radicals of the general formula -NRR', wherein R and R' are independently selected from hydrogen or a hydrocarbon radical.

"Acyl" used alone, as a prefix or suffix, means -C(=O)-R, wherein -R is an optionally substituted hydrocarbyl, hydrogen, amino or alkoxy. Acyl groups include, for example, acetyl, propionyl, benzoyl, phenyl acetyl, carboethoxy, and dimethylcarbamoyl.

Halogen includes fluorine, chlorine, bromine and iodine.

"Halogenated," used as a prefix of a group, means one or more hydrogens on the group is replaced with one or more halogens.

"RT" or "rt" means room temperature.

A first ring group being "fused" with a second ring group means the first ring and the second ring share at least two atoms therebetween.

"Link," "linked," or "linking," unless otherwise specified, means covalently linked or bonded.

When a first group, structure, or atom is "directly connected" to a second group, structure or atom, at least one atom of the first group, structure or atom forms a chemical bond with at least one atom of the second group, structure or atom.

"Saturated carbon" means a carbon atom in a structure, molecule or group wherein all the bonds connected to this carbon atom are single bond. In other words, there is no double or triple bonds connected to this carbon atom and this carbon atom generally adopts an $sp^3$ atomic orbital hybridization.

"Unsaturated carbon" means a carbon atom in a structure, molecule or group wherein at least one bond connected to this carbon atom is not a single bond. In other words, there is at least one double or triple bond connected to this carbon atom and this carbon atom generally adopts a *sp* or $sp^2$ atomic orbital hybridization.

In one aspect, an embodiment of the invention provides a compound of Formula I, a pharmaceutically acceptable salt thereof, diastereomers, enantiomers, or mixtures thereof:

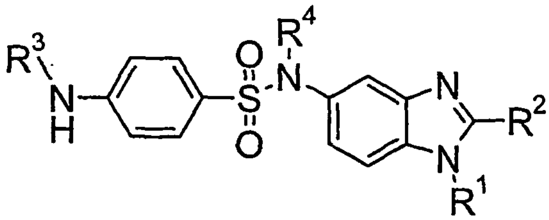

**I**

wherein

$R^1$ is selected from $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloalkyl, wherein said $C_{1-10}$alkyl, $C_{2-10}$alkenyl, $C_{3-10}$ocycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloalkyl used in defining $R^1$ is optionally substituted by one or more groups selected from halogen, cyano, nitro, methoxy, ethoxy, methyl, ethyl, hydroxy, amino, $C_{1-6}$alkylamino and di$C_{1-6}$alkylamino;

$R^2$ is selected from $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl, wherein said $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl used in defining $R^2$ is optionally substituted by one or more groups selected from halogen, methoxy, ethoxy, methyl, ethyl, hydroxy, amino, $C_{1-6}$alkylamino and di$C_{1-6}$alkylamino;

$R^3$ is selected from -H, $C_{1-6}$alkyl, $C_{2-6}$alkenyl, and $C_{1-6}$acyl, wherein said $C_{1-6}$alkyl, $C_{2-6}$alkenyl, and $C_{1-6}$acyl used in defining $R^3$ is optionally substituted with one or more groups selected from $CH_3C(=O)$-O-, halogen, cyano, methoxy, ethoxy, hydroxy, amino, alkylamino, dialkylamino, and $C_3$-$_6$heterocycloalkyl; and

$R^4$ is selected from -H, $C_{1-6}$alkyl, $C_{2-6}$alkenyl, $C_{3-6}$cycloalkyl, and $C_{3-6}$cycloalkyl-$C_{1-4}$alkyl.

Another embodiment of the invention provides a compound of Formula I, wherein

$R^1$ is selected from $C_{1-6}$alkyl, $C_{2-6}$alkenyl, $C_{3-6}$cycloalkyl-$C_{1-4}$alkyl, $C_{4-6}$cycloalkenyl-$C_{1-4}$alkyl and $C_{3-6}$heterocycloalkyl-$C_{1-4}$alkyl, wherein said $C_{1-6}$alkyl, $C_{2-6}$alkenyl, $C_{3-6}$cycloalkyl-$C_{1-4}$alkyl, $C_{4-6}$cycloalkenyl-$C_{1-4}$alkyl and $C_{3-6}$heterocycloalkyl-$C_{1-4}$alkyl used in defining $R^1$ is optionally substituted by one or more groups selected from halogen, methoxy, ethoxy, methyl, hydroxy, amino, $C_{1-6}$alkylamino and di$C_{1-6}$alkylamino;

$R^2$ is selected from $C_{1-6}$alkyl, $C_{2-6}$alkenyl, $C_{3-6}$cycloalkyl, $C_{3-6}$cycloalkyl-$C_{1-4}$alkyl, and $C_{4-6}$cycloalkenyl-$C_{1-4}$alkyl, wherein said $C_{1-6}$alkyl, $C_{2-6}$alkenyl, $C_{3-6}$cycloalkyl, $C_{3-6}$cycloalkyl-$C_{1-4}$alkyl, and $C_{4-6}$cycloalkenyl-$C_{1-4}$alkyl used in defining $R^2$ is optionally substituted by one or more groups selected from halogen, methoxy, ethoxy and hydroxy;

$R^3$ is selected from -H, $C_{1-6}$alkyl, $C_{2-6}$alkenyl, and $C_{1-6}$acyl, wherein said $C_{1-6}$alkyl, $C_{2-6}$alkenyl, and $C_{1-6}$acyl used in defining $R^3$ is optionally substituted with one or more groups selected from $CH_3C(=O)$-O-, halogen, methoxy, ethoxy, hydroxy, amino, methylamino, dimethylamino, and $C_{3-6}$heterocycloalkyl; and

$R^4$ is selected from -H and $C_{1-3}$alkyl.

A further embodiment of the invention provides a compound of Formula I,

wherein $R^1$ is selected from cyclopentyl-methyl, cyclohexyl-methyl, cyclobutyl-methyl, cyclopropyl-methyl, 4,4-difluorocyclohexyl-methyl, bicyclo[2.2.1]hept-5-en-2-yl-methyl, tetrahydropyranyl-methyl, tetrahydropyranyl-ethyl, tetrahydrofuranyl-methyl, morpholinyl-methyl, piperdinylethyl, N-methyl-piperdinylmethyl, and piperdinyl-methyl;

$R^2$ is selected from t-butyl, n-butyl, 2-methyl-2-butyl, isopentyl, 2-methoxy-2-propyl, 2-hydroxy-propyl, trifluoromethyl,

1,1-difluoroethyl, 2,2,2-trifluoroethyl, 1-methyl-propyl, 1,1-dimethyl-propyl, 1,1-dimethyl-3-buten-1-yl, ethyl, and 2-propyl; $R^3$ is selected from -H, $C_{1-6}$alkyl, and $C_{1-6}$acyl, wherein said $C_{1-6}$alkyl, and $C_{1-6}$acyl used in defining $R^3$ is optionally substituted with one or more groups selected from $CH_3C(=O)$-O-, halogen, methoxy, hydroxy, amino, methylamino, dimethylamino, pyrrolidinyl, piperidinyl and morpholinyl; and
$R^4$ is selected from -H and methyl.

An even further embodiment of the invention provides a compound of Formula I, wherein
$R^1$ is cyclohexyl-methyl, cyclobutyl-methyl, cyclopropyl-methyl, 4,4-difluorocyclohexyl-methyl, N-methylpiperidine-2-yl methyl, and tetrahydropyranylmethyl;
$R^2$ is t-butyl and 1,1-difluoroethyl;
$R^3$ is selected from -H, methyl, ethyl, propyl, 2-propyl, 2-hydroxyethyl, 2-methoxyethyl, formyl, acetyl, uriedo, N-isopropyl-ureido, ethylcarbonyl, 2-propylcarbonyl, t-butylcarbonyl, 2-amino-acetyl, 2-methylamino-acetyl, 2-dimethylamino-acetyl, 2-acetyloxy-acetyl, 2-hydroxy-acetyl, 2-bromo-acetyl, 2-(morpholin-1-yl)-acetyl, and 2-(pyrrolindin-1-yl)-acetyl; and
$R^4$ is selected from -H and methyl.

Another embodiment of the invention provides a compound of Formula IA, a pharmaceutically acceptable salt thereof, diastereomers, enantiomers, or mixtures thereof:

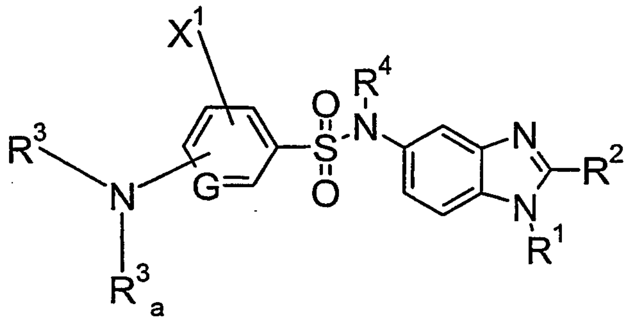

**IA**

wherein
G is CH or N;
$X^1$ is halogen;
$R^1$ is selected from $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloalkyl, wherein said $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloalkyl used in defining $R^1$ is optionally substituted by one or more groups selected from halogen, cyano, nitro, methoxy, ethoxy, methyl, ethyl, hydroxy, $CH_3C(=O)$-O-, amino, $C_{1-6}$alkylamino and di$C_{1-6}$alkylamino;
$R^2$ is selected from $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl, wherein said $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl used in defining $R^2$ is optionally substituted by one or more groups selected from halogen, $C_{3-5}$heteroaryl, methoxy, ethoxy, methyl, ethyl, hydroxy, amino, $C_{1-6}$alkylamino and di$C_{1-6}$alkylamino;
$R^3$ and $R^3{}_a$ are independently selected from -H, $C_{1-6}$alkyl, $C_{2-6}$alkenyl, $C_{1-3}$alkyl-O-C(=O)-, $C_{1-6}$alkyl-HN-C(=O)-, $H_2N$-C(=O)-, and $C_{1-6}$acyl, wherein said $C_{1-6}$alkyl, $C_{2-6}$alkenyl, and $C_{1-6}$acyl used in defining $R^3$ is optionally substituted with one or more groups selected from $CH_3C(=O)$-O-, halogen, cyano, methoxy, ethoxy, hydroxy, amino, $C_{1-6}$alkylamino, di$C_{1-6}$alkylamino, and $C_{3-6}$heterocycloalkyl; and
$R^4$ is selected from -H, $C_{1-6}$alkyl, $C_{2-6}$alkenyl, $C_{3-6}$cycloalkyl, and $C_{3-6}$cycloalkyl-$C_{1-4}$alkyl.

Another embodiment of the invention provides a compound of Formula IA, wherein
G is CH or N;
$X^1$ is halogen;
$R^1$ is selected from $C_{1-6}$alkyl, $C_{2-6}$alkenyl, $C_{3-6}$cycloakl-$C_{1-4}$alkyl, $C_{4-6}$cycloalkenyl-$C_{1-4}$alkyl and $C_{3-6}$heterocycloalkyl-$C_{1-4}$alkyl, wherein said $C_{1-6}$alkyl, $C_{2-6}$alkenyl, $C_{3-6}$cycloalkyl-$C_{1-4}$alkyl, $C_{4-6}$cycloalkenyl-$C_{1-4}$alkyl and $C_{3-6}$heterocycloalkyl-$C_{1-4}$alkyl used in defining $R^1$ is optionally substituted by one or more groups selected from halogen, methoxy, ethoxy, methyl, hydroxy, $CH_3C(=O)$-O-, amino, $C_{1-6}$alkylamino and di$C_{1-6}$alkylamino;
$R^2$ is selected from $C_{1-6}$alkyl, $C_{2-6}$alkenyl, $C_{3-6}$cycloalkyl, $C_{3-6}$cycloalkyl-$C_{1-4}$alkyl, and $C_{4-6}$cycloalkenyl-$C_{1-4}$alkyl, wherein said $C_{1-6}$alkyl, $C_{2-6}$alkenyl, $C_{3-6}$cycloalkyl, $C_{3-6}$cycloalkyl-$C_{1-4}$alkyl, and $C_{4-6}$cycloalkenyl-$C_{1-4}$alkyl used in defining $R^2$ is optionally substituted by one or more groups selected from halogen, $C_{3-5}$heteroaryl, methoxy, ethoxy and hydroxy;

$R^3$ is selected from -H, $C_{1-6}$alkyl, $C_{2-6}$alkenyl, $C_{1-3}$alkyl-O-C(=O)-, $C_{1-3}$alkyl-HN-C(=O)-, $H_2N$-C(=O)-, and $C_{1-6}$acyl, wherein said $C_{1-6}$alkyl, $C_{2-6}$alkenyl, and $C_{1-6}$acyl used in defining $R^3$ is optionally substituted with one or more groups selected from $CH_3C$(=O)-O-, halogen, methoxy, ethoxy, hydroxy, amino, methylamino, dimethylamino, and $C_{3-6}$heterocycloalkyl; and

$R^4$ is selected from -H and $C_{1-3}$alkyl.

A further embodiment of the invention provides a compound of Formula IA,

wherein G is CH or N; $X^1$ is halogen;

$R^1$ is selected from cyclopentyl-methyl, cyclohexyl-methyl, cyclobutyl-methyl, cyclopropyl-methyl, 4,4-difluorocyclohexanemethyl, bicyclo[2.2.1]hept-5-en-2-ylmethyl, tetrahydropyranyl-methyl, tetrahydropyranyl-ethyl, tetrahydrofuranyl-methyl, morpholinyl-methyl, piperdinylethyl, N-methyl-piperdinylmethyl, and piperdinyl-methyl;

$R^2$ is selected from t-butyl, n-butyl, 2-methyl-2-butyl, isopentyl, 2-methoxy-2-propyl, 2-hydroxy-propyl, trifluoromethyl, 1,1-difluoroethyl, 2,2,2-trifluoroethyl,1-methyl-propyl, 1,1-dimethyl-propyl, 1,1-dimethyl-3-buten-1-yl, ethyl, and 2-propyl;

$R^3$ is selected from -H, $C_{1-6}$alkyl, and $C_{1-6}$acyl, wherein said $C_{1-6}$alkyl, and $C_{1-6}$acyl used in defining $R^3$ is optionally substituted with one or more groups selected from $CH_3C$(=O)-O-, halogen, methoxy, hydroxy, amino, methylamino, dimethylamino, pyrrolidinyl, piperidinyl and morpholinyl; and

$R^4$ is selected from -H and methyl.

An even further embodiment of the invention provides a compound of Formula IA, wherein

G is CH or N; $X^1$ is bromo;

$R^1$ is cyclohexyl-methyl, cyclobutyl-methyl, 4,4-difluorocyclohexanemethyl, N-methylpiperidine-2-yl methyl, and tetrahydropyranyl-methyl;

$R^2$ is t-butyl and 1,1-difluoroethyl;

$R^3$ is selected from -H, methyl, ethyl, propyl, 2-propyl, 2-hydroxyethyl, 2-methoxyethyl, formyl, acetyl, uriedo, N-isopropylureido, ethylcarbonyl, 2-propylcarbonyl, t-butylcarbonyl, 2-amino-acetyl, 2-methylamino-acetyl, 2-dimethylamino-acetyl, 2-acetyloxy-acetyl, 2-hydroxy-acetyl, 2-bromo-acetyl, 2-(morpholin-1-yl)-acetyl, and 2-(pyrrolindin-1-yl)-acetyl; and

$R^4$ is selected from -H and methyl.

Another embodiment of the invention provides a compound of Formula IB, a pharmaceutically acceptable salt thereof, diastereomers, enantiomers, or mixtures thereof:

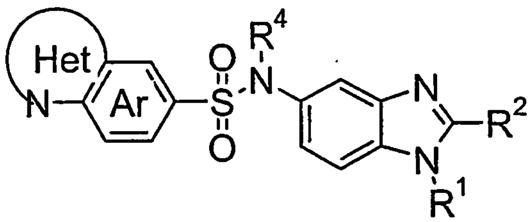

IB

wherein

$R^1$ is selected from $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloalkyl, wherein said $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloalkyl used in defining $R^1$ is optionally substituted by one or more groups selected from halogen, cyano, nitro, methoxy, ethoxy, methyl, ethyl, hydroxy, amino, $C_{1-6}$alkylamino and di$C_{1-6}$alkylamino;

$R^2$ is selected from $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl, wherein said $C_{1-10}$alkyl, $C_{2-10}$alkenyl, $C_{3-10}$cycloalkyl, $C_{3-10}$cycloakyl-$C_{1-4}$alkyl, and $C_{4-8}$cycloalkenyl-$C_{1-4}$alkyl used in defining $R^2$ is optionally substituted by one or more groups selected from halogen, methoxy, ethoxy, methyl, ethyl, hydroxy, amino, $C_{1-6}$alkylamino and di$C_{1-6}$alkylamino;

"Het" is a nitrogen (as shown in Formula IB) containing heterocycle ring that is fused with phenyl ring "Ar," wherein "Het" is optionally substituted with one or more groups selected from $C_{1-3}$alkyl, halogen, cyano, methoxy, ethoxy, hydroxy, and amino; and

$R^4$ is selected from -H, $C_{1-6}$alkyl, $C_{2-6}$alkenyl, $C_{3-6}$cycloalkyl, and $C_{3-6}$cycloalkyl-$C_{1-4}$alkyl.

Another embodiment of the invention provides a compound of Formula IB, wherein

$R^1$ is selected from $C_{1-6}$alkyl, $C_{2-6}$alkenyl, $C_{3-6}$cycloalkyl-$C_{1-4}$alkyl, $C_{4-6}$cycloalkenyl-$C_{1-4}$alkyl and $C_{3-6}$heterocycloalkyl-$C_{1-4}$alkyl, wherein said $C_{1-6}$alkyl, $C_{2-6}$alkenyl, $C_{3-6}$cycloalkyl-$C_{1-4}$alkyl, $C_{4-6}$cycloalkenyl-$C_{1-4}$alkyl and $C_{3-6}$heterocycloalkyl-$C_{1-4}$alkyl used in defining $R^1$ is optionally substituted by one or more groups selected from halogen,

methoxy, ethoxy, methyl, hydroxy, amino, $C_{1-6}$alkylamino and di$C_{1-6}$alkylamino;
$R^2$ is selected from $C_{1-6}$alkyl, $C_{2-6}$alkenyl, $C_{3-6}$cycloalkyl, $C_{3-6}$cycloalkyl-$C_{1-4}$alkyl, and $C_{4-6}$cycloalkenyl-$C_{1-4}$alkyl, wherein said $C_{1-6}$alkyl, $C_{2-6}$alkenyl, $C_{3-6}$cycloalkyl, $C_{3-6}$cycloalkyl-$C_{1-4}$alkyl, and $C_{4-6}$cycloalkenyl-$C_{1-4}$alkyl used in defining $R^2$ is optionally substituted by one or more groups selected from halogen, methoxy, ethoxy and hydroxy;
"Het" is morpholinyl, wherein said morpholinyl is optionally substituted with one or more groups selected from $C_{1-3}$alkyl, halogen, cyano, methoxy, ethoxy, hydroxy, and amino; and
$R^4$ is selected from -H and $C_{1-3}$alkyl.

A further embodiment of the invention provides a compound of Formula IB,
wherein $R^1$ is selected from cyclopentyl-methyl, cyclohexyl-methyl, cyclobutyl-methyl, cyclopropyl-methyl, 4,4-difluorocyclohexanemethyl, bicyclo[2.2.1]hept-5-en-2-ylmethyl, tetrahydropyranyl-methyl, tetrahydropyranyl-ethyl, tetrahydrofuranyl-methyl, morpholinyl-methyl, piperdinylethyl, N-methyl-piperdinylmethyl, and piperdinyl-methyl;
$R^2$ is selected from t-butyl, n-butyl, 2-methyl-2-butyl, isopentyl, 2-methoxy-2-propyl, 2-hydroxy-propyl, trifluoromethyl, 1,1-difluoroethyl, 2,2,2-trifluoroethyl,1-methyl-propyl, 1,1-dimethyl-propyl, 1,1-dimethyl-3-buten-1-yl, ethyl, and 2-propyl;
"Het" is morpholinyl, wherein said morpholinyl is optionally substituted with one or more $C_{1-3}$alkyl; and
$R^4$ is selected from -H and methyl.

An even further embodiment of the invention provides a compound of Formula IB, wherein
$R^1$ is cyclohexyl-methyl, cyclobutyl-methyl, 4,4-difluorocyclohexanemethyl, N-methylpiperidine-2-yl methyl, and tetrahydropyranyl-methyl;
$R^2$ is t-butyl and 1,1-difluoroethyl;
"Het" is morpholinyl, wherein said morpholinyl is optionally substituted with one or more $C_{1-3}$alkyl; and
$R^4$ is selected from -H and methyl.

It will be understood that when compounds of the present invention contain one or more chiral centers, the compounds of the invention may exist in, and be isolated as, enantiomeric or diastereomeric forms, or as a racemic mixture. The present invention includes any possible enantiomers, diastereomers, racemates or mixtures thereof, of a compound of Formula I, IA or IB. The optically active forms of the compound of the invention may be prepared, for example, by chiral chromatographic separation of a racemate, by synthesis from optically active starting materials or by asymmetric synthesis based on the procedures described thereafter.

It will also be appreciated that certain compounds of the present invention may exist as geometrical isomers, for example E and Z isomers of alkenes. The present invention includes any geometrical isomer of a compound of Formula I, IA or IB. It will further be understood that the present invention encompasses tautomers of the compounds of the Formula I, IA or IB.

It will also be understood that certain compounds of the present invention may exist in solvated, for example hydrated, as well as unsolvated forms. It will further be understood that the present invention encompasses all such solvated forms of the compounds of the Formula I, IA or IB.

Within the scope of the invention are also salts of the compounds of the Formula I, IA or IB. Generally, pharmaceutically acceptable salts of compounds of the present invention may be obtained using standard procedures well known in the art, for example by reacting a sufficiently basic compound, for example an alkyl amine with a suitable acid, for example, HCl or acetic acid, to afford a physiologically acceptable anion. It may also be possible to make a corresponding alkali metal (such as sodium, potassium, or lithium) or an alkaline earth metal (such as a calcium) salt by treating a compound of the present invention having a suitably acidic proton, such as a carboxylic acid or a phenol with one equivalent of an alkali metal or alkaline earth metal hydroxide or alkoxide (such as the ethoxide or methoxide), or a suitably basic organic amine (such as choline or meglumine) in an aqueous medium, followed by conventional purification techniques.

In one embodiment, the compound of Formula I, IA or IB above may be converted to a pharmaceutically acceptable salt or solvate thereof, particularly, an acid addition salt such as a hydrochloride, hydrobromide, phosphate, acetate, fumarate, maleate, tartrate, citrate, methanesulphonate orp-toluenesulphonate.

We have now found that the compounds of the invention have activity as pharmaceuticals, in particular as modulators or ligands such as agonists, partial agonists, inverse agonist or antagonists of $CB_1$ receptors. More particularly, the compounds of the invention exhibit selective activity as agonist of the $CB_1$ receptors and are useful in therapy, especially for relief of various pain conditions such as chronic pain, neuropathic pain, acute pain, cancer pain, pain caused by rheumatoid arthritis, migraine, visceral pain etc. This list should however not be interpreted as exhaustive. Additionally, compounds of the present invention are useful in other disease states in which dysfunction of $CB_1$ receptors is present or implicated. Furthermore, the compounds of the invention may be used to treat cancer, multiple sclerosis, Parkinson's disease, cancer, Huntington's chorea, Alzheimer's disease, anxiety disorders, gastrointestinal disorders and cardiovascular disorders.

Compounds of the invention are useful as immunomodulators, especially for autoimmune diseases, such as arthritis, for skin grafts, organ transplants and similar surgical needs, for collagen diseases, various allergies, for use as anti-tumour agents and anti viral agents.

Compounds of the invention are useful in disease states where degeneration or dysfunction of cannabinoid receptors is present or implicated in that paradigm. This may involve the use of isotopically labelled versions of the compounds of the invention in diagnostic techniques and imaging applications such as positron emission tomography (PET).

Compounds of the invention are useful for the treatment of diarrhoea, depression, anxiety and stress-related disorders such as post-traumatic stress disorders, panic disorder, generalized anxiety disorder, social phobia, and obsessive compulsive disorder, urinary incontinence, premature ejaculation, various mental illnesses, cough, lung oedema, various gastro-intestinal disorders, e.g. constipation, functional gastrointestinal disorders such as Irritable Bowel Syndrome and Functional Dyspepsia, Parkinson's disease and other motor disorders, traumatic brain injury, stroke, cardioprotection following miocardial infarction, spinal injury and drug addiction, including the treatment of alcohol, nicotine, opioid and other drug abuse and for disorders of the sympathetic nervous system for example hypertension.

Compounds of the invention are useful as an analgesic agent for use during general anaesthesia and monitored anaesthesia care. Combinations of agents with different properties are often used to achieve a balance of effects needed to maintain the anaesthetic state (e.g. amnesia, analgesia, muscle relaxation and sedation). Included in this combination are inhaled anaesthetics, hypnotics, anxiolytics, neuromuscular blockers and opioids.

Also within the scope of the invention is the use of any of the compounds according to the Formula I, IA or IB above, for the manufacture of a medicament for the treatment of any of the conditions discussed above.

Thus, the invention provides a compound of Formula I, IA or IB, or pharmaceutically acceptable salt or solvate thereof, as hereinbefore defined for use in therapy.

In a further aspect, the present invention provides the use of a compound of Formula I, IA or IB, or a pharmaceutically acceptable salt or solvate thereof, as hereinbefore defined in the manufacture of a medicament for use in therapy.

In the context of the present specification, the term "therapy" also includes "prophylaxis" unless there are specific indications to the contrary. The term "therapeutic" and "therapeutically" should be contrued accordingly. The term "therapy" within the context of the present invention further encompasses to administer an effective amount of a compound of the present invention, to mitigate either a pre-existing disease state, acute or chronic, or a recurring condition. This definition also encompasses prophylactic therapies for prevention of recurring conditions and continued therapy for chronic disorders.

The compounds of the present invention are useful in therapy, especially for the therapy of various pain conditions including, but not limited to: acute pain, chronic pain, neuropathic pain, back pain, cancer pain, and visceral pain.

In use for therapy in a warm-blooded animal such as a human, the compound of the invention may be administered in the form of a conventional pharmaceutical composition by any route including orally, intramuscularly, subcutaneously, topically, intranasally, intraperitoneally, intrathoracially, intravenously, epidurally, intrathecally, intracerebroventricularly and by injection into the joints.

In one embodiment of the invention, the route of administration may be oral, intravenous or intramuscular.

The dosage will depend on the route of administration, the severity of the disease, age and weight of the patient and other factors normally considered by the attending physician, when determining the individual regimen and dosage level at the most appropriate for a particular patient.

For preparing pharmaceutical compositions from the compounds of this invention, inert, pharmaceutically acceptable carriers can be either solid and liquid. Solid form preparations include powders, tablets, dispersible granules, capsules, cachets, and suppositories.

A solid carrier can be one or more substances, which may also act as diluents, flavoring agents, solubilizers, lubricants, suspending agents, binders, or table disintegrating agents; it can also be an encapsulating material.

In powders, the carrier is a finely divided solid, which is in a mixture with the finely divided compound of the invention, or the active component. In tablets, the active component is mixed with the carrier having the necessary binding properties in suitable proportions and compacted in the shape and size desired.

For preparing suppository compositions, a low-melting wax such as a mixture of fatty acid glycerides and cocoa butter is first melted and the active ingredient is dispersed therein by, for example, stirring. The molten homogeneous mixture in then poured into convenient sized moulds and allowed to cool and solidify.

Suitable carriers are magnesium carbonate, magnesium stearate, talc, lactose, sugar, pectin, dextrin, starch, tragacanth, methyl cellulose, sodium carboxymethyl cellulose, a low-melting wax, cocoa butter, and the like.

The term composition is also intended to include the formulation of the active component with encapsulating material as a carrier providing a capsule in which the active component (with or without other carriers) is surrounded by a carrier which is thus in association with it. Similarly, cachets are included.

Tablets, powders, cachets, and capsules can be used as solid dosage forms suitable for oral administration.

Liquid form compositions include solutions, suspensions, and emulsions. For example, sterile water or water propylene glycol solutions of the active compounds may be liquid preparations suitable for parenteral administration. Liquid compositions can also be formulated in solution in aqueous polyethylene glycol solution.

Aqueous solutions for oral administration can be prepared by dissolving the active component in water and adding suitable colorants, flavoring agents, stabilizers, and thickening agents as desired. Aqueous suspensions for oral use can be made by dispersing the finely divided active component in water together with a viscous material such as natural synthetic gums, resins, methyl cellulose, sodium carboxymethyl cellulose, and other suspending agents known to the pharmaceutical formulation art.

Depending on the mode of administration, the pharmaceutical composition will preferably include from 0.05% to 99%w (per cent by weight), more preferably from 0.10 to 50%w, of the compound of the invention, all percentages by weight being based on total composition.

A therapeutically effective amount for the practice of the present invention may be determined, by the use of known criteria including the age, weight and response of the individual patient, and interpreted within the context of the disease which is being treated or which is being prevented, by one of ordinary skills in the art.

Within the scope of the invention is the use of any compound of Formula I, IA or IB as defined above for the manufacture of a medicament.

Also within the scope of the invention is the use of any compound of Formula I, IA or IB for the manufacture of a medicament for the therapy of pain.

Additionally provided is the use of any compound according to Formula I, IA or IB for the manufacture of a medicament for the therapy of various pain conditions including, but not limited to: acute pain, chronic pain, neuropathic pain, back pain, cancer pain, and visceral pain. Additionally, there is provided a pharmaceutical composition comprising a compound of Formula I, IA or IB, or a pharmaceutically acceptable salt thereof, in association with a pharmaceutically acceptable carrier.

Particularly, there is provided a pharmaceutical composition comprising a compound of Formula I, IA or IB, or a pharmaceutically acceptable salt thereof, in association with a pharmaceutically acceptable carrier for therapy, more particularly for therapy of pain.

Further, there is provided a pharmaceutical composition comprising a compound of Formula I, IA or IB, or a pharmaceutically acceptable salt thereof, in association with a pharmaceutically acceptable carrier use in any of the conditions discussed above.

In a further aspect, the present invention provides a method of preparing the compounds of the present invention.

In one embodiment, the invention provides a process for preparing a compound of Formula I,

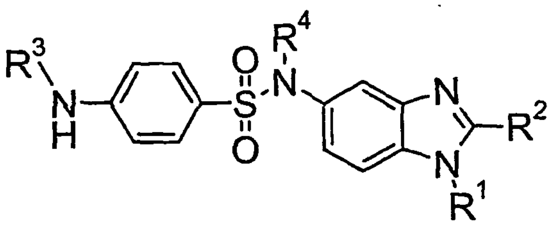

I

comprising the step of reacting a compound of Formula II,

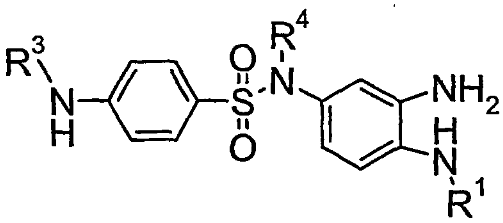

II

with a compound of $R^2COX$, in the presence of a base, such as an alkylamine, and optionally a coupling reagent, such as HATU, EDC, followed by treatment with an acid, such as HCl, acetic acid;

wherein

X is selected from Cl, Br, F and OH;

$R^1$ is selected from $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-

$C_{1-4}$alkyl, $C_{3-10}$cycloalkyl, $C_{4-8}$cycloalkenyl, and $C_{3-6}$heterocycloalkyl, wherein said $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloalkyl used in defining $R^1$ is optionally substituted by one or more groups selected from halogen, cyano, nitro, methoxy, ethoxy, methyl, ethyl, hydroxy,amino, $C_{1-6}$alkylamino and di$C_{1-6}$alkylamino;

$R^2$ is selected from $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl, wherein said $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl used in defining $R^2$ is optionally substituted by one or more groups selected from halogen, methoxy, ethoxy, methyl, ethyl, hydroxy, and amino;

$R^3$ is selected from -H, $C_{1-6}$alkyl and $C_{1-6}$acyl optionally substituted with one or more groups selected from $CH_3C(=O)$-O-, halogen, cyano, methoxy, ethoxy, hydroxy, amino, alkylamino, dialkylamino, and $C_{3-6}$heterocycloalkyl; and

$R^4$ is selected from -H, $C_{1-6}$alkyl, $C_{2-6}$alkenyl, $C_{3-6}$cycloalkyl, and $C_{3-6}$cycloalkyl-$C_{1-4}$alkyl.

Particularly, the present invention provides a method of preparing a compound of Formula I,

X is selected from Cl, Br, F and OH;

$R^1$ is selected from cyclopentyl-methyl, cyclohexyl-methyl, cyclobutyl-methyl, tetrahydropyranyl-methyl, tetrahydrofuranyl-methyl, morpholinyl-methyl, piperdinylethyl, 4,4-difluorocyclohexanemethyl, N-methyl-piperdinylmethyl, and piperdinyl-methyl;

$R^2$ is selected from t-butyl, n-butyl, 2-methyl-2-butyl, isopentyl, 2-methoxy-2-propyl, 2-hydroxy-propyl, trifluoromethyl, 1,1-difluoroethyl, 2,2,2-trifluoroethyl , 1-methyl-propyl, 1,1-dimethyl-propyl, 1,1-dimethyl-3-buten-1-yl, ethyl, and 2-propyl;

$R^3$ is selected from $C_{1-6}$alkyl and $C_{1-6}$acyl optionally substituted with one or more groups selected from $CH_3C(=O)$-O-, halogen, methoxy, hydroxy, amino, methylamino, dimethylamino, pyrrolidinyl, and morpholinyl; and

$R^4$ is selected from -H and methyl.

A further embodiment of the invention provides a method for preparing a compound of Formula IA,

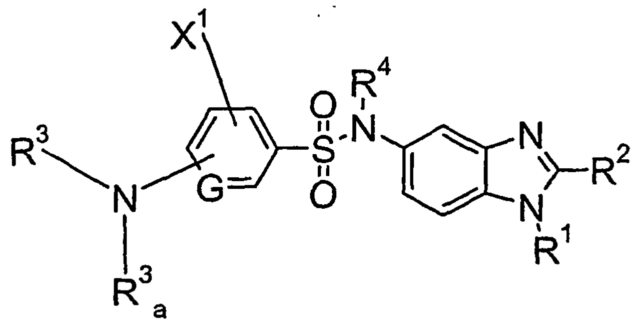

**IA**

comprising:

reacting a compound of Formula II,

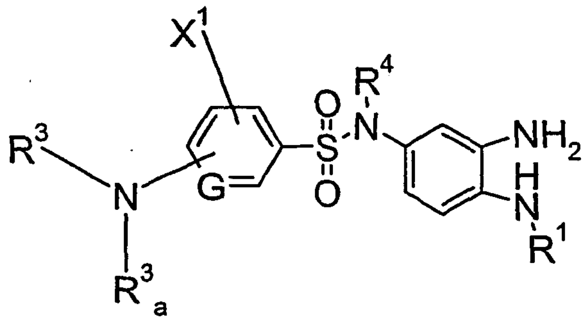

IIA

with a compound of $R^2COX$, in the presence of a base, such as an alkylamine, and optionally a coupling reagent, followed by treatment with an acid;

wherein
X and $X^1$ are independently selected from Cl, Br, F and OH;
G is CH or N;
$R^1$ is selected from $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloalkyl, wherein said $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloalkyl used in defining $R^1$ is optionally substituted by one or more groups selected from halogen, cyano, nitro, methoxy, ethoxy, methyl, ethyl, hydroxy, $CH_3C(=O)$-O-, amino, $C_{1-6}$alkylamino and di$C_{1-6}$alkylamino;
$R^2$ is selected from $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl, wherein said $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl used in defining $R^2$ is optionally substituted by one or more groups selected from halogen, $C_{3-5}$heteroaryl, methoxy, ethoxy, methyl, ethyl, hydroxy, amino, $C_{1-6}$alkylamino and di$C_{1-4}$alkylamino;
$R^3$ and $R^3_a$ are independently selected from -H, $C_{1-6}$alkyl, $C_{2-6}$alkenyl, $C_{1-3}$alkyl-O-C(=O)-, $C_{1-6}$alkyl-HN-C(=O)-, $H_2N$-C(=O)-, and $C_{1-6}$acyl, wherein said $C_{1-6}$alkyl, $C_{2-6}$alkenyl, and $C_{1-6}$acyl used in defining $R^3$ is optionally substituted with one or more groups selected from $CH_3C(=O)$-O-, halogen, cyano, methoxy, ethoxy, hydroxy, amino, $C_{1-6}$alkylamino, di$C_{1-6}$alkylamino, and $C_{3-6}$heterocycloalkyl; and
$R^4$ is selected from -H, $C_{1-6}$alkyl, $C_{2-6}$alkenyl, $C_{3-6}$cycloalkyl, and $C_{3-6}$cycloalkyl-$C_{1-4}$alkyl.

Compounds of the present invention may also be prepared according to the synthetic routes as depicted in Schemes 1-7.

**Scheme 1**

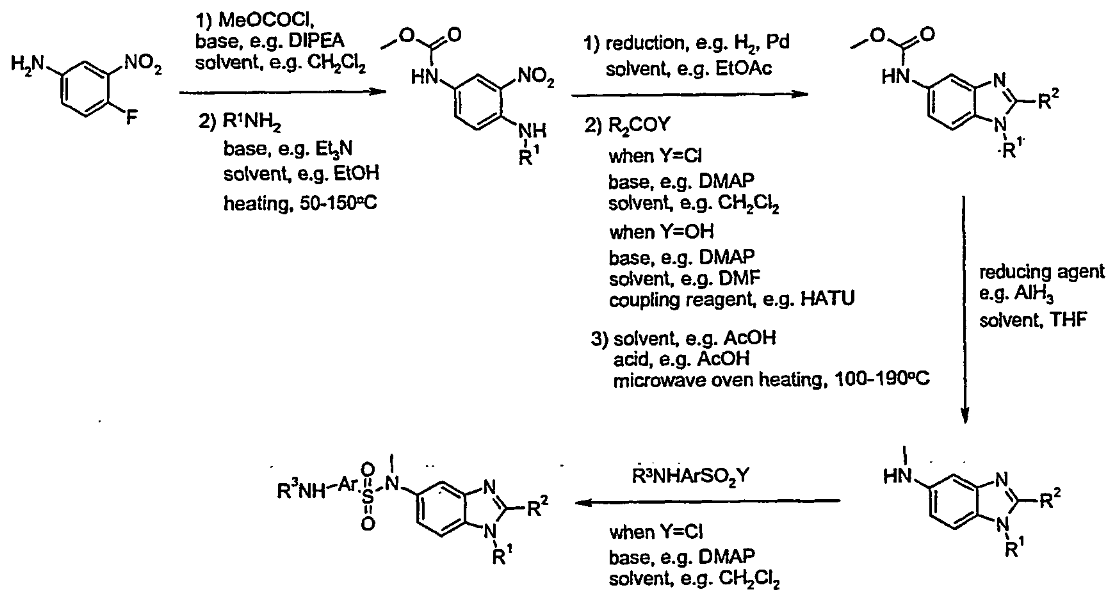


$R^1$, $R^2$, $R^3$, and $R^4$ are as defined in the specifications.
Ar is phenylene or pyridinylene.

**Scheme 2**

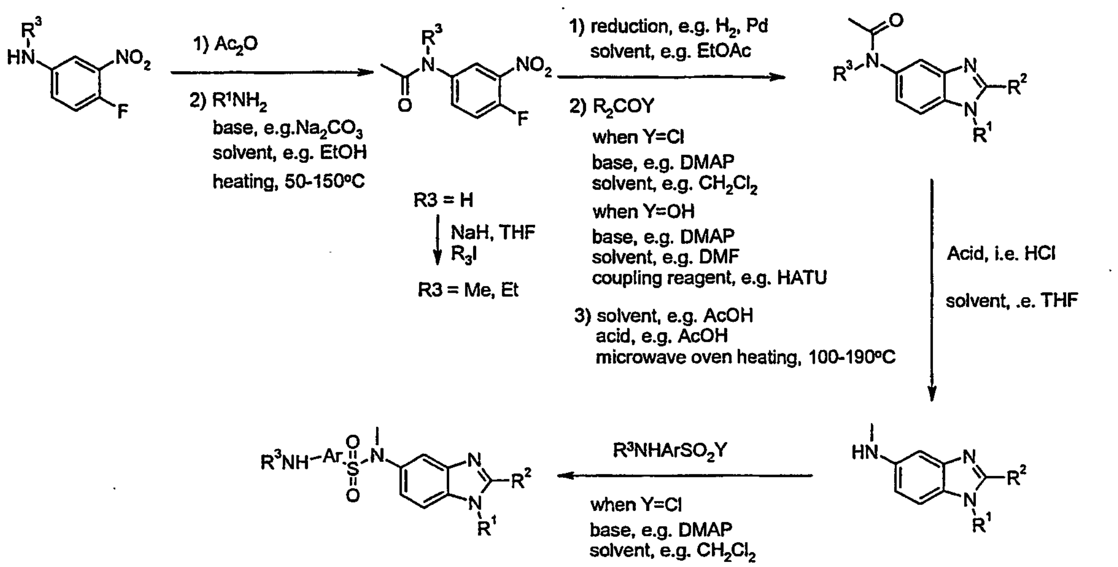


$R^1$, $R^2$, $R^3$, and $R^4$ are as defined in the specifications.
Ar is phenylene or pyridinylene.

**Scheme 3**

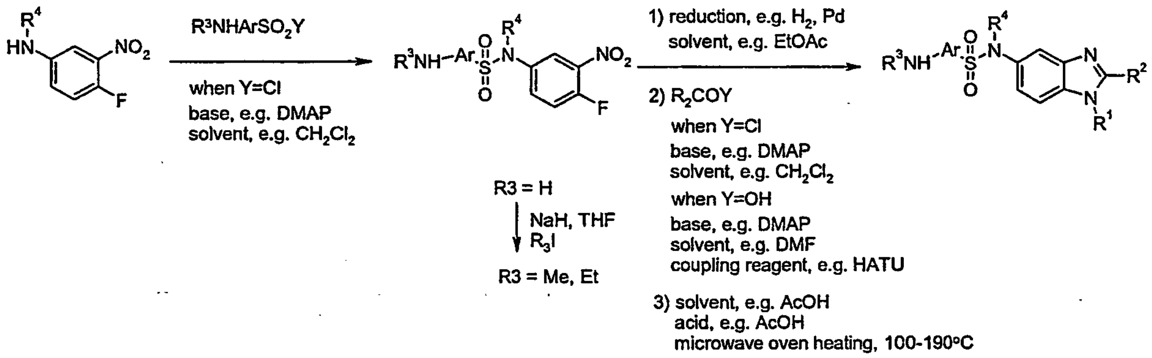


$R^1$, $R^2$, $R^3$, and $R^4$ are as defined in the specifications.
Ar is phenylene or pyridinylene.

## Scheme 4

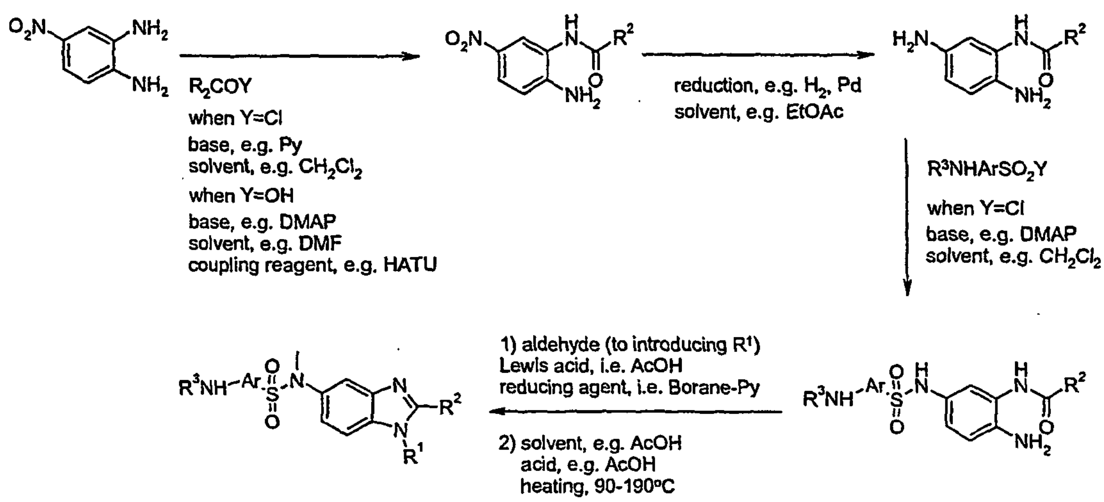


$R^1$, $R^2$, $R^3$, and $R^4$ are as defined in the specifications.
Ar is phenylene or pyridinylene.

## Scheme 5

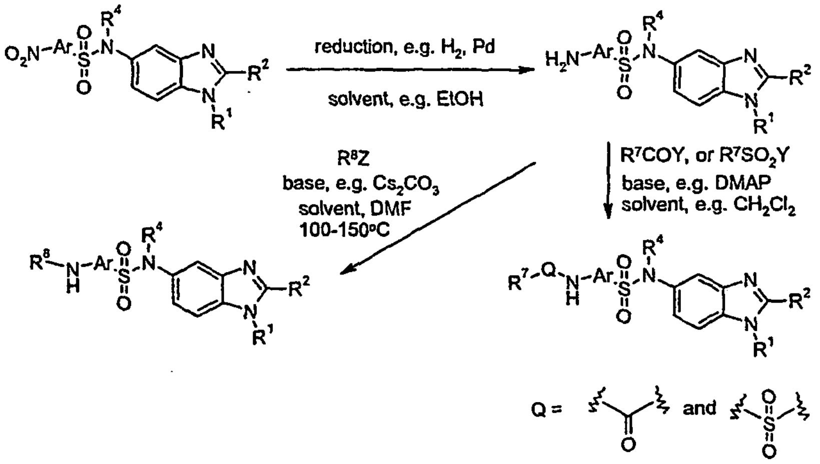


$R^1$, $R^2$, and $R^4$ are as defined in the specifications;
$R^7$ and $R^8$ are optionally substituted C1-6alkyl;
Y and Z are halogen or -OH.
Ar is phenylene or pyridinylene.

**Scheme 6**

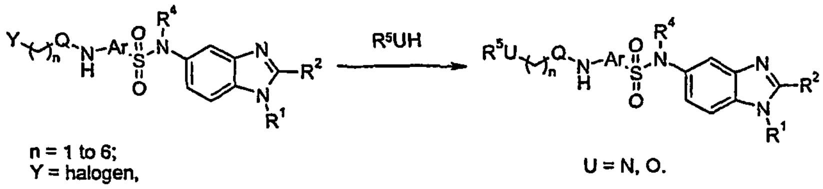


n = 1 to 6;
Y = halogen,

U = N, O.

Q = 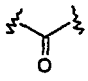 and 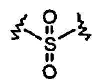

$R^1$, $R^2$, and $R^4$ are as defined in the specifications.
Ar is phenylene or pyridinylene.
$R^5$ is $C_{1-6}$alkyl.

**Scheme 7**

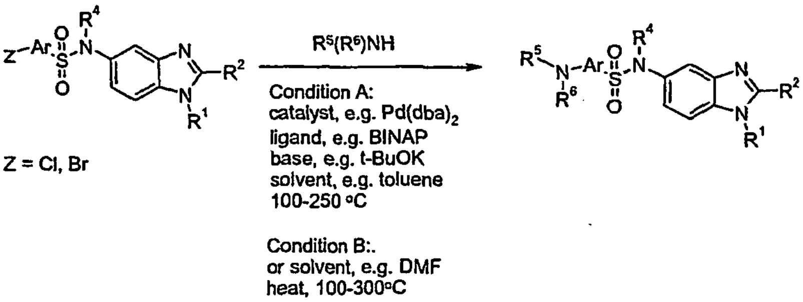


Z = Cl, Br

$R^1$, $R^2$, and $R^4$ are as defined in the specifications.
$R^5$, $R^6$ are -H or $C_{1-6}$alkyl,
and Ar is phenylene or pyridinylene.

## Biological Evaluation

### $hCB_1$ and $hCB_2$ receptor binding

Human $CB_1$ receptor from Receptor Biology ($hCB_1$) or human $CB_2$ receptor from BioSignal ($hCB_2$) membranes are thawed at 37 °C, passed 3 times through a 25-gauge blunt-end needle, diluted in the cannabinoid binding buffer (50 mM Tris, 2.5 mM EDTA, 5 mM $MgCl_2$, and 0.5 mg/mL BSA fatty acid free, pH 7.4) and aliquots containing the appropriate amount of protein are distributed in 96-well plates. The $IC_{50}$ of the compounds of the invention at $hCB_1$ and $hCB_2$ are evaluated from 10-point dose-response curves done with $^3$H-CP55,940 at 20000 to 25000 dpm per well (0.17-0.21 nM) in a final volume of 300 μl. The total and non-specific binding are determined in the absence and presence of 0.2 μM of HU210 respectively. The plates are vortexed and incubated for 60 minutes at room temperature, filtered through Unifilters GF/B (presoaked in 0.1% polyethyleneimine) with the Tomtec or Packard harvester using 3 mL of wash buffer (50 mM Tris, 5 mM $MgCl_2$, 0.5 mg BSA pH 7.0). The filters are dried for 1 hour at 55 °C. The radioactivity (cpm) is counted in a TopCount (Packard) after adding 65 μl/well of MS-20 scintillation liquid.

### $hCB_1$ and $hCB_2$ GTPγS binding

Human $CB_1$ receptor from Receptor Biology ($hCB_1$) or human $CB_2$ receptor membranes (BioSignal) are thawed at 37 °C, passed 3 times through a 25-gauge blunt-end needle and diluted in the GTPγS binding buffer (50 mM Hepes, 20 mM NaOH, 100 mM NaCl, 1 mM EDTA, 5 mM $MgCl_2$, pH 7.4, 0.1% BSA). The $EC_{50}$ and $E_{max}$ of the compounds of the invention are evaluated from 10-point dose-response curves done in 300μl with the appropriate amount of membrane protein and 100000-130000 dpm of GTPg$^{35}$S per well (0.11-0.14 nM). The basal and maximal stimulated binding is determined in absence and presence of 1 μM ($hCB_2$) or 10 μM ($hCB_1$) Win 55,212-2 respectively. The membranes are

pre-incubated for 5 minutes with 56.25 μM (hCB2) or 112.5 μM ($hCB_1$) GDP prior to distribution in plates (15 μM ($hCB_2$) or 30 μM ($hCB_1$) GDP final). The plates are vortexed and incubated for 60 minutes at room temperature, filtered on Unifilters GF/B (presoaked in water) with the Tomtec or Packard harvester using 3 ml of wash buffer (50 mM Tris, 5 mM $MgCl_2$, 50 mM NaCl, pH 7.0). The filters are dried for 1 hour at 55 °C. The radioactivity (cpm) is counted in a TopCount (Packard) after adding 65 μl/well of MS-20 scintillation liquid. Antagonist reversal studies are done in the same way except that (a) an agonist dose-response curve is done in the presence of a constant concentration of antagonist, or (b) an antagonist dose-response curve is done in the presence of a constant concentration of agonist.

Based on the above assays, the dissociation constant (Ki) for a particular compound of the invention towards a particular receptor is determined using the following equation:

$$Ki = IC_{50}/(1+[rad]/Kd),$$

Wherein $IC_{50}$ is the concentration of the compound of the invention at which 50% displacement has been observed;
[rad] is a standard or reference radioactive ligand concentration at that moment; and
Kd is the dissociation constant of the radioactive ligand towards the particular receptor.

Using the above-mentioned assays, the Ki towards human $CB_1$ receptors for most compounds of the invention is measured to be in the range of 0.72-7170 nM. The Ki towards human $CB_2$ receptors for most compounds of the invention is measured to be in the range of about 0.36-24.7 nM. The $EC_{50}$ towards human $CB_1$ receptors for most compounds of the invention is measured to be in the range of about 0.85-785 nM. The $E_{max}$ towards human $CB_1$ receptors for most compounds of the invention is measured to be in the range of about 39-140%.

In one embodiment, the Ki towards human $CB_1$ receptors for most compounds of the invention is measured to be in the range of 0.72-15 nM. The Ki towards human $CB_2$ receptors for most compounds of the invention is measured to be in the range of about 0.36- 3 nM. The $EC_{50}$ towards human $CB_1$ receptors for most compounds of the invention is measured to be in the range of about 0.85-25 nM. The $E_{max}$ towards human $CB_1$ receptors for most compounds of the invention is measured to be in the range of about 85-131%.

## EXAMPLES

The invention will further be described in more detail by the following Examples which describe methods whereby compounds of the present invention may be prepared, purified, analyzed and biologically tested, and which are not to be construed as limiting the invention.

### Example 1

***N*-(4-{[[2-*tert*-butyl-1-(cyclohexylmethyl)-1*H*-benzimidazol-5-yl](methyl)amino]sulfonyl}phenyl) acetamide**

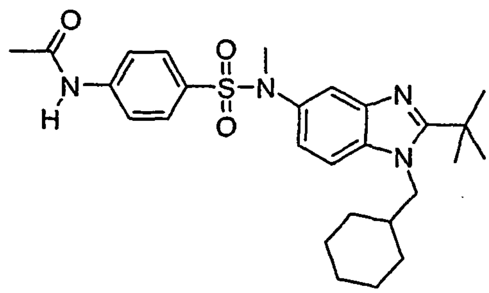

**Step A. *N*-(4-{[[2-*tert*-butyl-1-(cyclohexylmethyl)-1*H*-benzimidazol-5-yl](methyl)amino]sulfonyl}phenyl) acetamide**

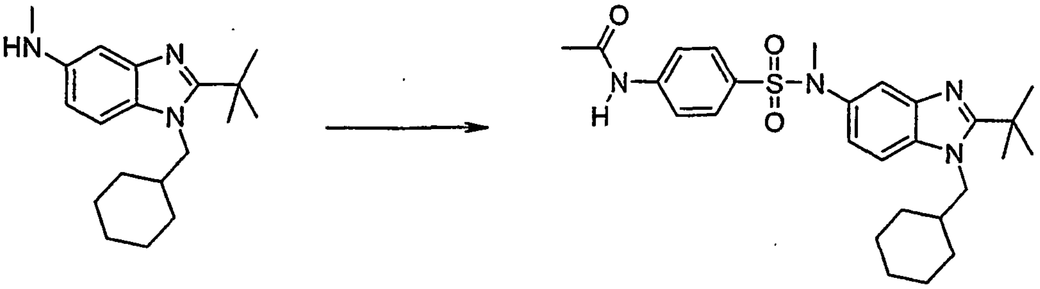

2-*tert*-Butyl-1-(cyclohexylmethyl)-*N*-methyl-1*H*-benzimidazol-5-amine (40 mg, 0.133 mmol) (for preparation, see the following steps B, C, D, E and F) and 4-acetamidobenzene sulfonyl chloride (37 mg, 0.160 mmol) were stirred in 3 mL of dichloromethane containing a catalytic amount of DMAP overnight at rt. The solvent was evaporated. The product was purified by reversed-phase HPLC using 20-80% $CH_3CN/H_2O$ and then lyophilized affording the title compound as the corresponding TFA salt. Yield: 63 mg (78%); $^1H$ NMR (400 MHz, METHANOL-$D_4$): δ 1.21 (m, 5 H), 1.61 (m, 3 H), 1.64 (s, 9 H), 1.67 (m, 1 H), 1.75 (m, 2 H), 2.07 (m, 1 H), 2.11 (s, 3 H), 3.22 (s, 3 H), 4.42 (d, J=7.62 Hz, 2 H), 7.29 (dd, J=9.08, 2.05 Hz, 1 H), 7.42 (d, J=8.98 Hz, 2 H), 7.50 (d, J=1.56 Hz, 1 H), 7.68 (d, J=8.98 Hz, 2 H), 7.82 (d, J=8.98 Hz, 1 H); MS (ESI) $(M+H)^+$: 497.2; Anal. Calcd for $C_{27}H_{36}N_4O_3S$ + 1.4 TFA + 0.4 $H_2O$: C, 53.94; H, 5.80; N, 8.44. Found: C, 53.98; H, 5.79; N, 8.50.

**Step B. Methyl (4-fluoro-3-nitrophenyl)carbamate**

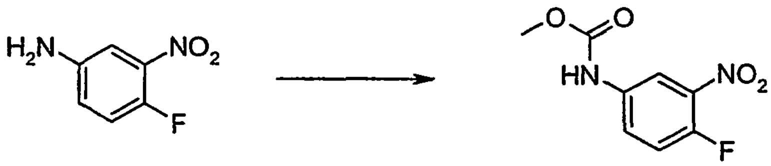

Methyl chloroformate (13.2 mL, 170.2 mmol) was added dropwise to a cold (0°C) dichloromethane (200 mL) solution of 4-fluoro-3-nitro aniline (24.15 g, 154.7 mmol) and DIPEA (35 mL, 201 mmol). The reaction mixture was stirred at rt overnight. The solution was then diluted with 200 mL of dichloromethane and washed with 2M HCl, brine and dried over anhydrous $MgSO_4$. The solvent was concentrated and the product was directly used for next step without further purification. Yield: 35.5 g (99%); $^1H$ NMR (400 MHz, CHLOROFORM-D) δ 3.81 (s, 3 H), 7.02 (s, 1 H), 7.23 (m, 1 H), 7.72 (d, *J*=8.59 Hz, 1 H), 8.17 (dd, *J*=6.35, 2.64 Hz, 1 H).

**Step C. Methyl {4-[(cyclohexylmethyl)amino]-3-nitrophenyl}carbamate**

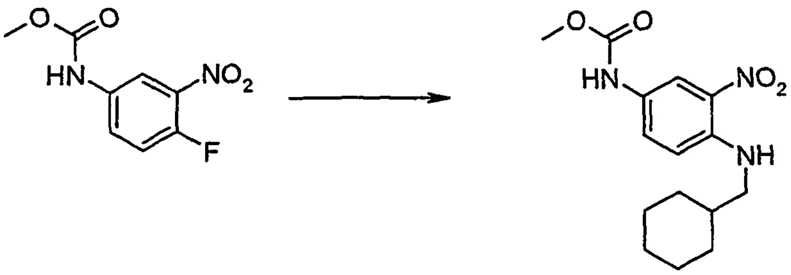

Methyl (4-fluoro-3-nitrophenyl)carbamate (1.00 g, 4.67 mmol) and cyclohexylmethyl amine (0.730 mL, 5.60 mmol) were stirred in EtOH (20 mL) containing TEA (1.0 mL, 7.00 mmol) at 75°C for 24h. The solvent was concentrated. The residue was dissolved in EtOAc and washed with 5% $KHSO_4$ solution, saturated $NaHCO_3$ solution, brine and dried

over anhydrous $MgSO_4$. The crude product was purified by flash chromatography using 4:1/hex:EtOAc on silica gel. Yield: 1.05 g (73%); $^1$H NMR (400 MHz, CHLOROFORM-D): δ1.04 (ddd, J = 24.02, 12.11, 2.93Hz, 2H), 1.25 (m, 3H), 1.69 (m, 2H), 1.76 (m, 1H), 1.79 (m, 1H), 1.83 (m, 1H), 1.86 (m, 1H), 3.14 (dd, J = 6.44, 5.66Hz, 2H), 3.78 (s, 3H), 6.46 (m, 1H), 6.84 (d, J = 9.37 Hz, 1H), 7.63 (m, 1H), 8.05 (d, J = 2.54 Hz, 1H), 8.09 (m, 1H).

**Step D. Methyl {3-amino-4-[(cyclohexylmethyl)amino]phenyl}carbamate**

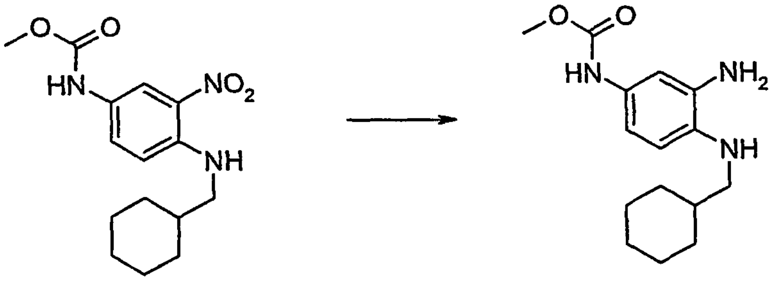

Methyl {4-[(cyclohexylmethyl)amino]-3-nitrophenyl}carbamate (1.05 g, 3.42 mmol) was dissolved in 30 mL of EtOAc containing a catalytic amount of 10% Pd/C. The solution was shaken in a Parr hydrogenation apparatus under $H_2$ atmosphere (40 psi) at rt overnight. The solution was filtered through Celite and the solvent was evaporated. The product was directly used for the next step without further purification. Yield: 950 mg (99%); MS (ESI) $(M+H)^+$: 277.9.

**Step E. Methyl [2-*tert*-butyl-1-(cyclohexylmethyl)-1*H*-benzimidazol-5-yl]carbamate**

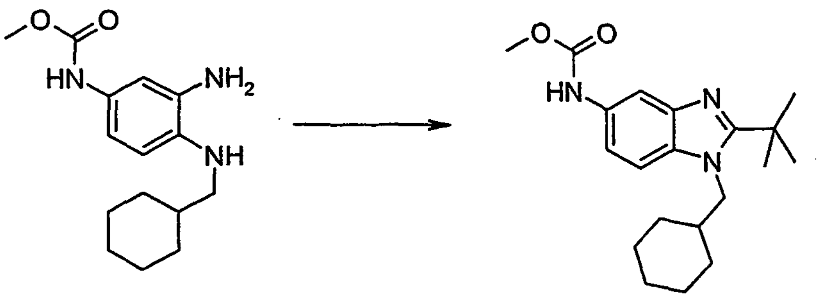

Methyl {3-amino-4-[(cyclohexylmethyl)amino]phenyl}carbamate (950 mg, 3.43 mmol) and DMAP (100 mg, 0.858 mmol) were dissolved in 25 mL of dichloromethane. Trimethylacetyl chloride (0.460 mL, 3.77 mmol) was added dropwise and the solution was stirred at rt for 1h. The solvent was concentrated. The residue was divided in two portions and each of them dissolved in 3 mL of glacial AcOH in a sealed tube. The solutions were heated at 150°C using a Personal Chemistry Smith Synthesizer microwave instrument for three intervals of 30 min (3 X 30 min). The two tubes were combined and the solvent was evaporated. The residue was dissolved in EtOAc and washed with saturated $NaHCO_3$ solution, brine and dried over anhydrous $MgSO_4$. The crude product was purified by flash chromatography using 3: 1/dichloromethane:diethyl ether. Yield: 656 mg (56%); $^1$H NMR (400 MHz, CHLOROFORM-D): δ 1.08 (m, 2H), 1.18 (m, 3H), 1.54 (s, 9H), 1.65 (m, 1H), 1.69 (m, 2H), 1.73 (dd, J = 5.96, 3.22 Hz, 2H), 2.02 (m, 1H), 3.78 (s, 3H), 4.10 (d, J = 7.42 Hz, 2H), 6.64 (m, 1H), 7.25 (d, J = 8.79 Hz, 1H), 7.39 (m, 1H), 7.59 (d, J = 1.76 Hz, 1H).

**Step F. 2-*tert*-Butyl-1-(cyclohexylmethyl)-*N*-methyl-1*H*-benzimidazol-5-amine**

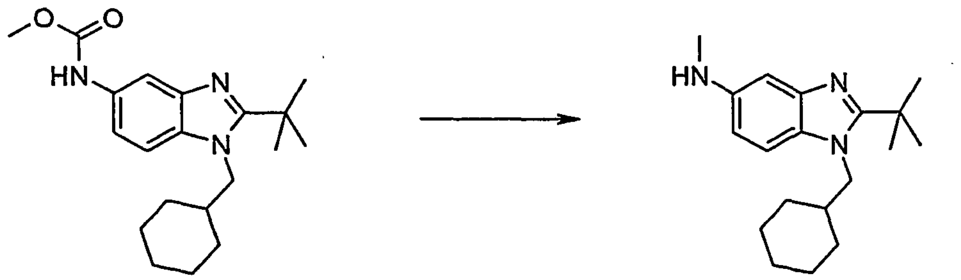

Methyl [2-*tert*-butyl-1-(cyclohexylmethyl)-1*H*-benzimidazol-5-yl]carbamate (650 mg, 1.89 mmol) was dissolved in 20 mL of THF at 0°C under nitrogen. 1M HCl/ether (2.65 mL, 2.65 mmol) was added dropwise and the solution stirred at 0°C for 15min. $LiA1H_4$ (360 mg, 9.45 mmol) was then slowly added and the solution was stirred at rt overnight. The reaction mixture was quenched at 0°C by addition of MeOH (5 mL) followed by water (10 mL). The solution was diluted with EtOAc and washed with saturated $NaHCO_3$ solution, brine and dried over anhydrous $MgSO_4$. The solvent was evaporated and the product was used directly for Step A without further purification. Yield: 544 mg (96%); $^1$H NMR (400 MHz, CHLOROFORM-D): δ 1.08 (s, 2 H), 1.17 (m, 3 H), 1.54 (s, 9 H), 1.64 (m, 2 H), 1.67 (m, 2 H), 1.72 (m, 2 H), 2.02 (m, 1 H), 2.87 (s, 3 H), 4.06 (d, *J*=7.62 Hz, 2 H), 6.60 (dd, *J*=8.69, 2.25 Hz, 1 H), 7.00 (d, *J*=1.76 Hz, 1 H), 7.12 (d, *J*=8.59 Hz, 1 H).

**Example 2**

***N*-[2-*tert*-Butyl-1-(cyclohexylmethyl)-1*H*-benzimidazol-5-yl]-*N*-methyl-4-nitrobenzenesulfonamide**

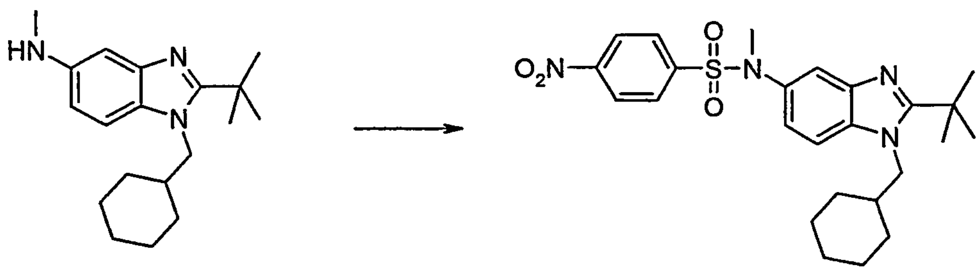

Following the procedure for Step A in Example 1, using 2-*tert*-btyl-1-(cyclohexylmethyl)-*N*-methyl-1*H*-benzimidazol-5-amine (253 mg, 0.845 mmol) (prepared according to procedures in Example 1, Steps B-F), 4-nitrobenzenesulfonyl chloride (245 mg, 1.10 mmol) and DMAP (catalytic) in 20mL of DCM. The solution was washed with saturated $NaHCO_3$ aqueous solution, brine and dried over anhydrous $MgSO_4$. The crude product was purified by flash chromatography on silica gel using 2:1 / hexanes:EtOAc as eluent to afford the title product. Yield: 380 mg (93%); $^1$H NMR (400 MHz, CHLOROFORM-D): δ 1.09 (m, 2 H), 1.21 (m, 3 H), 1.54 (s, 9 H), 1.64 (m, 1 H), 1.67 (m, 1 H), 1.71 (m, 1 H), 1.76 (m, 2 H), 2.03 (m, 1 H), 3.27 (s, 3 H), 4.12 (d, *J*=7.23 Hz, 2 H), 7.18 (m, *J*=8.98 Hz, 2 H), 7.30 (d, *J*=8.98 Hz, 1 H), 7.77 (d, *J*=9.18 Hz, 2 H), 8.30 (d, *J*=9.18 Hz, 2 H).

**Example 3**

**4-Amino-*N*-[2-*tert*-butyl-1-(cyclohexylmethyl)-1*H*-benzimidazol-5-yl]-*N*-methylbenzenesulfonamide**

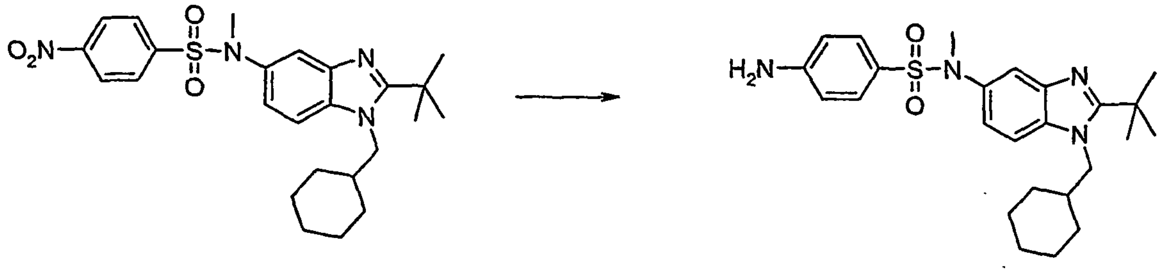

*N*-[2-*tert*-Butyl-1-(cyclohexylmethyl)-1*H*-benzimidazol-5-yl]-*N*-methyl-4-nitrobenzenesulfonamide (375mg, 0.774 mmol) (prepared according to the procedure in Example 2) was dissolved in 20 mL of EtOH containing a catalytic amount of 10% Pd/C. The solution was shaken in a Parr hydrogenation apparatus under $H_2$ atmosphere (40 psi) at rt for 3h. The solution was filtered through celite and the solvent was concentrated. Yield: 332 mg (94%); $^1$H NMR (400 MHz, METHANOL-$D_4$): δ 1.22 (m, 6 H), 1.60 (m, 1 H), 1.64 (s, 9 H), 1.67 (m, 1 H), 1.75 (m, 2 H), 2.08 (m, 1 H), 3.17 (s, 3 H), 4.42 (d, J=7.42 Hz, 2 H), 6.56 (d, J=8.79 Hz, 2 H), 7.14 (d, J=8.79 Hz, 2 H), 7.32 (dd, J=8.98, 1.95 Hz, 1 H), 7.49 (d, J=1.95 Hz, 1 H), 7.81 (d, J=8.98 Hz, 1 H); MS (ESI) $(M+H)^+$: 455.0; Anal. Calcd for $C_{25}H_{34}N_4O_2S$ + 1.5 TFA + 0.4 $H_2O$: C, 53.14; H, 5.78; N, 8.85. Found: C, 53.10; H, 5.67; N, 8.92.

**Example 4**

***N*-(4-{[[2-*tert*-Butyl-1-(cyclohexylmethyl)-1*H*-benzimidazol-5-yl](methyl)amino]sulfonyl}phenyl)propanamide**

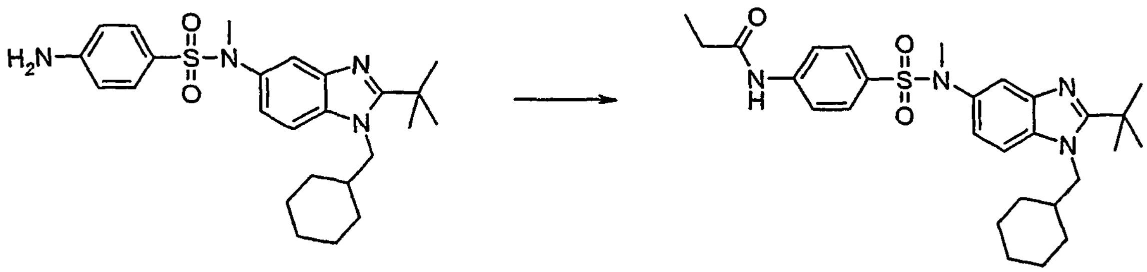

4-Amino-*N*-[2-*tert*-butyl-1-(cyclohexylmethyl)-1*H*-benzimidazol-5-yl]-*N*-methylbenzenesulfonamide (50 mg, 0.110 mmol) and propionyl chloride (0.012 mL, 0.143 mmol) were stirred in 3 mL of DCM containing a catalytic amount of DMAP at rt for 12h. The solvent was concentrated and the crude product was purified by reversed-phase HPLC using 20-80% $CH_3CN/H_2O$ and then lyophilized affording the title compound as the corresponding TFA salt. Yield: 68 mg (99%); $^1$H NMR (400 MHz, METHANOL-$D_4$): δ 1.16 (t, J=7.62 Hz, 3 H), 1.21 (m, 5 H), 1.60 (m, 2 H), 1.64 (s, 9 H), 1.67 (m, 1 H), 1.75 (m, 2 H), 2.07 (m, 1 H), 2.38 (q, J=7.62 Hz, 2 H), 3.22 (s, 3 H), 4.42 (d, J=7.62 Hz, 2 H), 7.29 (dd, J=9.08, 2.05 Hz, 1 H), 7.42 (d, J=8.98 Hz, 2 H), 7.49 (d, J=1.76 Hz, 1 H), 7.69 (d, J=8.98 Hz, 2 H), 7.81 (d, J=8.98 Hz, 1 H); MS (ESI) $(M+H)^+$: 511.2; Anal. Calcd for $C_{28}H_{38}N_4O_3S$ + 1.5 TFA + 0.2 $H_2O$: C, 54.33; H, 5.87; N, 8.18. Found: C, 54.32; H, 5.84; N, 8.25.

**Example 5**

***N*-(4-{[[2-*tert*-Butyl-1-(cyclohexylmethyl)-1*H*-benzimidazol-5-yl](methyl)amino]sulfonyl}phenyl)-2-methylpropanamide**

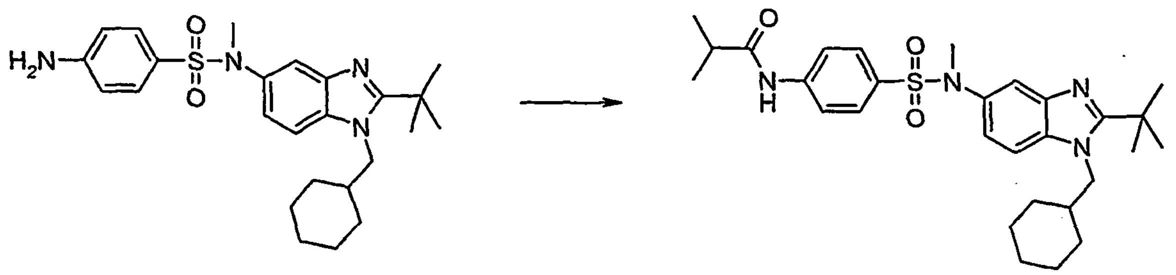

Following the procedure for Example 4, using 4-amino-*N*-[2-*tert*-butyl-1-(cyclohexylmethyl)-1*H*-benzimidazol-5-yl]-*N*-methylbenzenesulfonamide (50 mg, 0.110 mmol), isobutyryl chloride (0.015 mL, 0.143 mmol) and a catalytic amount of DMAP in 3mL of DCM. Yield: 73 mg (99%); $^1$H NMR (400 MHz, METHANOL-$D_4$): δ 1.15 (d, J=6.83 Hz, 6 H), 1.21 (m, 5 H), 1.62 (m, 2 H), 1.64 (s, 9 H), 1.67 (m, 1 H), 1.75 (m, 2 H), 2.08 (m, 1 H), 2.61 (dt, J=13.82, 6.86 Hz, 1 H), 3.23 (s, 3 H), 4.42 (d, J=7.62 Hz, 2 H), 7.29 (dd, J=8.98, 1.95 Hz, 1 H), 7.42 (d, J=8.98 Hz, 2 H), 7.50 (d, J=1.95 Hz, 1 H), 7.71 (d, J=8.98 Hz, 2 H), 7.82 (d, J=8.98 Hz, 1 H); MS (ESI) $(M+H)^+$: 525.3; Anal. Calcd for $C_{29}H_{40}N_4O_3S$ + 1.7 TFA + 0.3 $H_2O$: C, 53.75; H, 5.89; N, 7.74. Found: C, 53.75; H, 5.87; N, 7.73.

**Example 6**

***N*-(4-{[[2-*tert*-Butyl-1-(cyclohexylmethyl)-1*H*-benzimidazol-5-yl](methyl)amino]sulfonyl}phenyl)-2,2-dimethyl-propanamide**

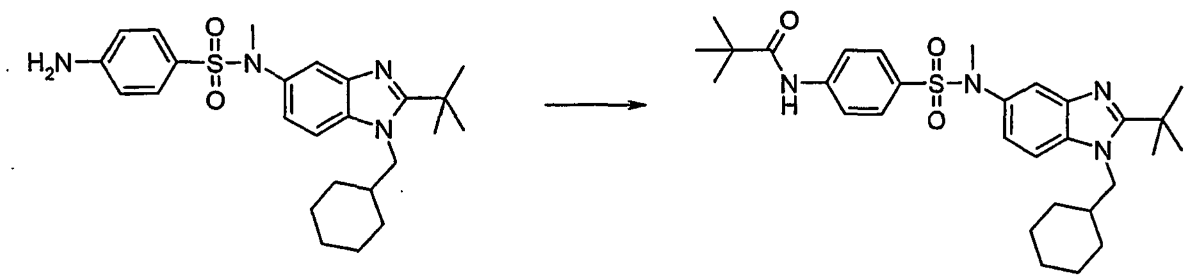

Following the procedure for Example 4, using 4-amino-*N*-[2-*tert*-butyl-1-(cyclohexylmethyl)-1*H*-benzimidazol-5-yl]-*N*-methylbenzenesulfonamide (50 mg, 0.110 mmol), trimethylacetyl chloride (0.018 mL, 0.143 mmol) and a catalytic amount of DMAP in 3mL of DCM. Yield: 76 mg (99%); $^1$H NMR (400 MHz, METHANOL-$D_4$): δ 1.21 (m, 5 H), 1.26 (s, 9 H), 1.62 (m, 2 H), 1.64 (s, 9 H), 1.67 (m, 1 H), 1.75 (m, 2 H), 2.07 (m, 1 H), 3.23 (s, 3 H), 4.42 (d, J=7.62 Hz, 2 H), 7.29 (dd, J=9.08, 2.05 Hz, 1 H), 7.42 (d, J=8.98 Hz, 2 H), 7.49 (d, J=1.76 Hz, 1 H), 7.73 (d, J=8.98 Hz, 2 H), 7.82 (d, J=8.98 Hz, 1 H); MS (ESI) $(M+H)^+$: 539.2; Anal. Calcd for $C_{30}H_{42}N_4O_3S$ + 1.4 TFA + 0.5 $H_2O$: C, 55.69; H, 6.33; N, 7.92. Found: C, 55.70; H, 6.31; N, 7.92.

**Example 7**

***N*-[2-*tert*-Butyl-1-(cyclohexylymethyl)-1*H*-benzimidazol-5-yl]-4-(ethylamino)-*N*-methylbenzenesulfonamide**

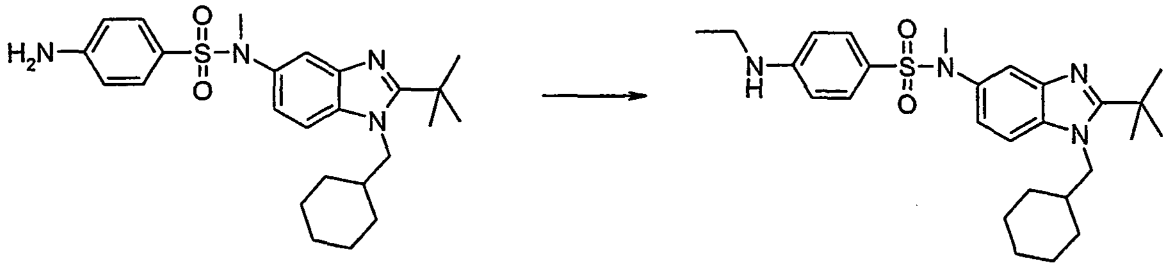

4-Amino-*N*-[2-*tert*-butyl-1-(cyclohexylmethyl)-1*H*-benzimidazol-5-yl]-*N*-methylbenzenesulfonamide (55 mg, 0.121 mmol), cesium carbonate (78 mg, 0.242 mmol) and ethyl iodide (0.011 mL, 0.133mmol) were dissolved in 1 mL of DMF in a sealed tube flushed with nitrogen. The solution was heated at 125°C in a Personal Chemistry SmithSynthesizer microwave instrument for 10 min. Another 0.133 mmol (0.011 mL) of ethyl iodide was added and the solution was heated for another 10 min. This procedure was then repeated 3 more times. The solvent was then concentrated. The residue was dissolved in EtOAc and washed with saturated $NaHCO_3$ aqueous solution, brine and dried over anhydrous $MgSO_4$. The solvent was concentrated and the crude product was purified by reversed-phase HPLC using 20-80% $CH_3CN/H_2O$ and then lyophilized affording the title compound as the corresponding TFA salt. Yield: 39 mg (54%); $^1H$ NMR (400 MHz, METHANOL-$D_4$): δ 1.19 (t, J=7.23 Hz, 3 H), 1.23 (m, 5 H), 1.62 (m, 2 H), 1.64 (s, 9 H), 1.67 (m, 1 H), 1.75 (m, 2 H), 2.08 (m, 1 H), 3.10 (q, J=7.23 Hz, 2 H), 3.17 (s, 3 H), 4.43 (d, J=7.62 Hz, 2 H), 6.52 (d, J=8.98 Hz, 2 H), 7.18 (d, J=8.98 Hz, 2 H), 7.32 (dd, J=8.98, 1.95 Hz, 1 H), 7.51 (d, J=2.15 Hz, 1 H), 7.81 (d, J=8.98 Hz, 1 H); MS (ESI) $(M+H)^+$: 483.3; Anal. Calcd for $C_{27}H_{38}N_4O_2S$ + 1.8 TFA: C, 53.43; H, 5.83; N, 8.14. Found: C, 53.51; H, 5.81; N, 8.13.

**Example 8**

***N*-[2-*tert*-Butyl-1-(cyclohexylmethyl)-1*H*-benzimidazol-5-yl]-4-(formylamino)-*N*-methylbenzenesulfonamide**

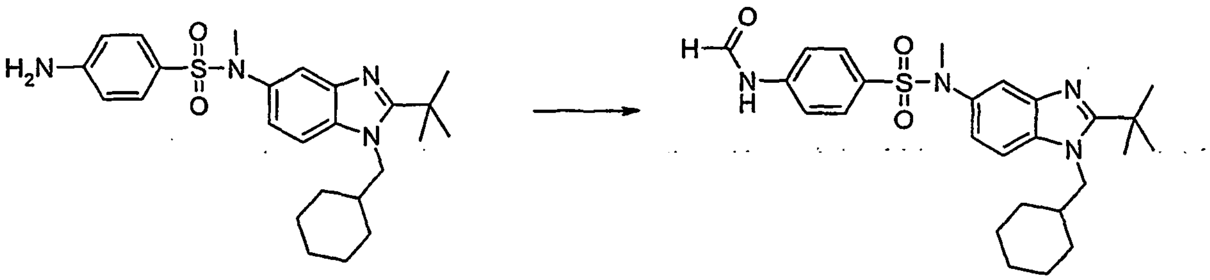

4-Amino-*N*-[2-*tert*-butyl-1-(cyclohexylmethyl)-1*H*-benzimidazol-5-yl]-*N*-methylbenzenesulfonamide (45 mg, 0.099 mmol), was heated in 1 mL of formic acid in a sealed tube at 125°C for 15 min using a Personal Chemistry SmithSynthesizer microwave instrument. The solvent was concentrated and the product was purified by reversed-phase HPLC using 20-80% $CH_3CN/H_2O$ and then lyophilized affording the title compound as the corresponding TFA salt. Yield: 61 mg (99%); $^1H$ NMR (400 MHz, METHANOL-$D_4$): δ 1.21 (m, 5 H), 1.62 (m, 2 H), 1.64 (s, 9 H), 1.67 (m, 1 H), 1.75 (m, 2 H), 2.08 (m, 1 H), 3.23 (s, 3 H), 4.42 (d, J=7.62 Hz, 2 H), 7.29 (dd, J=8.98, 1.95 Hz, 1 H), 7.45 (d, J=8.98 Hz, 2 H), 7.50 (d, J=1.76 Hz, 1 H), 7.70 (d, J=8.79 Hz, 2 H), 7.82 (d, J=8.98 Hz, 1 H), 8.31 (s, 1 H); MS (ESI) $(M+H)^+$: 483.0; Anal. Calcd for $C_{26}H_{34}N_4O_3S$ + 1.4 TFA + 0.5 $H_2O$: C, 53.11; H, 5.63; N, 8.60. Found: C, 53.02; H, 5.62; N, 8.71.

**Example 9**

***N*-(4-{[[2-*tert*-Butyl-1-(cyclohexylmethyl)-1*H*-benzimidazol-5-yl](methyl)amino]sulfonyl}phenyl)-2-pyrrolidin-1-ylacetamide**

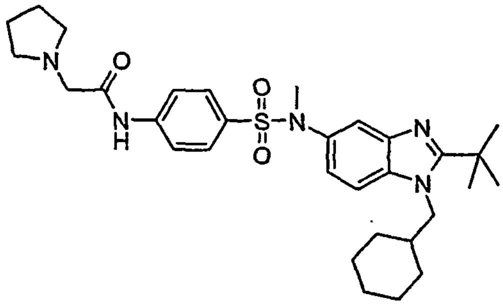

**Step A. *N*-(4-{[[2-*tert*-Butyl-1-(cyclohexylmethyl)-1*H*-benzimidazol-5-yl](methyl)amino]sulfonyl}phenyl)-2-pyrrolidin-1-ylacetamide**

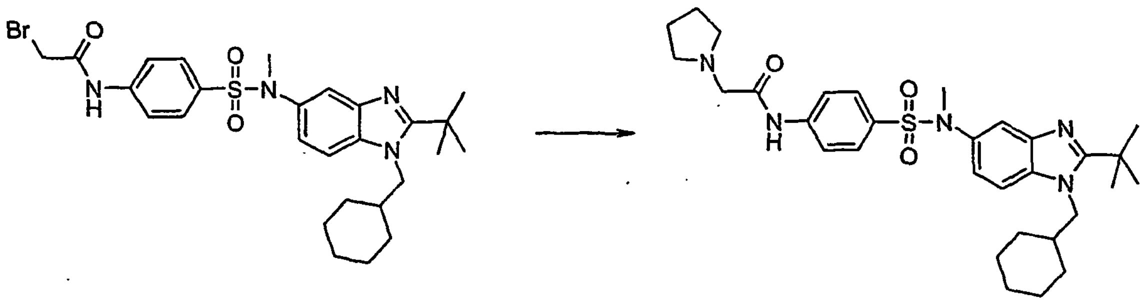

2- Bromo- *N*-(4- { [ [2-*tert*-butyl- 1-(cyclohexylmethyl)- 1*H*-benzimidazol- 5- yl] (methyl) amino] sulfonyl} phenyl) acetamide (42 mg, 0.0730 mmol) and pyrrolidine (0.030 mL, 0.365 mmol) were dissolved in 1 mL of DMF in a sealed tube. The solution was heated at 125°C in a Personal Chemistry SmithSynthesizer microwave instrument for 15 min. The solvent was concentrated. The residue was dissolved in EtOAc and was washed with saturated $NaHCO_3$ aqueous solution, brine and dried over anhydrous $MgSO_4$. The solvent was concentrated and the crude product was purified by reversed-phase HPLC using 20-80% $CH_3CN/H_2O$ and then lyophilized affording the title compound as the corresponding TFA salt. Yield: 51 mg (88%); $^1H$ NMR (400 MHz, METHANOL-$D_4$): δ 1.24 (m, 5 H), 1.63 (m, 2 H), 1.67 (s, 11 H), 1.70 (m, 1 H), 1.78 (m, 2 H), 2.09 (m, 2 H), 2.17 (m, 2 H), 3.19 (m, 1 H), 3.27 (s, 3 H), 3.78 (m, 1 H), 4.27 (s, 2 H), 4.44 (d, J=7.62 Hz, 2 H), 7.28 (dd, J=8.98, 1.95 Hz, 1 H), 7.53 (d, J=8.98 Hz, 2 H), 7.59 (d, J=1.76 Hz, 1 H), 7.77 (d, J=8.98 Hz, 2 H), 7.82 (d, J=8.98 Hz, 1 H); MS (ESI) $(M+H)^+$: 566.2; Anal. Calcd for $C_{31}H_{43}N_5O_3S$ + 2.7 TFA + 0.4 $H_2O$: C, 49.63; H, 5.32; N, 7.95. Found: C, 49.63; H, 5.33; N, 7.93.

**Step B. 2-Bromo-*N*-(4-{[[2-*tert*-butyl-1-(cyclohexylmethyl)-1*H*-benzimidazol-5-yl](methyl)amino]sulfonyl}phenyl)acetamide**

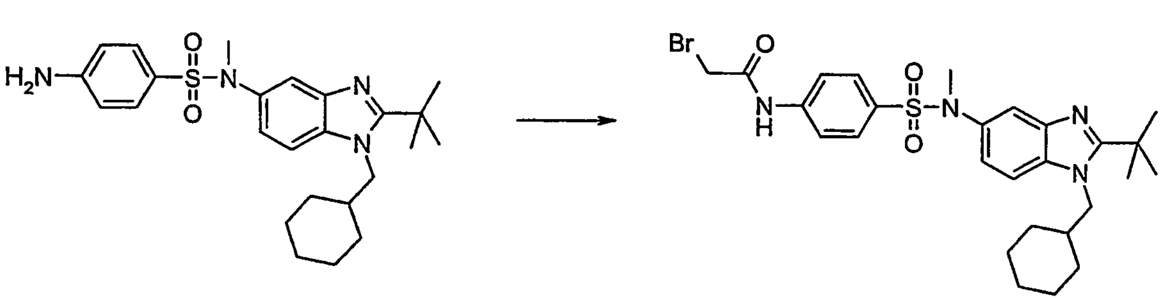

4-Amino-*N*-[2-*tert*-butyl-1-(cyclohexylmethyl)-1*H*-benzimidazol-5-yl]-*N*-methylbenzenesulfonamide (155 mg,

0.341 mmol) was dissolved in 5 mL of DCM containing a catalytic amount of DMAP. Bromoacetyl chloride (0.035 mL, 0.409 mmol) was added and the solution was stirred at rt for 3h. The solution was washed with saturated $NaHCO_3$ aqueous solution, brine and dried over anhydrous $MgSO_4$. The crude product was purified by flash chromatography on silica gel using 50-75% EtOAc in hexanes as eluent to afford the title product. Yield: 175 mg (89%); $^1$H NMR (400 MHz, CHLOROFORM-D): δ 1.09 (m, 1H), 1.12 (m, 1H), 1.15 (m, 1H), 1.19 (d J=8.59 Hz, 2H), 1.54 (s, 9H), 1.65 (m, 1H), 1.68 (m, 1H), 1.72 (m, 1H), 1.75 (m, 2H), 2.04 (m, 1H), 3.21 (d, J=1.17 Hz, 3H), 4.04 (s, 1H), 4.12 (m, 2H), 4.22 (s, 1H), 7.20 (m, 1H), 7.23 (m, 1H), 7.28 (m, 1H), 7.57 (m, 2H), 7.66 (t, J=8.49 Hz, 2H), 8.44 (d, J=8.40 Hz, 1H).

**Example 10**

***N*¹-(4-{[[2-*tert*-Butyl-1-(cyclohexylmethyl)-1*H*-benzimidazol-5-yl](methyl)amino]sulfonyl}phenyl)-*N*², *N*²-dimethylglycinamide**

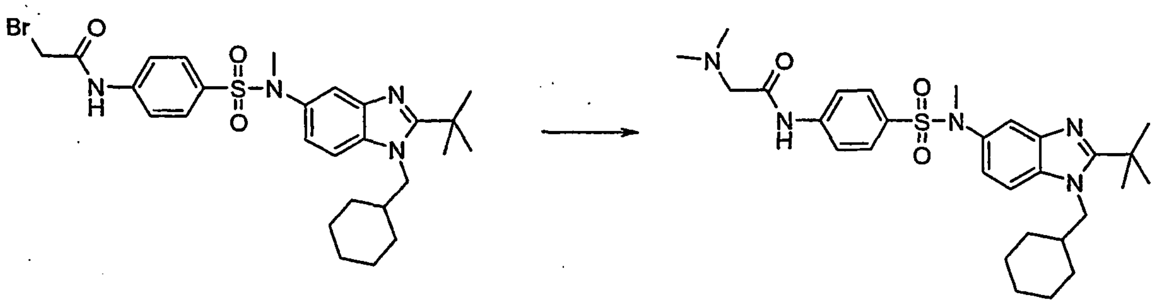

Following the procedure for Example 9, using 2-bromo-*N*-(4-{[[2-*tert*-butyl-1-(cyclohexylmethyl)-1*H*-benzimidazol-5-yl](methyl)amino]sulfonyl}phenyl) acetamide (40 mg, 0.0695 mmol), dimethylamine hydrochloride (0.030 mg, 0.348 mmol) and DIPEA (0.060 mL, 0.348 mmol) in 1 mL of DMF. Yield: 35 mg (77%); $^1$N MR (400 MHz, METHANOL-$D_4$): δ 1.24 (m, 5 H), 1.64 (m, 2 H), 1.67 (s, 9 H), 1.70 (m, 1 H), 1.78 (m, 2 H), 2.10 (m, 1 H), 3.00 (s, 6 H), 3.27 (s, 3 H), 4.18 (s, 2 H), 4.45 (d, J=7.62 Hz, 2 H), 7.29 (dd, J=8.98, 1.95 Hz, 1 H), 7.54 (d, J=8.98 Hz, 2 H), 7.60 (d, J=1.76 Hz, 1 H), 7.77 (d, J=8.98 Hz, 2 H), 7.82 (d, J=8.98 Hz, 1 H); MS (ESI) $(M+H)^+$: 540.3; Anal. Calcd for $C_{29}H_{41}N_5O_3S$ + 2.9 TFA + 0.5 $H_2O$: C, 47.53; H, 5.15; N, 7.96. Found: C, 47.57; H, 5.11; N, 7.99.

**Example 11**

***N*-(4-{[[2-*tert*-Butyl-1-(cyclohexylmethyl)-1*H*-benzimidazol-5-yl](methyl)amino]sulfonyl}phenyl)-2-morpholin-4-ylacetamide**

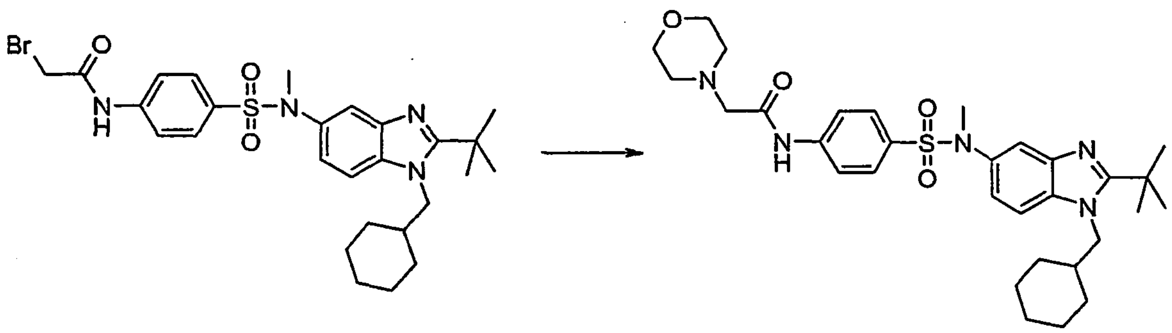

Following the procedure for Example 9, using 2-bromo-*N*-(4-{[[2-*tert*-butyl-1-(cyclohexylmethyl)-1*H*-benzimidazol-5-yl](methyl)amino]sulfonyl}phenyl) acetamide (56 mg, 0.0973 mmol) and morpholine (0.045 mL, 0.486 mmol) in

1 mL of DMF. Yield: 15mg (26%); $^1$H NMR (400 MHz, METHANOL-$D_4$): δ 1.24 (m, 5 H), 1.64 (m, 2 H), 1.67 (s, 9 H), 1.70 (m, 1 H), 1.78 (m, 2 H), 2.11 (m, 1 H), 3.27 (s, 3 H), 3.42 (m, 4 H), 3.96 (m, 4 H), 4.17 (s, 2 H), 4.45 (d, J=7.62 Hz, 2 H), 7.29 (dd, J=8.98, 2.15 Hz, 1 H), 7.54 (d, J=9.18 Hz, 2 H), 7.60 (d, J=1.56 Hz, 1 H), 7.77 (d, J=8.98 Hz, 2 H), 7.83 (d, J=8.98 Hz, 1 H); MS (ESI) $(M+H)^+$: 582.2; Anal. Calcd for $C_{31}H_{43}N_5O_4S$ + 3.2 TFA + 0.2 $H_2O$: C, 47.27; H, 4.94; N, 7.37. Found: C, 47.23; H, 4.92; N, 7.49.

**Example 12**

***N*^1-(4-{[[2-*tert*-Butyl-1-(cyclohexylmethyl)-1*H*-benzimidazol-5-yl](methyl)amino]sulfonyl}phenyl)glycinamide**

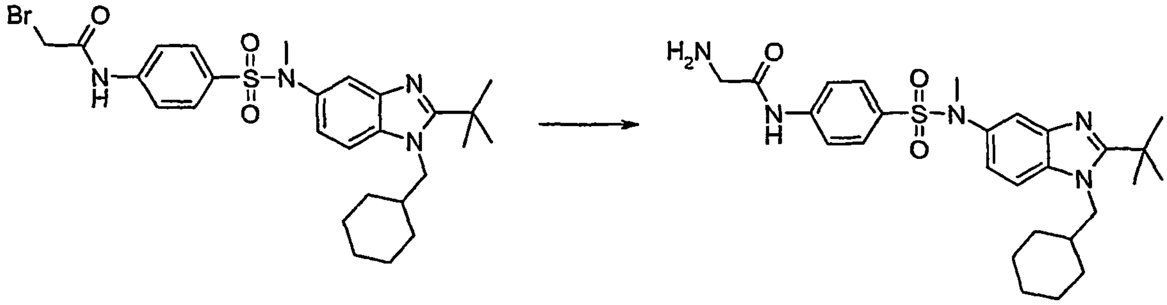

Following the procedure for Example 9, using 2-bromo-*N*-(4-{[[2-*tert*-butyl-1-(cyclohexylmethyl)-1*H*-benzimidazol-5-yl](methyl)amino]sulfonyl}phenyl) acetamide (37 mg, 0.0608 mmol) and ammonium hydroxide (28% aqueous) (0.5 mL, excess) in 1 mL of DMF. Yield: 28 mg (74%); $^1$H NMR (400 MHz, METHANOL-$D_4$): δ 1.24 (m, 5 H), 1.64 (m, 2 H), 1.67 (s, 9 H), 1.70 (m, 1 H), 1.78 (m, 2 H), 2.10 (m, 1 H), 3.27 (s, 3 H), 3.89 (s, 2 H), 4.44 (d, J=7.62 Hz, 2 H), 7.30 (dd, J=8.98,1.95 Hz, 1 H), 7.53 (d, J=9.18 Hz, 2 H), 7.58 (d, J=1.76 Hz, 1 H), 7.76 (d, J=8.98 Hz, 2 H), 7.82 (d, J=9.18 Hz, 1 H); MS (ESI) $(M+H)^+$: 512.0; Anal. Calcd for $C_{27}H_{37}N_5O_3S$ + 2.6 TFA + 1.6 $H_2O$: C, 46.21; H, 5.15; N, 8.37. Found: C, 46.22; H, 5.09; N, 8.43.

**Example 13**

**2-[(4-{[[2-*tert*-Butyl-1-(cyclohexylmethyl)-1*H*-benzimidazol-5-yl](methyl)amino]sulfonyl}phenyl)amino]-2-oxoethyl acetate**

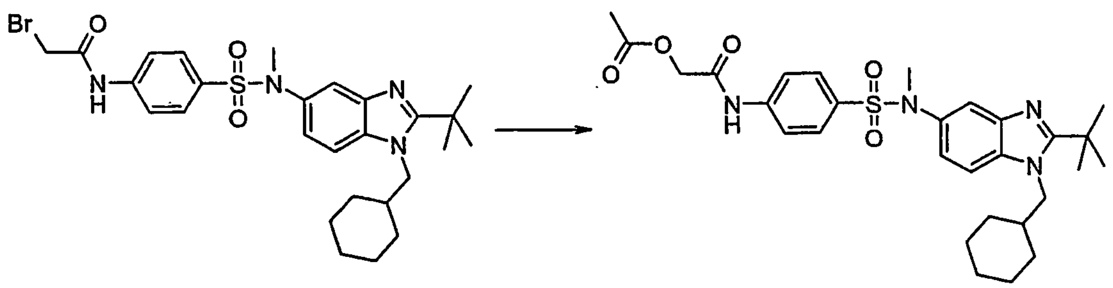

Following the procedure for Example 9, using 2-bromo-*N*-(4-{[[2-*tert*-butyl-1-(cyclohexylmethyl)-1*H*-benimidazol-5-yl](methyl)amino]sulfonyl}phenyl) acetamide (50 mg, 0.0869 mmol) and sodium acetate (35 mg, 0.434 mmol) in 2 mL of DMF. The product was used directly for the next step without any further purification. Yield: 48 mg (99%); MS (ESI) $(M+H)^+$: 555.2.

**Example 14**

***N*-(4-{[[2-*tert*-Butyl-1-(cyclohexylmethyl)-1*H*-benzimidazol-5-yl](methyl)amino]sulfonyl}phenyl)-2-hydroxyacetamide**

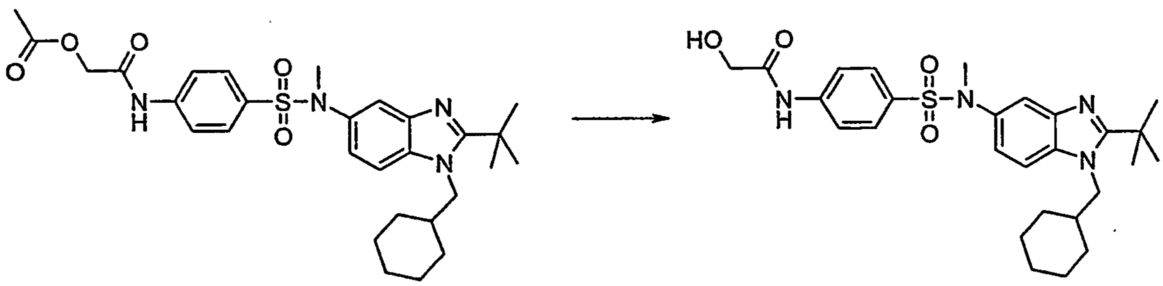

2-[(4-{[[2-*tert*-Butyl-1-(cyclohexylmethyl)-1*H*-benzimidazol-5-yl](methyl)amino]sulfonyl}phenyl)amino]-2-oxoethyl acetate (48 mg, 0.0869 mmol) was refluxed in 2 mL of EtOH containing 1M LiOH (0.5 mL, excess) for 2h. The solvent was concentrated and the residue was dissolved in EtOAc. The organic phase was washed with saturated $NaHCO_3$ aqueous solution, brine and dried over anhydrous $MgSO_4$. The solvent was concentrated and the crude product was purified by reversed-phase HPLC using 20-80% $CH_3CN/H_2O$ and then lyophilized affording the title compound as the corresponding TFA salt. Yield: 16 mg (29%); $^1H$ NMR (400 MHz, METHANOL-$D_4$): δ 1.24 (m, 5 H), 1.63 (m, 2 H), 1.67 (s, 9 H), 1.71 (m, 1 H), 1.78 (m, 2 H), 2.10 (m, 1 H), 3.27 (s, 3 H), 4.13 (s, 2 H), 4.45 (d, J=7.62 Hz, 2 H), 7.31 (dd, J=9.08, 2.05 Hz, 1 H), 7.49 (d, J=8.79 Hz, 2 H), 7.53 (d, J=1.95 Hz, 1 H), 7.83 (m, 3 H); MS (ESI) $(M+H)^+$: 513.0.

**Example 15**

**5-Bromo-*N*-[2-*tert*-butyl-1-(cyclohexylmethyl)-1*H*-benzimidazol-5-yl]-6-chloro-*N*-methylpyridine-3-sulfonamide**

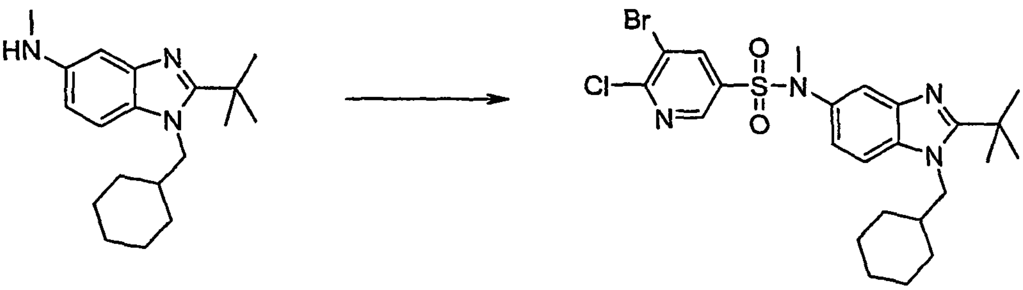

2-*tert*-Butyl-1-(cyclohexylmethyl)-*N*-methyl-1*H*-benzimidazol-5-amine (for parathion, see Example 1) (80 mg, 0.267 mmol) and 3-bromo-2-chloro pyridine-5-sulphonyl chloride (95 mg, 0.320 mmol) were stirred in 3 mL of DCM containing a catalytic amount of DMAP at rt overnight. The solution was washed with saturated aqueous $NaHCO_3$ solution, brine and dried over anhydrous $MgSO_4$. The crude product was purified by silica gel flash chromatography using 2:1 / hexanes:EtOAc as eluent. Yield: 127 mg (86%); $^1H$ NMR (400 MHz, CHLOROFORM-D): δ 1.09 (m, 1 H), 1.11 (m, 1 H), 1.15 (m, 1 H), 1.18 (m, 1 H), 1.20 (m, 1 H), 1.54 (s, 9 H), 1.64 (m, 1 H), 1.67 (m, 1 H), 1.70 (m, 1 H), 1.75 (m, 2 H), 2.02 (m, 1 H), 3.29 (s, 3 H), 4.13 (d, J=7.42 Hz, 2 H), 7.12 (dd, J=8.79, 1.56 Hz, 1 H), 7.31 (m, 2 H), 8.06 (d, J=2.15 Hz, 1 H), 8.41 (d, J=2.15 Hz, 1 H); MS (ESI) $(M+H)^+$: 553.0; Anal. Calcd for $C_{24}H_{30}N_4O_2SClBr$ + 0.1 $H_2O$: C, 51.87; H, 5.48; N, 10.08. Found: C, 52.01; H, 5.54; N, 9.83.

**Example 16**

**5-Bromo-*N*-[2-*tert*-butyl-1-(cyclohexylmethyl)-1*H*-benzimidazol-5-yl]-6-[(2-hydroxyethyl)ammo]-*N*-methylpyridine-3-sulfonamide**

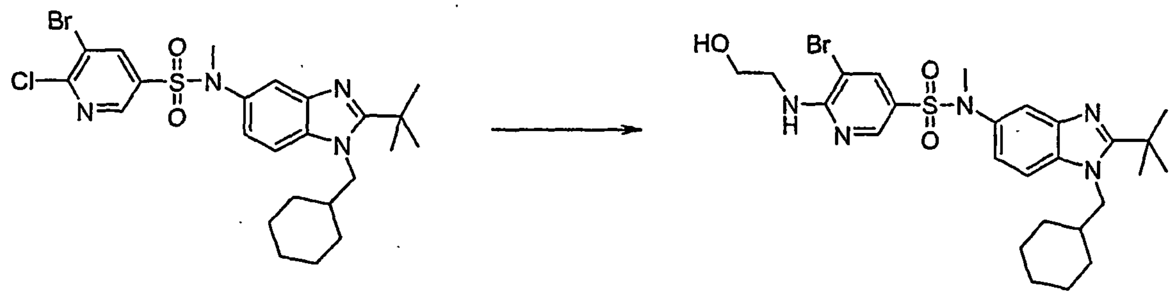

5-Bromo-*N*-[2-*tert*-butyl-1-(cyclohexylmethyl)-1*H*-benzimidazol-5-yl]-6-chloro-*N*-methylpyridine-3-sulfonamide (50 mg, 0.0903 mmol) and ethanolamine (0.025 mL, 0.451 mmol) were dissolved in 2 mL of DMF. The solution was heated at 120°C for 30 min using a Personal Chemistry microwaves instrument. The solvent was evaporated. The product was directly purified by reversed-phase HPLC using 10-60% $CH_3CN/H_2O$ and lyophilized affording the title compound as the corresponding TFA salt. Yield: 45 mg (72%); $^1H$ NMR (400 MHz, METHANOL-D4): δ 1.25 (m, 5 H), 1.65 (m, 2 H), 1.68 (s, 9 H), 1.71 (m, 1 H), 1.78 (m, 2 H), 2.11 (m, 1 H), 3.27 (s, 3 H), 3.61 (t, J=5.37 Hz, 2 H), 3.70 (t, J=5.47 Hz, 2 H), 4.47 (d, J=7.62 Hz, 2 H), 7.38 (dd, J=9.08, 2.05 Hz, 1 H), 7.61 (d, J=2.15 Hz, 1 H), 7.63 (d, J=1.56 Hz, 1 H), 7.89 (d, J=8.98 Hz, 1 H), 8.09 (d, J=2.15 Hz, 1 H); MS (ESI) $(M+H)^+$ 578.3; Anal. Calcd for $C_{26}H_{38}N_5O_3SBr$ + 1.9 TFA + 0.2 $H_2O$: C, 44.69; H, 5.07; N, 8.74. Found: C, 44.71; H, 5.13; N, 8.74.

**Example 17**

***N*-[2-*tert*-Butyl-1-(cyclohexylmethyl)-1*H*-benzimidazol-5-yl]-6-[(2-hydroxyethyl)amino]-*N*-methylpyridine-3-sulfonamide**

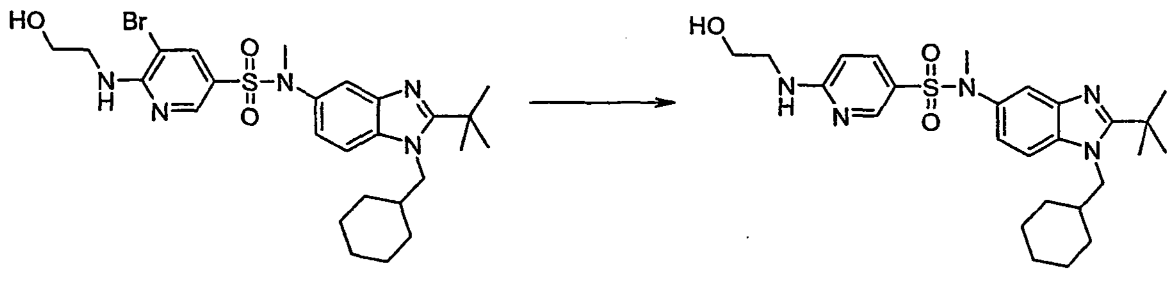

5-Bromo-*N*-[2-*tert*-butyl-1-(cyclohexylmethyl)-1*H*-benzimidazol-5-yl]-6-[(2-hydroxyethyl)amino]-*N*-methylpyridine-3-sulfonamide (55 mg, 0.0794 mmol) was dissolved in 15 mL of EtOH containing a catalytic amount of 10% Pd/C. The solution was shaken under $H_2$ atmosphere (45 psi) using a Parr hydrogenation apparatus at rt for 5h. The solution was filtered through celite and the solvent was evaporated. The product was purified by reversed-phase HPLC using 10-60% $CH_3CN/H_2O$ and lyophilized affording the title compound as the corresponding TFA salt. Yield: 31 mg (64%); $^1H$ NMR (400 MHz, METHANOL-$D_4$): δ 1.24 (m, 5 H), 1.64 (m, 2 H), 1.68 (s, 9 H), 1.71 (m, 1 H), 1.78 (m, 2 H), 2.12 (m, 1 H), 3.27 (s, 3 H), 3.49 (t, J=5.57 Hz, 2 H), 3.70 (t, J=5.57 Hz, 2 H), 4.47 (d, J=7.62 Hz, 2 H), 6.63 (d, J=9.18 Hz, 1 H), 7.41 (dd, J=8.98, 1.95 Hz, 1 H), 7.47 (d, J=7.42 Hz, 1 H), 7.61 (d, J=1.95 Hz, 1 H), 7.88 (d, J=8.98 Hz, 1 H), 8.01 (d, J=2.34 Hz, 1 H); MS (ESI) $(M+H)^+$: 500.3; Anal. Calcd for $C_{26}H_{37}N_5O_3S$ + 2.5 TFA + 1.7 $H_2O$: C, 45.67; H, 5.30; N, 8.59. Found: C, 45.69; H, 5.32; N, 8.43.

**Example 18**

***N*-(5-{[[2-*tert*-Butyl-1-(cyclohexylmethyl)-1*H*-benzimidazol-5-yl](methyl)amino]sulfonyl}pyridin-2-yl)acetamide**

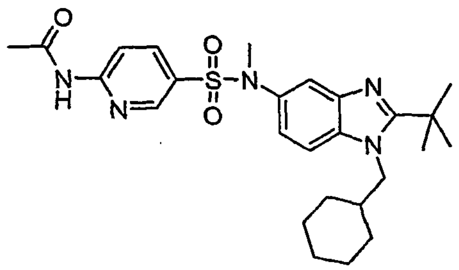

**Step A. *N*-(5-{[[2-*tert*-Butyl-1-(cyclohexylmethyl)-1*H*-benzimidazol-5-yl](methyl)amino]sulfonyl}pyridin-2-yl) acetamide**

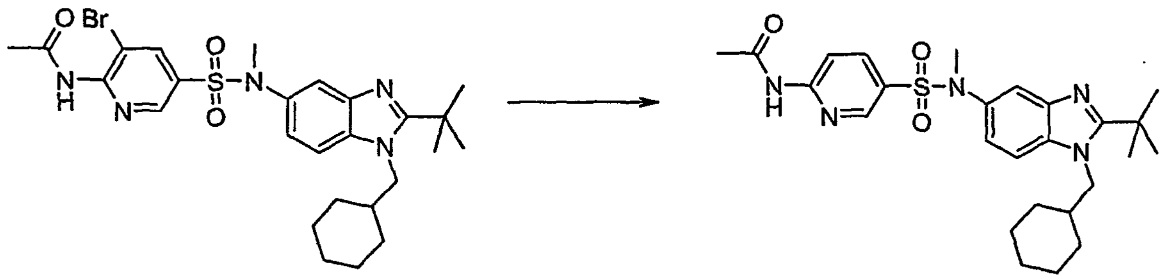

Following the same procedure as in Example 17 using *N*-(3-bromo-5-{[[2-*tert*-butyl-1-(cyclohexylmethyl)-1*H*-benzimidazol-5-yl](methyl)amino]sulfonyl}pyridin-2-yl)acetamide (see Step B for preparation) (16 mg, 0.0278 mmol). The product was purified by reversed-phase HPLC using 10-60% $CH_3CN/H_2O$ and lyophilized affording the title compound as the corresponding TFA salt. Yield: 13 mg (76%); $^1$H NMR (400 MHz, METHANOL-$D_4$): δ 1.24 (m, 5 H), 1.64 (m, 2 H), 1.67 (s, 9 H), 1.70 (m, 1 H), 1.78 (m, 2 H), 2.11 (m, 1 H), 2.19 (s, 3 H), 3.30 (m, 3 H), 4.46 (d, J=7.62 Hz, 2 H), 7.36 (dd, J=8.98, 2.15 Hz, 1 H), 7.58 (d, J=1.95 Hz, 1 H), 7.81 (dd, J=8.89, 2.25 Hz, 1 H), 7.87 (d, J=8.98 Hz, 1 H), 8.25 (d, J=8.98 Hz, 1 H), 8.40 (s, 1 H); MS (ESI) $(M+H)^+$: 498.2; Anal. Calcd for $C_{26}H_{35}N_5O_3S$ + 2.0 TFA + 1.0 $H_2O$: C, 48.45; H, 5.29; N, 9.42. Found: C, 48.37; H, 5.16; N, 9.64.

**Step B. *N*-(3-Bromo-5-{[[2-*tert*-butyl-1-(cyclohexylmethyl)-1*H*-benzimidazol-5-yl](methyl)amino]sulfonyl}pyridin-2-yl)acetamide**

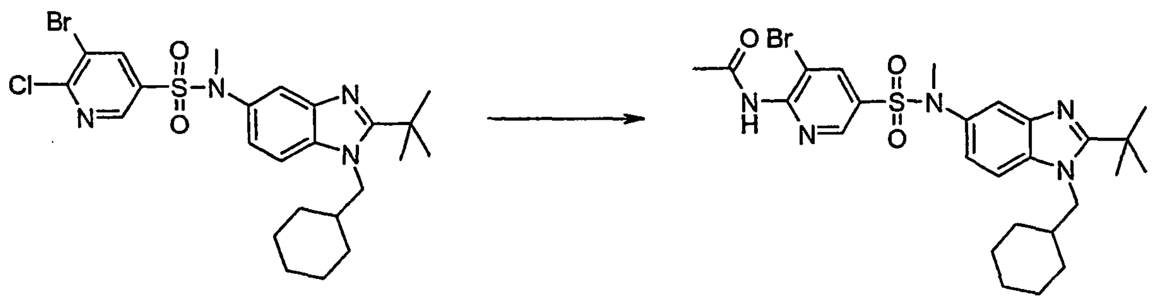

5-Bromo-*N*-[2-*tert*-butyl-1-(cyclohexylmethyl)-1*H*-benzimidazol-5-yl]-6-chloro-*N*-methylpyridine-3-sulfonamide (87 mg, 0.157 mmol) was dissolved in 2 mL of DMF containing ammonia (28% w/v in water) (1 mL). The solution was heated at 120°C for 30 min using a Personal Chemistry microwaves instrument. The solvent was evaporated. The residue was dissolved in EtOAc and washed with saturated aqueous $NaHCO_3$ solution, brine and dried over anhydrous $MgSO_4$. The solvent was evaporated. The product was dissolved in 2 mL of DCE containing a catalytic amount of DMAP. Acetyl chloride (0.055 mL, 0.785 mmol) was added and the solution was heated at 120°C for 30 min using a Personal Chemistry microwaves instrument. The solution was washed with saturated aqueous $NaHCO_3$ solution, brine and dried over anhydrous $MgSO_4$. The crude product was purified by silica gel flash chromatography using 1:1 / hexanes:EtOAc as eluent. Yield: 16 mg (18%); MS (ESI) $(M+H)^+$: 578.28.

**Example 19**

***N*-(3-{[[2-*tert*-Butyl-1-(cyclohexylmethyl)-1*H*-benzimidazol-5-yl](methyl)amino]sulfonyl}phenyl)acetamide**

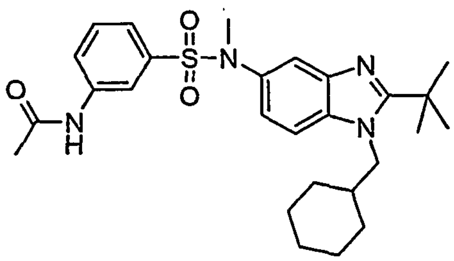

**Step A. *N*-(3-{[[2-*tert*-Butyl-1-(cyclohexylmethyl)-1*H*-benzimidazol-5-yl](methyl)amino]sulfonyl}phenyl)acetamide**

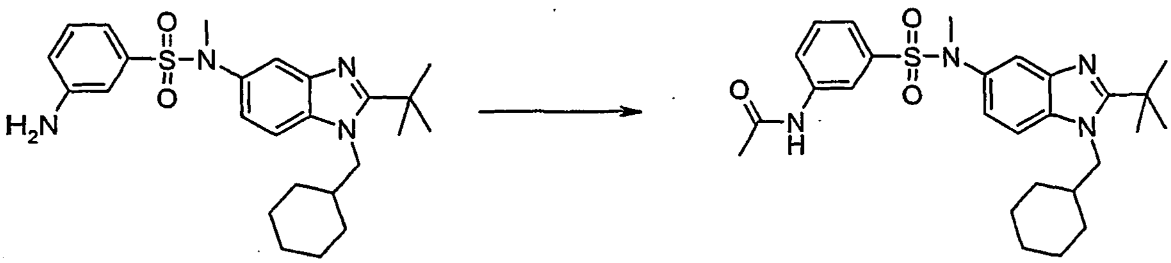

3-Amino-*N*-[2-*tert*-butyl-1-(cyclohexylmethyl)-1*H*-benzimidazol-5-yl]-*N*-methylbenzenesulfonamide (see Step B and C for preparation) (40 mg, 0.0880 mmol) and acetyl chloride (0.008 mL, 0.106 mmol) were stirred in 2 mL of DCM containing a catalytic amount of DMAP at rt for 1h. The solvent was evaporated. The product was purified by reversed-phase HPLC using 10-60% $CH_3CN/H_2O$ and lyophilized affording the title compound as the corresponding TFA salt. Yield: 38 mg (71%); $^1H$ NMR (400 MHz, METHANOL-$D_4$): δ 1.24 (m, 5 H), 1.65 (m, 2 H), 1.68 (s, 9 H), 1.71 (m, 1 H), 1.77 (m, 2 H), 2.07 (s, 3 H), 2.11 (m, 1 H), 3.28 (s, 3 H), 4.46 (d, J=7.42 Hz, 2 H), 7.32 (m, 2 H), 7.47 (t, J=8.01 Hz, 1 H), 7.59 (m, 2 H), 7.84 (d, J=8.98 Hz, 1 H), 7.93 (t, J=1.86 Hz, 1 H); MS (ESI) $(M+H)^+$: 497.2; Anal. Calcd for $C_{27}H_{36}N_4O_3S$ + 1.7 TFA + 0.5 $H_2O$: C, 52.20; H, 5.58; N, 8.01. Found: C, 52.14; H, 5.48; N, 8.08.

**Step B. *N*-[2-*tert*-Butyl-1-(cyclohexylmethyl)-1*H*-benzimidazol-5-yl]-*N*-methyl-3-nitrobenzenesulfonamide**

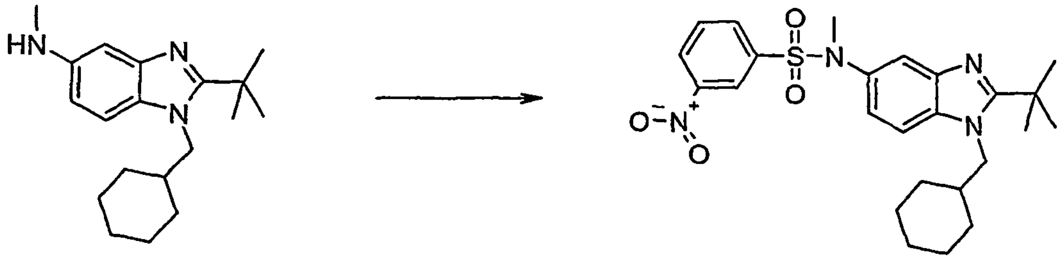

2-*tert*-Butyl-1-(cyclohexylmethyl)-*N*-methyl-1*H*-benzimidazol-5-amine (50 mg, 0.167 mmol) and 3-nitrophenylsulphonyl chloride (44 mg, 0.200 mmol) were stirred in 3 mL of DCM containing a catalytic amount of DMAP at rt overnight. The solution was washed with saturated aqueous $NaHCO_3$ solution, brine and dried over anhydrous MgSO4. The crude product was purified by silica gel flash chromatography using 1:1 / hexanes:EtOAc as eluent. Yield: 75 mg (94%); $^1H$ NMR (400 MHz, CHLOROFORM-D): δ 1.09 (m, 1 H), 1.11 (m, 1 H), 1.15 (m, 1 H), 1.18 (m, 1 H), 1.21 (m, 1 H), 1.53 (s, 9 H), 1.64 (m, 1 H), 1.67 (m, 1 H), 1.72 (m, 1 H), 1.76 (m, 1 H), 2.03 (m, 1 H), 3.29 (s, 3 H), 4.12 (d, J=7.62 Hz, 2 H), 7.18 (m, 2 H), 7.31 (d, J=8.40 Hz, 1 H), 7.67 (t, J=8.01 Hz, 1 H), 7.91 (m, 1 H), 8.39 (t, J=1.76 Hz, 1 H), 8.43 (ddd, J=8.10, 2.25, 0.98 Hz, 1 H).

**Step C. 3-Amino-*N*-[2-*tert*-butyl-1-(cyclohexylmethyl)-1*H*-benzimidazol-5-yl]-*N*-methylbenzenesulfonamide**

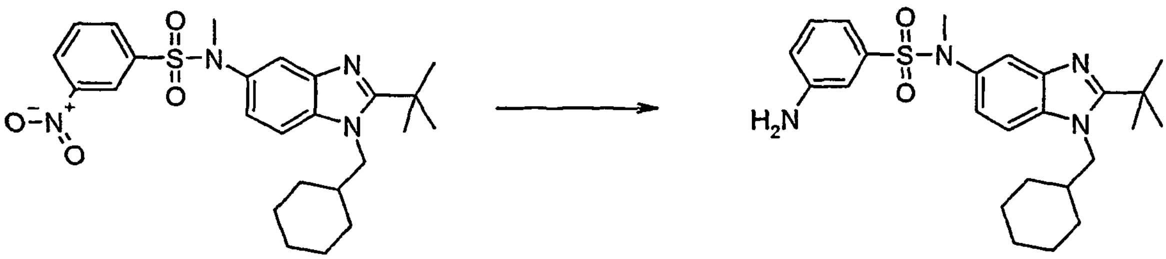

*N*-[2-*tert*-Butyl-1-(cyclohexylmethyl)-1*H*-benzimidazol-5-yl]-*N*-methyl-3-nitrobenzenesulfonamide (72 mg, 0.149 mmol) was dissolved in 15 mL of EtOH containing a catalytic amount of 10% Pd/C. The solution was shaken under H2 atmosphere (45 psi) using a Parr hydrogenation apparatus at rt for 6h. The solution was filtered through celite and the solvent was evaporated. The crude product was purified by silica gel flash chromatography using 1:1 / hexanes: EtOAc as eluent. Yield: 43 mg (63%); $^1H$ NMR (400 MHz, CHLOROFORM-D): δ 1.08 (m, 1 H), 1.11 (m, 1 H), 1.15 (m, 1 H), 1.18 (m, 1 H), 1.20 (m, 1 H), 1.54 (s, 9 H), 1.65 (m, 1 H), 1.68 (m, 1 H), 1.71 (m, 1 H), 1.75 (m, 2 H), 2.03 (m, 1 H), 3.22 (s, 3 H), 3.82 (m, 2 H), 4.11 (d, J=7.42 Hz, 2 H), 6.83 (ddd, J=8.01, 2.44, 0.88 Hz, 1 H), 6.90 (t, J=1.95 Hz, 1 H), 6.97 (m, 1 H), 7.22 (m, 2 H), 7.28 (m, 1 H), 7.33 (m, 1 H).

**Example 20**

***N*¹-(4-{[[2-*tert*-Butyl-1-(cyclohexylmethyl)-1*H*-benzimidazol-5-yl](methyl)amino]sulfonyl}phenyl)-*N*²-(2-hydroxyethyl)glycinamide**

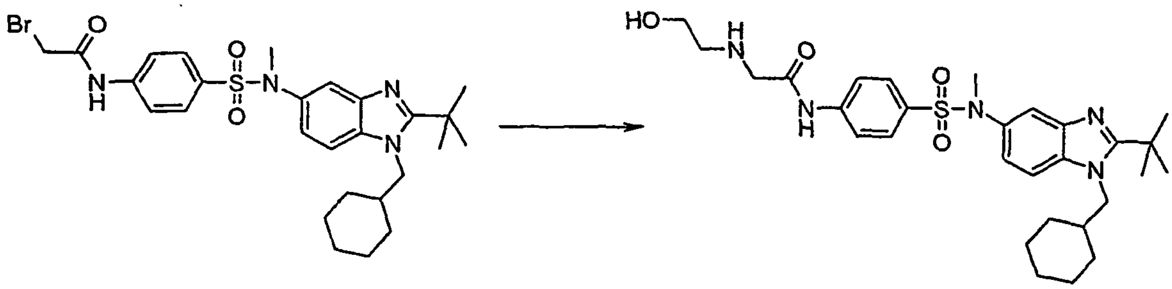

2- Bromo- *N*-(4- { [ [2-*tert*-butyl- 1-(cyclohexylmethyl)- 1*H*-benzimidazol- 5- yl] (methyl) amino] sulfonyl} phenyl) acetamide (for preparation, see Example 9, Step B) (56 mg, 0.0973 mmol) and ethanolamine (0.030 mL, 0.487 mmol) were heated in 1 mL of DMF at 125°C for 15 min using a Personal Chemistry microwaves instrument. The solvent was evaporated. The residue was dissolved in EtOAc and washed with saturated aqueous $NaHCO_3$ solution, brine and dried over anhydrous $MgSO_4$. The solvent was evaporated. The product was purified by reversed-phase HPLC using 10-70% $CH_3CN/H_2O$ and lyophilized affording the title compound as the corresponding TFA salt. Yield: 20 mg (31%); $^1H$ NMR (400 MHz, METHANOL-$D_4$): δ 1.24 (m, 5 H), 1.63 (m, 2 H), 1.67 (s, 9 H), 1.70 (m, 1 H), 1.78 (m, 2 H), 2.11 (m, 1 H), 3.24 (m, 2 H), 3.27 (s, 3 H), 3.85 (m, 2 H), 4.07 (s, 2 H), 4.45 (d, J=7.62 Hz, 2 H), 7.31 (dd, J=9.08, 2.05 Hz, 1 H), 7.53 (d, J=8.98 Hz, 2 H), 7.59 (d, J=1.76 Hz, 1 H), 7.77 (d, J=8.98 Hz, 2 H), 7.84 (d, J=8.98 Hz, 1 H); MS (ESI) $(M+H)^+$: 556.3.

**Example 21**

**4-[(Aminocarbonyl)amino]-*N*-[2-*tert*-butyl-1-(tetrahydro-2*H*-pyran-4-ylmethyl)-1*H*-benzimidazol-5-yl]-*N*-methylbenzenesulfonamide**

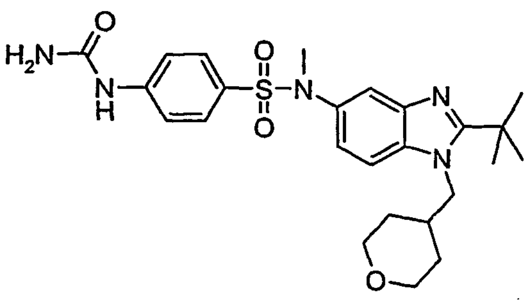

**Step A. 4-[(Aminocarbonyl)anino]-*N*-[2-*tert*-butyl-1-(tetrahydro-2*H*-pyran-4-ylmethyl)-1*H*-benzimidazol-5-yl]-*N*-methylbenzenesulfonamide**

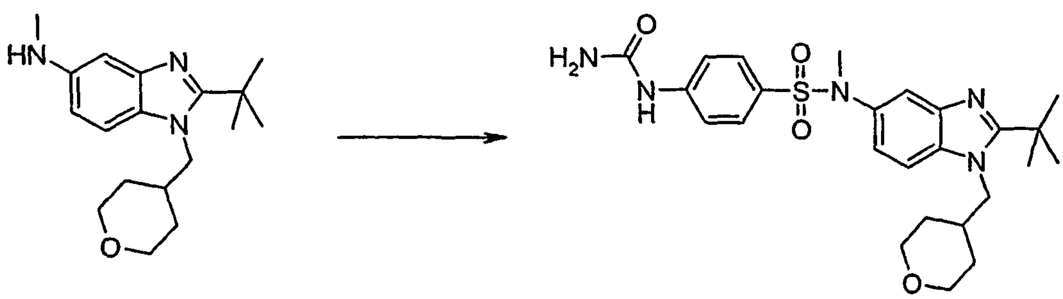

2-tert-Butyl-N-methyl-1-(tetrahydro-2H-pyran-4-ylmethyl)-1H-benzimidazol-5-amine (see following Steps B, C,

D, E and F for preparation) (30 mg, 0.0995 mmol) and 4-ureido-benzenesulfonyl chloride (28 mg, 0.119 mmol) were stirred in 3 mL of DMF containing a catalytic amount of DMAP at rt for 4h. The solvent was evaporated. The product was purified by reversed-phase HPLC using 10-70% $CH_3CN/H_2O$ and lyophilized affording the title compound as the corresponding TFA salt. Yield: 24 mg (39%); $^1$H NMR (400 MHz, METHANOL-$D_4$): δ 1.50 - 1.55 (m, 2 H), 1.56 - 1.63 (m, 2 H), 1.67 (s, 9 H), 2.32 - 2.40 (m, 1 H), 3.23 (s, 3 H), 3.34 (dt, J=11.42, 2.34 Hz, 2 H), 3.92 (d, J=3.12 Hz, 1 H), 3.95 (d, J=3.12 Hz, 1 H), 4.51 (d, J=7.42 Hz, 2 H), 7.32 (ddd, J=9.03, 2.00, 0.88 Hz, 1 H), 7.38 (d, J=8.20 Hz, 2 H), 7.49 - 7.54 (m, 3 H), 7.88 (d, J=8.98 Hz, 1 H); MS (ESI) $(M+H)^+$: 500.0; Anal. Calcd for $C_{25}H_{33}N_5O_4S$ + 1.7 TFA + 0.6 $H_2O$: C, 48.43; H, 5.14; N, 9.94. Found: C, 48.44; H, 5.04; N, 10.04.

**Step B: Methyl (4-fluoro-3-nitrophenyl)carbamate**

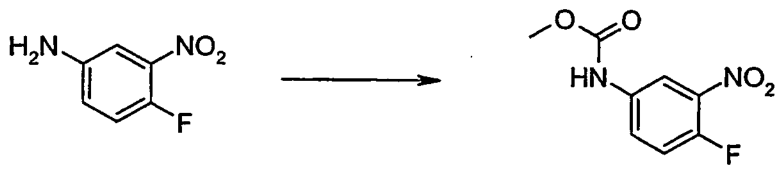

Methyl chloroformate (13.2 mL, 170.2 mmol) was added dropwise to a cold (0°C) dichloromethane (200 mL) solution of 4-fluoro-3-nitro aniline (24.15 g, 154.7 mmol) and DIPEA (35 mL, 201 mmol). The reaction mixture was stirred at rt overnight. The solution was then diluted with 200 mL of dichloromethane and washed with 2M HCl, brine and dried over anhydrous $MgSO_4$. The solvent was concentrated and the product was directly used for next step without further purification. Yield: 35.5 g (99%); $^1$H NMR (400 MHz, CHLOROFORM-D): δ 3.81 (s, 3H), 7.02 (s, 1H), 7.23 (m, 1H), 7.72 (d, J = 8.59Hz, 1H), 8.17 (dd, J = 6.35, 2.64Hz, 1H).

**Step C. Methyl {3-nitro-4-[(tetrahydro-2H-pyran-4-ylmethyl)amino]phenyl}carbamate**

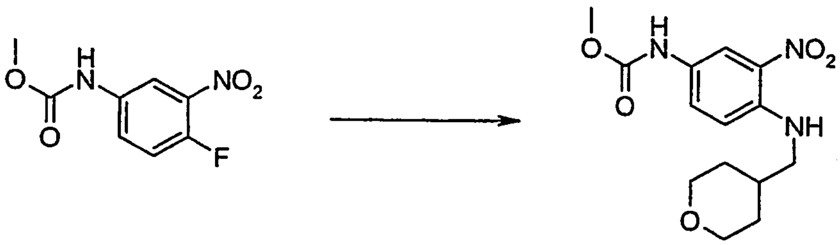

Methyl (4-fluoro-3-nitrophenyl)carbamate (2.0 g, 9.32 mmol) and 4-aminomethyl tetrahydropyran (1.28g, 11.2 mmol) were stirred in 50 mL of EtOH containing TEA (2.0 mL, 14.0 mmol) at 75°C for 48 h. The solvent was evaporated. The residue was dissolved in EtOAc and washed with aqueous 5% $KHSO_4$, saturated aqueous $NaHCO_3$ solution, brine and dried over anhydrous $MgSO_4$. The crude product was purified by silica gel flash chromatography using 1:1 / hexanes : EtOAc as eluent. Yield: 2.53 g (88%); $^1$H NMR (400 MHz, CHLOROFORM-D): δ 1.42 (ddd, *J*=25.24, 12.06, 4.49 Hz, 2 H), 1.73 (d, *J*=1.76 Hz, 1 H), 1.76 (d, *J*=1.95 Hz, 1 H), 1.88 - 2.01 (m, 1 H), 3.22 (dd, *J*=6.74, 5.57 Hz, 2 H), 3.42 (td, *J*=11.86, 2.05 Hz, 2 H), 3.78 (s, 3 H), 4.01 (d, *J*=4.30 Hz, 1 H), 4.04 (d, *J*=3.51 Hz, 1 H), 6.48 (br.s, 1 H), 6.85 (d, *J*=9.37 Hz, 1 H), 7.65 (br.s, 1 H), 8.03 - 8.09 (m, 2 H).

**Step D. Methyl {3-amino-4-[(tetrahydro-2*H*-pyran-4-ylmethyl)amino]phenyl}carbamate**

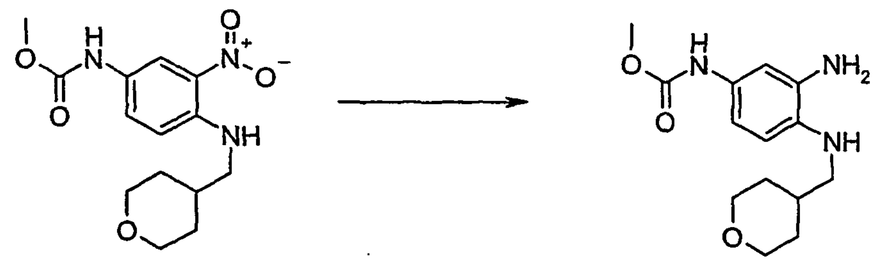

,

Methyl {3-nitro-4-[(tetrahydro-2H-pyran-4-ylmethyl)amino]phenyl}carbamate (2.53 g, 8.18 mmol) was dissolved in 50 mL of EtOAc containing a catalytic amount of 10% Pd/C. The solution was shaken under $H_2$ atmosphere (40 psi) using a Parr hydrogenation apparatus overnight at rt. The solution was filtered through celite and the solvent was evaporated. Yield: 2.29 g (99%); $^1$H NMR (400 MHz, CHLOROFORM-D): δ 1.40 (ddd, *J*=25.09, 12.01, 4.49 Hz, 2 H), 1.70 - 1.74 (m, 1 H), 1.74 - 1.77 (m, 1 H), 1.81 - 1.92 (m, 1 H), 2.99 (d, *J*=6.64 Hz, 2 H), 3.34 (br.s, 2 H), 3.41 (dt, *J*=11.81, 2.15 Hz, 2 H), 3.74 (s, 3 H), 3.99 (d, *J*=3.51 Hz, 1 H), 4.02 (d, *J*=3.51 Hz, 1 H), 6.38 (br.s, 1 H), 6.55 - 6.60 (m, 1 H), 6.62 - 6.68 (m, 1 H), 6.95 (br.s, 1 H).

**Step E. Methyl [2-tert-butyl-1-(tetrahydro-2H-pyran-4-ylmethyl)-1H-benzimidazol-5-yl]carbamate**

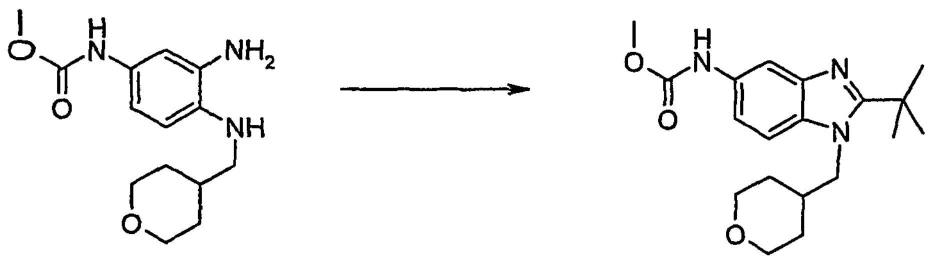

Methyl {3-amino-4-[(tetrahydro-2*H*-pyran-4-ylmethyl)amino]phenyl}carbamate (2.29 g, 8.20 mmol) and DMAP (0.20 g, 1.64 mmol) were dissolved in 75 mL of DCM. Trimethylacetyl chloride (1.10 mL, 9.02 mmol) was added dropwise and the solution was stirred at rt for 2h. The solution was washed with aqueous $NaHCO_3$ solution, brine and dried over anhydrous $MgSO_4$. The residue was dissolved in 25 mL of AcOH and was heated at 125°C for 1h using a Personal Chemistry microwave apparatus. The solvent was evaporated. The residue was dissolved in EtOAc and washed with aqueous $NaHCO_3$ solution, brine and dried over anhydrous $MgSO_4$. The crude product was purified by silica gel flash chromatography using 4:3 / hexanes : acetone as eluent. Yield: 1.81 g (64%); $^1$H NMR (400 MHz, CHLOROFORM-D): δ 1.48 - 1.54 (m, 4 H) 1.56 (s, 9 H) 2.23 - 2.35 (m, 1 H) 3.27 - 3.35 (m, 2 H) 3.78 (s, 3 H) 3.96 (t, *J*=2.93 Hz, 1 H) 3.99 (t, *J*=3.03 Hz, 1 H) 4.18 (d, *J*=7.42 Hz, 2 H) 6.63 (br.s, 1 H) 7.24 - 7.28 (m, 1 H) 7.41 (br.s, 1 H) 7.61 (d, *J*=1.95 Hz, 1 H).

**Step F: 2-tert-Butyl-N-methyl-1-(tetrahydro-2H-pyran-4-ylmethyl)-1H-benzimidazol-5-amine**

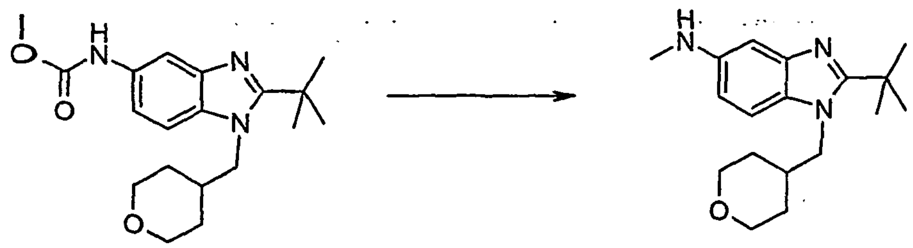

Methyl [2-tert-butyl-1-(tetrahydro-2H-pyran-4-ylmethyl)-1H-benzimidazol-5-yl]carbamate (1.80g, 5.21 mmol) was dissolved in 75 mL of THF at 0°C. 1M HCl/ether (7.3 mL, 7.29 mmol) was added dropwise and the solution was stirred at 0°C for 15 min. $LiA1H_4$ (988 mg, 26.1 mmol) was added slowly and the solution was stirred at rt overnight. The reaction was quenched at 0°C by the addition of MeOH (5 mL) followed by water (10 mL) and the solution was left to stir at rt for 30 min. Anhydrous $Na_2SO_4$ (10 g) was added and the solution was stirred at rt for another 30 min. The solution was filtered and the solvent was evaporated. The residue was dissolved in EtOAc and washed with aqueous $NaHCO_3$ solution, brine and dried over anhydrous $MgSO_4$. The solvent was evaporated. Yield: 1.54g (98%); $^1$H NMR (400 MHz, CHLOROFORM-D): δ 1.49 - 1.53 (m, 4 H), 1.53 - 1.57 (m, 9 H), 2.22 - 2.32 (m, 1 H), 2.87 (s, 3 H), 3.26 - 3.35 (m, 2 H), 3.95 (t, *J*=3.03 Hz, 1 H), 3.97 - 4.00 (m, 1 H), 4.13 (d, *J*=7.42 Hz, 2 H), 6.61 (dd, *J*=8.59, 2.15 Hz, 1 H), 6.99 (d, *J*=1.95 Hz, 1 H), 7.11 (d, *J*=8.59 Hz, 1 H).

**Example 22**

***N*-(4-{[[2-*tert*-Butyl-1-(tetrahydro-2*H*-pyran-4-ylmethyl)-1*H*-benzimidazol-5-yl](methyl)amino]sulfonyl}phenyl) acetamide**

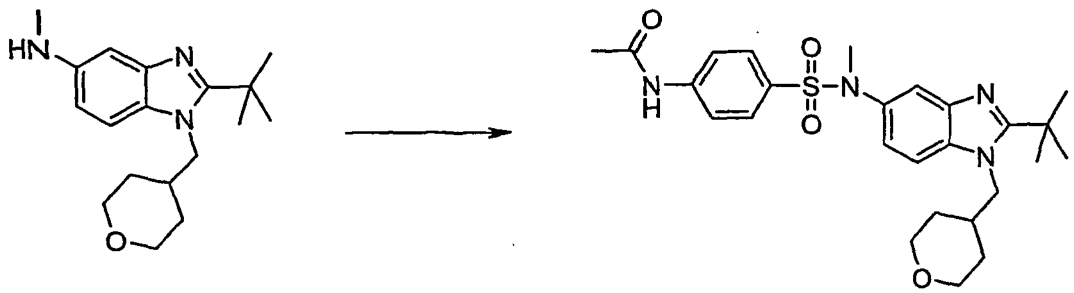

2-tert-Butyl-N-methyl-1-(tetrahydro-2H-pyran-4-ylmethyl)-1H-benzimidazol-5-amine (200 mg, 0.663 mmol) and N-acetylsulfanilyl chloride (186 mg, 0.796 mmol) were stirred in 10 mL of DCM containing DMAP (16 mg, 0.133 mmol) at rt for 48h. The solution was washed with saturated aqueous $NaHCO_3$ solution, brine and dried over anhydrous $MgSO_4$. The solvent was evaporated. The product was purified by reversed-phase HPLC using 10-60% $CH_3CN/H_2O$ and lyophilized affording the title compound as the corresponding TFA salt. Yield: 353 mg (87%); $^1$H NMR (400 MHz, METHANOL-$D_4$): δ 1.51 - 1.57 (m, 2 H), 1.56 - 1.65 (m, 2 H), 1.68 (s, 9 H), 2.14 (s, 3 H), 2.32 - 2.41 (m, 1 H), 3.25 (s, 3 H), 3.35 (td, *J*=11.47, 2.64 Hz, 2 H), 3.93 (d, *J*=2.93 Hz, 1 H), 3.96 (d, *J*=3.71 Hz, 1 H), 4.52 (d, *J*=7.62 Hz, 2 H), 7.32 (dd, *J*=8.98, 2.15 Hz, 1 H), 7.45 (d, *J*=8.98 Hz, 2 H), 7.54 (d, *J*=1.56 Hz, 1 H), 7.71 (d, *J*=8.98 Hz, 2 H), 7.88 (d, *J*=8.98 Hz, 1 H); MS (ESI) $(M+H)^+$: 499.0.

**Example 23**

***N*-(4-{[[2-*tert*-Butyl-1-(tetrahydro-2*H*-pyran-4-ylmethyl)-1*H*-benzimidazol-5-y](methyl)amino]sulfonyl}phenyl)-*N*-methylacetamide**

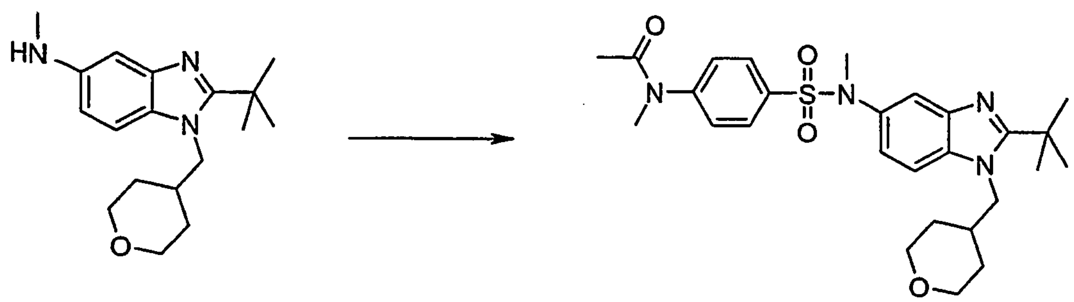

2-tert-Butyl-N-methyl-1-(tetrahydro-2H-pyran-4-ylmethyl)-1H-benzimidazol-5-amine (37 mg, 0.123 mmol) and N-acetylsulfanilyl chloride (37 mg, 0.160 mmol) were stirred in 5 mL of DCM containing DMAP (catalytic) at rt overnight. The solution was washed with saturated aqueous $NaHCO_3$ solution, brine and dried over anhydrous $MgSO_4$. The product was passed through a plug of silica gel using EtOAc as eluent and the solvent was evaporated. The product was dissolved in 5 mL of DMF at 0°C and NaH (60% dispersion in oil) (7 mg, 0.185 mmol) was added followed by iodomethane (0.012 mL, 0.185 mmol). The solution was stirred at rt for 2h. The solvent was evaporated. The residue was dissolved in EtOAc and washed with saturated aqueous $NaHCO_3$ solution, brine and dried over anhydrous $MgSO_4$. The solvent was evaporated. The product was purified by reversed-phase HPLC using 10-60% $CH_3CN/H_2O$ and lyophilized affording the title compound as the corresponding TFA salt. Yield: 28 mg (36%); $^1$H NMR (400 MHz, METHANOL-$D_4$): δ 1.51 - 1.57 (m, 2 H), 1.57 - 1.64 (m, 2 H), 1.69 (s, 9 H), 2.00 (br.s, 3 H), 2.32 - 2.41 (m, 1 H), 3.29 - 3.30 (m, 6 H), 3.32 - 3.39 (m, 2 H), 3.93 (d, J=2.93 Hz, 1 H), 3.96 (d, J=3.51 Hz, 1 H), 4.53 (d, J=7.42 Hz, 2 H), 7.34 (dd, J=8.98, 1.95 Hz, 1 H), 7.48 (d, J=8.79 Hz, 2 H), 7.59 - 7.65 (m, 3 H), 7.90 (d, J=8.98 Hz, 1 H); MS (ESI) $(M+H)^+$: 513.0; Anal. Calcd for $C_{27}H_{36}N_4O_4S$ + 2.3 TFA + 0.2 $H_2O$: C, 48.75; H, 5.01; N, 7.20. Found: C, 48.69; H, 4.97; N, 7.39.

## Example 24

**_N_-(4-{[[2-_tert_-Butyl-1-(tetrahydro-2_H_-pyran4-ylmethyl)-1_H_-benzimidazol-5-yl](methyl)amino]sulfonyl}phenyl)-2,2-dimethylpropanamide**

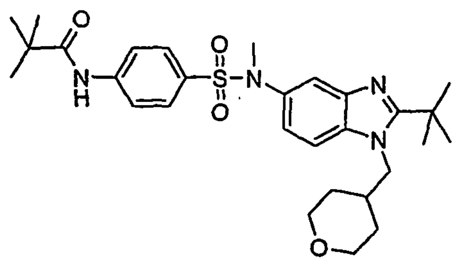

**Step A. _N_-(4-{[[2-_tert_-Butyl-1-(tetrahydro-2_H_-pyran-4-ylmethyl)-1_H_-benzimidazol-5-yl](methyl)amino]sulfonyl} phenyl)-2,2-dimethylpropanamide**

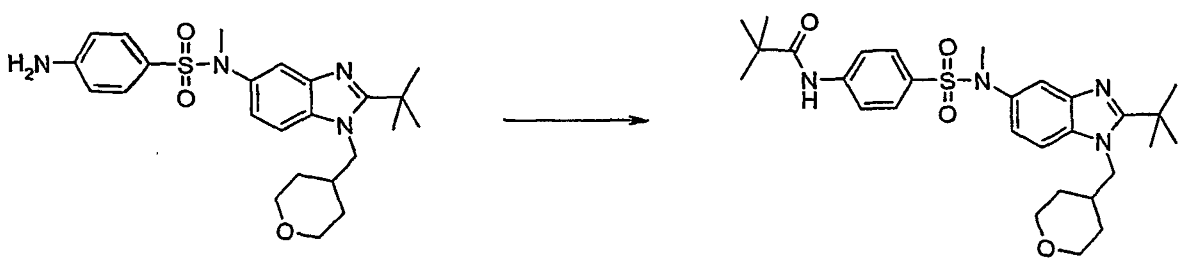

4-Amino-*N*-[2-*tert*-butyl-1-(tetrahydro-2*H*-pyran-4-ylmethyl)-1*H*-benzimidazol-5-yl]-*N*-methylbenzenesulfonamide (see following Steps B and C for preparation) (375 mg, 0.821 mmol) and trimethylacetyl chloride (0.120 mL, 0.985 mmol) were stirred in 20 mL of DCM containing a catalytic amount of DMAP at rt for 3h. The solution was washed with saturated aqueous $NaHCO_3$ solution, brine and dried over anhydrous $MgSO_4$. The product was purified by reversed-phase HPLC using 10-75% $CH_3CN/H_2O$ and lyophilized affording the title compound as the corresponding TFA salt. Yield: 445 mg (83%); $^1$H NMR (400 MHz, METHANOL-$D_4$): δ 1.29 (s, 9 H), 1.50 - 1.56 (m, 2 H), 1.57 - 1.65 (m, 2 H), 1.67 (s, 9 H), 2.32 - 2.40 (m, 1 H), 3.26 (s, 3 H), 3.35 (td, J=11.47, 2.64 Hz, 2 H), 3.93 (d, J=2.93 Hz, 1 H), 3.96 (d, J=3.32 Hz, 1 H), 4.51 (d, J=7.42 Hz, 2 H), 7.30 (dd, J=9.08, 2.05 Hz, 1 H), 7.45 (d, J=8.98 Hz, 2 H), 7.51 (d, J=1.95 Hz, 1 H), 7.75 (d, J=2.34 Hz, 1 H), 7.77 (d, J=2.34 Hz, 1 H), 7.86 (d, J=8.98 Hz, 1 H), 9.39 (s, 1 H); MS (ESI) $(M+H)^+$: 541.0.

**Step B. *N*-[2-*tert*-Butyl-1-(tetrahydro-2*H*-pyran-4-ylmethyl)-1*H*-benzimidazol-5-yl]-*N*-methyl-4-nitrobenzenesulfonamide**

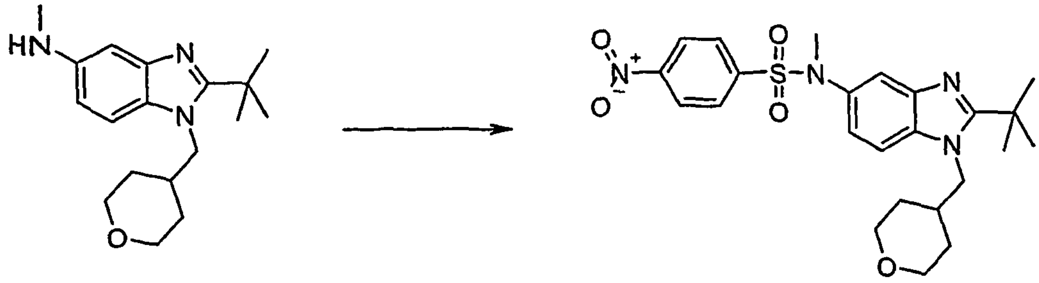

2-tert-Butyl-N-methyl-1-(tetrahydro-2H-pyran-4-ylmethyl)-1H-benzimidazol-5-amine (280 mg, 0.929 mmol) and 4-nitrobenzenesulfonyl chloride (247 mg, 1.11 mmol) were stirred in 10 mL of DCM containing a catalytic amount of DMAP at rt overnight. The solution was washed with saturated aqueous $NaHCO_3$ solution, brine and dried over anhydrous $MgSO_4$. The crude product was purified by silica gel flash chromatography using 1:1 / hexanes : EtOAc as eluent. Yield: 404 mg (89%); $^1H$ NMR (400 MHz, CHLOROFORM-D): δ 1.51 - 1.57 (m, 13 H), 2.24 - 2.34 (m, 1 H), 3.27 (s, 3 H), 3.30 - 3.38 (m, 2 H), 3.99 (t, *J*=2.93 Hz, 1 H), 4.02 (t, *J*=3.03 Hz, 1 H), 4.20 (d, *J*=7.42 Hz, 2 H), 7.19 - 7.23 (m, 2 H), 7.29 - 7.33 (m, 1 H), 7.77 (d, *J*=8.98 Hz, 2 H), 8.30 (d, *J*=8.79 Hz, 2 H).

**Step C. 4-Amino-*N*-[2-*tert*-butyl-1-(tetrahydro-2*H*-pyran-4-ylmethyl)-1*H*-benzimidazol-5-yl]-*N*-methylbenzenesulfonamide**

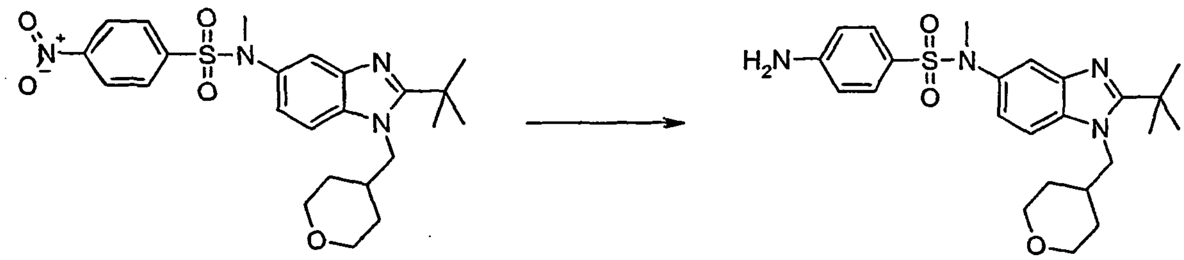

*N*-[2-*tert*-Butyl-1-(tetrahydro-2*H*-pyran-4-ylmethyl)-1*H*-benzimidazol-5-yl]-*N*-methyl-4-nitrobenzenesulfonamide (400 mg, 0.822 mmol) was dissolved in 30 mL of 1:1 / EtOAc:EtOH containing a catalytic amount of 10% Pd/C. The solution was shaken under $H_2$ atmosphere (40 psi) using a Parr hydrogenation apparatus overnight at rt. The solution was filtered through celite and the solvent was evaporated. Yield: 375 mg (99%); MS (ESI) $(M+H)^+$: 457.32.

**Example 25**

***N*-(4-{[[2-*tert*-Butyl-1-(tetrahydro-2*H*-pyran-4-ylmethyl)-1*H*-benzimidazol-5-yl](methyl)amino]sulfonyl}phenyl)-2-hydroxyacetamide**

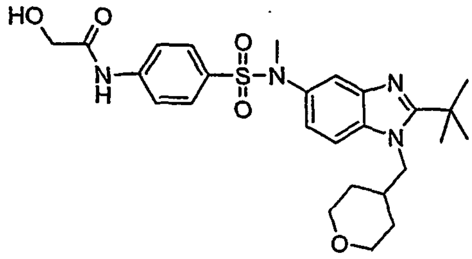

**Step A. *N*-(4-{[[2-*tert*-Butyl-1-(tetrahydro-2*H*-pyran-4-ylmethyl)-1*H*-benzimidazol-5-yl](methyl)amino]sulfonyl} phenyl)-2-hydroacyacetamide**

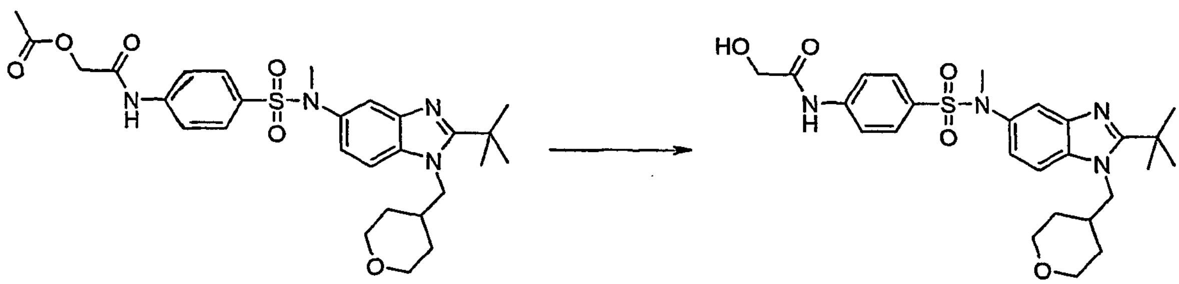

2-[(4-{[[2-*tert*-Butyl-1-(tetrahydro-2*H*-pyran-4-ylmethyl)-1*H*-benzimidazol-5-yl](methyl)amino]sulfonyl}phenyl) amino]-2-oxoethyl acetate (see following Step B for preparation) (45 mg, 0.0808 mmol) was dissolved in 3 mL of MeOH at 0°C. 25% NaOMe/MeOH (pH adjusted to 9.0) was added and solution was stirred at 0°C for 2h. The solvent was evaporated. The residue was dissolved in EtOAc and washed with aqueous 5% $KHSO_4$ solution. The aqueous phase was basified with saturated aqueous $NaHCO_3$ solution and extracted with EtOAc (2X). The organic phase was washed with brine and dried over anhydrous $MgSO_4$. The product was purified by reversed-phase HPLC using 10-60% $CH_3CN/H_2O$ and lyophilized affording the title compound as the corresponding TFA salt. Yield: 35 mg (69%); $^1H$ NMR (400 MHz, METHANOL-$D_4$): δ 1.51 - 1.57 (m, 2 H), 1.57 - 1.65 (m, 2 H), 1.68 (s, 9 H), 2.32 - 2.41 (m, 1 H), 3.26 (s, 3 H), 3.35 (dt, J=11.47, 2.64 Hz, 2 H), 3.93 (d, J=2.93 Hz, 1 H), 3.96 (d, J=3.71 Hz, 1 H), 4.13 (s, 2 H), 4.53 (d, J=7.62 Hz, 2 H), 7.33 (dd, J=9.08, 2.05 Hz, 1 H), 7.48 (d, J=8.98 Hz, 2 H), 7.54 (d, J=1.56 Hz, 1 H), 7.81 (d, J=8.98 Hz, 2 H), 7.89 (d, J=8.98 Hz, 1 H); MS (ESI) $(M+H)^+$: 515.0; Anal. Calcd for $C_{26}H_{34}N_4O_5S$ + 2.2 TFA + 1.6 $H_2O$: C, 42.84; H, 4.48; N, 6.25. Found: C, 42.77; H, 4.28; N, 6.65.

**Step B. 2-[(4-{[[2-*tert*-Butyl-1-(tetrahydro-2*H*-pyran-4-ylmethyl)-1*H*-benzimidazol-5-yl](methyl)amino]sulfonyl} phenyl)amino]-2-oxoethyl acetate**

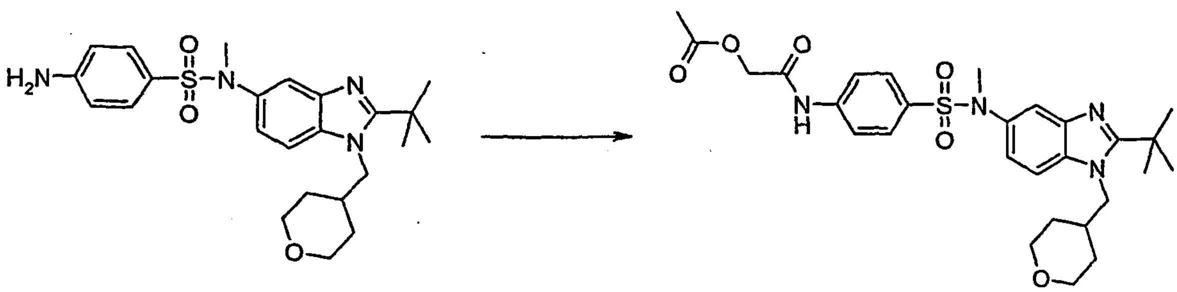

4-Amino-*N*-[2-*tert*-butyl-1-(tetrahydro-2*H*-pyran-4-ylmethyl)-1*H*-benzimidazol-5-yl]-*N*-methylbenzenesulfonamide (45 mg, 0.0986 mmol) and acetoxyacetyl chloride (0.013 mL, 0.118 mmol) were stirred in 2 mL of DCM containing a catalytic amount of DMAP at rt for 3h. The solution was washed with saturated aqueous $NaHCO_3$ solution, brine and

dried over anhydrous $MgSO_4$. The crude product was purified by silica gel flash chromatography using EtOAc as eluent. Yield: 45 mg (82%); $^1H$ NMR (400 MHz, CHLOROFORM-D): δ 1.50 - 1.55 (m, 13 H), 2.23 (s, 3 H), 2.25 - 2.33 (m, 1 H), 3.18 (s, 3 H), 3.29 - 3.37 (m, 2 H), 3.97 (t, J=2.83 Hz, 1 H), 4.00 (t, J=2.64 Hz, 1 H), 4.18 (d, J=7.23 Hz, 2 H) 4.67 - 4.71 (m, 2 H), 7.18 - 7.24 (m, 2 H), 7.24 - 7.29 (m, 1 H), 7.51 (d, J=8.79 Hz, 2 H), 7.62 (d, J=8.79 Hz, 2 H), 8.31 (s, 1 H).

**Example 26**

***N*¹-(4-{[[2-*tert*-Butyl-1-(tetrahydro-2*H*-pyran-4-ylmethyl)-1*H*-benzimidazol-5-yl](methyl)amino]sulfonyl}phenyl)-*N*²,*N*²-dimethylglycinamide**

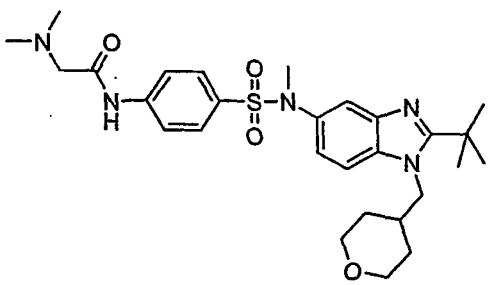

**Step A. *N*¹-(4-{[[2-*tert*-Butyl-1-(tetrahydro-2*H*-pyran-4-ylmethyl)-1*H* benzimidazol-5-yl](methyl)amino]sulfonyl} phenyl)-*N*²,*N*²-dimethylglycinamide**

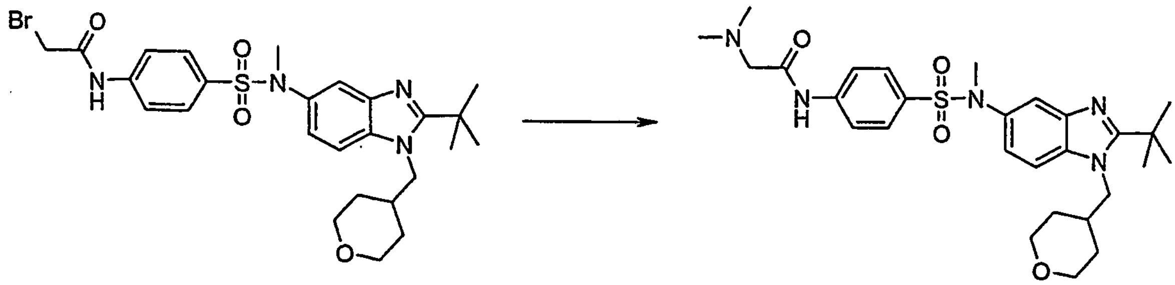

2-Bromo-*N*-(4-{[[2-*tert*-butyl-1-(tetrahydro-2*H*-pyran-4-ylmethyl)-1*H*-benzimidazol-5-yl](methyl)amino]sulfonyl}phenyl)acetamide (see following Step B for preparation) (36 mg, 0.0625 mmol) and dimethylamine hydrochloride (25 mg, 0.311 mmol) were stirred in 2 mL of DMF containing DIPEA (0.054 mL, 0.311 mmol) at 125°C for 15 min using a Personal Chemistry microwaves instrument. The solvent was evaporated. The product was purified by reversed-phase HPLC using 10-60% $CH_3CN/H_2O$ and lyophilized affording the title compound as the corresponding TFA salt. Yield: 34 mg (83%); $^1H$ NMR (400 MHz, METHANOL-$D_4$): δ 1.50 - 1.55 (m, 2 H), 1.56 - 1.64 (m, 2 H), 1.66 (s, 9 H), 2.31 - 2.40 (m, 1 H), 2.99 (s, 6 H), 3.26 (s, 3 H), 3.35 (dt, J=11.47, 2.64 Hz, 2 H), 3.93 (d, J=2.93 Hz, 1 H), 3.96 (d, J=3.7 Hz, 1 H), 4.18 (s, 2 H), 4.49 (d, J=7.62 Hz, 2 H), 7.26 (dd, J=8.98, 2.15 Hz, 1 H), 7.52 (d, J=8.98 Hz, 2 H), 7.55 (d, J=1.95 Hz, 1 H), 7.76 (d, J=8.98 Hz, 2 H), 7.81 (d, J=8.79 Hz, 1 H); MS (ESI) $(M+H)^+$: 542.3; Anal. Calcd for $C_{28}H_{39}N_5O_4S$ + 2.3 TFA + 1.0 $H_2O$: C, 47.64; H, 5.31; N, 8.52. Found: C, 47.68; H, 5.27; N, 8.55.

**Step B. 2-Bromo-*N*-(4-{[[2-*tert*-butyl-1-(tetrahydro-2*H*-pyran-4-ylmethyl)-1*H* benzimidazol-5-yl](methyl)amino] sulfonyl}phenyl)acetamide**

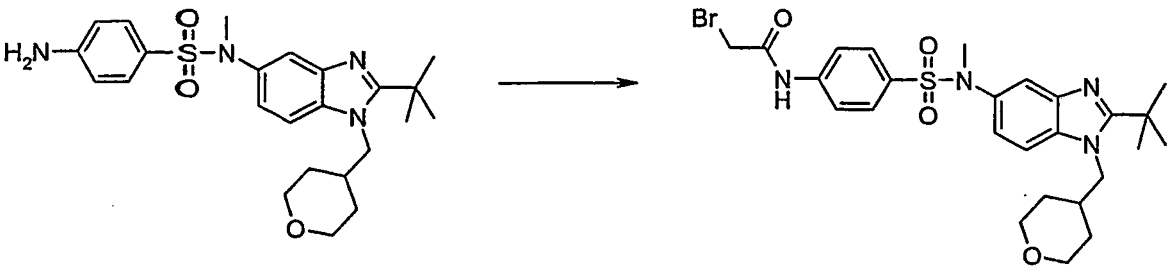

4-Amino-*N*-[2-*tert*-butyl-1-(tetrahydro-2*H*-pyran-4-ylmethyl)-1*H*-benzimidazol-5-yl]-*N*-methylbenzenesulfonamide (160 mg, 0.350 mmol) and bromoacetyl chloride (0.035 mL, 0.420 mmol) were stirred in 5 mL of DCM containing a catalytic amount of DMAP at rt for 3h. The solution was washed with saturated aqueous $NaHCO_3$ solution, brine and dried over anhydrous $MgSO_4$. The crude product was purified by silica gel flash chromatography using EtOAc as eluent. Yield: 127 mg (63%); $^1$H NMR (400 MHz, CHLOROFORM-D): δ 1.50 - 1.58 (m, 11 H), 1.59 -1.67 (m, 2 H), 2.25 - 2.36 (m, 1 H), 3.21 (s, 3 H), 3.30 - 3.39 (m, 2 H), 3.99 (br.s, 1 H), 4.01 (br.s, 1 H), 4.05 (s, 1 H), 4.20 (d, J=7.42 Hz, 2 H) 4.23 (s, 1 H), 7.22 (s, 1 H), 7.24 - 7.30 (m, 2 H), 7.53 - 7.59 (m, 2 H), 7.63 - 7.70 (m, 2 H), 8.43 (d, J=14.84 Hz, 1 H).

## Example 27

***N*^1-(4-{[[2-*tert*-Butyl-1-(tetrahydro-2*H*-pyran-4-ylmethyl)-1*H*-benzimidazol-5-yl](methyl)amino]sulfonyl}phenyl) glycinamide**

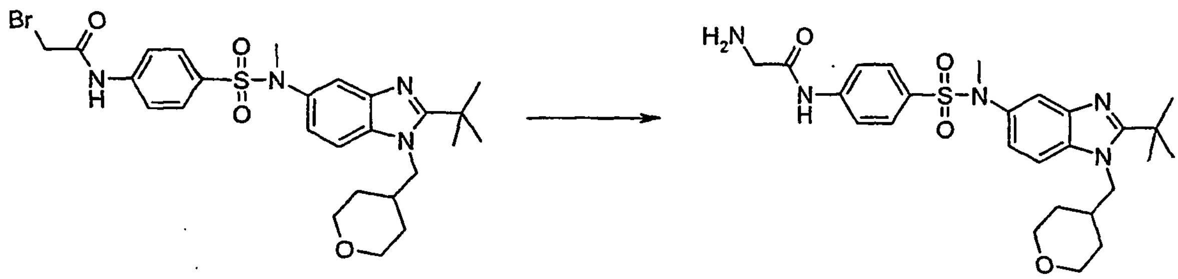

Same procedure as in Step A in Example 26 using 2-bromo-*N*-(4-{[[2-tert-butyl-1-(tetrahydro-2*H*-pyran-4-ylmethyl)-1*H*-benzimidazol-5-yl](methyl)amino]sulfonyl}phenyl)acetamide (50 mg, 0.0866 mmol) and 28% (w/v) ammonia in water (0.5 mL) in 2 mL of DMF. The product was purified by reversed-phase HPLC using 10-60% $CH_3CN/H_2O$ and lyophilized affording the title compound as the corresponding TFA salt. Yield: 41 mg (75%); $^1$H NMR (400 MHz, METHANOL-$D_4$): δ 1.50 - 1.56 (m, 2 H), 1.56 - 1.64 (m, 2 H), 1.67 (s, 9 H), 2.32 - 2.40 (m, 1 H), 3.26 (s, 3 H), 3.35 (dt, J=11.47, 2.64 Hz, 2 H), 3.89 (s, 2 H), 3.94 (d, J=2.93 Hz, 1 H), 3.96 (d, J=3.32 Hz, 1 H), 4.50 (d, J=7.42 Hz, 2 H), 7.27 (dd, J=9.08, 2.05 Hz, 1 H), 7.51 (d, J=8.98 Hz, 2 H), 7.56 (d, J=1.76 Hz, 1 H), 7.75 (d, J=9.18 Hz, 2 H), 7.83 (d, J=8.79 Hz, 1 H); MS (ESI) $(M+H)^+$: 514.0.

## Example 28

***N*^1-(4-{[[2-*tert*-Butyl-1-(tetrahydro-2*H*-pyran-4-ylmethyl)-1*H*-benzimidazol-5-yl](methyl)amino]sulfonyl}phenyl)-*N*^2-methylglycinamide**

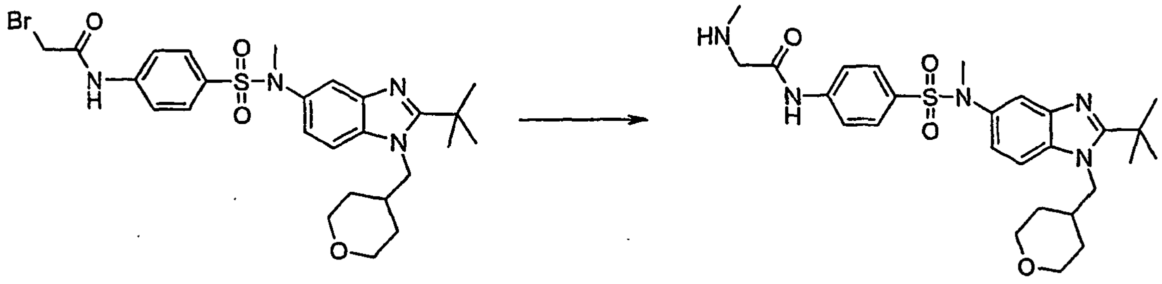

Same procedure as in Step A in Example 26 using 2-bromo-*N*-(4-{[[2-*tert*-butyl-1-(tetrahydro-2*H*-pyran-4-ylmethyl)-1*H*-benzimidazol-5-yl](methyl)amino]sulfonyl}phenyl)acetamide (30 mg, 0.0519 mmol), DIPEA (0.045 mL, 0.260 mmol) and methylamine hydrochloride (18 mg, 0.260 mmol) in 2 mL of DMF. The product was purified by reversed-phase HPLC using 10-60% $CH_3CN/H_2O$ and lyophilized affording the title compound as the corresponding TFA salt. Yield: 20 mg (60%); $^1H$ NMR (400 MHz, METHANOL-$D_4$): δ 1.49 - 1.57 (m, 2 H), 1.55 - 1.63 (m, 2 H), 1.66 (s, 9 H), 2.32 - 2.39 (m, 1 H), 2.78 (s, 3 H), 3.26 (s, 3 H), 3.35 (dt, J=11.42, 2.54 Hz, 2 H), 3.93 (d, J=2.93 Hz, 1 H), 3.96 (d, J=3.12 Hz, 1 H), 4.00 (s, 2 H), 4.48 (d, J=7.62 Hz, 2 H), 7.25 (dd, J=8.98, 1.95 Hz, 1 H), 7.52 (d, J=8.79 Hz, 2 H), 7.54 (d, J=1.95 Hz, 1 H), 7.75 (d, J=8.98 Hz, 2 H), 7.80 (d, J=8.98 Hz, 1 H); MS (ESI) $(M+H)^+$: 528.0.

**Example 29**

***N*-[2-*tert*-Butyl-1-(tetrahydro-2*H*-pyran-4-ylmethyl)-1*H*-benzimidazol-5-yl]-6-[(2-hydroxyethyl)amino]-*N*-methylpyridine-3-sulfonamide**

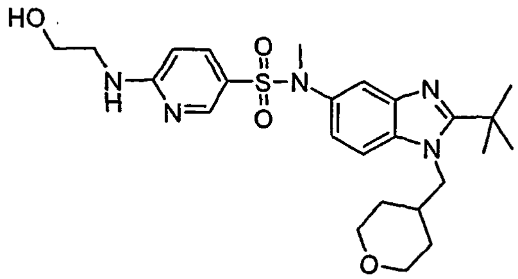

**Step A. *N*-[2-*tert*-Butyl-1-(tetrahydro-2*H*-pyran-4-ylmethyl)-1*H*-benzimidazol-5-yl]-6-[(2-hydroxyethyl)amino]-*N*-methylpyridine-3-sulfonamide**

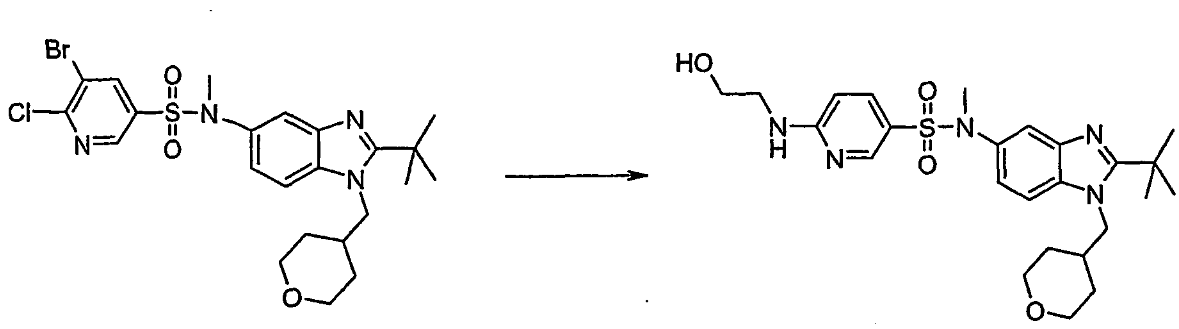

5-Bromo-*N*-[2-*tert*-butyl-1-(tetrahydro-2*H*-pyran-4-ylmethyl)-1*H*-benzimidazol-5-yl]-6-chloro-*N*-methylpyridine-3-sulfonamide (see following Step B for preparation) (270 mg, 0.484 mmol) and ethanolamine (0.145 mL, 2.42 mmol)

were stirred in 5 mL of DMF at 120°C for 3h. The solvent was concentrated. The product precipitated and was rinsed with ether. The product was dissolved in a 5:1 / EtOH:AcOH mixture (40 mL) containing a catalytic amount of 10% Pd/C and was shaken under $H_2$ atmosphere (50 psi) using a Parr hydrogenation apparatus at rt for 24h. The solution was filtered through celite and the solvent was evaporated. The product was purified by reversed-phase HPLC using 10-60% $CH_3CN/H_2O$ and lyophilized affording the title compound as the corresponding TFA salt. Yield: 240 mg (81%); $^1H$ NMR (400 MHz, METHANOL-$D_4$): δ 1.52 - 1.57 (m, 2 H), 1.58 - 1.65 (m, 2 H), 1.69 (s, 9 H), 2.33-2.41 (m, 1 H), 3.26 (s, 3 H), 3.36 (td, J=11.47, 2.64 Hz, 2 H), 3.48 (t, J=5.66 Hz, 2 H), 3.70 (t, J=5.57 Hz, 2 H), 3.93 (d, J=2.93 Hz, 1 H), 3.96 (d, J=3.71 Hz, 1 H), 4.53 (d, J=7.42 Hz, 2 H), 6.61 (d, J=8.98 Hz, 1 H), 7.41 (dd, J=9.08, 2.05 Hz, 1 H), 7.47 (dd, J=8.98, 1.95 Hz, 1 H), 7.61 (d, J=1.56 Hz, 1 H), 7.92 (d, J=9.18 Hz, 1 H), 7.99 (dd, J=2.44, 0.68 Hz, 1 H); MS (ESI) $(M+H)^+$: 502.0; Anal. Calcd for $C_{25}H_{35}N_5O_4S$ + 2.7 TFA: C, 45.11; H, 4.69; N, 8.65. Found: C, 45.18; H, 4.73; N, 8.43.

**Step B. 5-Bromo-*N*-[2-*tert*-butyl-1-(tetrahydro-2*H*-pyran4-ylmethyl)-1*H*-benzimidazol-5-yl]-6-chloro-*N*-methylpyridine-3-sulfonamide**

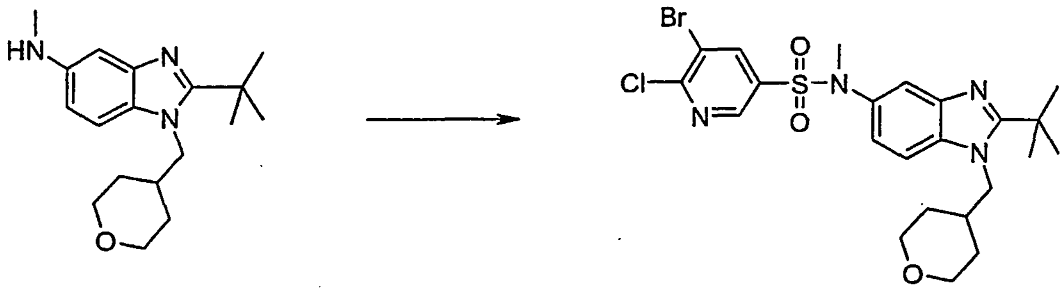

2-tert-Butyl-N-methyl-1-(tetrahydro-2H-pyran-4-ylmethyl)-1H-benzimidazol-5-amine (180 mg, 0.597 mmol) and 3-bromo, 2-chloro-pyridine-5-sulphonyl chloride (225 mg, 0.776 mmol) were stirred in 5 mL of DCM containing a catalytic amount of DMAP at rt for 4h. The solution was washed with saturated aqueous $NaHCO_3$ solution, brine and dried over anhydrous $MgSO_4$. The crude product was purified by silica gel flash chromatography using 1:1 / hexanes: EtOAc as eluent. Yield: 275 mg (83%); $^1H$ NMR (400 MHz, CHLOROFORM-D): δ 1.51 - 1.60 (m, 13 H), 2.24 - 2.34 (m, 1 H), 3.30 (s, 3 H), 3 .30 - 3.38 (m, 2 H), 3.99 (t, *J*=2.93 Hz, 1 H), 4.02 (t, *J*=2.93 Hz, 1 H), 4.20 (d, *J*=7.42 Hz, 2 H), 7.15 (dd, *J*=8.79, 1.76 Hz, I H), 7.29 - 7.33 (m, 2 H), 8.08 (d, *J*=2.15 Hz, 1 H), 8.39 (d, *J*=2.15 Hz, 1 H).

**Example 30**

***N*-[2-*tert*-Butyl-1-(tetrahydro-2*H*-pyran-4-ylmethyl)-1*H*-benzimidazol-5-yl]-6-[(2-methoxyethyl)amino]-*N*-methylpyridine-3-sulfonamide**

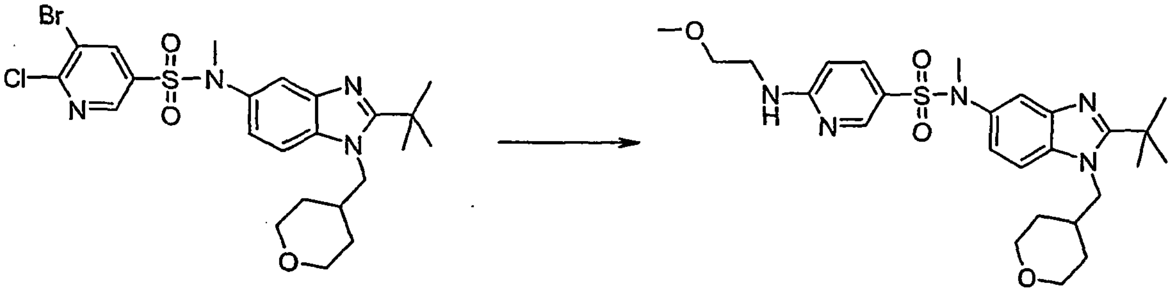

Following the same procedure as in Step A in Example 29 using 5-bromo-*N*-[2-*tert*-butyl-1-(tetrahydro-2*H*-pyran-4-ylmethyl)-1*H*-benzimidazol-5-yl]-6-chloro-*N*-methylpyridine-3-sulfonamide (70 mg, 0.126 mmol) and 2-methoxyethylamine (0.055 mL, 0.630 mmol) in 2 ml of DMF. The product was purified by reversed-phase HPLC using

10-60% $CH_3CN/H_2O$ and lyophilized affording the title compound as the corresponding TFA salt. Yield: 36 mg (45%); $^1H$ NMR (400 MHz, METHANOL-$D_4$): δ 1.52 -1.57 (m, 2 H), 1.57 - 1.66 (m, 2 H), 1.69 (s, 9 H), 2.33 - 2.42 (m, 1 H), 3.26 (s, 3 H), 3.32 - 3.39 (m, 5 H), 3.52 - 3.56 (m, 4 H), 3.93 (d, J=3.32 Hz, 1 H), 3.96 (d, J=3.91 Hz, 1 H), 4.53 (d, J=7.42 Hz, 2 H), 6.59 (d, J=9.18 Hz, 1 H), 7.41 (dd, J=9.08, 2.05 Hz, 1 H), 7.45 (dd, J=9.18, 1.95 Hz, 1 H), 7.61 (d, J=1.76 Hz, 1 H), 7.92 (d, J=8.98 Hz, 1 H), 8.00 (d, J=1.95 Hz, 1 H); MS (ESI) $(M+H)^+$: 516.0.

**Example 31**

***N*-[2-*tert*-Butyl-1-(tetrahydro-2*H*-pyran-4-ylmethyl)-1*H*-benzimidazol-5-yl]-6-(formylamino)-*N*-methylpyridine-3-sulfonamide**

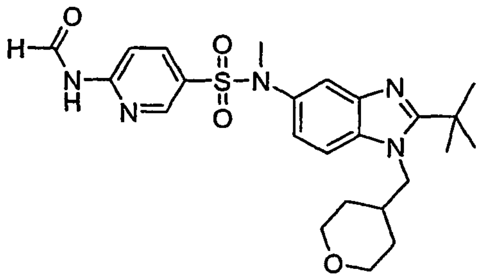

**Step A. *N*-[2-*tert*-Butyl-(tetrahydro-2*H*-pyran-4-ylmethyl)-1*H*-benzimidazol-5-yl]-6-(formylamino)-*N*-methylpyridine-3-sulfonamide**

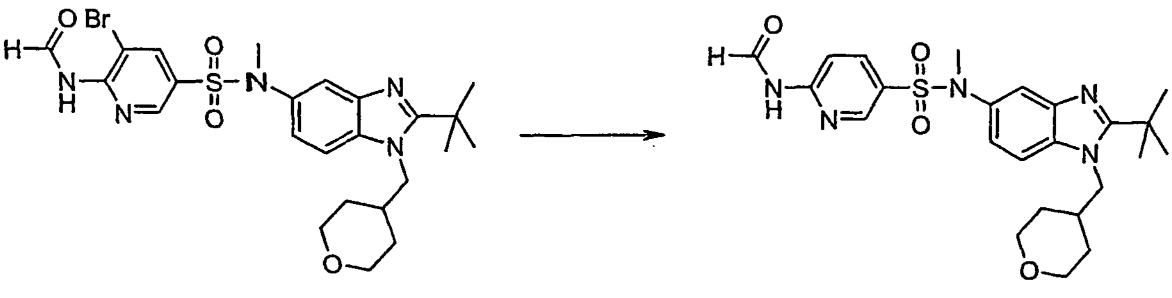

*N*-(3-Bromo-5-{[[2-*tert*-butyl-1-(tetrahydro-2*H*-pyran-4-ylmethyl)-1*H*-benzimidazol-5-yl](methyl)amino]sulfonyl}pyridin-2-yl)acetamide (see following Step B for preparation) (56 mg, 0.0992 mmol) was dissolved in 20 mL of EtOH containing a catalytic amount of 10% Pd/C. The solution was shaken under $H_2$ atmosphere (40 psi) using a Parr hydrogenation apparatus at rt overnight. The solution was filtered through celite and the solvent was evaporated. The product was purified by reversed-phase HPLC using 10-60% $CH_3CN/H_2O$ and lyophilized affording the title compound as the corresponding TFA salt. Yield: 36 mg (45%); $^1H$ NMR (400 MHz, METHANOL-$D_4$): δ 1.52 - 1.57 (m, 2 H), 1.57 - 1.66 (m, 2 H), 1.69 (s, 9 H), 2.33 - 2.42 (m, 1 H), 3.25 (s, 3 H), 3.35 (td, J=11.47, 2.83 Hz, 2 H), 3.93 (d, J=3.12 Hz, 1 H), 3.96 (d, J=3.71 Hz, 1 H), 4.54 (d, J=7.42 Hz, 2 H), 6.70 (d, J=9.18 Hz, 1 H), 7.40 (dd, J=8.98, 2.15 Hz, 1 H), 7.58 (dd, J=9.37, 2.54 Hz, 1 H), 7.60 (d, J=1.76 Hz, 1 H), 7.92 (d, J=8.98 Hz, 1 H), 8.05 (dd, J=2.54, 0.39 Hz, 1 H); MS (ESI) $(M+H)^+$: 486.0.

**Step B. 5-Bromo-*N*-[2-*tert*-butyl-1-(tetrahydro-2*H*-pyran-4-ylmethyl)-1*H*-benzimidazol-5-yl]-6-(formylamino)-*N*-methylpyridine-3-sulfonamide**

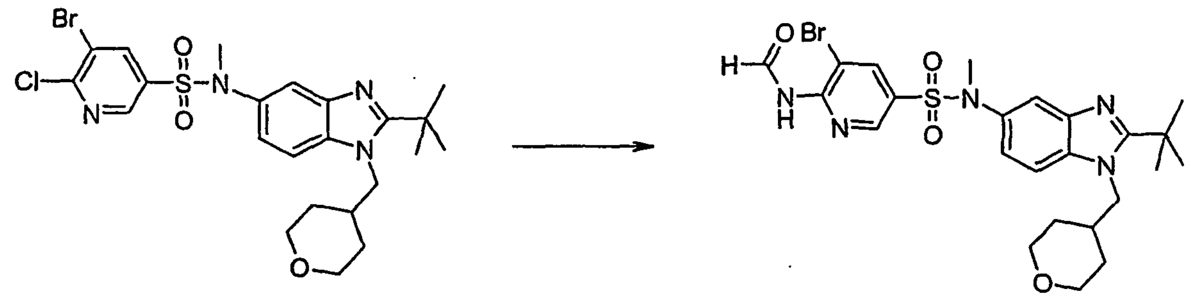

5-Bromo-*N*-[2-*tert*-butyl-1-(tetrahydro-2*H*-pyran-4-ylmethyl)-1*H*-benzimidazol-5-yl]-6-chloro-*N*-methylpyridine-3-sulfonamide (81 mg, 0.146 mmol) and 28% (w/v) ammonia in water (0.5 mL) were stirred in 3 mL of DMF at 120°C using a Personal Chemistry microwaves instrument for 30 min. The solvent was evaporated. The product was dissolved in EtOAc and washed with saturated aqueous $NaHCO_3$ solution, brine and dried over anhydrous $MgSO_4$. The crude product was purified by silica gel flash chromatography using EtOAc as eluent. Yield: 56 mg (68%). MS (ESI) $(M+H)^+$: 564.21.

## Example 32

### *N*-(5-{[[2-*tert*-Butyl-1-(tetrahydro-2*H*-pyran-4-ylmethyl)-1*H*-benzimidazol-5-yl](methyl)amino]sulfonyl}pyridin-2-yl)acetamide

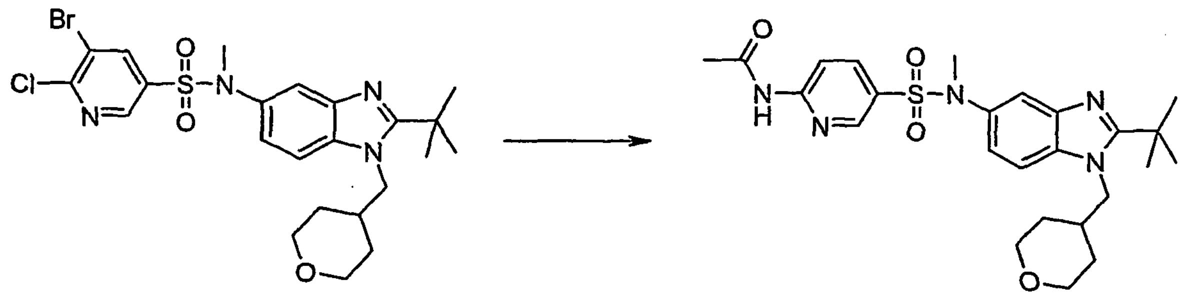

5-Bromo-*N*-[2-*tert*-butyl-1-(tetrahydro-2*H*-pyran-4-ylmethyl)-1*H*-benzimidazol-5-yl]-6-chloro-*N*-methylpyridine-3-sulfonamide (275 mg, 0.495 mmol) and 28% (w/v) ammonia in water (1 mL) were dissolved in 4 mL of dioxane. The solution was stirred at 125°C using a Personal Chemistry microwaves instrument for 1h. The solvent was evaporated. The product was dissolved in EtOAc and washed with saturated aqueous $NaHCO_3$ solution, brine and dried over anhydrous $MgSO_4$. The product was dissolved in 25 mL of EtOH containing a catalytic amount of 10% Pd/C. The solution was shaken under $H_2$ atmosphere (40 psi) using a Parr hydrogenation apparatus at rt overnight. The solution was filtered through celite and the solvent was evaporated. The residue was dissolved in 10 mL of 1:1 / DCE:pyridine and acetyl chloride (0.070 mL, 0.990 mmol) was added dropwise. The solution was stirred at rt for 3h. The solvent was evaporated. The product was dissolved in EtOAc and washed with saturated aqueous $NaHCO_3$ solution, brine and dried over anhydrous $MgSO_4$. The product was purified by reversed-phase HPLC using 10-60% $CH_3CN/H_2O$ and lyophilized affording the title compound as the corresponding TFA salt. Yield: 170 mg (56%); $^1H$ NMR (400 MHz, METHANOL-$D_4$): δ 1.51 - 1.56 (m, 2 H), 1.57 - 1.64 (m, 2 H), 1.68 (s, 9 H), 2.18 (s, 3 H), 2.32 - 2.41 (m, 1 H), 3.29 - 3.31 (m, 3 H), 3.35 (td, J=11.52, 2.54 Hz, 2 H), 3.93 (d, J=3.12 Hz, 1 H), 3.96 (d, J=2.93 Hz, 1 H), 4.52 (d, J=7.42 Hz, 2 H), 7.35 (dd, J=9.08, 2.05 Hz, 1 H), 7.57 (d, J=1.95 Hz, 1 H), 7.80 (dd, J=8.88, 2.44 Hz, 1 H), 7.90 (d, J=8.98 Hz, 1 H), 8.24 (d, J=8.79 Hz, 1 H), 8.39 (d, J=2.15 Hz, 1 H); MS (ESI) $(M+H)^+$:500.0; Anal. Calcd for $C_{25}H_{33}N_5O_4S$ + 1.4 TFA + 0.3 $H_2O$: C, 50.24; H, 5.31; N, 10.54. Found: C, 50.25; H, 5.30; N, 10.44.

**Example 33**

***N*-[4-({[2-*tert*-Butyl-1-(tetrahydro-2*H*-pyran-4-ylmethyl)-1*H*-benzimidazol-5-yl]amino}sulfonyl)phenyl]acetamide**

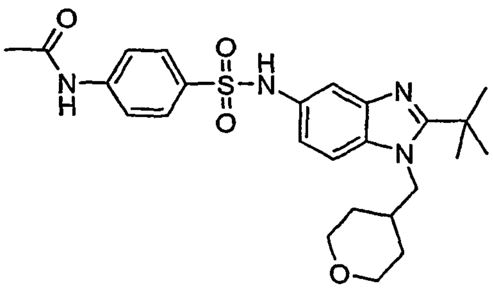

**Step A. *N*-[4-({[2-*tert*-Butyl-1-(tetrahydro-2*H*-pyran-4-ylmethyl)-1*H*-benzimidazol-5-yl]amino}sulfonyl)phenyl] acetamide**

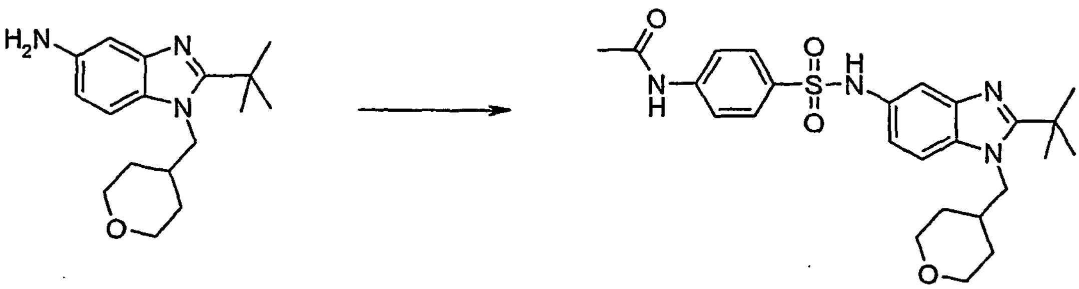

2-*tert*-Butyl-1-(tetrahydro-2*H*-pyran-4-ylmethyl)-1*H*-benzimidazol-5-amine (for preparation see following Steps B, C, D, E and F) (30 mg, 0.104 mmol) and 4-acetamidophenyl sulphonyl chloride (29 mg, 0.125 mmol) were stirred in 2 mL of DMF containing a catalytic amount of DMAP at rt for 4h. The solvent was evaporated and the product was purified by reversed-phase HPLC using 10-60% $CH_3CN/H_2O$ and lyophilized affording the title compound as the corresponding TFA salt. Yield: 23 mg (37%); $^1H$ NMR (400 MHz, METHANOL-$D_4$): δ 1.45 - 1.51 (m, 2 H), 1.52 - 1.60 (m, 2 H), 1.63 (s, 9 H), 2.09 (s, 3 H), 2.26 - 2.36 (m, 1 H), 3.32 (dt, J= 11.42,2.34 Hz, 2 H), 3.89 (d, J=2.93 Hz, 1 H), 3.92 (d, J=3.12 Hz, 1 H), 4.44 (d, J=7.62 Hz, 2 H), 7.24 (dd, J=8.98, 2.15 Hz, 1 H), 7.60 - 7.66 (m, 3 H), 7.62 - 7.73 (m, 2 H), 7.78 (d, J=8.98 Hz, 1 H); MS (ESI) $(M+H)^+$: 485.0; Anal. Calcd for $C_{25}H_{32}N_4O_4S$ + 1.8 TFA + 0.5 $H_2O$: C, 49.15; H, 5.02; N, 8.02. Found: C, 49.09; H, 5.00; N, 8.21.

**Step B. *N*-(4-Fluoro-3-nitrophenyl)acetamide**

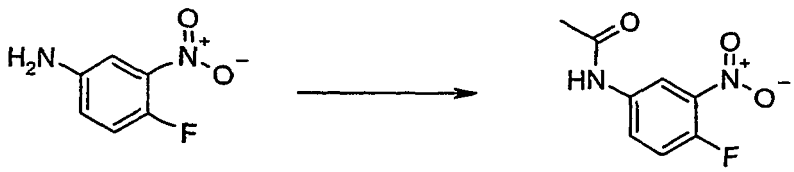

4-Fluoro-3-nitroaniline (5.0g, 32.0 mmol) was dissolved in 50 mL of DCM at 0°C containing TEA (6.7 mL, 48.0

mmol). Acetyl chloride (2.75 mL, 38.4 mmol) was added dropwise and the solution was stirred at rt overnight. The solution was washed with aqueous 5% $KHSO_4$ solution, saturated aqueous $NaHCO_3$ solution, brine and dried over anhydrous $MgSO_4$. The product was crystallized from DCM. Yield: 5.3g (84%); $^1H$ NMR (400 MHz, CHLOROFORM-D): δ 2.04 (s, 3 H), 7.51 (dd, J=11.23, 9.08 Hz, 1 H), 7.80 (ddd, J=9.08, 4.00, 2.93 Hz, 1 H), 8.47 (dd, J=7.03, 2.73 Hz, 1 H), 10.38 (s, 1 H).

**Step C. *N*-{3-Nitro-4-[(tetrahydro-2*H*-pyran-4-ylmethyl)amino]phenyl}acetamide**

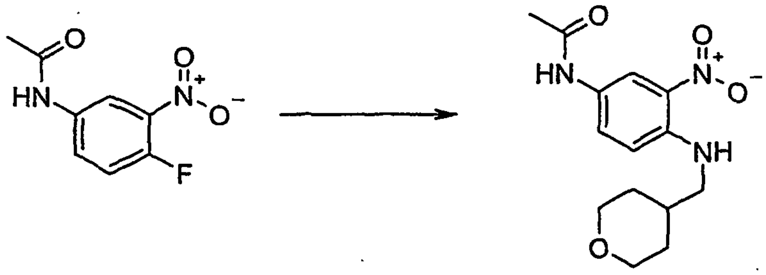

*N*-(4-Fluoro-3-nitrophenyl)acetamide (500 mg, 2.52 mmol) and 4-aminomethyl tetrahydropyran (350 mg, 3.02 mmol) were stirred in 20 mL of EtOH containing TEA (0.525 mL, 3.78 mmol) at 75°C overnight. The solvent was concentrated. The residue was dissolved in EtOAc and washed with aqueous 5% $KHSO_4$, saturated aqueous $NaHCO_3$ solution, brine and dried over anhydrous $MgSO_4$. The crude product was purified by silica gel flash chromatography using EtOAc as eluent. Yield: 611 mg (83%); $^1H$ NMR (400 MHz, CHLOROFORM-D): δ 1.42 (ddd, J=25.19, 12.11, 4.49 Hz, 2 H), 1.74 (dd, J=12.89, 1.95 Hz, 2 H), 1.89 - 2.00 (m, 1H), 2.18 (s, 3 H), 3.22 (dd, J=6.44, 5.66 Hz, 2 H), 3.42 (dt, J=11.86, 2.05 Hz, 2 H), 4.02 (dd, J=10.94, 3.71 Hz, 2 H), 6.84 (d, J=9.37 Hz, 1 H), 7.20 (br.s, 1 H), 7.81 (dd, J=9.37, 2.54 Hz, 1 H), 8.09 (d, J=2.54 Hz, 1 H), 8.10 - 8.12 (m, 1 H).

**Step D. *N*-{3-Amino-4-[(tetrahydro-2*H*-pyran-4-ylmethyl)amino]phenyl}acetamide**

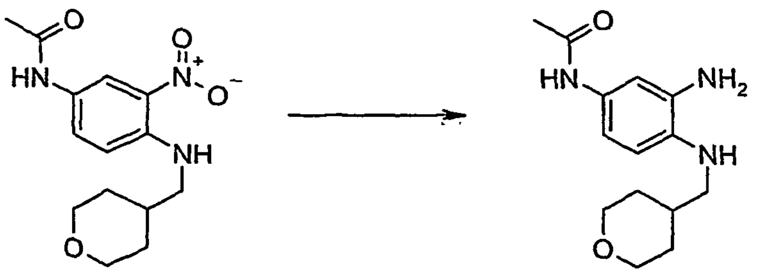

*N*-{3-Nitro-4-[(tetrahydro-2*H*-pyran-4-ylmethyl)amino]phenyl} acetamide (605mg, 2.06 mmol) was dissolved in 50 mL of EtOAc containing a catalytic amount of 10% Pd/C. The solution was shaken under $H_2$ atmosphere (40 psi) using a Parr hydrogenation apparatus overnight at rt. The solution was filtered through celite and the solvent was evaporated. Yield: 315g (58%); $^1H$ NMR (400 MHz, CHLOROFORM-D): δ 1.40 (ddd, J=25.14, 12.06, 4.39 Hz, 2 H), 1.74 (dd, J=12.89, 1.95 Hz, 2 H), 1.82 -1.91 (m, 1H), 2.13 (s, 3 H), 2.99 (d, J=6.64, 2 H), 3.42 (dt, J=11.86, 2.05 Hz, 2 H), 4.02 (dd, J=10.94, 3.71 Hz, 2 H), 6.84 (d, J=9.37 Hz, 1 H), 7.20 (br.s, 1 H), 7.81 (dd, J=9.37, 2.54 Hz, 1 H), 8.09 (d, J=2.54 Hz, 1 H), 8.10 - 8.12 (m, 1 H).

**Step E. *N*-[2-*tert*-Butyl-1-(tetrahydro-2*H*-pyran-4-ylmethyl)-1*H*-benzimidazol-5-yl]acetamide**

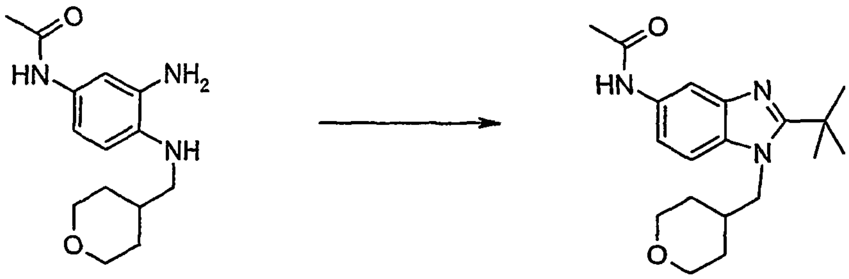

*N*-{3-Amino-4-[(tetrahydro-2*H*-pyran-4-ylmethyl)amino]phenyl}acetamide (315 mg, 1.20 mmol) and DMAP (30 mg, 0.240 mmol) were dissolved in 20 mL of DCM. Trimethylacetyl chloride (0.160 mL, 1.32 mmol) was added dropwise and the solution was stirred at rt for 2h. The solution was washed with aqueous $NaHCO_3$ solution, brine and dried over anhydrous $MgSO_4$. The residue was dissolved in 3 mL of AcOH and was heated at 125°C for 1h using a Personal Chemistry microwave apparatus. The solvent was evaporated. The residue was dissolved in EtOAc and washed with aqueous $NaHCO_3$ solution, brine and dried over anhydrous $MgSO_4$. The crude product was purified by silica gel flash chromatography using 1:1 / hexanes : acetone as eluent. Yield: 135 mg (34%); $^1$H NMR (400 MHz, CHLOROFORM-D): δ 1.48 - 1.54 (m, 4 H), 1.56 (s, 9 H), 2.20 (s, 3 H), 2.24 - 2.35 (m, 1 H), 3.28 - 3.35 (m, 2 H), 3.96 (t, J= 2.83 Hz, 1 H), 3.99 (t, J= 3.03 Hz, 1 H), 4.19 (d, J=7.42 Hz, 2 H), 7.27 (d, J=8.59 Hz, 1 H), 7.34 (br.s, 1 H), 7.57 (dd, J=8.79, 1.95 Hz, 1 H), 7.67 (d, J=1.95 Hz, 1 H).

**Step F. 2-*tert*-Butyl-1-(tetrahydro-2*H*-pyran-4-ylmethyl)-1*H*-benzimidazol-5-amine**

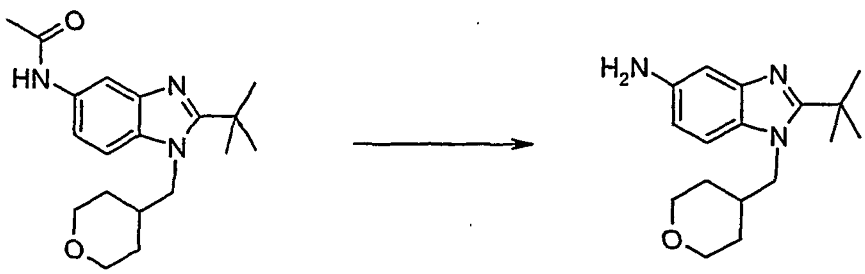

*N*-[2-*tert*-Butyl-1-(tetrahydro-2*H*-pyran-4-ylmethyl)-1*H*-benzimidazol-5-yl]acetamide (135 mg, 0.409 mmol) was dissolved in 4 mL of 1:1 / EtOH:2M HCl. The solution was heated at 120°C for 30 min using a Personal Chemistry microwave apparatus. The solvent was evaporated. The residue was dissolved in EtOAc and washed with 2M NaOH solution, brine and dried over anhydrous $MgSO_4$. The solvent was evaporated. Yield: 117 mg (99%); $^1$H NMR (400 MHz, CHLOROFORM-D): δ 1.47 - 1.52 (m, 4 H), 1.54 (s, 9 H), 2.23 - 2.31 (m, 1 H), 3.28 - 3.36 (m, 2 H), 3.96 (t, J= 3.12 Hz, 1 H), 3.97 - 4.00 (m, 1 H), 4.13 (d, J=7.62 Hz, 2 H), 6.66 (dd, J=8.40, 2.15 Hz, 1 H), 7.06 (d, J=2.15 Hz, 1 H), 7.10 (d, J=8.40 Hz, 1 H).

**Example 34**

***N*-[4-({[2-*tert*-Butyl-1-(cyclohexylmethyl)-1*H*-benzimidazol-5-yl]amino}sulfonyl)phenyl]acetamide**

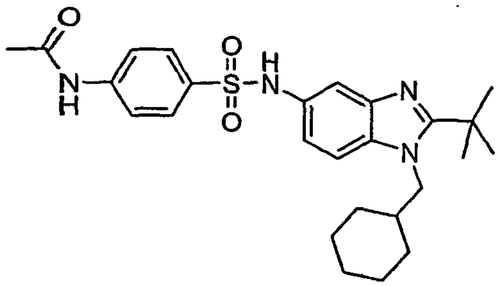

**Step A. *N*-[4-({[2-*tert*-Butyl-1-(cylohexylmethyl)-1*H*-benzimidazol-5-yl]amino}sulfonyl)phenyl]acetamide**

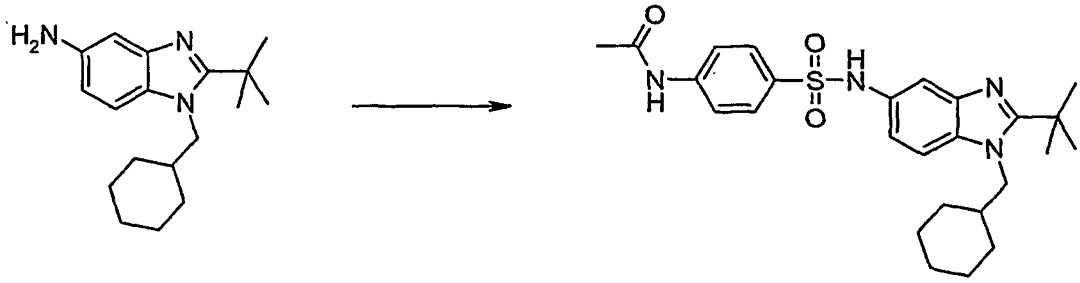

2-*tert*-Butyl-1-(cyclohexylmethyl)-1*H*-benzimidazol-5-amine (for preparation see following Steps, B, C, D, and E) (50 mg, 0.175 mmol) and 4-acetamidophenyl sulphonyl chloride (49 mg, 0.210 mmol) were stirred in 3 mL of DCM containing a catalytic amount of DMAP at rt for 4h. The solvent was evaporated and the product was purified by reversed-phase HPLC using 10-70% $CH_3CN/H_2O$ and lyophilized affording the title compound as the corresponding TFA salt. Yield: 80 mg (77%); $^1H$ NMR (400 MHz, METHANOL-$D_4$): δ 1.21 (m, 5 H), 1.59 (m, 1 H), 1.61 (m, 1 H), 1.63 (s, 9 H), 1.68 (m, 1 H), 1.75 (m, 2 H), 2.06 (m, 1 H), 2.10 (s, 3 H), 4.38 (d, J=7.62 Hz, 2 H), 7.25 (dd, J=9.08, 2.05 Hz, 1 H), 7.61 (d, J=1.56 Hz, 1 H), 7.66 (m, J=8.98 Hz, 2 H), 7.72 (m, 2 H), 7.76 (d, J=8.98 Hz, 1 H); MS (ESI) $(M+H)^+$: 483.3; Anal. Calcd for $C_{26}H_{34}N_4O_3S$ + 1.4 TFA + 0.5 $H_2O$: C, 53.11; H, 5.63; N, 8.60. Found: C, 53.03; H, 5.64; N, 8.72.

**Step B. *N*-{4-[(Cyclohexylmethyl)amino]-3-nitrophenyl}acetamide**

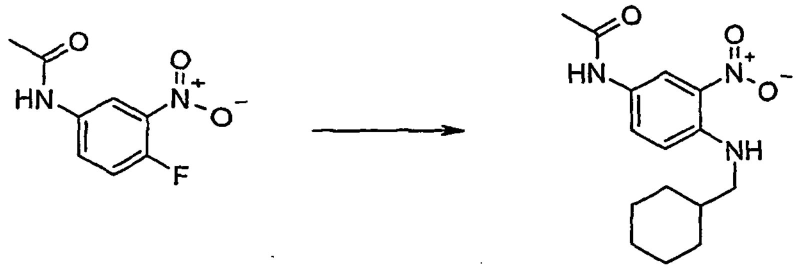

*N*-(4-Fluoro-3-nitrophenyl)acetamide (500 mg, 2.52 mmol) and cyclohexanemethylamine (0.400 mL, 3.02 mmol) were stirred in 15 mL of EtOH containing TEA (0.525 mL, 3.78 mmol) at 75°C overnight. The solvent was concentrated. The residue was dissolved in EtOAc and washed with aqueous 5% $KHSO_4$, saturated aqueous $NaHCO_3$ solution, brine and dried over anhydrous $MgSO_4$. The solvent was evaporated. Yield: 735 mg (99%); $^1H$ NMR (400 MHz, CHLOROFORM-D): δ 1.03 (m, 2 H), 1.25 (m, 3 H), 1.62 (m, 1 H), 1.69 (m, 1 H), 1.76 (m, 1 H), 1.79 (m, 1 H), 1.82 (m, 1 H), 1.86 (m,1 H), 2.17 (s, 3 H), 3.14 (dd, J=6.25, 4.30 Hz, 2 H), 6.83 (d, J=9.37 Hz, 1 H), 7.20 (m, 1 H), 7.78 (dd, J=9.28, 2.64 Hz, 1 H), 8.07 (d, J=2.54 Hz, 1 H), 8.12 (m, 1 H).

**Step C. *N*-{3-Amino-4-[(cyclohexylmethyl)amino]phenyl}acetamide**

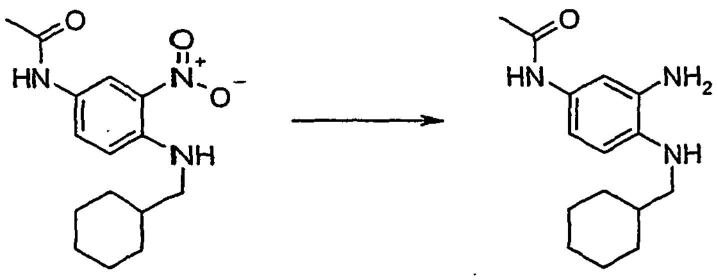

*N*-{4-[(Cyclohexylmethyl)amino]-3-nitrophenyl}acetamide (730 mg, 2.51 mmol) was dissolved in 40 mL of EtOAc containing a catalytic amount of 10% Pd/C. The solution was shaken under $H_2$ atmosphere (45 psi) using a Parr hydrogenation apparatus overnight at rt. The solution was filtered through celite and the solvent was evaporated. Yield: 629 mg (96%); $^1$H NMR (400 MHz, CHLOROFORM-D): δ 1.00 (m, 2 H), 1.25 (m, 4 H), 1.60 (m,1 H), 1.69 (m, 1 H), 1.73 (m, 1 H), 1.76 (m, 1 H), 1.83 (m, 1 H), 1.86 (m, 1 H), 2.13 (s, 3 H), 2.91 (d, J=6.64, 2 H), 3.38 (m, 2 H), 6.56 (d, J=8.40 Hz, 1 H), 6.69 (dd, J=8.40, 2.15 Hz, 1 H), 7.01 (m, 1 H), 7.11 (d, J=2.34 Hz, 1 H).

**Step D. *N*-[2-*tert*-Butyl-1-(cyclohexylmethyl)-1*H*-benzimidazol-5-yl]acetamide**

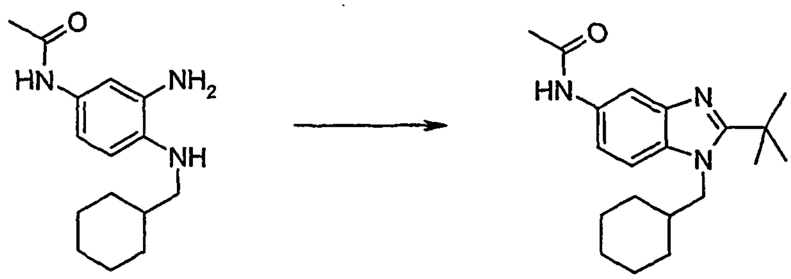

*N*-{3-Amino-4-[(cyclohexylmethyl)amino]phenyl}acetamide (367 mg, 1.40 mmol) and DMAP (34 mg, 0.280 mmol) were dissolved in 10 mL of DCM. Trimethylacetyl chloride (0.190 mL, 1.54 mmol) was added dropwise and the solution was stirred at rt for 1h. The solvent was evaporated. The product was dissolved in 4 mL of AcOH and was stirred at 150°C for 45 min. The solvent was evaporated. The residue was dissolved in EtOAc and washed with saturated aqueous $NaHCO_3$ solution, brine and dried over anhydrous $MgSO_4$. The crude product was purified by silica gel flash chromatography using 2:1 / hexanes : acetone as eluent. Yield: 268 mg (58%); $^1$H NMR (400 MHz, CHLOROFORM-D): δ 1.09 (m, 2 H), 1.17 (m, 3 H), 1.55 (s, 9 H), 1.62 (m, 1 H), 1.65 (m, 1 H), 1.69 (m, 1 H), 1.73 (m, 2 H), 2.03 (m, 1 H), 2.19 (s, 3 H), 4.11 (d, J=7.42, 2 H), 4.11 (d, J=7.42 Hz, 2 H), 7.27 (m, 1 H), 7.37 (m, 1 H), 7.55 (dd, J=8.69, 2.05 Hz, 1 H), 7.65(d, J=1.95 Hz, 1 H).

**Step E. 2-*tert*-Butyl-1-(tetrahydro-2*H*-pyran-4-ylmethyl)-1*H*-benzimidazol-5-amine**

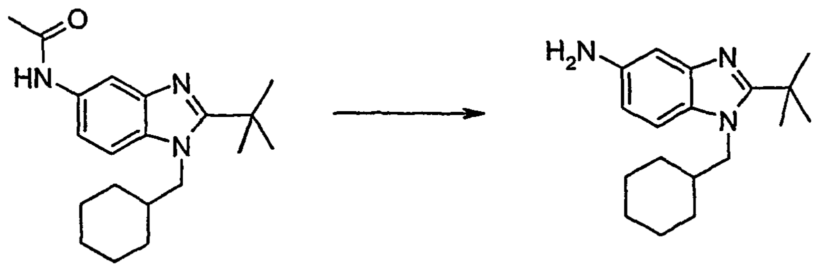

*N*-[2-*tert*-Butyl-1-(cyclohexylmethyl)-1*H*-benzimidazol-5-yl]acetamide (260 mg, 0.794 mmol) was dissolved in 4 mL of 1:1 / EtOH:2M HCl mixture. The solution was stirred at 170°C using a Personal Chemistry microwaves instrument for 30 min. The solvent was evaporated. The residue was dissolved in EtOAc and washed with saturated aqueous $NaHCO_3$ solution, brine and dried over anhydrous $MgSO_4$. The solvent was evaporated. Yield: 205 mg (90%); $^1H$ NMR (400 MHz, CHLOROFORM-D): δ 1.08 (m, 2 H), 1.17 (m, 3 H), 1.53 (s, 9 H), 1.63 (m, 1 H), 1.67 (m, 1 H), 1.72 (m, 1 H), 2.01 (m, 1 H), 3.58 (m, 1 H), 4.05 (d, J=7.42, 2 H), 6.64 (dd, J=8.59, 2.15 Hz, 2 H), 7.06 (d, J=1.95 Hz, 1 H), 7.11(d, J=8.40 Hz, 1 H).

**Example 35**

***N*-(4-{[[2-*tert*-Butyl-1-(2-piperidin-1-ylethyl)-1*H*-benzimidazol-5-yl](methyl)amino]sulfonyl}phenyl)acetamide**

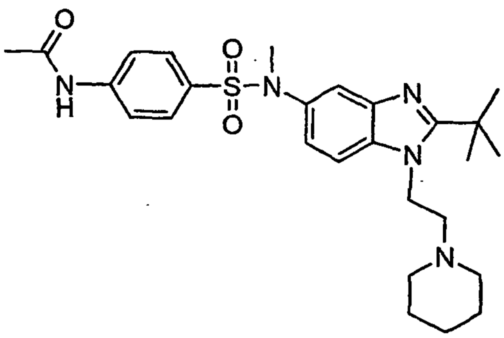

**Step A. *N*-(4-{[[2-*tert*-Butyl-1-(2-piperidin-1-ylethyl)-1*H*-benzimidazol-5-yl](methyl)amino]sulfonyl}phenyl) acetamide**

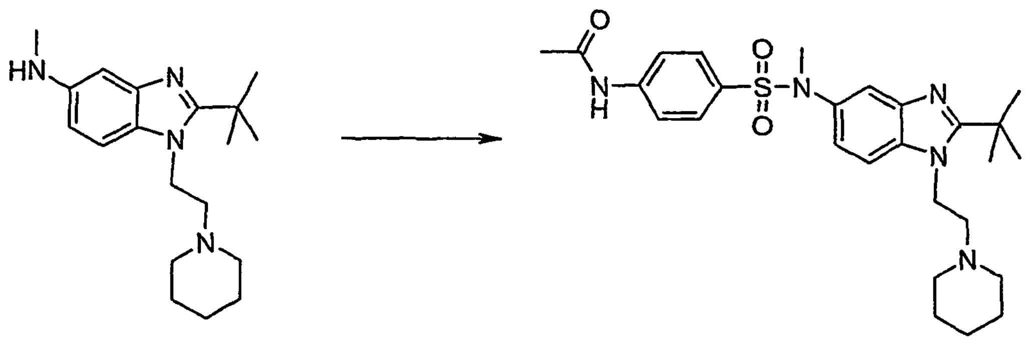

Same procedure used as in Step A of Example 34 using 2-*tert*-butyl-*N*-methyl-1-(2-piperidin-1-ylethyl)-1*H*-benzimidazol-5-amine (for preparation see following Steps B, C, D and E) (22 mg, 0.070 mmol) and 4-acetamidophenyl

sulphonyl chloride (20 mg, 0.084 mmol) in 5 mL of DCM. The solvent was evaporated and the product was purified by reversed-phase HPLC using 10-50% $CH_3CN/H_2O$ and lyophilized affording the title compound as the corresponding TFA salt. Yield: 30 mg (68%); $^1H$ NMR (400 MHz, METHANOL-$D_4$): δ 1.64 (s, 9 H), 1.94 (m, 6 H), 2.15 (s, 3 H), 3.18 (m, 2 H), 3.25 (s, 3 H), 3.57 (m, 2 H), 4.98 (m, 2 H), 7.33 (dd, J=8.88, 2.05 Hz, 1 H), 7.44 (d, J=1.95 Hz, 1 H), 7.46 (d, J=8.98 Hz, 2 H), 7.72 (m, 3 H); MS (ESI) $(M+H)^+$: 512.3; Anal. Calcd for $C_{27}H_{37}N_5O_3S$ + 3.0 TFA + 0.8 $H_2O$: C, 45.66; H, 4.83; N, 8.07. Found: C, 45.67; H, 4.81; N, 8.02.

**Step B. Methyl {3-nitro-4-[(2-piperidin-1-ylethyl)amino]phenyl}carbamate**

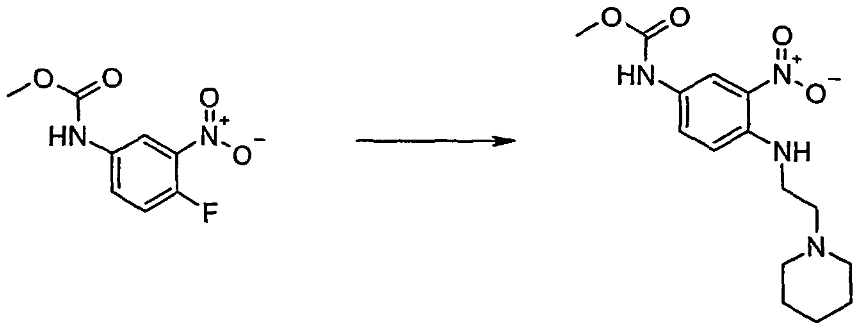

Same procedure used as in Step B of Example 34 using methyl (4-fluoro-3-nitrophenyl)carbamate (75 mg, 0.350 mmol), TEA (0.075 mL, 0.525 mmol) and 1-aminoethylpiperidine (0.060 mL, 0.420 mmol). The crude product was purified by silica gel flash chromatography using EtOAc as eluent. Yield: 81 mg (72%); $^1H$ NMR (400 MHz, CHLOROFORM-D): δ 1.46 (m, 2 H), 1.62 (m, 4 H), 2.45 (m, 4 H), 2.66 (t, J=6.35 Hz, 2 H), 3.36 (m, 2 H), 3.78 (s, 3 H), 6.46 (s, 1 H), 6.83 (d, J=9.37 Hz, 1 H), 7.64 (s, 1 H), 8.05 (d, J=2.73 Hz, 1 H), 8.41 (m, 1 H).

**Step C. Methyl {3-amino-4-[(2-piperidin-1-ylethyl)amino]phenyl}carbamate**

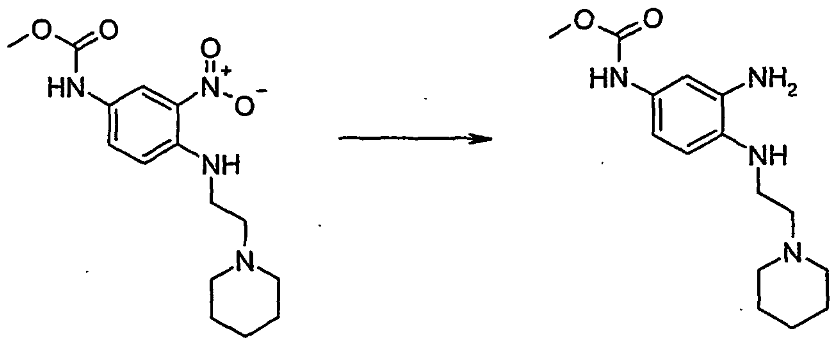

Same procedure used as in Step C of Example 34 using methyl {3-nitro-4-[(2-piperidin-1-ylethyl)amino]phenyl} carbamate (78 mg, 0.242 mmol) and a catalytic amount of 10% Pd/C in 15 mL of EtOAc. Yield: 56 mg (79%). MS (ESI) $(M+H)^+$: 293.22.

**Step D. Methyl [2-*tert*-butyl-1-(2-piperidin-1-ylethyl)-1*H*-benzimidazol-5-yl]carbamate**

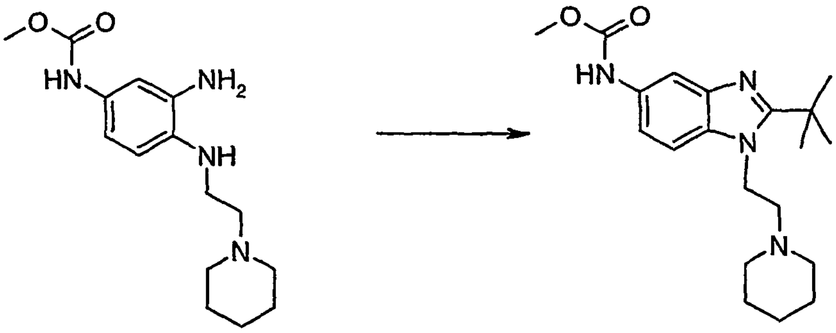

Methyl {3-amino-4-[(2-piperidin-1-ylethyl)amino]phenyl}carbamate (55 mg, 0.188 mmol) and trimethylacetyl chloride (0.025 mL, 0.207 mmol) were stirred in 5 mL of DCM containing a catalytic amount of DMAP at rt for 1h. The solution was washed with saturated aqueous $NaHCO_3$ solution, brine and dried over anhydrous $MgSO_4$. The solvent was evaporated. The residue was dissolved in 2 mL of AcOH and stirred at 150°C in a Personal Chemistry microwaves instrument for 40 min. The solvent was evaporated. The residue was dissolved in EtOAc and washed with saturated aqueous $NaHCO_3$ solution, brine and dried over anhydrous $MgSO_4$. The product was purified by reversed-phase HPLC using 10-50% $CH_3CN/H_2O$. The fractions were concentrated. The residue was dissolved in EtOAc and washed with aqueous 2M NaOH solution, brine and dried over anhydrous $MgSO_4$. Yield: 27 mg (40%); $^1H$ NMR (400 MHz, CHLOROFORM-D): δ 1.49 (m, 2 H), 1.55 (m, 9 H), 1.65 (m, 6 H), 2.55 (m, 2 H), 2.73 (m, 2 H), 3.78 (s, 3 H), 4.45 (m, 2 H), 4.45 (m, 2 H), 6.62 (m, 1 H), 7.26 (m, 1 H), 7.40 (m, 1 H), 7.61 (d, J=1.95 Hz, 1 H).

**Step E. 2-*tert*-Butyl-*N*-methyl-1-(2-piperidin-1-ylethyl)-1*H*-benzimidazol-5-amine**

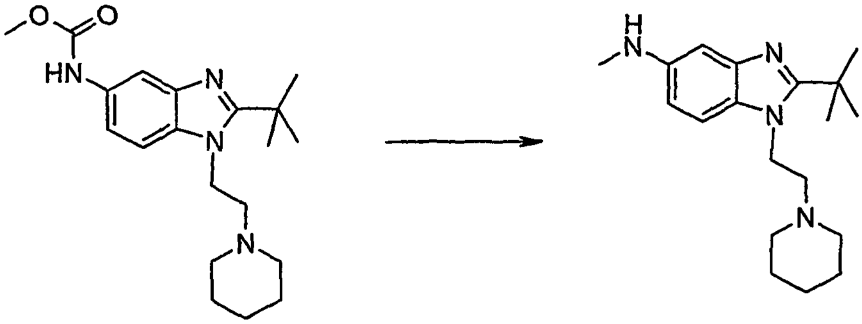

Methyl [2-*tert*-butyl-1-(2-piperidin-1-ylethyl)-1*H*-benzimidazol-5-yl]carbamate (27 mg, 0.0753 mmol) was dissolved in 5 mL of THF at 0°C. 1M HCl/ether (0.115 mL, 0.113 mmol) was added and the solution was stirred at 0°C for 15 min. $LiA1H_4$ (15 mg, 0.377 mmol) was added and the solution was stirred at rt for 24h. The reaction was quenched at 0°C by the addition of MeOH (0.5 mL) and water (0.5 mL). Solid $Na_2SO_4$ (1g) was added and the solution was stirred at rt for 1h. The solution was filtered and rinsed with THE. The solvent was evaporated. Yield: 22 mg (93%); MS (ESI) $(M+H)^+$: 315.03.

**Example 36**

***N*-(4-{[[2-*tert*-Butyl-1-(1,4-dioxan-2-ylmethyl)-1*H*-benzimidazol-5-yl](methyl)amino]sulfonyl}phenyl)acetamide**

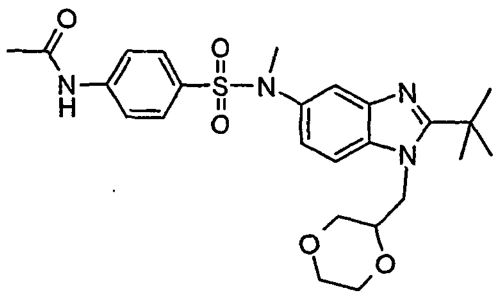

**Step A. *N*-(4-{[[2-*tert*-Butyl-1-(1,4-dioxan-2-ylmethyl)-1*H*-benzimidazol-5-yl](methyl)amino]sulfonyl}phenyl) acetamide**

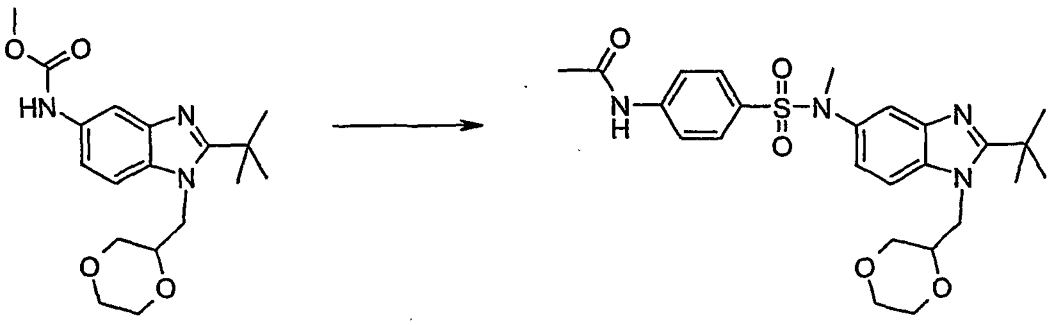

Methyl [2-*tert*-butyl-1-(1,4-dioxan-2-ylmethyl)-1*H*-benzimidazol-5-yl]carbamate (for preparation see following Steps B, C and D) (45 mg, 0.130 mmol) was dissolved in 5 mL of THF at 0°C. 1M HCl/ether (0.195 mL, 0.195 mmol) was added and the solution was stirred at 0°C for 15min. $LiA1H_4$ (25 mg, 0.650 mmol) was added and the solution was stirred at rt for 24h. The reaction was quenched at 0°C by the addition of MeOH (0.5 mL) and water (0.5 mL). Solid $Na_2SO_4$ (1g) was added and the solution was stirred at rt for 1h. The solution was filtered and rinsed with THF. The solvent was evaporated. The residue was dissolved in 3 mL of 1:1 / DCM:DMF solution containing a catalytic amount of DMAP. 4-Acetamidophenylsulfonyl chloride (35 mg, 0.156 mmol) was added and the solution was stirred at rt for 2h. The solvent was evaporated. The residue was dissolved in EtOAc and washed with saturated aqueous $NaHCO_3$ solution, brine and dried over anhydrous $MgSO_4$. The product was purified by reversed-phase HPLC using 10-50% $CH_3CN/H_2O$ affording the title compound as its corresponding TFA salt. Yield: 26 mg (33%); $^1H$ NMR (400 MHz, METHANOL-$D_4$): δ 1.65 (s, 9 H), 2.13 (s, 3 H), 3.25 (s, 3 H), 3.50 (m, 2 H), 3.61 (dt, J=11.28, 2.44 Hz, 1 H), 3.67 (m, 1 H), 3.70 (m, 1 H), 4.00 (dd, J=11.52, 2.54 Hz, 1 H), 4.10 (m, 1 H), 4.63 (m, 2 H), 5.47 (s, 2 H), 7.28 (dd, J=9.08, 2.05 Hz, 1 H), 7.50 (d, J=1.76 Hz, 1 H), 7.70 (d, J=8.98 Hz, 2 H), 7.86 (d, J=8.79 Hz, 1 H); MS (ESI) $(M+H)^+$: 501.0; Anal. Calcd for $C_{25}H_{32}N_4O_5S$ + 1.5 TFA + 0.9 $H_2O$: C, 48.89; H, 5.17; N, 8.14. Found: C, 48.82; H, 5.12; N, 8.16.

**Step B. Methyl {4-[(1,4-dioxan-2-ylmethyl)amino]-3-nitrophenyl}carbamate**

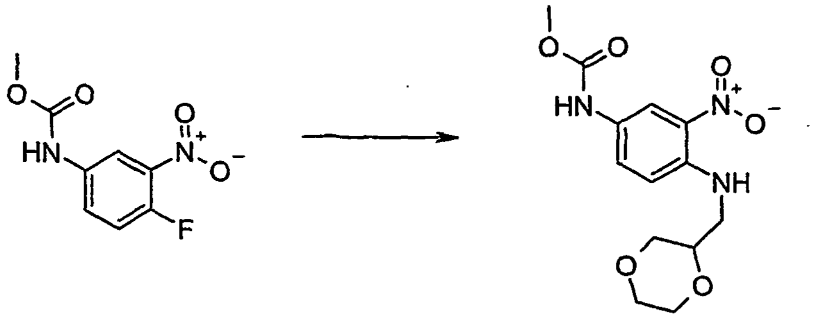

Same procedure used as in Step B of Example 34 using methyl (4-fluoro-3-nitrophenyl)carbamate (125 mg, 0.583 mmol), TEA (0.120 mL, 0.875 mmol) and C-[1,4]dioxane-2-yl-methylamine (82 mg, 0.700 mmol). The crude product was purified by silica gel flash chromatography using 50 to 75% EtOAc/hexanes as eluent. Yield: 94 mg (52%); $^1$H NMR (400 MHz, CHLOROFORM-D): δ 3.31 (m, 2 H), 3.46 (dd, J=11.42, 9.86 Hz, 1 H), 3.64 (dd, J=3.03, 0.88 Hz, 1 H), 3.66 (d, J=3.12 Hz, 1 H), 3.73 (m, 1 H), 3.76 (m, 4 H), 3.81 (dd, J=4.20, 2.64 Hz, 1 H), 3.84 (m, 1 H), 3.87 (m, 1 H), 6.46 (m, 1 H), 6.81 (d, J=9.18 Hz, 1 H), 7.63 (m, 1 H), 8.06 (d, J=2.54 Hz, 1 H).

**Step C. Methyl {3-amino-4-[(1,4-dioxan-2-ylmethyl)amino]phenyl}carbamate**

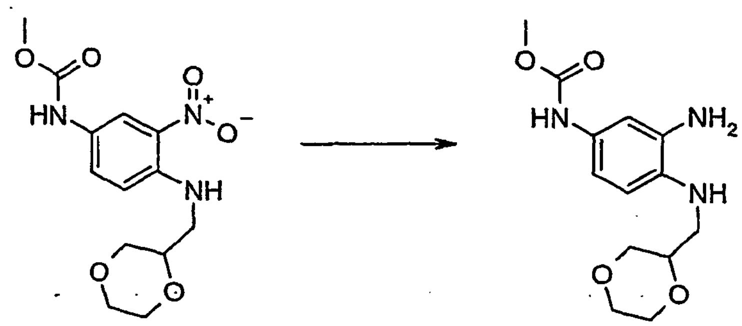

Same procedure used as in Step C of Example 34 using methyl {4-[(1,4-dioxan-2-ylmethyl)amino]-3-nitrophenyl} carbamate (90 mg, 0.289 mmol) and a catalytic amount of 10% Pd/C in 15 mL of EtOAc. Yield: 81 mg (99%); MS (ESI) $(M+H)^+$: 281.88.

**Step D. Methyl [2-*tert*-butyl-1-(1,4-dioxan-2-ylmethyl)-1*H*-benzimidazol-5-yl]carbamate**

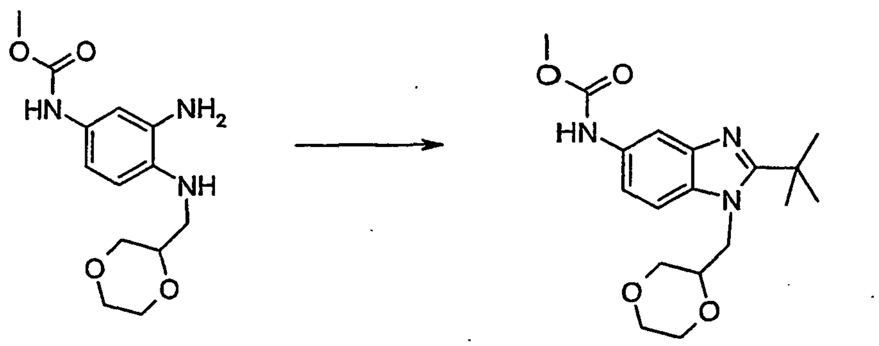

Same procedure as in Step D of Example 35 using methyl {3-amino-4-[(1,4-dioxan-2-ylmethyl)amino]phenyl} carbamate (81 mg, 0.288 mmol) and trimethylacetyl chloride (0.039 mL, 0.317 mmol). The crude product was purified by silica gel flash chromatography using EtOAc as eluent. Yield: 45 mg (45%).

**Example 37**

***N*-(4-{[{2-*tert*-Butyl-1-[(1-methylpiperidin-2-yl)methyl]-1*H*-benzimidazol-5-yl}(methyl)amino]sulfonyl}phenyl) acetamide**

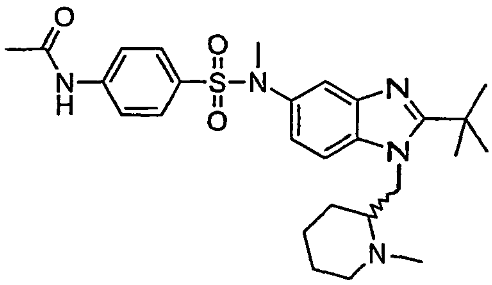

**Step A. *N*-(4-{[{2-*tert*-Butyl-1-[(1-methylpiperidin-2-yl)methyl]-1*H*-benzimidazol-5-yl}(methyl)amino]sulfonyl} phenyl)acetamide**

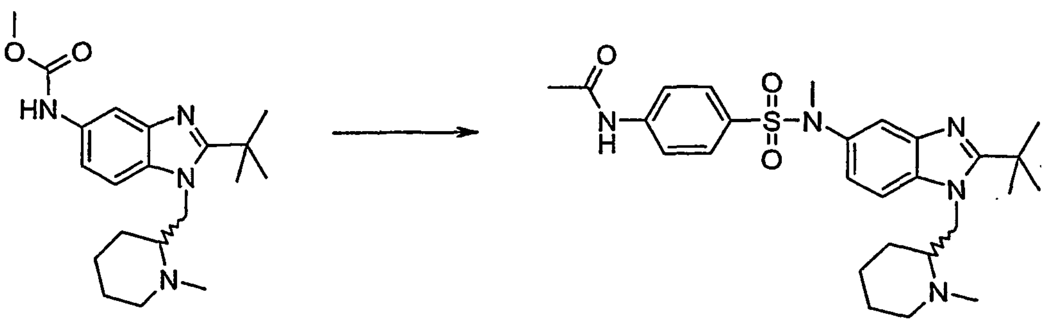

Same procedure as in Step A of Example 36 using methyl {2-*tert*-butyl-1-[(1-methylpiperidin-2-yl)methyl]-1*H*-benzimidazol-5-yl}carbamate (for preparation see following Steps B, C, D, and E) (38 mg, 0.106 mmol), 1M HCl/ ether (0.150 mL, 0.159 mmol), $LiA1H_4$ (20 mg, 0.530 mmol) in 5 mL of THF and 4-acetamidophenylsulfonyl chloride (30 mg, 0.127 mmol) in 5 mL of DCM. The product was purified by reversed-phase HPLC using 10-50% $CH_3CN/H_2O$ affording the title compound as its corresponding TFA salt. Yield: 43 mg (65%); $^1$H NMR (400 MHz, METHANOL-$D_4$): δ 1.39 (m, 2 H), 1.66 (s, 9 H), 1.84 (m, 2 H), 1.91 (m, 1 H), 2.15 (s, 3 H), 3.16 (s, 3 H), 3.25 (s, 3 H), 3.63 (m, 1 H), 4.02 (m, 1 H), 4.88 (m, 1 H), 5.19 (m, 1 H), 7.29 (dd, J=8.98, 2.15 Hz, 1 H), 7.46 (d, J=8.98 Hz, 2 H), 7.49 (d, J=1.76 Hz, 1 H), 7.71 (d, J=8.98 Hz, 2 H), 7.84 (d, J=8.98 Hz, 1 H); MS (ESI) $(M+H)^+$: 512.3; Anal. Calcd for $C_{27}H_{37}N_5O_3S$ + 2.7 TFA + 1.0 $H_2O$: C, 46.46; H, 5.02; N, 8.36. Found: C, 46.46; H, 4.92; N, 8.59.

**Step B. *tert*-Butyl 2-[({4-[(methoxycarbonyl)amino]-2-nitrophenyl}amino)methyl]piperidine-1-carboxylate**

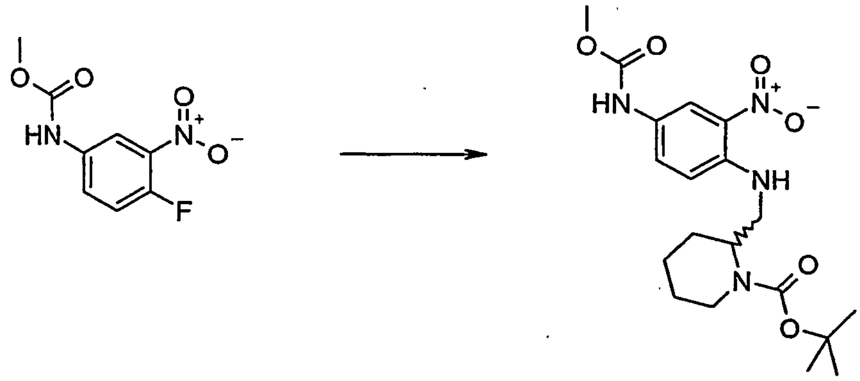

Same procedure used as in Step B of Example 34 using methyl (4-fluoro-3-nitrophenyl)carbamate (100 mg, 0.467 mmol), TEA (0.100 mL, 0.700 mmol) and 2-(aminomethyl)-1-N-Boc-piperidine (120 mg, 0.560 mmol). The crude product was purified by silica gel flash chromatography using 35 to 55% EtOAc/hexanes as eluent. Yield: 121 mg (63%); $^1$H NMR (400 MHz, CHLOROFORM-D) □ ppm 1.46 (s, 9 H), 1.53 (m, 1 H), 1.65 (m, 1 H), 1.69 (m, 2 H), 1.72 (m, 2 H), 2.79 (m, 1 H), 3.33 (m, 1 H), 3.57 (m, 1 H), 3.78 (s, 3 H), 4.07 (m, 1 H), 6.47 (m, 1 H), 6.97 (d, J=9.57 Hz, 1 H), 7.66 (m, 1 H), 8.04 (m, 1 H), 8.07 (d, J=2.54 Hz, 1 H).

**Step C. Methyl (4-{[(1-methylpiperidin-2-yl)methyl]amino}-3-nitrophenyl)carbamate**

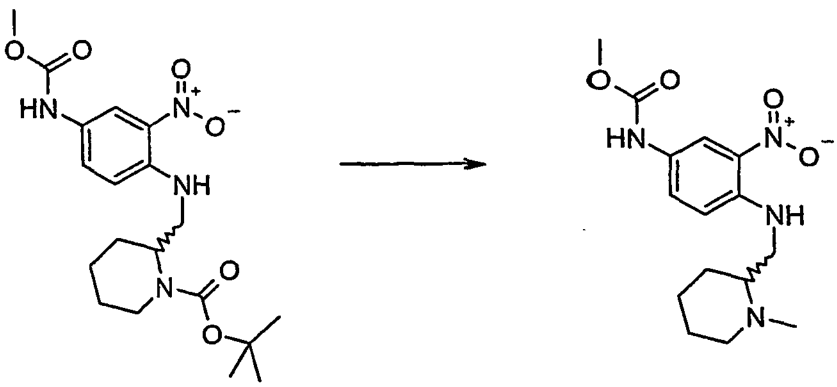

*tert*-Butyl 2-[({4-[(methoxycarbonyl)amino]-2-nitrophenyl} amino)methyl]piperidine-1-carboxylate (118 mg, 0.289 mmol) was stirred in 3 mL of 1M HCl/AcOH at rt for 1h. The solvent was evaporated. The residue was dissolved in 5 mL of MeOH and 37% HCHO/water (1 mL) was added, followed by $NaBH(OAc)_3$ (120 mg, 0.578 mmol). The solution was stirred at rt for 1h. The solvent was evaporated. The residue was dissolved in EtOAc and washed with saturated aqueous $NaHCO_3$ solution, brine and dried over anhydrous $MgSO_4$. The solvent was evaporated. Yield: 87 mg (93%); $^1$H NMR (400 MHz, CHLOROFORM-D): δ 1.29 (m, 2 H), 1.61 (m, 5 H), 1.67 (m, 1 H), 1.78 (m, 1 H), 2.15 (m, 1 H), 2.22 (m, 1 H), 2.29 (s, 3 H), 2.93 (m, 1 H), 3.26 (m, 1 H), 3.43 (m, 1 H), 6,46 (m, 1 H), 6.79 (d, J=9.37 Hz, 1 H), 7.64 (m, 1 H), 8.05 (d, J=2.54 Hz, 1 H), 8.34 (m, 1 H).

**Step D. Methyl (3-amino-4-{[(1-methylpiperidin-2-yl)methyl]amino}phenyl)carbamate**

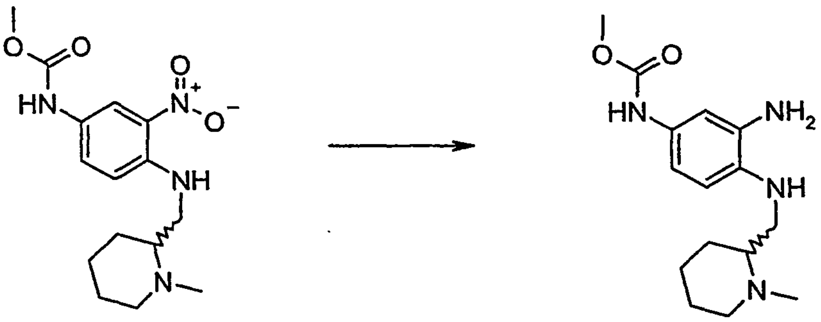

Same procedure used as in Step C of Example 34 using methyl (4-{[(1-methylpiperidin-2-yl)methyl]amino}-3-nitrophenyl)carbamate (83 mg, 0.257 mmol) and a catalytic amount of 10% Pd/C in 20 mL of EtOAc. Yield: 75 mg (99%); MS (ESI) $(M+H)^+$: 293.26.

**Step E. Methyl {2-*tert*-butyl-1-[(1-methylpiperidin-2-yl)methyl]-1*H*-benzimidazol-5-yl}carbamate**

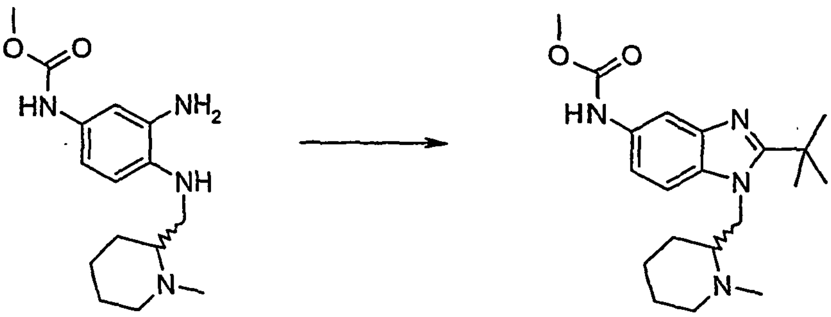

Same procedure as in Step D of Example 35 using methyl (3-amino-4-{[(1-methylpiperidin-2-yl)methyl]amino} phenyl)carbamate (72 mg, 0.246 mmol) and trimethylacetyl chloride (0.033 mL, 0.271 mmol). The product was purified by reversed-phase HPLC using 10-50% $CH_3CN/H_2O$. The fractions were concentrated. The residue was dissolved in EtOAc and washed with aqueous 2M NaOH solution, brine and dried over anhydrous $MgSO_4$. Yield: 38 mg (43%); $^1H$ NMR (400 MHz, CHLOROFORM-D): δ 1.07 (m, 2 H), 1.25 (m, 1 H), 1.56 (m, 9 H), 1.59 (m, 1 H), 1.63 (m, 1 H), 1.75 (m, 1 H), 2.20 (m, 1 H), 2.47 (s, 3 H), 2.68 (m, 1 H), 2.93 (d, J=11.52 Hz, 1 H), 3.78 (s, 3 H), 4.24 (dd, J=14.25, 10.15 Hz, 1 H), 4.66 (dd, J=14.35, 5.17 Hz, 1 H), 6.66 (m, 1 H), 7.31 (d, J=8.59 Hz, 1 H), 7.39 (m, 1 H), 7.60 (s, 1 H).

**Example 38**

***N*-(4-{[(2-*tert*-Butyl-1-{[(2*R*)-1-methylpiperidin-2-yl]methyl}-1*H*-benzimidazol-5-yl)(methyl)amino]sulfonyl}phenyl)acetamide**

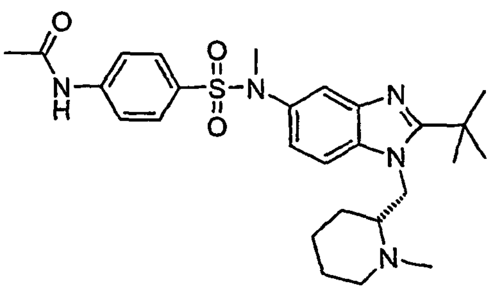

**Step A. *N*-(4-{[(2-*tert*-Butyl-1-{[(2*R*)-1-methylpiperidin-2-yl]methyl}-1*H*-benzimidazol-5-yl)(methyl)amino]sulfonyl}phenyl)acetamide**

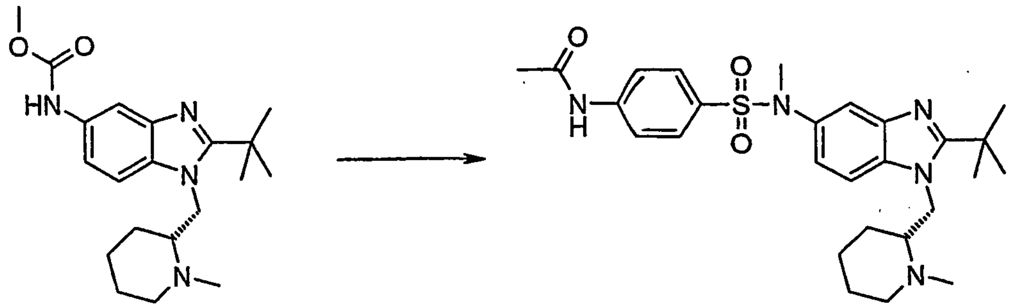

Same procedure as in Step A of Example 36 using methyl (2-*tert*-butyl-1-{[(2*R*)-1-methylpiperidin-2-yl]methyl}-1*H*-benzimidazol-5-yl)carbamate (for preparation see following Steps B, C, D, and E) (51 mg, 0.142 mmol), 1M HCl/ether (0.215 mL, 0.213 mmol), $LiA1H_4$ (27 mg, 0.710 mmol) in 5 mL of THF and 4-acetamidophenylsulfonyl chloride (40 mg, 0.170 mmol) in 5 mL of DCM. The product was purified by reversed-phase HPLC using 10-50% $CH_3CN/H_2O$ affording the title compound as its corresponding TFA salt. Yield: 59 mg (66%); $^1$H NMR (400 MHz, METHANOL-$D_4$): δ 1.41 (m, 2 H), 1.65 (s, 9 H), 1.82 (m, 2 H), 1.89 (m, 2 H), 2.13 (s, 3 H), 3.15 (s, 3 H), 3.24 (s, 3 H), 3.62 (m, 1 H), 4.00 (m, 1 H), 4.86 (m, 1 H), 5.17 (m, 1 H), 7.29 (dd, J=8.98, 1.95 Hz, 1 H), 7.45 (d, J=8.79 Hz, 2 H), 7.49 (d, J=1.95 Hz, 1 H), 7.70 (d, J=8.79 Hz, 2 H), 7.83 (d, J=8.98 Hz, 1 H); MS (ESI) $(M+H)^+$: 512.3; Anal. Calcd for $C_{27}H_{37}N_5O_3S$ + 2.9 TFA + 1.2 $H_2O$: C, 45.60; H, 4.93; N, 8.11. Found: C, 45.64; H, 4.95; N, 8.05.

**Step B. *tert*-Butyl (2*R*)-2-[({4-[(methoxycarbonyl)amino]-2-nitrophenyl}amino)methyl]piperidine-1-carboxylate**

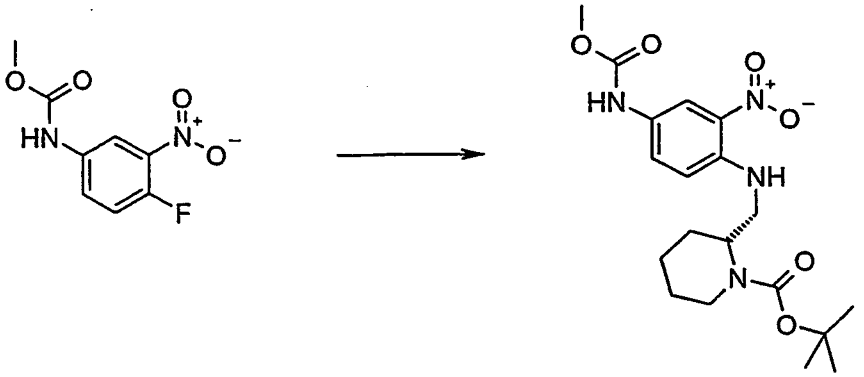

Same procedure used as in Step B of Example 34 using methyl (4-fluoro-3-nitrophenyl)carbamate (300 mg, 1.40 mmol), TEA (0.300 mL, 2.10 mmol) and 2-R-(aminomethyl)-1-N-Boc-piperidine (360 mg, 1.68 mmol). The crude product was purified by silica gel flash chromatography using 30 to 50% EtOAc/hexanes as eluent. Yield: 285 mg (50%); $^1$H NMR (400 MHz, CHLOROFORM-D): δ 1.46 (s, 9 H), 1.53 (m, 1 H), 1.65 (m, 1 H), 1.69 (m, 2 H), 1.72 (m, 2 H), 2.79 (m, 1 H), 3.33 (m, 1 H), 3.57 (m, 1 H), 3.78 (s, 3 H), 4.07 (m, 1 H), 6.47 (m, 1 H), 6.97 (d, J=9.57 Hz, 1 H), 7.66 (m, 1 H), 8.04 (m, 1 H), 8.07 (d, J=2.54 Hz, 1 H).

**Step C. Methyl [4-({[(2*R*)-1-methylpiperidin-2-yl]methyl}amino)-3-nitrophenyl]carbamate**

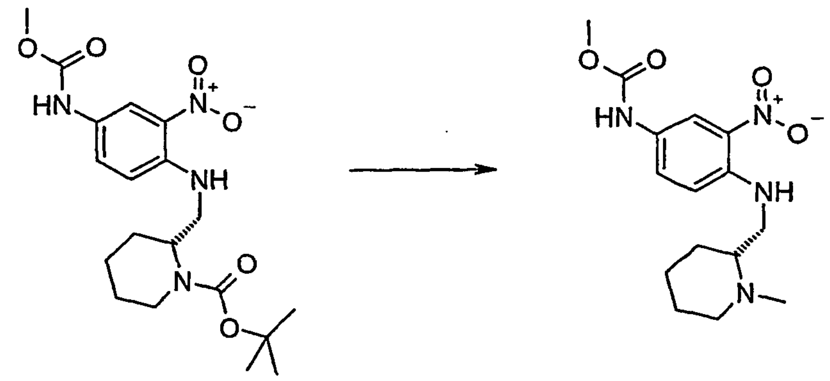

Same procedure used as in Step C of Example 37 using *tert*-butyl (2*R*)-2-[({4-[(methoxycarbonyl)amino]-2-nitrophenyl}amino)methyl]piperidine-1-carboxylate (280 mg, 0.686 mmol), 1M HCl/AcOH (3 mL), 37% HCHO/water (1 mL) and $NaBH(OAc)_3$ (290 mg, 1.37 mmol) in 5 mL of THF. Yield: 187 mg (85%); MS (ESI) $(M+H)^+$: 323.27.

**Step D. Methyl [3-aminl-4-({[(2*R*)-1-methylpiperidin-2-yl]methyl}amino)phenyl]carbamate**

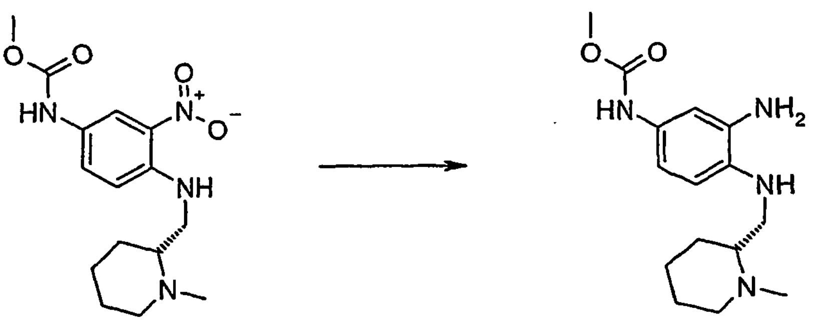

Same procedure used as in Step C of Example 34 using methyl [4-({[(2*R*)-1-methylpiperidin-2-yl]methyl}amino)-3-nitrophenyl]carbamate (187 mg, 0.580 mmol) and a catalytic amount of 10% Pd/C in 25 mL of EtOAc. Yield: 164 mg (97%); MS (ESI) $(M+H)^+$: 293.24.

**Step E. Methyl (2-*tert*-butyl-1-{[(2*R*)-1-methylpiperidin-2-yl]methyl}-1*H*-benzimidazol-5-yl)carbamate**

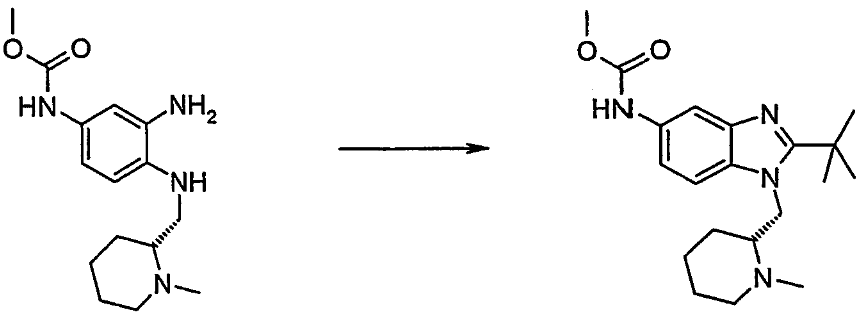

Same procedure as in Step D of Example 35 using methyl [3-amino-4-({[(2*R*)-1-methylpiperidin-2-yl]methyl} amino)phenyl]carbamate (160 mg, 0.547 mmol) and trimethylacetyl chloride (0.075 mL, 0.602 mmol). The product was purified by reversed-phase HPLC using 10-50% $CH_3CN/H_2O$. The fractions were concentrated. The residue was dissolved in EtOAc and washed with aqueous 2M NaOH solution, brine and dried over anhydrous $MgSO_4$. Yield: 55 mg

(28%); $^1H$ NMR (400 MHz, CHLOROFORM-D): δ 1.08 (m, 2 H), 1.29 (m, 1 H), 1.57 (s, 9 H), 1.61 (m, 3 H), 2.22 (m, 1 H), 2.49 (s, 3 H), 2.72 (m, 1 H), 2.96 (m, 1 H), 3.78 (s, 3 H), 4.26 (dd, J=14.35,4.98 Hz, 1 H), 6.62 (s, 1 H), 7.32 (d, J=8.59 Hz, 1 H), 7.39 (m, 1 H), 7.61 (s, 1 H).

**Example 39**

***N*-[4-({methyl[1-(tetrahydro-2*H*-pyran-4-ylmethyl)-2-(trifluoromethyl)-1*H*-benzimidazol-5-yl]amino}sulfonyl) phenyl]acetamide**

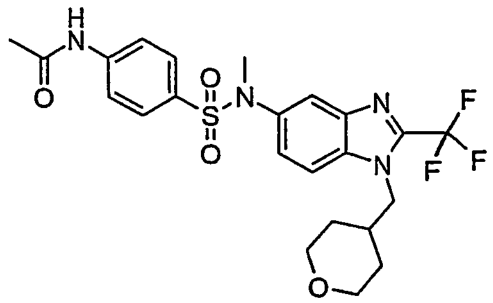

**Step A. *N*-[4-({methyl[1-(tetrahydro-2*H*-pyran-4-ylmethyl)-2-(trifluoromethyl)-1*H*-benzimidazol-5-yl]amino}sulfonyl)phenyl]acetamide**

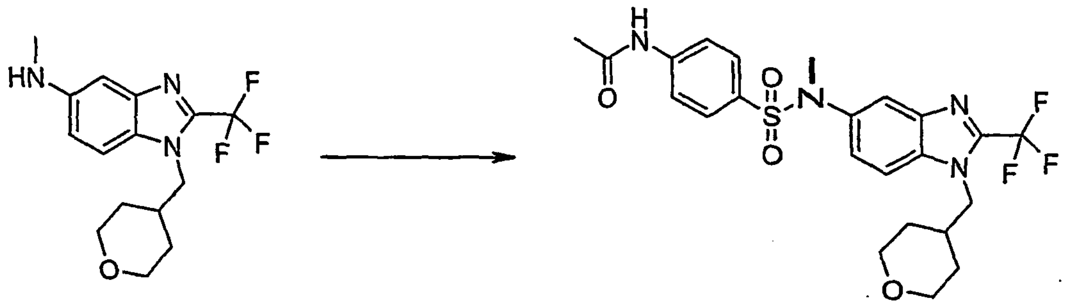

*N*-methyl-1-(tetrahydro-2*H*-pyran-4-ylmethyl)-2-(trifluoromethyl)-1*H*-benzimidazol-5-amine hydrochloride (76.1 mg, 0.2 mmol) (for preparation, see the following steps B, C, D, E and F), DMAP (97.7 mg, 0.8 mmol) and 4-(acetylamino)benzenesulfonyl chloride (93.5 mg, 0.4 mmol) in MeCN (5 mL) were stirred overnight at room temperature. The reaction mixture was quenched with $H_2O$ (6 mL). Upon evaporation, the crude product was purified by reversed-phase HPLC using 20-70% $CH_3CN/H_2O$ and then lyophilized affording the title compound as the corresponding TFA salt. Yield: 49.1 mg (48%); $^1H$NMR (400 MHz, $CD_3OD$): 1.39 -1.56 (m, 4 H), 2.14 (s, 3 H), 2.19 - 2.32 (m, 1 H), 3.24 (s, 3 H), 3.31 - 3.39 (m, 2 H), 3.85 - 4.01 (m, 2 H), 4.32 (d, J=7.42 Hz, 2 H), 7.32 (dd, J=8.88, 2.05 Hz, 1 H), 7.40 (d, J=1.95 Hz, 1 H), 7.43 - 7.49 (m, 2 H), 7.67 - 7.75 (m, 3 H); MS (ESI) $(M+H)^+$: 511.0; Anal. Calcd for $C_{23}H_{25}F_3N_4O_4S$+ 0.4 TFA+0.2 $H_2O$ (559.75): C, 51.07, H, 4.65, N, 10.01; Found: C, 51.16; H, 4.74; N, 9.65.

**Step B. *N*-(4-fluoro-3-nitrophenyl)-*N*-methylacetamide**

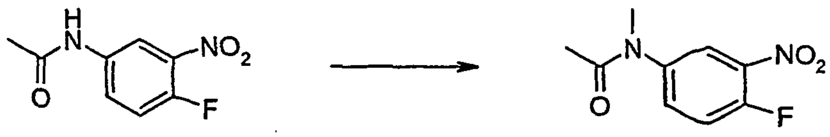

Sodium hydride (2.40 g, 60 mmol) was added in portions to a solution of *N*-(4-fluoro-3-nitrophenyl)acetamide (7.93 g, 40 mmol) (for preparation see Example 33, Step B) in THF (120 mL) at 0 °C. Stirring for 20 min, iodomethane (17.0 g, 120 mmol) was added. The reaction mixture was stirred at room temperature for 2 h, quenched with saturaed $NaHCO_3$ (30 mL) and extracted with EtOAc (3x100 mL). The combined organic phases were washed with saturated NaCl (2x30 mL). After filtration and concentration, 8.73 g (100%) of the title compound was obtained as a brown solid. $^1H$ NMR (400 MHz, $CDCl_3$): δ 1.92 (s, 3 H), 3.30 (s, 3 H), 7.38 (s, 1 H), 7.52 (s, 1 H), 7.95 (s, 1 H).

**Step C. *N*-methyl-*N*-{3-nitro-4-[(tetrahydro-2*H*-pyran-4-ylmethyl)amino]phenyl}acetamide**

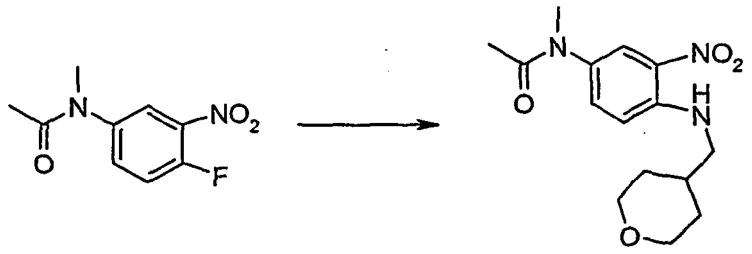

4-Aminomethylpyran (2.50 g, 21.7 mmol ) was added to a mixture of *N*-(4-fluoro-3-nitrophenyl)-*N*-methylacetamide (4.61 g, 21.27 mmol) and sodium carbonate (5.10 g, 47.7 mmol) in EtOH (120 mL) at room temperature. The reaction mixture was heated for 3 days at 60 °C. Upon evaporation of ethanol, the residue was dissolved in EtOAc (400 mL), washed with $H_2O$ (3x50 mL), saturated NaCl (3x50 mL), and dried over $Na_2SO_4$. After filtation and concentration, 6.62 g (100%) of the title compound was obtained as an orange-red solid. $^1H$ NMR (400 MHz, $CDCl_3$): δ 1.38 - 1.52 (m, 2 H), 1.72 - 1.81 (m, 2 H), 1.90 (s, 3 H), 1.93 - 2.02 (m, 1 H), 3.23 (s, 3 H), 3.23 - 3.27 (m, 2 H), 3.36 - 3.49 (m, 2 H), 4.01 - 4.07 (m, 2 H), 6.91 (d, *J*=9.18 Hz, 1 H), 7.29 (dd, *J*=9.08, 2.64 Hz, 1 H), 8.05 (d, J=2.34 Hz, 1 H), 8.22 (t, *J*=5.37 Hz, 1 H); MS (ESI) $(M+H)^+$: 309.12.

**Step D. *N*-{3-amino-4-[(tetrahydro-2*H*-pyran-4-ylmethyl)amino]phenyl}-*N*-methylacetamide**

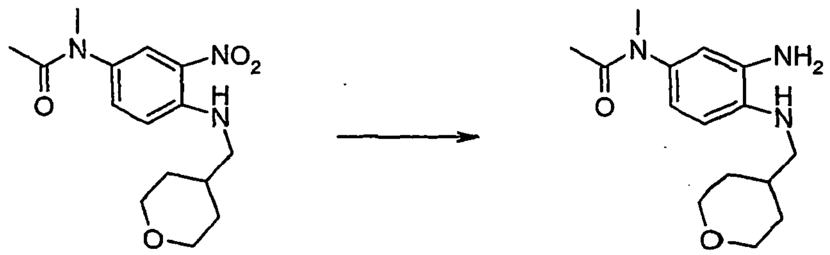

*N*-methyl-*N*-{3-nitro-4-[(tetrahydro-2*H*-pyran-4-ylmethyl)amino]phenyl}acetamide (5.39 g, 16.7 mmol) was hydrogenated in ethyl acetate (200 mL) catalyzed by 10% Pd/C (0.2 g) at 30-40 psi $H_2$ in Parr shaker for 18 h at room temperature. After filtration through celite and concentration, 6.0 g (100%) of a purple solid was obtained as HCl salt, which was used in the next step without further purification. $^1H$ NMR (400 MHz, $CD_3OD$): δ 1.32 - 1.46 (m, 2 H), 1.78 - 1.84 (m, 2 H), 1.85 (s, 3 H), 1.91 - 2.06 (m, 1 H), 3.16 (d, *J*=6.83 Hz, 2 H), 3.20 (s, 3 H), 3.39 - 3.51 (m, 2 H), 3.94 - 4.03 (m, 2 H), 7.01 (d, *J*=8.59 Hz, 1 H), 7.12 (d, *J*=2.15 Hz, 1 H), 7.17 (dd, *J*=8.49, 4.39 Hz, 1 H); MS (ESI) $(M+H)^+$: 278.7.

**Step E. *N*-methyl-*N*-[1-(tetrahydro-2*H*-pyran-4-ylmethyl)-2-(trifluoromethyl)-1*H*-benzimidazol-5-yl]acetamide**

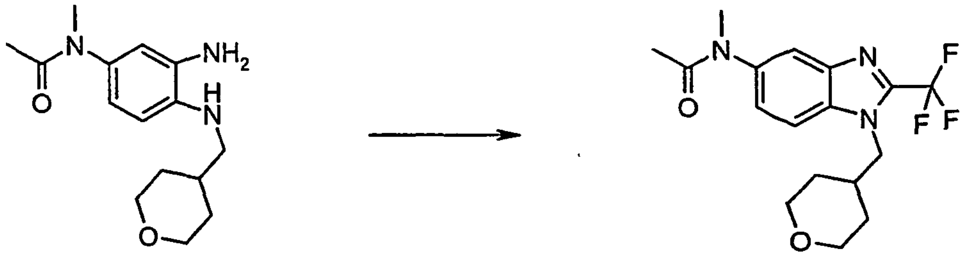

A solution of *N*-{3-amino-4-[(tetrahydro-2*H*-pyran-4-ylmethyl)amino]phenyl}-*N*-methylacetamide hydrochoride (395.1 mg, 1.42 mmol) in trifluoroacetic acid (10 mL) was heated to reflux for 20 h. After evaporation of the solvent, the crude product was used directly for next step without further purification. MS (ESI) $(M+H)^+$: 356.02.

**Step F. *N*-methyl-1-(tetrahydro-2*H*-pyran-4-ylmethyl)-2-(trifluoromethyl)-1*H*-benzimidazol-5-amine**

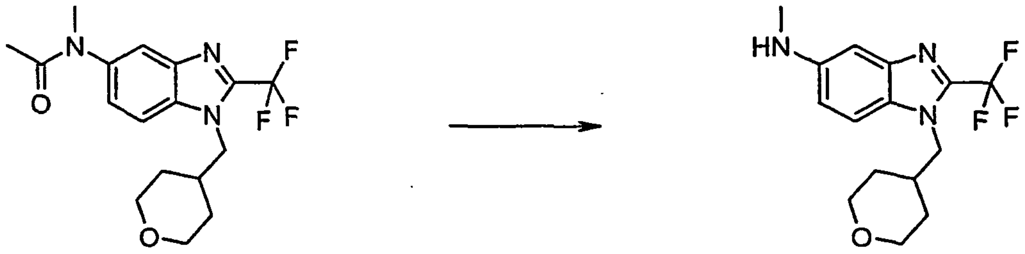

The crude *N*-methyl-*N*-[1-(tetrahydro-2*H*-pyran-4-ylmethyl)-2-(trifluoromethyl)-1*H*-benzimidazol-5-yl]acetamide (~500 mg, 1.42 mmol) was dissolved in 10 mL of EtOH-2*N* HCl (3:2), and then heated at 120°C in a Personal Chemistry SmithSynthesizer microwave instrument for 4 h. After concentration and dried *in vacuo*, 539 mg (100%) of a grey white solid was obtained as the title product, which was used directly for Step A. MS (ESI) $(M+H)^+$: 314.20.

**Example 40**

**4-Bromo-*N*-[1-(cyclohexylmethyl)-2-(1,1-dimethylethyl)-1*H*-benzimidazol-5-yl]-*N*-methyl-benzenesulfonamide**

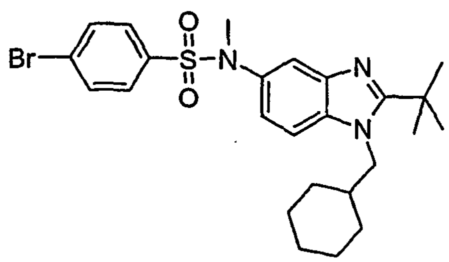

**Step A. 4-Bromo-*N*-[1-(cyclohexylmethyl)-2-(1,1-dimethylethyl)-1*H*-benzimidazol-5-yl]-*N*-methyl-benzenesulfonamide**

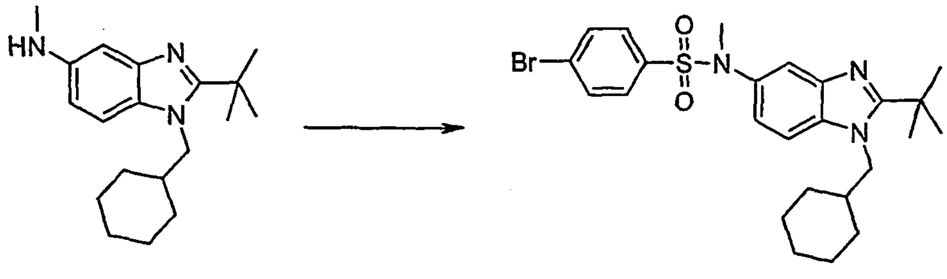

N-1-(Cyclohexylmethyl)-2-(1,1-dimethylethyl)-*N*-methyl-1*H*-benzimidazol-5-amine hydrochloride (532.2 mg, 1.39 mmol) (for preparation, see the following steps B, C, D, E and F), DMAP (679.3 mg, 5.56 mmol) and 4-bromobenzenesulfonyl chloride (426.7 mg, 1.67 mmol) in MeCN (50 mL) were stirred overnight at room temperature. The reaction mixture was quenched with saturated $NaHCO_3$ (10 mL), evaporated to small volume and extracted with EtOAc (3x50 mL). The combined organic phases were washed with brine and dried over $Na_2SO_4$. After evaporation of the solvent, the product was purified by MPLC using Hexanes/EtOAc (1:1) on silica gel to give 529.6 mg (74%) of a white solid as the title product. A small amount of the title product was converted to the corresponding TFA salt. $^1$H NMR (400 MHz, $CD_3OD$): δ 1.26 (m, 5 H), 1.64 (m, 2 H), 1.67 (s, 9 H), 1.71 (m, 1 H), 1.78 (m, 2 H), 2.11 (m, 1 H), 3.29 (s, 3 H), 4.45 (d, J=7.62 Hz, 2 H), 7.31(m, 1 H), 7.45 (m, 2 H), 7.53 (d, J=1.56 Hz, 1 H), 7.72 (m, 2 H), 7.85 (d, J=8.98 Hz, 1 H); MS (ESI) $(M+H)^+$: 518.2; Anal. Calcd for $C_{25}H_{32}BrN_3O_2S$+1.00 TFA+1.40 $H_2O$ (639.75): C, 50.69; H, 5.33; N, 6.57; Found: C, 50.75; H, 5.40; N, 6.47.

**Step B. *N*-{4-[(cyclohexylmethyl)amino]-3-nitrophenyl}acetamide**

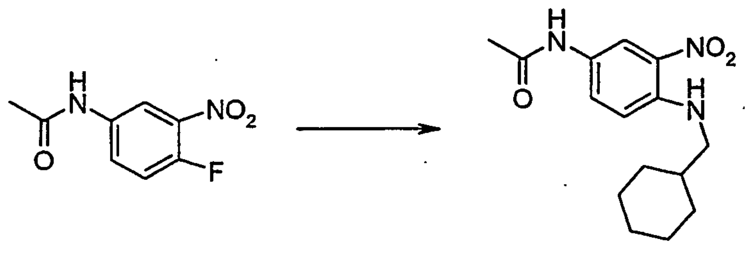

Cyclohexylmethylamine (2.86 mL, 2.49 g, 22.0 mmol ) was added to a mixture of *N*-(4-fluoro-3-nitrophenyl) acetamide (3.96 g, 20.0 mmol) (for preparation, see Example 33, Step B) and sodium carbonate (4.66 g, 44 mmol) in EtOH (50 mL) at room temperature. The reaction mixture was heated for 48 h at 60 °C, and diluted with $H_2O$ (800 mL). The orange solid was precipitated out and collected to give the title product (6.60 g, 100%). MS (ESI) $(M+H)^+$: 292.3.

**Step C. *N*-{3-amino-4-[(cyclohexylmethyl)amino]phenyl}acetamide**

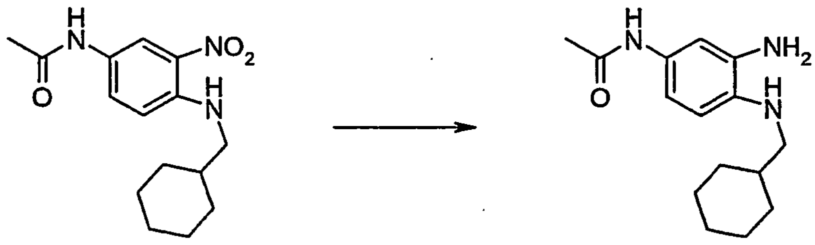

(*N*-{4-[(Cyclohexylmethyl)amino]-3-nitrophenyl}acetamide) (6.60 g, 20 mmol) was hydrogenated in ethyl acetate (300 mL) catalyzed by 10% Pd/C (0.5 g) at 20-30 psi $H_2$ in Parr shaker for 4.5 h at room temperature. After filtration through celite and concentration, 5.08 g (97%) of a purple solid was obtained, which was used in the next step without purification. $^1$H NMR (400 MHz, $CDCl_3$): δ 1.00 (m, 2 H), 1.24 (m, 3 H), 1.59 (m, 2 H), 1.72 (m, 2 H), 1.84 (m, 2 H), 2.13 (s, 3 H), 2.91 (d, *J*=6.64 Hz, 2 H), 3.37 (s broad, 3 H), 6.56 (d, *J*=8.40 Hz, 1 H), 6.69 (dd, *J*=8.30, 2.25 Hz, 1 H), 6.98 (s, 1 H), 7.12 (d, *J*=2.34 Hz, 1 H). MS (ESI) $(M+H)^+$= 262.3.

**Step D. *N*-[1-(cyclohexylmethyl)-2-(1,1-dimethylethyl)-1*H*-benzimidazol-5-yl]acetamide**

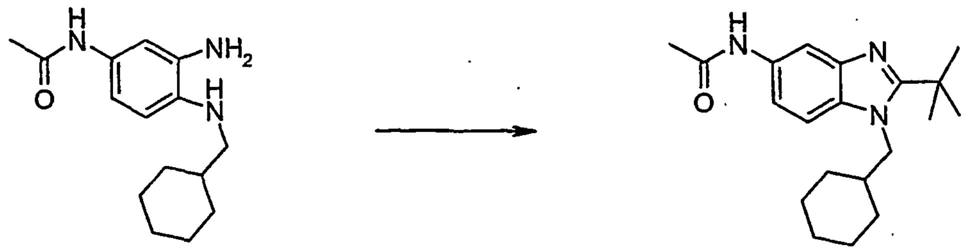

DMAP (0.15 g, 1.2 mmol) was added to a solution of *N*-{3-amino-4-[(cyclohexylmethyl)amino]phenyl}acetamide (1.57 g, 6.0 mmol) in dichloromethane (70 mL) at 0 °C, followed by addition of trimethylacetyl chloride (0.85 mL, 0.83 g, 6.6 mmol). The resulting mixture was stirred overnight at room temperature. After evaporation of the solvent, the residue was dissolved in dichloroethane (40 mL) and then divided to 8 sealed test tubes. The mixture was heated at 170°C in a Personal Chemistry SmithSynthesizer microwave instrument for 2 h. The combined reaction mixture was dissolved in EtOAc (200 mL), washed with 2 *N* NaOH aqueous solution (2x10 mL), brine (2x10 mL) and dried over $Na_2SO_4$. After filtration and evaporation, the residue was purified by MPLC using EtOAc as eluent on silica gel to give the title compound as a white solid (1.42 g, 72%). $^1$H NMR (400 MHz, $CD_3OD$): δ 1.24 (m, 5 H), 1.64 (m, 2 H), 1.67 (s, 9 H), 1.70 (m, 1 H), 1.77 (m, 2 H), 2.12 (m, 1 H), 2.18 (s, 3 H), 4.45 (d, J=7.62 Hz, 2 H), 7.50 (m, 1 H), 7.84 (d, J=8.98 Hz, 1 H), 8.43 (d, J=1.95 Hz, 1 H); MS (ESI) $(M+H)^+$: 328.2.

**Step E. *N*-[1-(cyclohexylmethyl)-2-(1,1-dimethylethyl)-1*H*-benzimidazol-5-yl]-*N*-methyl-acetamide**

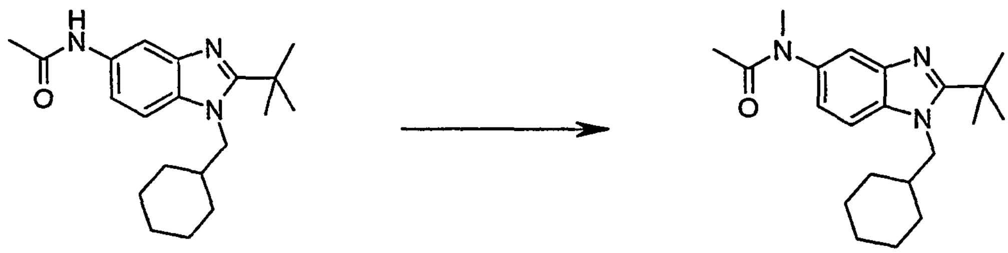

Sodium hydride (60%, 201.5 mg, 5.04 mmol) was added to a solution of *N*-[1-(cyclohexylmethyl)-2-(1,1-dimethylethyl)-1*H*-benzimidazol-5-yl]acetamide (549.8 mg, 1.68 mmol) in THF (50 mL) at 0 °C. Stirring for 1h, iodomethane (0.31 mL, 715.4 mg, 5.04 mmol) was added. The mixture was stirred overnight at room temperature, quenched with saturated $NaHCO_3$ (5 mL), and extracted with EtOAc (3x20 mL). The combined organic phases were washed with saturated $NaHCO_3$ (20 mL), brine (20 mL) and dried over $Na_2SO_4$. After filtration and concentration, the residue was purified by MPLC using EtOAc on silica gel to give 580.5 mg (100%) of the title compound as a white solid. $^1H$ NMR (400 MHz, $CD_3OD$): δ 1.26 (m, 5 H), 1.67 (m, 2 H), 1.69 (s, 9 H), 1.71 (m, 1 H), 1.78 (m, 2 H), 1.87 (s, 3 H), 2.14 (m, 1 H), 3.30 (s, 3 H), 4.49 (d, J=7.62 Hz, 2 H), 7.55 (d, J=8.40 Hz, 1 H), 7.71 (s, 1 H), 8.00 (d, J=8.40 Hz, 1 H). MS (ESI) $(M+H)^+$: 342.2.

**Step F. *N*-1-(Cyclohexylmethyl)-2-(1,1-dimethylethyl)-*N*-methyl-1*H*-benzimidazol-5-amine**

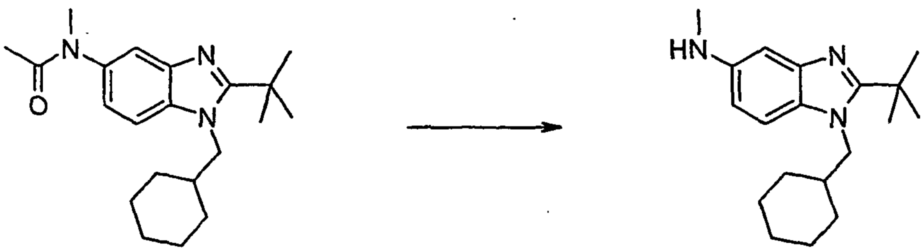

*N*-[1-(cyclohexylmethyl)-2-(1,1-dimethylethyl)-1*H*-benzimidazol-5-yl]acetamide (540.6 mg, 1.58 mmol) was dissolved in 20 mL ofEtOH-2NHCl (3:2), and then heated at 120°C in a Personal Chemistry SmithSynthesizer microwave instrument for 30 min. After concentration and dried *in vacuo*, 603.5 mg (100%) of a white solid was obtained as the title product. $^1H$ NMR (400 MHz, $CD_3OD$): δ 1.26 (m, 5 H), 1.65 (m, 3 H), 1.71 (s, 9 H), 1.78 (m, 2 H), 2.11 (m, 1 H), 3.17 (s, 3 H), 4.53 (d, *J*=7.62 Hz, 2 H), 7.75 (m, 1 H), 8.03 (m, 1 H), 8.17 (m, 1 H); MS (ESI) $(M+H)^+$: 300.1.

**Example 41**

***N*-[2-*tert*-butyl-1-(cyclohexylmethyl)-1*H*-benzimidazol-5-yl]-4-[(2-hydroxyethyl)ammo]-*N*-methylbenzenesulfonamide**

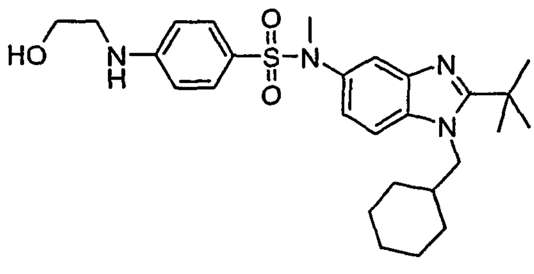

4- Bromo- *N*-[1-(cyclohexylmethyl)- 2-(1,1- dimethylethyl)- 1*H*-benzimidazol- 5- yl]-*N*- methyl- benzenesulfonamide (21.0 mg, 0.0405 mmol) (for preparation, see Example 40) and ethanolamine (1.0 mL) were placed in a sealed tube, The mixture was heated at 220°C in a Personal Chemistry SmithSynthesizer microwave instrument for 1.5 h, and purified by reversed-phase HPLC using 15-60% $CH_3CN/H_2O$ and then lyophilized affording the title compound as the corresponding TFA salt. Yield: 20.8 mg (84%); $^1H$ NMR (400 MHz, $CD_3OD$): δ 1.24 (m, 5 H), 1.63 (m, 2 H), 1.66 (s, 9 H), 1.71 (m, 1 H), 1.78 (m, 2 H), 2.11 (m, 1 H), 3.20 (s, 3 H), 3.26 (t, J=5.76 Hz, 2 H), 3.70 (t, J=5.86 Hz, 2 H), 4.44 (d, J=7.62 Hz, 2 H), 6.61 (m, 2 H), 7.23 (m, 2 H), 7.32 (dd, J=8.98, 2.15 Hz, 1 H), 7.51 (d, J=1.95 Hz, 1 H), 7.81 (d, J=9,18 Hz, 1 H); MS (ESI) $(M+H)^+$: 499.2; Anal. Calcd for $C_{27}H_{38}N_4O_3S$+1.60 TFA+2.30 $H_2O$ +0.3 MeCN (734.88): C, 50.34; H, 6.19; N; 8.20; Found: C, 50.40; H, 6.17; N, 8.18.

**Example 42**

***N*-[2-*tert*-butyl-1-(cyclohexylmethyl)-1*H*-benzimidazol-5-yl]-4-(dimethylamino)-*N*-methylbenzenesulfonamide**

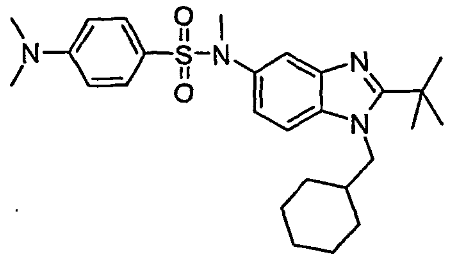

Following the procedure for Example 41, using 4-Bromo-*N*-[1-(cyclohexylmethyl)-2-(1,1-dimethylethyl)-1*H*-benzimidazol-5-yl]-*N*-methyl-benzenesulfonamide (31.6 mg, 0.0609 mmol) (for preparation, see Example 40) and ethanolamine (0.5 mL) in DMF (1.0 mL), the crude product was purified by reversed-phase HPLC using 15-60% $CH_3CN/H_2O$ and then lyophilized affording 20.4 mg (56%) of the title compound and 12.8 mg (34%) of the title compound in Example 41 as the corresponding TFA salt. $^1H$ NMR (400 MHz, $CD_3OD$): δ 1.25 (m, 5 H), 1.63 (m, 2 H), 1.67 (s, 9 H), 1.71 (m, 1 H), 1.78 (m, 2 H), 2.11 (m, 1 H), 3.03 (s, 6 H), 3.21 (s, 3 H), 4.44 (d, J=7.62 Hz, 2 H), 6.70 (m, 2 H), 7.31 (m, 3 H), 7.52 (d, J=1.95 Hz, 1 H), 7.82 (d, J=8.98 Hz, 1 H); MS (ESI) $(M+H)^+$: 483.3; Anal. Calcd for $C_{27}H_{38}N_4O_2S$+1.50 TFA+1.10 $H_2O$ (673.55): C, 53.50; H, 6.24; N, 8.32, Found: C, 53.42; H, 6.20; N, 8.42.

**Example 43**

**4-[bis(2-hydroxyethyl)amino]-*N*-[2-*tert*-butyl-1-(cyclohexylmethyl)-1*H*-benzimidazol-5-yl]-*N*-methylbenzenesulfonamide**

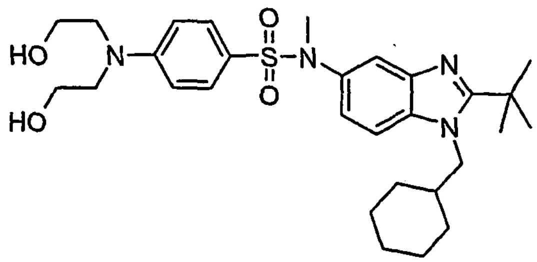

Following the procedure for Example 41, using 4-Bromo-*N*-[1-(cyclohexylmethyl)-2-(1,1-dimethylethyl)-1*H*-benzimidazol-5-yl]-*N*-methyl-benzenesulfonamide (31.2 mg, 0.0602 mmol) (for preparation, see Example 40) and 2,2'-iminodiethanol (1.0 mL), the crude product was purified by reversed-phase HPLC using 15-60% $CH_3CN/H_2O$ and then lyophilized affording the title compound as the corresponding TFA salt. Yield: 25.3 mg (64%); $^1H$ NMR (400 MHz, $CD_3OD$): 1.25 (m, 5 H), 1.64 (m, 2 H), 1.67 (s, 9 H), 1.71 (m, 1 H), 1.78 (m, 2 H), 2.10 (m, 1 H), 3.22 (s, 3 H), 3.60 (t, J=5.86 Hz, 4 H), 3.72 (t, J=5.86 Hz, 4 H), 4.45 (d, J=7.62 Hz, 2 H), 6.77 (m, 2 H), 7.30 (m, 2 H), 7.33 (m, 1 H), 7.54 (d, J=1.95 Hz, 1 H), 7.83 (d, J=8.98 Hz, 1 H); MS (ESI) $(M+H)^+$: 543.2; Anal. Calcd for $C_{29}H_{42}N_4O_4S$+1.60 TFA+0.4 $H_2O$ (732.39): C, 52.81; H, 6.11; N, 7.65, Found: C, 52.85; H, 6.06; N, 7.69.

**Example 44**

***N*-[2-*tert*-butyl-1-(cyclohexylmethyl)-1*H*-benzimidazol-5-yl]-*N*,4-dimethyl-3,4-dihydro-2*H*-1,4-benzoxazine-7-sulfonamide**

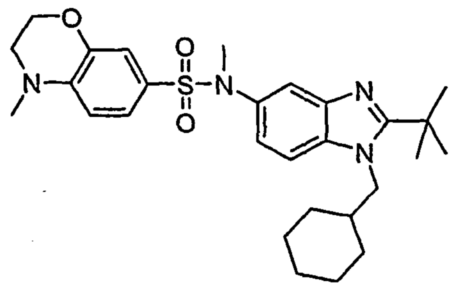

Following the procedure for the Step A in Example 40, using *N*-1-(Cyclohexylmethyl)-2-(1,1-dimethylethyl)-*N*-methyl-1*H*-benzimidazol-5-amine hydrochloride (33.0 mg, 0.0886 mmol) (for preparation, see the step F in Example 40), DMAP (43.3 mg, 0.354 mmol) and 4-methyl-3,4-dihydro-2*H*-1,4-benzoxazine-7-sulfonyl chloride (28.5 mg, 0.115 mmol) in MeCN (5 mL), the crude product was purified by by reversed-phase HPLC using 20-70% $CH_3CN/H_2O$ and then lyophilized affording the title compound as the corresponding TFA salt. Yield: 43.3 mg (78%); $^1H$ NMR (400 MHz, $CD_3OD$): δ 1.25 (m, 5 H), 1.64 (m, 2 H), 1.67 (s, 9 H), 1.70 (m, 1 H), 1.78 (m, 2 H), 2.11 (m, 1 H), 2.72 (s, 3 H), 3.24 (s, 3 H), 3.28 (m, 2 H), 4.31 (m, 2 H), 4.46 (d, J=7.42 Hz, 2 H), 6.66 (s, 1 H), 6.77 (m, 2 H), 7.32 (m, 1 H), 7.57 (d, J=1.37 Hz, 1 H), 7.85 (d, J=7.62 Hz, 1 H);. MS (ESI) $(M+H)^+$: 511.2; Anal. Calcd for $C_{28}H_{38}N_4O_3S$+1.40 TFA+0.40 $H_2O$ (677.54): C, 54.60; H, 5.98; N, 8.27, Found: C, 54.48; H, 5.89; N, 8.52.

**Example 45**

***N*-[4-({methyl[2-(1-methyl-1-pyridin-2-ylethyl)-1-(tetrahydro-2*H*-pyran-4-ylmethyl)-1*H*-benzimidazol-5-yl]amino}sulfonyl)phenyl]acetamide**

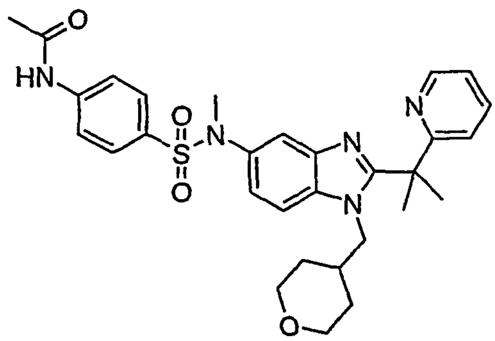

**Step A. *N*-[4-({methyl[2-(1-methyl-1-pyridin-2-ylethyl)-1-(tetrahydro-2*H*-pyran-4-ylmethyl)-1*H*-benzimidazol-5-yl]amino}sulfonyl)phenyl]acetamide**

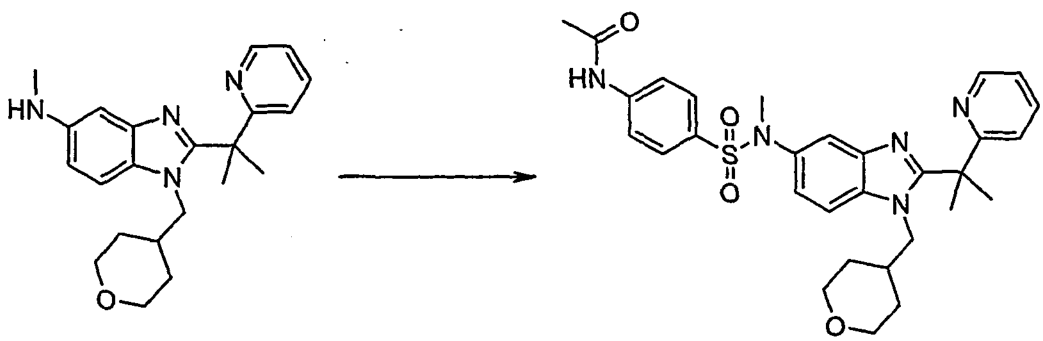

*N*-methyl-2-(1-methyl-1-pyridin-2-ylethyl)-1-(tetrahydro-2*H*-pyran-4-ylmethyl)-1*H*-benzimidazol-5-amine hydrochloride (85.0 mg, 0.13 mmol) (for preparation, see the following steps B, C, D, E and F), DMAP (64.0 mg, 0.53 mmol) and 4-(acetylamino)benzenesulfonyl chloride (60.7 mg, 0.26 mmol) in MeCN (5 mL) were stirred for 8 h at room temperature. The reaction mixture was quenched with $H_2O$ (3 mL). Upon evaporation, the residue was purified by reversed-phase HPLC using 10-50% $CH_3CN/H_2O$ and then lyophilized affording the title compound as the corresponding TFA salt. Yield: 45.8 mg (63%);. $^1$HNMR (400 MHz, $CD_3OD$): δ 1.07 - 1.13 (m, 4 H), 1.14 - 1.21 (m, 1 H). 2.02 (s, 6 H), 2.14 (s, 3 H), 2.92 - 3.09 (m, 2 H), 3.27 (s, 3 H), 3.71 - 3.80 (m, 2 H), 3.95 (d, J=6.64 Hz, 2 H), 7.28 (dd, J=8.98, 1.95 Hz, 1 H), 7.41 - 7.46 (m, 1 H), 7.46 - 7.51 (m, 2 H), 7.57 (d, J=1.76 Hz, 1 H), 7.67 - 7.80 (m, 4 H), 7.91 - 8.02 (m, 1 H), 8.43 - 8.55 (m, 1 H); MS (ESI) $(M+H)^+$: 562.0; Anal. Calcd for $C_{30}H_{35}N_5O_4S$+ 1.20TFA+0.40$H_2O$ +0.50 $CH_3OH$ (721.61): C, 54.75; H, 5.45; N, 9.70; Found: C, 54.76 ; H, 5.46; N, 9.76.

**Step B. *N*-methyl-*N*-{3-nitro-4-[(tetrahydro-2*H*-pyran-4-ylmethyl)amino]phenyl}acetamide**

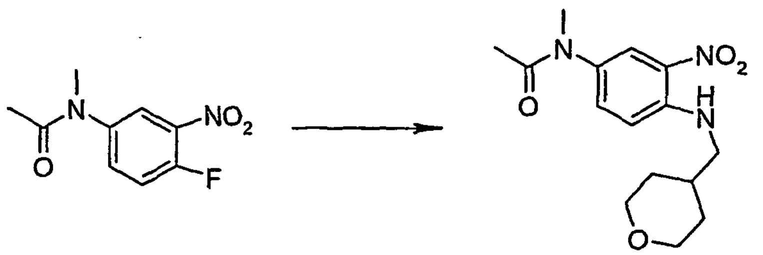

4-Aminomethylpyran (2.50 g, 21.7 mmol ) was added to a mixture of *N*-(4-fluoro-3-nitrophenyl)-*N*-methylacetamide (4.61 g, 21.27 mmol) (for preparation, see Example 39, Step B) and sodium carbonate (5.10 g, 47.7mmol) in EtOH (120 mL) at room temperature. The reaction mixture was heated for 3 days at 60 °C. Upon evaporation of ethanol, the residue was dissolved in EtOAc (400 mL), washed with $H_2O$ (3x50 mL), saturated NaCl (3x50 mL), and dried over $Na_2SO_4$. After filtation and concentration, 6.62 g (100%) of the title compound was obtained as an orange-red solid. $^1$H NMR (400 MHz, $CDCl_3$); δ 1.38 -1.52 (m, 2 H), 1.72 - 1.81 (m, 2 H), 1.90 (s, 3 H), 1.93 - 2.02 (m, 1 H), 3.23 (s, 3 H), 3.23 - 3.27 (m, 2 H), 3.36 - 3.49 (m, 2 H), 4.01 - 4.07 (m, 2 H), 6.91 (d, *J*=9.18 Hz, 1 H), 7.29 (dd, *J*=9.08, 2.64 Hz, 1 H), 8.05 (d, *J*=2.34 Hz, 1 H), 8.22 (t, *J*=5.37 Hz, 1 H); MS (ESI) $(M+H)^+$: 309.12.

**Step C. *N*-{3-amino-4-[(tetrahydro-2*H*-pyran-4-ylmethyl)amino]phenyl}-*N*-methylacetamide**

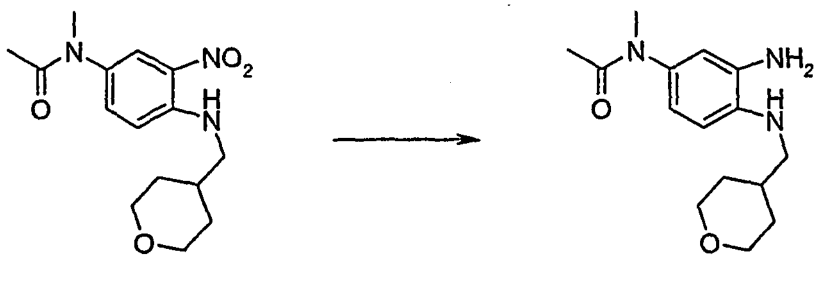

*N*-methyl-*N*-{3-nitro-4-[(tetrahydro-2*H*-pyran-4-ylmethyl)amino]phenyl}acetamide (5.39 g, 16.7 mmol) was hydrogenated in ethyl acetate (200 mL) catalyzed by 10% Pd/C (0.2 g) at 30-40 psi $H_2$ in Parr shaker for 18 h at room temperature. After filtration through celite and concentration, 6.0 g (100%) of a purple solid was obtained as HCl salt, which was used in the next step without purification. $^1$H NMR (400 MHz, $CD_3OD$): δ 1.32 - 1.46 (m, 2 H), 1.78 - 1.84 (m, 2 H), 1.85 (s, 3 H), 1.91 - 2.06 (m, 1 H), 3.16 (d, *J*=6.83 Hz, 2 H), 3.20 (s, 3 H), 3.39 - 3.51 (m, 2 H), 3.94 - 4.03 (m, 2 H), 7.01 (d, *J*=8.59 Hz, 1 H), 7.12 (d, *J*=2.15 Hz, 1 H), 7.17 (dd, *J*=8.49, 4.39 Hz, 1 H); MS (ESI) $(M+H)^+$: 278.7

**Step D. *N*-methyl-*N*-[2-(pyridin-2-ylmethyl)-1-(tetrahydro-2*H*-pyran-4-ylmethyl)-1*H*-benzimidazol-5-yl]acetamide**

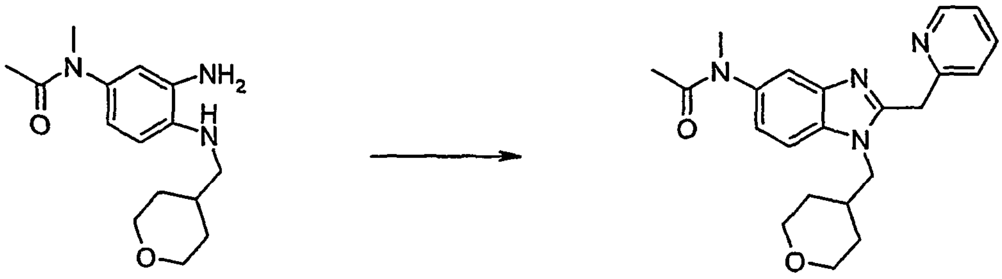

Diisopropylethylamine (0.970 g, 7.50 mmol) was added into a solution of *N*-{3-amino-4-[(tetrahydro-2*H*-pyran-4-ylmethyl)amino]phenyl}-*N*-methylacetamide hydrochoride (0.416 g, 1.33 mmol) and 2-pyridylacetic acid hydrochloride (0.286 g, 1.65 mmol) in DMF (15 mL) at 0 °C. Stirring for 20 min HATU (0.680 g, 1.80 mmol) was added. The reaction mixture was stirred for 4 h at room temperature, quenched with water (5 mL), concentrated to small volume, dissolved EtOAc (150 mL), washed with saturated NaCl (10 mL) and dried with anhydrous $Na_2SO_4$. After filtration and concentration, the residue was dissolved in acetic acid (20 mL) and heated for 18 h at 80 °C. Upon evaporation of the solvent, the residue was diluted with EtOAc (150 mL), washed with 2 *N* NaOH(10 mL) and satturated NaCl (2x10 mL), and dried over $Na_2SO_4$. After filtration and evaporation, the crude product was purified by MPLC using $CH_2Cl_2$/MeOH (10:1) on silica gel to give 0.3 1 g (61 %) of a yellow solid as the title compound. MS (ESI) $(M+H)^+$: 379.0.

**Step E. *N*-methyl-*N*-[2-(1-methyl-1-pyridin-2-ylethyl)-1-(tetrahydro-2*H*-pyran-4-ylmethyl)-1*H*-benzimidazol-5-yl] acetamide**

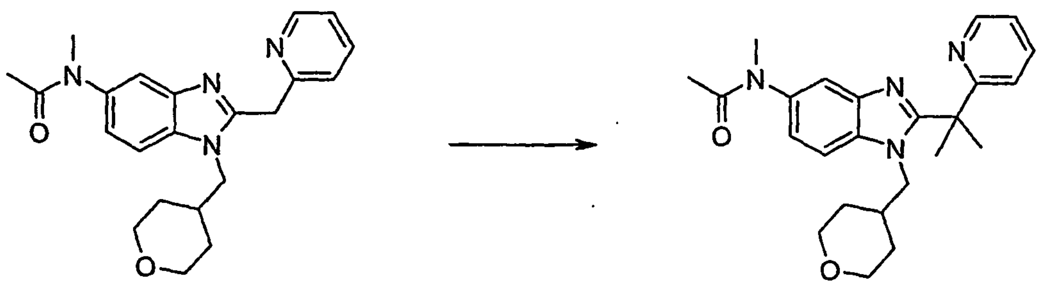

KHMDS (1.6 mL, 0.5 *M*, 0.8 mmol) was added to a solution of *N*-methyl-*N*-[2-(pyridin-2-ylmethyl)-1-(tetrahydro-2*H*-pyran-4-ylmethyl)-1*H*-benzimidazol-5-yl]acetamide (248.4 mg, 0.656 mmol) in THF (25 mL) at -78 °C. Stirring for 10 min, iodomethane (113.6 mg, 50 uL, 0.80 mmol) was added. The mixture was stirred for 30 min at -78 °C and 30 min at room temperature, then cooled down to -78 °C again. Another 1.2 equivalent KHMDS and iodomethane were added. The resulting mixture was stirred for 30 min at -78 °C and 45 min at room temperature, quenched with saturated $NaHCO_3$ (5 mL), and extracted with EtOAc (3x20 mL). The combined organic phases were washed with saturated $NaHCO_3$ (20 mL), brine (20 mL) and dried over $Na_2SO_4$. After filtration and concentration, the residue was purified by MPLC using EtOAc/MeOH (20:1) on silica gel to give 218.1 mg (90%) of the title compound as a white solid. $^1H$ NMR (400 MHz, $CDCl_3$): δ 1.02 - 1.12 (m, 2 H), 1.13 - 1.19 (m, 2 H), 1.19 - 1.27 (m, 1 H), 1.90 (s, 3 H), 1.97 (s, 6 H), 2.90 - 3.11 (m, 2 H), 3.31 (s, 3 H), 3.68 (d, *J*=7.22 Hz, 2 H), 3.81 (m, 2 H), 7.04 (dd, *J*=8.49, 2.05 Hz, 1 H), 7.18 - 7.32 (m, 3 H), 7.57 - 7.70 (m, 2 H), 8.53 - 8.70 (m, 1 H); MS (ESI) $(M+H)^+$: 407.03.

**Step F. *N*-methyl-2-(1-methyl-1-pyridin-2-ylethyl)-1-(tetrahydro-2*H*-pyran-4-ylmethyl)-1*H*-benzimidazol-5-amine**

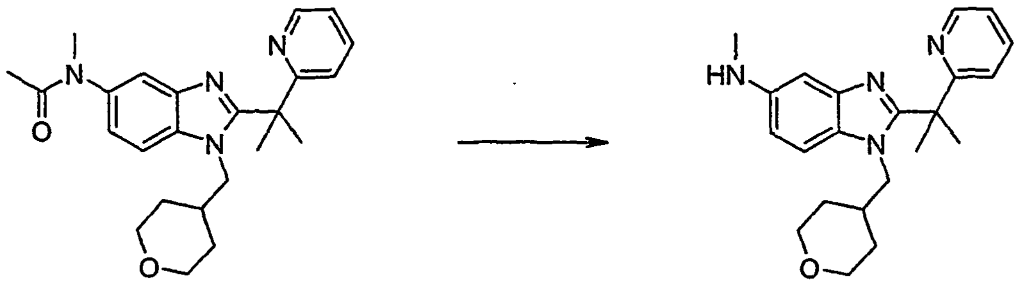

*N*-methyl-*N*-[2-(1-methyl-1-pyridin-2-ylethyl)-1-(tetrahydro-2*H*-pyran-4-ylmemyl)-1*H*-benzimidazol-5-yl]acetamide (214.0mg, 0.526 mmol) was dissolved in 5 mL of EtOH-2NHCl (3:2), and then heated at 120°C in a Personal Chemistry SmithSynthesizer microwave instrument for 1h. After concentration and dried *in vacuo*, 331 mg (100%) of a grey white solid was obtained as the title product. $^1$H NMR (400 MHz, DMSO-$D_6$): δ 0.86 - 1.08 (m, 4 H), 1.94 (s, 6 H), 1.96 - 2.03 (m, 1 H), 2.71 - 2.92 (m, 5 H), 3.55 - 3.70 (m, 2 H), 3.86 (d, *J*=5.47 Hz, 2 H), 7.31 - 7.48 (m, 2 H), 7.69 (d, *J*=7.42 Hz, 1 H), 7.74 - 7.84 (m, 1 H), 7.93 (t, *J*=8.30 Hz, 1 H), 8.48 (d, *J*=4.10 Hz, 2 H); MS (ESI) $(M+H)^+$: 365.04.

**Example 46**

***N*-(4-{[[2-*tert*-butyl-1-(tetrahydro-2*H*-pyran-4-ylmethyl)-1*H*-benzimidazol-5-yl](ethyl)amino]sulfonyl}phenyl) acetamide**

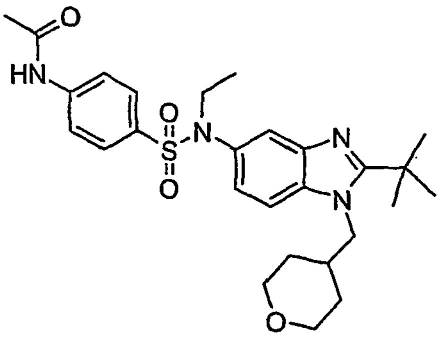

**Step A *N*-(4-{[[2-*tert*-butyl-1-(tetrahydro-2*H*-pyran4-ylmethyl)-1*H*-benzimidazol-5-yl](ethyl)amino]sulfonyl}phenyl)acetamide**

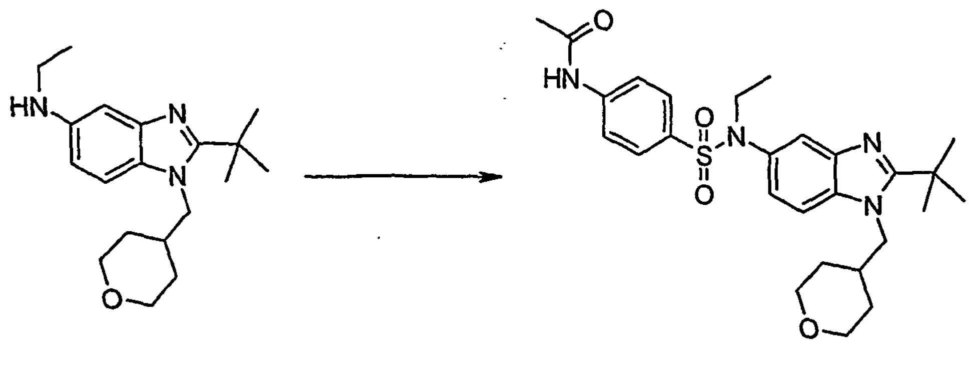

2-*tert*-butyl-*N*-ethyl-1-(tetrahydro-2*H*-pyran-4-ylmethyl)-1*H*-benzimidazol-5-amine hydrochloride (52.8 mg, 0.15 mmol) (for preparation, see the following steps B, C, D, E and F), DMAP (73.3 mg, 0.60 mmol) and 4-(acetylamino) benzenesulfonyl chloride (70.1 mg, 0.30 mmol) in MeCN (5 mL) were stirred overnight at room temperature. The reaction mixture was diluted with EtOAc (100 mL), washed with saturated $NaHCO_3$ (10 mL) and saturated NaCl (10 mL), and dried over $Na_2SO_4$. Upon evaporation, the residue was purified by MPLC using EtOAc/MeOH (20:1) on silica gel to give 60.3 mg (78%) of a white solid as the title compound. $^1$HNMR (400 MHz, $CD_3OD$): δ 1.07 (t, J=7.13 Hz, 3 H), 1.51 - 1.64 (m, 4 H), 1.68 (s, 9 H), 2.15 (s, 3 H), 2.29 - 2.47 (m, 1 H), 3.32 - 3.42 (m, 2 H), 3.72 (q, J=7.22 Hz, 2 H), 3.90 - 4.01 (m, 2 H), 4.53 (d, J=7.42 Hz, 2 H), 7.25 (dd, J=8.98, 1.95 Hz, 1 H), 7.47 - 7.55 (m, 3 H), 7.66 - 7.78 (m, 2 H), 7.90 (d, J=8.98 Hz, 1 H); MS (ESI) $(M+H)^+$: 513.0; Anal. Calcd for $C_{27}H_{36}N_4O_4S$+ 1.30 TFA+0.30 $CH_3OH$ (670.52): C, 53.56; H, 5.79; N, 8.36; Found: C, 53.66 ; H, 5.75; N, 8.10..

**Step B. *N*-ethyl-*N*-(4-fluoro-3-nitrophenyl)acetamide**

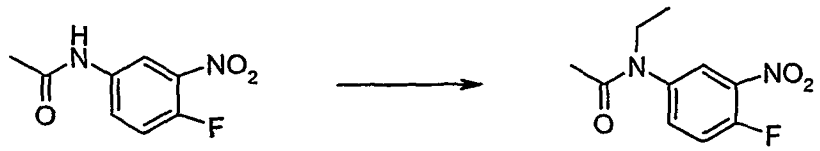

Sodium hydride (1.20g, 30 mmol) was added in portions to a solution of *N*-(4-fluoro-3-nitrophenyl)acetamide (3.96 g, 20 mmol) (for preparation see the step B in Example 33) in THF (100 mL) at 0 °C. Stirring for 20 min, iodoethane (9.32 g, 60 mmol) was added. The reaction mixture was stirred overnight at room temperature, quenched with saturated $NaHCO_3$ (30 mL) and extracted with EtOAc (3x100 mL). The combined organic phases were washed with saturated NaCl (2x30 mL). After filtration and concentration, the residue was purified by MPLC using Hex/EtOAc (1:1) on silica gel to give 2.36 g (52%) of a yellow solid as the title compound. $^1$H NMR (400 MHz, $CDCl_3$): δ 1.14 (t, J=6.93 Hz, 3 H), 1.88 (s, 3 H), 3.70 - 3.84 (q, J=7.0 Hz, 2 H), 7.34 - 7.43 (m, 1 H), 7.48 (s, 1 H), 7.87 - 7.98 (m, 1 H).

**Step C. *N*-ethyl-*N*-{3-nitro-4-[(tetrahydro-2*H*-pyran-4-ylmethyl)amino]phenyl}acetamide**

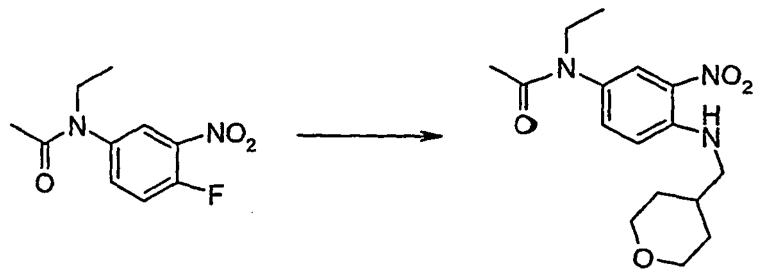

4-Aminomethylpyran (1.32 g, 11.4 mmol ) was added to a mixture of *N*-ethyl-*N*-(4-fluoro-3-nitrophenyl)acetamide (2.36 g, 10.4 mmol) and sodium carbonate (2.43 g, 22.9 mmol) in EtOH (70 mL) at room temperature. The reaction mixture was heated for a weekend at 60 °C. Upon evaporation of ethanol, the residue was diluted with $H_2O$ (50 mL), and extracted with EtOAc (3x100 mL). The combined organic phases weer washed saturated NaCl (2x50 mL) and dried over $Na_2SO_4$. After filtation and concentration, the residue was purified by MPLC using Hex/EtOAc (1:1) on silica gel to give 2.83 g (85%) of an orange-red solid as the title compound. $^1$H NMR (400 MHz, $CDCl_3$): δ 1.11 (t, *J*=7.13 Hz, 3 H), 1.38 - 1.52 (m, 2 H), 1.78 (m, 2 H), 1.86 (s, 3 H), 1.92 - 2.04 (m, 1 H), 3.20 - 3.29 (m, 2 H), 3.39 - 3.49 (m, 2 H), 3.71 (q, *J*=7.09 Hz, 2 H), 4.00 - 4.08 (m, 2 H), 6.91 (d, *J*=8.98 Hz, 1 H), 7.24 (d, *J*=2.54 Hz, 1 H), 8.01 (d, *J*=2.54 Hz, 1 H), 8.22 (t, *J*=4.98 Hz, 1 H).

**Step D. *N*-{3-amino-4-[(tetrahydro-2*H*-pyran-4-ylmethyl)amino]phenyl}-*N*-ethylacetamide**

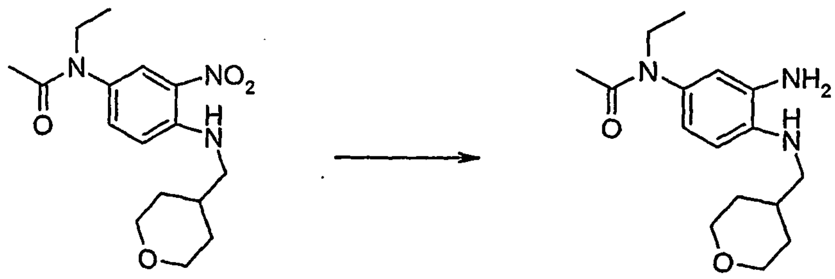

*N*-ethyl-*N*-{3-nitro-4-[(tetrahydro-2*H*-pyran-4-ylmethyl)amino]phenyl}acetamide (2.83 g, 8.79 mmol) was hydrogenated in ethyl acetate (200 mL) catalyzed by 10% Pd/C (0.2 g) at 30-40 psi $H_2$ in Parr shaker for 16 h at room temperature. After filtration through celite and concentration, 2.45 g (95%) of a light yellow solid was obtained, which was used in the next step without purification. MS (ESI) $(M+H)^+$: 292.3

**Step E. *N*-[2-*tert*-butyl-1-(tetrahydro-2*H*-pyran-ylmethyl)-1*H*-benzimidazol-5-yl]-*N*-ethylacetamide**

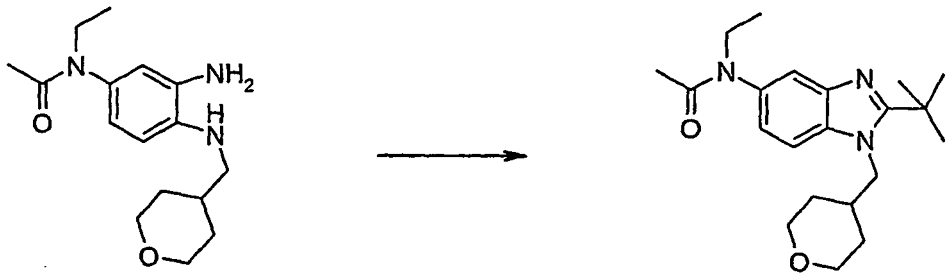

Following the procedure for Step D in Example 40, using *N*-{3-amino-4-[(tetrahydro-2*H*-pyran4-ylmethyl)amino] phenyl}-*N*-ethylacetamide (803.1 mg, 2.75 mmol), DMAP (671.9 mg, 5.50 mmol) and trimethylacetyl chloride (380.9 mg, 3.16 mmol) in $CH_2Cl_2$ (60 mL) and then in DCE (30 mL), the crude product was purified by MPLC using EtOAc/MeOH (20:1) on silica gel. Yield: 694.1 mg (71%); $^1H$ NMR (400 MHz, $CDCl_3$): δ 1.12 (t, *J*=7.13 Hz, 3 H), 1.51 - 1.57 (m, 4 H), 1.58 (s, 9 H), 1.83 (s, 3 H), 2.21 - 2.40 (m, 1 H), 326 - 3.43 (m, 2 H), 3.78 (q, *J*=7.23 Hz, 2 H), 3.94 - 4.07 (m, 2 H), 4.22 (d, *J*=7.42 Hz, 2 H), 7.02 (dd, *J*=8.59, 1.95 Hz, 1 H), 7.34 (d, *J*=8.59 Hz, 1 H), 7.54 (d, *J*=0.98 Hz, 1 H); MS (ESI) $(M+H)^+$: 358.07.

**Step F. 2-*tert*-butyl-*N*-ethyl-1-(tetrahydro-2*H*-pyran-4-ylmethyl)-1*H*-benzimidazol-5-amine**

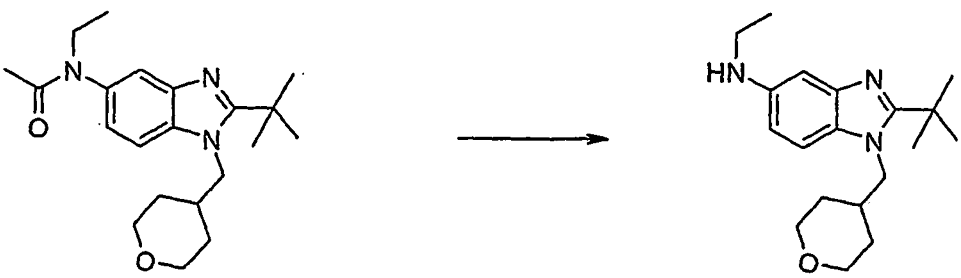

*N*-[2-*tert*-butyl-1-(tetrahydro-2*H*-pyran-4-ylmethyl)-1*H*-benzimidazol-5-yl]-*N*-ethylacetamide (648.3 mg, 2.06 mmol) was dissolved in 15 mL of EtOH-2*N* HCl (3:2), and then heated at 120°C in a Personal Chemistry SmithSynthesizer

microwave instrument for 3h. After concentration and dried *in vacuo*, 754.71 mg (100%) of a grey white solid was obtained as the title product. MS (ESI) $(M+H)^+$: 316.3.

**Example 47**

**4-[(aminocarbonyl)amino]-*N*-[2-*tert*-butyl-1-(tetrahydro-2*H*-pyran-4-ylmethyl)-1*H*-benzimidazol-5-yl]-*N*-ethyl-benzenesulfonamide**

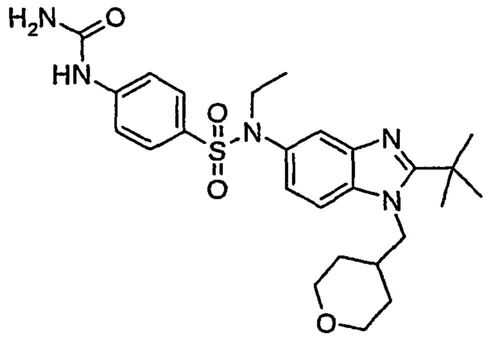

Following the procedure for the step A in Example 46, using 2-*tert*-butyl-*N*-ethyl-1-(tetrahydro-2*H*-pyran-4-ylmethyl)-1*H*-benzimidazol-5-amine hydrochloride (52.8 mg, 0.15 mmol) (for preparation, see the steps B, C, D, E, and F in example 46), DMAP (73.3 mg, 0.60 mmol) and 4-[(aminocarbonyl)amino]benzenesulfonyl chloride (70.3 mg, 0.30 mmol) in MeCN (5 mL), the crude product was purified by MPLC using EtOAc/MeOH (20:1) on silica gel to give 59.9 mg (78%) of a white solid as the title compound. $^1$HNMR (400 MHz, $CD_3OD$): δ 1.06 (t, J=7.13 Hz, 3 H), 1.51 -1.64 (m, 4 H), 1.68 (s, 9 H), 2.29 - 2.48 (m, 1 H), 3.31 - 3.43 (m, 2 H), 3.71 (q, J=7.03 Hz, 2 H), 3.86 - 4.01 (m, 2 H), 4.52 (d, J=7.62 Hz, 2 H), 7.26 (dd, J=8.88, 1.85 Hz, 1 H), 7.41 - 7.49 (m, 3 H), 7.51 - 7.59 (m, 2 H), 7.90 (d, J=8.98 Hz, 1 H); MS (ESI) $(M+H)^+$: 514.0; Anal. Calcd for $C_{26}H_{35}N_5O_4S$+ 1.30 TFA+0.40 $CH_3OH$ (674.71): C, 51.63; H, 5.66; N, 10.38; Found: C, 51.65; H, 5.63; N,10.38.

**Example 48**

***N*-[2-*tert*-butyl-1-(tetrahydro-2*H*-pyran-4-ylmethyl)-1*H*-benzimidazol-5-yl]-*N*-ethyl-4-{[(methylamino)carbonyl] amino}benzenesulfonamide**

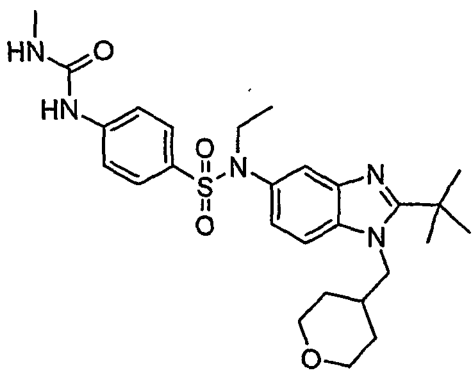

Following the procedure for the step A in Example 46, using 2-*tert*-butyl-*N*-ethyl-1-(tetrahydro-2*H*-pyran-4-ylmethyl)-1*H*-benzimidazol-5-amine hydrochloride (52.8 mg, 0.15 mmol) (for preparation, see the steps B, C, D, E and F in Example 46), DMAP (73.3 mg, 0.60 mmol) and 4-{[(methylamino)carbonyl]amino}benzenesulfonyl chloride (74.6

mg, 0.30 mmol) in MeCN (5 mL), the crude product was purified by MPLC using EtOAc/MeOH (20:1) on silica gel to give 63.2 mg (80%) of a white solid as the title compound. $^1$HNMR (400 MHz, $CD_3OD$): δ 1.06 (t, J=7.13 Hz, 3 H), 1.50 - 1.64 (m, 4 H), 1.68 (s, 9 H), 2.29 - 2.46 (m, 1 H), 2.77 (s, 3 H), 3.31 - 3.41 (m, 2 H), 3.71 (q, J=7.16 Hz, 2 H), 3.91 - 4.00 (m, 2 H), 4.52 (d, J=7.42 Hz, 2 H), 7.26 (dd, J=8.88, 2.05 Hz, 1 H), 7.40 - 7.46 (m, 2 H) ,7.48 (d, J=1.76 Hz, 1 H) , 7.50 - 7.55 (m, 2 H), 7.89 (d, J=8.98 Hz, 1 H); MS (ESI) $(M+H)^+$: 528.0; Anal. Calcd for $C_{27}H_{37}N_5O_4S$+ 1.40 TFA+0.50 $H_2O$ (696.33): C, 51.40; H, 5.70; N, 10.06; Found: C, 51.38 ; H, 5.69; N, 10.09.

**Example 49**

**4-amino-*N*-[2-*tert*-butyl-1-(tetrahydro-2*H*-pyran-4-ylmethyl)-1*H*-benzimidazol-5-yl]-*N*-ethylbenzenesulfonamide**

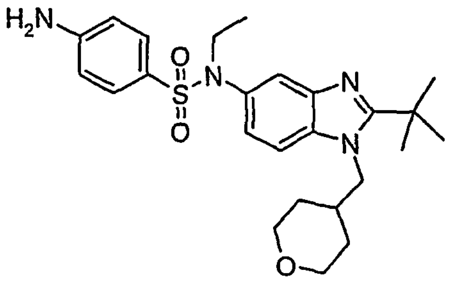

**Step A. 4-amino-*N*-[2-*tert*-butyl-1-(tetrahydro-2*H*-pyran-4-ylmethyl)-1*H*-benzimidazol-5-yl]-*N*-ethylbenzenesulfonamide**

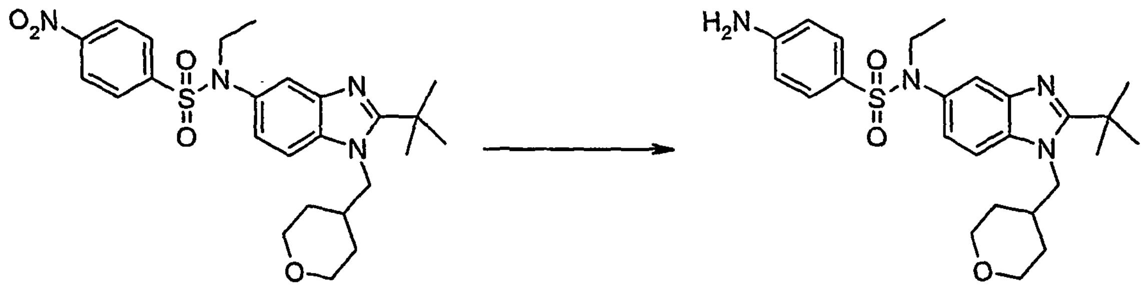

*N*-[2-*tert*-butyl-1-(tetrahydro-2*H*-pyran-4-ylmethyl)-1*H*-benzimidazol-5-yl]-*N*-ethyl-4-nitrobenzenesulfonamide (399.6 mg, 0.798 mmol) (for preparation, see the following step B) was hydrogenated in ethyl acetate (50 mL) catalyzed by 10% Pd/C (100 mg) at 30-40 psi $H_2$ in Parr shaker for 6 h at room temperature. After filtration through celite and concentration, 457.9 mg (100%) of a white solid was obtained. Small amounts of the crude product was purified by reversed-phase HPLC using 20-50% $CH_3CN/H_2O$ and then lyophilized affording the title compound as the corresponding TFA salt. $^1$HNMR (400 MHz, $CD_3OD$): δ 1.04 (t, J=7.13 Hz, 3 H), 1.49 - 1.65 (m, 4 H), 1.68 (s, 9 H), 2.25 - 2.55 (m, 1 H,) 3.32 - 3.43 (m, 2 H), 3.66 (q, J=7.03 Hz, 2 H), 3.88 - 4.04 (m, 2 H), 4.53 (d, J=7.42 Hz, 2 H), 6.50 - 6.69 (m, 2 H), 7.19 - 7.26 (m, 2 H), 7.28 (dd, J=8.98, 1.95 Hz, 1 H), 7.50 (d, J=1.76 Hz, 1 H), 7.90 (d, J=8.98 Hz, 1 H); MS (ESI) $(M+H)^+$: 471.0; Anal. Calcd for $C_{25}H_{34}N_4O_3S$+ 1.80 TFA+0.30 $H_2O$ (681.29): C, 50.42; H, 5.39; N, 8.22; Found: C, 50.38 ; H, 5.21; N, 8.44.

**Step B. *N*-[2-*tert*-butyl-1-(tetrahydro-2*H*-pyran-4-ylmethyl)-1*H*-benzimidazol-5-yl]-*N*-ethyl-4-nitrobenzenesulfonamide**

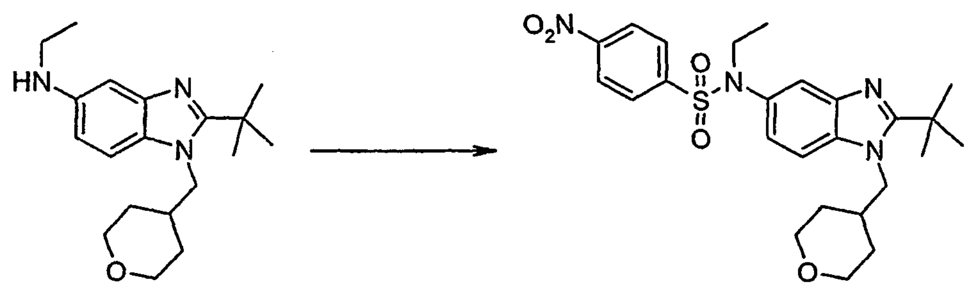

Following the procedure for the step A in Example 46, using 2-*tert*-butyl-*N*-ethyl-1-(tetrahydro-2*H*-pyran-4-ylmethyl)-1*H*-benzimidazol-5-amine hydrochloride (354.1 mg, 1.01 mmol) (for preparation, see the steps B, C, D, E and F in example 46), DMAP (491.7 mg, 4.03 mmol) and 4-nitrobenzenesulfonyl chloride (445.9 mg, 2.01 mmol) in MeCN (20 mL), the crude product was purified by MPLC using Hex/EtOAc (1:1) on silica gel to give 399.6 mg (80%) of a yellow solid as the title compound. MS (ESI) $(M+H)^+$: 501.0.

**Example 50**

***N*-(4-{[[2-*tert*-butyl-1-(tetrahydro-2*H*-pyran-4-ylmethyl)-1*H*-benzimidazol-5-yl](ethyl)amino]sulfonyl}phenyl)-2,2-dimethylpropanamide**

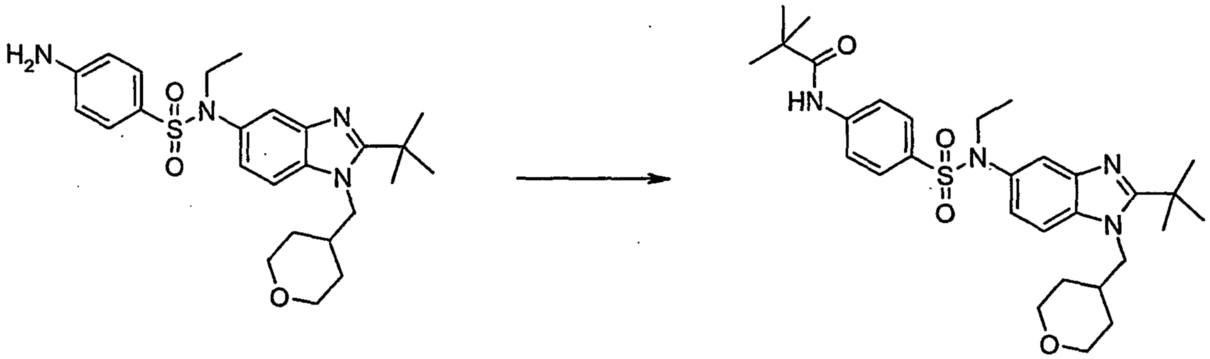

4-amino-*N*-[2-*tert*-butyl-1-(tetrahydro-2*H*-pyran-4-ylmethyl)-1*H*-benzimidazol-5-yl]-N-ethylbenzenesulfonamide (56.7 mg, 0.10 mmol) (for preparation, see the step A in Example 49), DMAP (48.9 mg, 0.40 mmol) and trimethylacetyl chloride (24.6 mg, 0.20 mmol) in MeCN (5 mL) were stirred for 4 h at room temperature. The reaction mixture was diluted with EtOAc (100 mL), washed with saturated $NaHCO_3$ (10 mL) and saturated NaCl (10 mL), and dried over $Na_2SO_4$. Upon evaporation, the residue was purified by reversed-phase HPLC using 20-70% $CH_3CN/H_2O$ and then lyophilized affording the title compound as the corresponding TFA salt. Yield: 41.3 mg (74%); $^1$HNMR (400 MHz, $CD_3OD$): δ 1.07 (t, J=7.13 Hz, 3 H), 1.29 (s, 9 H), 1.52 - 1.63 (m, 4 H), 1.67 (s, 9 H), 2.31 - 2.44 (m, 1 H), 3.31 - 3.41 (m, 2 H), 3.72 (q, J=7.03 Hz, 2 H), 3.95 (m, 2 H), 4.51 (d, J=7.62 Hz, 2 H), 7.24 (dd, J=8.98, 1.95 Hz, 1 H), 7.47 (d, J=1.95 Hz, 1 H), 7.48 - 7.56 (m, 2 H), 7.73 - 7.82 (m, 2 H), 7.88 (d, J=8.98 Hz, 1 H), 9.39 (s, 1 H); MS (ESI) $(M+H)^+$: 555.0; Anal. Calcd for $C_{30}H_{43}N_{34}O_4S$+ 1.80 TFA+0.30 $H_2O$ (765.40): C, 52.73; H, 5.85; N, 7.32; Found: C, 52.67; H, 5.75; N, 7.45.

**Example 51**

**2-[(4-{[[2-*tert*-butyl-1-(tetrahydro-2*H*-pyran-4-ylmethyl)-1*H*-benzimidazol-5-yl](ethyl)amino]sulfonyl}phenyl) amino]-2-oxoethyl acetate**

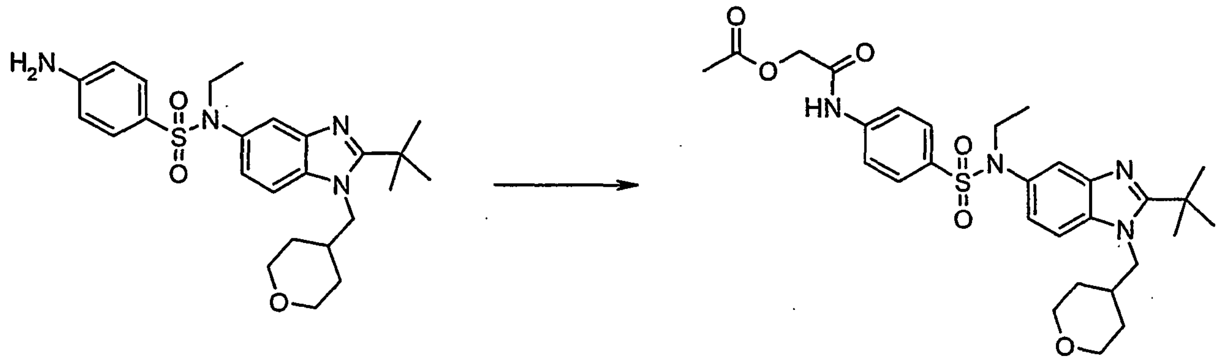

Following the procedure for Example 50, using 4-amino-*N*-[2-*tert*-butyl-1-(tetrahydro-2*H*-pyran-4-ylmethyl)-1*H*-benzimidazol-5-yl]-*N*-ethylbenzenesulfonamide (113.4 mg, 0.20 mmol) (for preparation, see the step A in Example 49), DMAP (97.7 mg, 0.80 mmol) and 2-chloro-2-oxoethyl acetate (54.6 mg, 0.40 mmol) in MeCN (10 mL), the crude product was purified by MPLC using EtOAc on silica gel to give 102.6 mg (90%) of a white solid as the title compound. $^1$NMR (400 MHz, $CD_3OD$): δ 1.07 (t, J=7.13 Hz, 3 H), 1.51 - 1.64 (m, 4 H), 1.67 (s, 9 H), 2.16 (s, 3 H), 2.30 - 2.45 (m, 1 H), 3.32 - 3.44 (m, 2 H), 3.73 (q, J=7.23 Hz, 2 H), 3.84 - 4.04 (m, 2 H), 4.51 (d, J=7.42 Hz, 2 H), 4.69 (s, 2 H), 7.24 (dd, J=8.98, 1.95 Hz, 1 H), 7.47 (d, J=1.76 Hz, 1 H), 7.50 - 7.57 (m, 2 H), 7.68 - 7.79 (m, 2 H), 7.89 (d, J=8.98 Hz, 1 H); MS (ESI) $(M+H)^+$: 571.0; Anal. Calcd for $C_{29}H_{38}N_4O_6S$+ 0.90 TFA (673.33): C, 54.94; H, 5.82; N, 8.32; Found: C, 54.95; H, 5.79; N, 8.13.

## Example 52

**N-(4-{[[2-*tert*-butyl-1-(tetrahydro-2*H*-pyran-4-ylmethyl)-1*H*-benzimidazol-5-yl](ethyl)amino]sulfonyl}phenyl)-2-hydroxyacetamide**

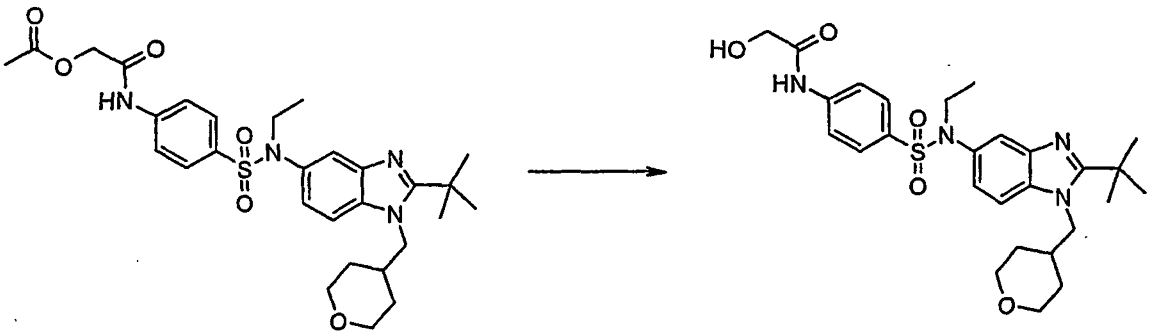

2-[(4-{[[2-*tert*-butyl-1-(tetrahydro-2*H*-pyran-4-ylmethyl)-1*H*-benzimidazol-5-yl](ethyl)amino]sulfonyl}phenyl) amino]-2-oxoethyl acetate (70.3 mg, 0.123 mmol) (for preparation, see the Example 51) and a drop of sodium methoxide (25% in MeOH) in MeOH (5 mL) was stirred overnight at room temperature. After evaporation, the crude product was purified by reversed-phase HPLC using 10-50% $CH_3CN/H_2O$ and then lyophilized affording the title compound as the corresponding TFA salt. Yield: 58.5 mg (90%); $^1$HNMR (400 MHz, $CD_3OD$): δ 1.07 (t, J=7.13 Hz, 3 H), 1.51 - 1.64 (m, 4 H), 1.68 (s, 9 H), 2.25 - 2.48 (m, 1 H), 3.31 - 3.41 (m, 2 H), 3.73 (q, J=7.16 Hz, 2 H), 3.95 (m, 2 H), 4.13 (s, 2 H), 4.52 (d, J=7.42 Hz, 2 H), 7.25 (dd, J=8.98, 1.95 Hz, 1 H), 7.49 (d, J=1.76 Hz, 1 H), 7.50 - 7.58 (m, 2 H), 7.78 - 7.85 (m, 2 H), 7.89 (d, J=8.98 Hz, 1 H); MS (ESI) $(M+H)^+$: 529.0; Anal. Calcd for $C_{27}H_{36}N_4O_5S$+ 1.50 TFA+0.20 $H_2O$ +0.30 $CH_3CN$ (715.63): C, 51.36; H, 5.47; N, 8.42; Found: C, 51.35; H, 5.47; N, 8.35.

**Example 53**

***N*-[2-*tert*-butyl-1-(tetrahydro-2*H*-pyran-ylmethyl)-1*H*-benzimidazol-5-yl]-*N*-ethyl-4-{[(isopropylamino)carbonyl] amino}benzenesulfonamide**

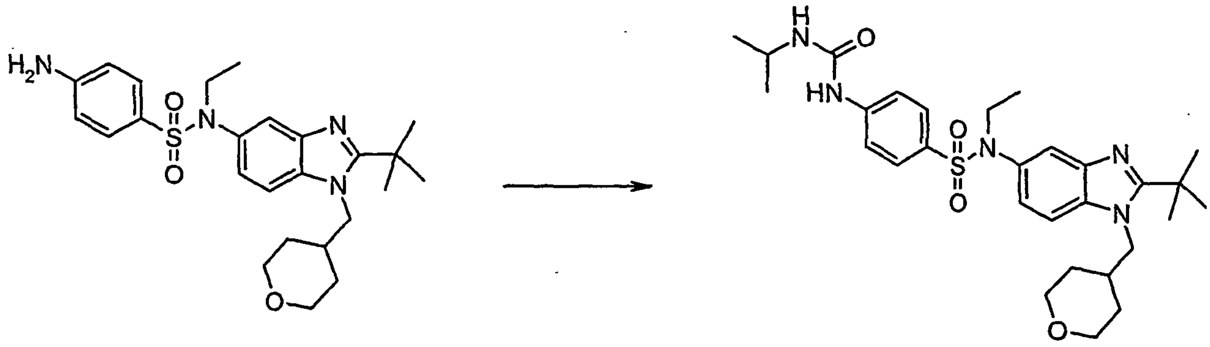

4-Amino-*N*-[2-*tert*-butyl-1-(tetrahydro-2*H*-pyran-4-ylmethyl)-1*H*-benzimidazol-5-yl]-N-ethylbenzenesulfonamide (56.7 mg, 0.1 mmol) (for preparation, see the step A in Example 49) and 2-isocyanatopropane (0.1 mL) in DCE (5 mL) was heated overnight at 80 °C. After evaporation, the crude product was purified by reversed-phase HPLC using 20-50% $CH_3CN/H_2O$ and then lyophilized affording the title compound as the corresponding TFA salt. Yield: 39.5 mg (71%); $^1$HNMR (400 MHz, $CD_3OD$): δ 1.06 (t, J=7.13 Hz, 3 H), 1.18 (d, J=6.64 Hz, 6 H), 1.51 - 1.63 (m, 4 H), 1.68 (s, 9 H), 2.27 - 2.42 (m, 1 H), 3.32 - 3.42 (m, 2 H), 3.70 (q, J=7.03 Hz, 2 H), 3.83 - 3.92 (m, 1 H), 3.92 - 3.99 (m, 2 H), 4.52 (d, J=7.42 Hz, 2 H), 7.25 (dd, J=8.88, 2.05 Hz, 1 H), 7.40 - 7.46 (m, 2 H), 7.47 (d, J=1.95 Hz, 1 H), 7.48 - 7.54 (m, 2 H), 7.89 (d, J=8.98 Hz, 1 H); MS (ESI) $(M+H)^+$: 556.0; Anal. Calcd for $C_{29}H_{41}N_5O_4S$+ 1.80 TFA+0.20 $H_2O$ +0.50 $CH_3CN$ (785.12): C, 51.40; H, 5.74; N, 9.81; Found: C, 51.40; H, 5.72; N, 9.79.

**Example 54**

***N*-[4-({ethyl[2-(1-methoxy-1-methylethyl)-1-(tetrahydro-2*H*-pyran4-ylmethyl)-1*H*-benzinidazol-5-yl]anlino}sulfonyl)phenyl]acetamide**

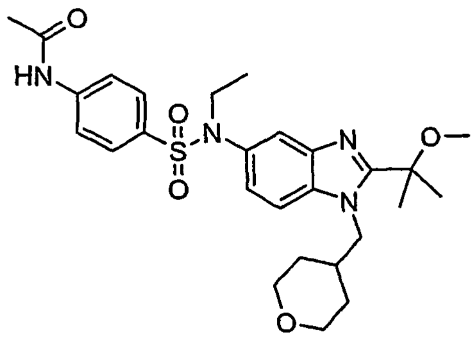

**Step A. *N*-[4-({ethyl[2-(1-methoxy-1-methylethyl)-1-(tetrahydro-2*H*-pyran-4-ylmethyl)-1*H*-benzimidazol-5-yl] amino}sulfonyl)phenyl]acetamide**

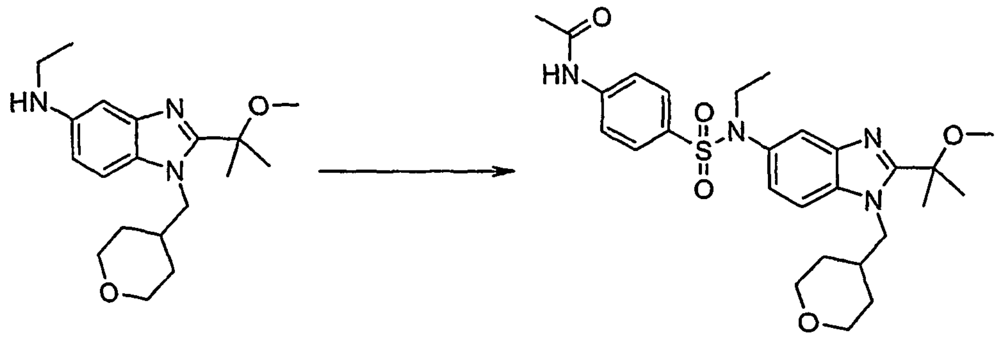

Following the procedure for the step A in Example 46, using *N*-ethyl-2-(1-methoxy-1-methylethyl)-1-(tetrahydro-2*H*-pyran-4-ylmethyl)-1*H*-benzimidazol-5-amine hydrochloride (50.0 mg, 0.136 mmol) (for preparation, see the following steps B, C and D), DMAP (64.5 mg, 0.50 mmol) and 4-(acetylamino)benzenesulfonyl chloride (61.2 mg, 0.26 mmol) in MeCN (5 mL), the crude product was purified by reversed-phase HPLC using 10-50% $CH_3CN/H_2O$ and then lyophilized affording the title compound as the corresponding TFA salt. Yield: 42.0 mg (58%); $^1$HNMR (400 MHz, $CD_3OD$): δ 1.06 (t, J=7.03 Hz, 3 H), 1.42 - 1.61 (m, 4 H), 1.80 (s, 6 H), 2.15 (s, 3 H), 2.31 - 2.46 (m, 1 H), 3.34 (s, 3 H), 3.35 - 3.43 (m, 2 H), 3.71 (q, J=7.23 Hz, 2 H), 3.89 - 4.02 (m, 2 H), 4.53 (d, J=7.42 Hz, 2 H), 7.20 (dd, J=8.88, 1.85 Hz, 1 H), 7.43 (d, J=1.76 Hz, 1 H), 7.48 - 7.57 (m, 2 H), 7.68 - 7.76 (m, 2 H), 7.81 (d, J=8.79 Hz, 1 H); MS (ESI) $(M+H)^+$: 529.0; Anal. Calcd for $C_{27}H_{36}N_4O_5S$+ 1.20 TFA+0.20 $H_2O$ (669.11): C, 52.78; H, 5.66; N, 8.37; Found: C, 52.80; H, 5.59; N, 8.51.

**Step B. *N*-ethyl-*N*-[2-(1-hydroxy-1-methylethyl)-1-(tetrahydro-2*H*-pyran-4-ylmethyl)-1*H*-benzimidazol-5-yl] acetamide**

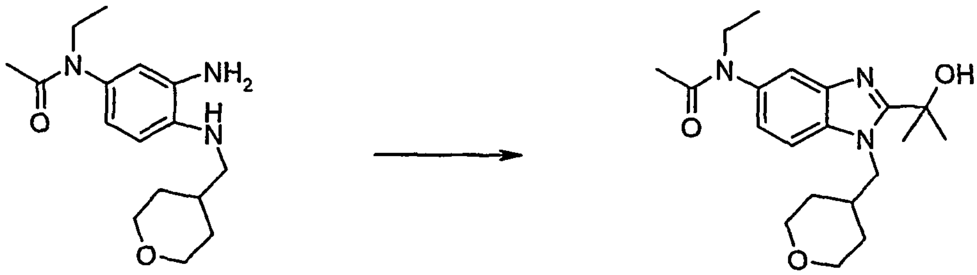

Diisopropylethylamine (0.558 g, 4.32 mmol) was added into a solution of *N*-{3-amino-4-[(tetrahydro-2*H*-pyran-4-ylmethyl)amino]phenyl}-*N*-ethylacetamide (0.841 g, 2.88 mmol) (for preparation, see the steps B, C and D in Example 46) and 2-hydroxy-2-methylpropanoic acid (0.330 g, 3.17 mmol) in DMF (40 mL) at 0 °C. Stirring for 30 min, HATU (1.31 g, 3.46 mmol) was added. The reaction mixture was stirred for overnight at room temperature and quenched with water (5 mL). After concentration, the residue was dissolved in acetic acid (50 mL) in sealed tubes. The solutions were heated at 140°C using a Personal Chemistry Smith Synthesizer microwave instrument for 35 min. Upon evaporation of the solvent, the residue was diluted with EtOAc (100 mL), washed with 2 *N* NaOH(10 mL) and satturated NaCl (2x10 mL), and dried over $Na_2SO_4$. After filtration and evaporation, 1.78 g (purity >43%) of the crude product was obtained, which was used directly for next step without purification. MS (ESI) $(M+H)^+$: 360.04.

**Step C. *N*-ethyl-*N*-[2-(1-methoxy-1-methylethyl)-1-(tetrahydro-2*H*-pyran-4-ylmethyl)-1*H*-benzimidazol-5-yl] acetamide**

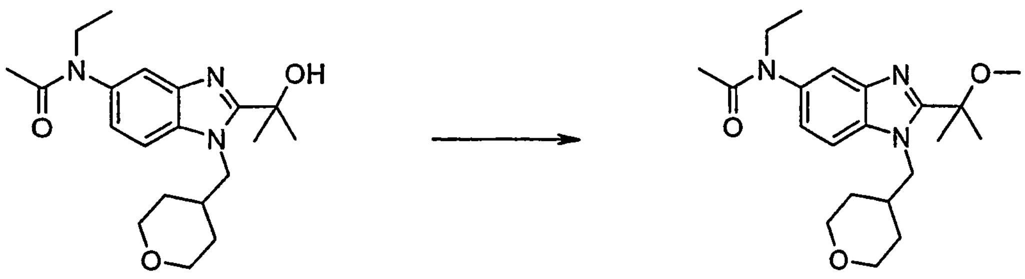

Sodium hydride (0.35 g, 60%, 8.64 mmol) was added in portions to a solution of *N*-ethyl-*N*-[2-(1-hydroxy-1-methylethyl)-1-(tetrahydro-2*H*-pyran-4-ylmethyl)-1*H*-benzimidazol-5-yl]acetamide (1.78 g of the above crude product, 2.88 mmol) in THF (100 mL) at 0 °C. Stirring for 20 min, iodomethane (1.23 g, 8.64 mmol) was added. The reaction mixture was stirred overnight at room temperature, quenched with saturated $NH_4Cl$ (20 mL) and diluted with EtOAc (100 mL), washed with saturated NaCl (2x20 mL) and dried over $Na_2SO_4$. After filtration and concentration, the residue was purified by MPLC using EtOAc/MeOH (20:1) on silica gel to give 0.423 g (39%) of a grey white solid as the title compound. MS (ESI) $(M+H)^+$: 374.03.

**Step D. *N*-ethyl-2-(1-methoxy-1-methylethyl)-1-(tetrahydro-2*H*-pyran-4-ylmethyl)-1*H*-benzimidazol-5-amine**

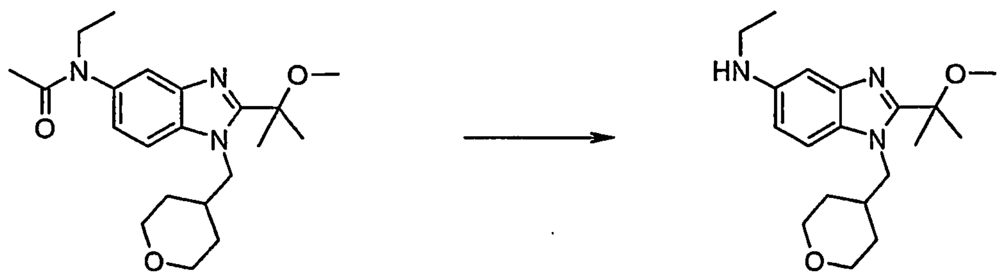

*N*-ethyl-*N*-[2-(1-methoxy-1-methylethyl)-1-(tetrahydro-2*H*-pyran-4-ylmethyl)-1*H*-benzimidazol-5-yl]acetamide (422.5 mg, 1.13 mmol) was dissolved in 15 mL of EtOH-2*N* HCl (3:2), and then heated at 120°C in a Personal Chemistry SmithSynthesizer microwave instrument for 3.5 h. After concentration and dried *in vacuo*, 441.9 mg (100%) of a light brown solid was obtained as the title product. MS (ESI) $(M+H)^+$: 332.04.

**Example 55**

**4-[(aminocarbonyl)amino]-*N*-ethyl-*N*-[2-(1-methoxy-1-methylethyl)-1-(tetrahydro-2*H*-pyran4-ylmethyl)-1*H*-benzimidazol-5-yl]benzenesulfonamide**

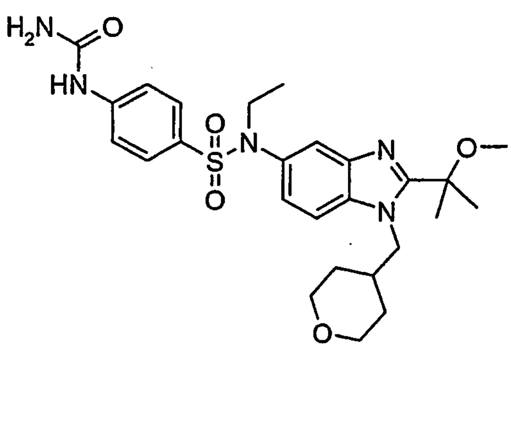

Following the procedure for the step A in Example 47, using *N*-ethyl-2-(1-methoxy-1-methylethyl)-1-(tetrahydro-2*H*-pyran-4-ylmethyl)-1*H*-benzimidazol-5-amine hydrochloride (50.0 mg, 0.136 mmol) (for preparation, see the steps B, C and D in example 15), DMAP (64.5 mg, 0.50 mmol) and 4-[(aminocarbonyl)amino]benzenesulfonyl chloride (64.5 mg, 0.26 mmol) in MeCN (5 mL), the crude product was purified by reversed-phase HPLC using 10-45% $CH_3CN/H_2O$ and then lyophilized affording the title compound as the corresponding TFA salt. Yield: 31.1 mg (43%); $^1$HNMR (400 MHz, $CD_3OD$): δ 1.06 (t, J=7.13 Hz, 3 H), 1.50 - 1.58 (m, 4 H), 1.81 (s, 6 H), 2.29 - 2.48 (m, 1 H), 3.35 (s, 3 H), 3.36 - 3.43 (m, 2 H), 3.70 (q, J=7.03 Hz, 2 H), 3.89 - 4.02 (m, 2 H), 4.54 (d, J=7.22 Hz, 2 H), 7.22 (dd, J=8.98, 1.95 Hz, 1 H), 7.40 - 7.49 (m, 3 H), 7.51 - 7.58 (m, 2 H), 7.77 - 7.90 (m, 1 H); MS (ESI) $(M+H)^+$: 530.0; Anal. Calcd for $C_{26}H_{35}N_5O_5S$+ 1.20 TFA+1.10 $H_2O$ +0.10 $CH_3OH$ (689.51): C, 49.65; H, 5.67; N, 10.16; Found: C, 49.67; H, 5.67; N, 10.19.

## Example 56

**N-ethyl-N-[2-(1-methoxy-1-methylethyl)-1-(tetrahydro-2H-pyran-4-ylmethyl)-1H-benzimidazol-5-yl]-4-{[(methylamino)carbonyl]amino}benzenesulfonamide**

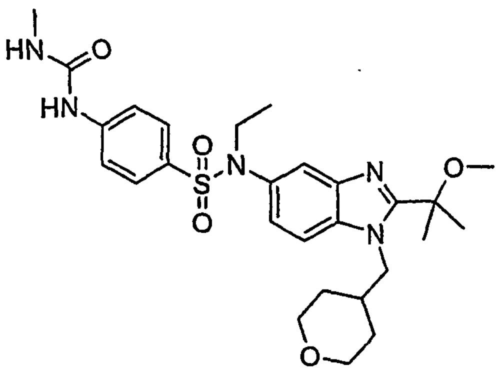

Following the procedure for the step A in Example 47, using *N*-ethyl-2-(1-methoxy-1-methylethyl)-1-(tetrahydro-2*H*-pyran-4-ylethyl)-1*H*-benzimidazol-5-amine hydrochloride (50.0 mg, 0.136 mmol) (for preparation, see the steps B, C and D in example 54), DMAP (64.5 mg, 0.50 mmol) and 4-{[(methylamino)carbonyl]amino}benzenesulfonyl chloride (62.0 mg, 0.26 mmol) in MeCN (5 mL), the crude product was purified by reversed-phase HPLC using 10-45% $CH_3CN/H_2O$ and then lyophilized affording the title compound as the corresponding TFA salt. Yield: 36.9 mg (50%); $^1$HNMR (400 MHz, $CD_3OD$): δ 1.06 (t, J=7.13 Hz, 3 H),1.51 - 1.59 (m, 4 H), 1.81 (s, 6 H), 2.31 - 2.47 (m, 1 H), 2.77 (s, 3 H), 3.35 (s, 3 H) ,3.36 - 3.41 (m, 2 H), 3.70 (q, J=7.16 Hz, 2 H), 3.91 - 3.99 (m, 2 H), 4.54 (d, J=7.42 Hz, 2 H), 7.20 - 7.25 (m, 1 H), 7.44 (d, J=2.15 Hz, 1 H), 7.44 - 7.48 (m, 2 H), 7.50 - 7.56 (m, 2 H), 7.85 (d, J=9.18 Hz, 1 H); MS (ESI) $(M+H)^+$: 544.0; Anal. Calcd for $C_{27}H_{37}N_5O_5S$+ 0.80 TFA+0.50 $H_2O$ (643.92): C, 53.35; H, 6.07; N, 10.88; Found: C, 53.25; H, 6.05; N, 10.99.

## Example 57

**4-amino-N-ethyl-N-[2-(1-methoxy-1-methylethyl)-1-(tetrahydro-2H-pyran-4-ylmethyl)-1H-benzimidazol-5-yl] benzenesulfonamide**

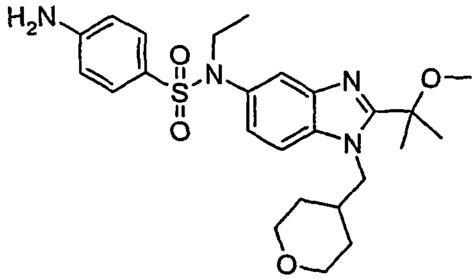

**Step A. 4-amino-*N*-ethyl-*N*-[2-(1-methoxy-1-methylethyl)-1-(tetrahydro-2*H*-pyran4-ylmethyl)-1*H*-benzimidazol-5-yl]benzenesulfonamide**

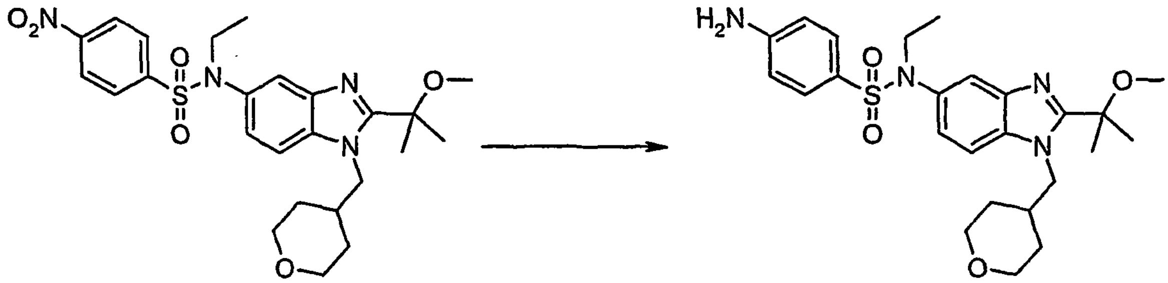

Following the procedure for the step A in Example 49, using *N*-ethyl-*N*-[2-(1-methoxy-1-methylethyl)-1-(tetrahydro-2*H*-pyran-4-ylmethyl)-1*H*-benzimidazol-5-yl]-4-nitrobenzenesulfonamide (276.0 mg, 0.798 mmol) (for preparation, see the following step B) and 10% Pd/C (50 mg) in ethyl acetate (50 mL), 287.7 mg (100%) of a white solid was obtained. Small amounts of the crude product was purified by reversed-phase HPLC using 10-50% $CH_3CN/H_2O$ and then lyophilized affording the title compound as the corresponding TFA salt. $^1$HNMR (400 MHz, $CD_3OD$): δ 1.03 (t, J=7.13 Hz, 3 H), 1.50 - 1.58 (m, 4 H), 1.81 (s, 6 H), 2.31 - 2.48 (m, 1 H), 3.35 (s, 3 H), 3.36 - 3.42 (m, 2 H), 3.65 (q, J=7.03 Hz, 2 H), 3.90 - 3.99 (m, 2 H), 4.54 (d, J=7.42 Hz, 2 H), 6.60 - 6.66 (m, 2 H), 7.21 - 7.27 (m, 3 H), 7.44 (d, J=1.95 Hz, 1 H), 7.82 (d, J=8.79 Hz, 1 H); MS (ESI) $(M+H)^+$: 487.0; Anal. Calcd for $C_{25}H_{34}N_4O_4S$+ 1.50 TFA (657.67): C, 51.14; H, 5.44; N, 8.52; Found: C, 51.31; H, 5.44; N, 8.40.

**Step B. *N*-ethyl-*N*-[2-(1-methoxy-1-methylethyl)-1-(tetrahydro-2*H*-pyran-4-ylmethyl)-1*H*-benzimidazol-5-yl]-4-nitrobenzenesulfonamide**

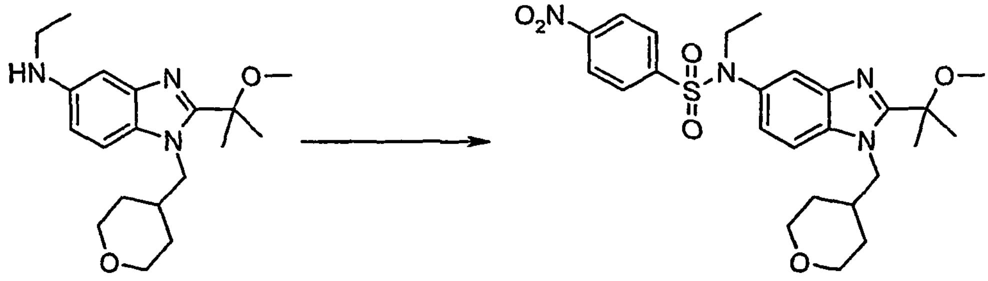

Following the procedure for the step A in Example 46, using *N*-ethyl-2-(1-methoxy-1-methylethyl)-1-(tetrahydro-2*H*-pyran-4-ylmethyl)-1*H*-benzimidazol-5-amine hydrochloride (248.2 mg, 0.675 mmol) (for preparation, see the steps B, C and D in example 54), DMAP (329.9 mg, 2.70 mmol) and 4-nitrobenzenesulfonyl chloride (299.0 mg, 1.35 mmol)

in MeCN (15 mL), the crude product was purified by MPLC using Hex/EtOAc (1:1) on silica gel to give 276.0 mg (79%) of a yellow solid as the title compound. MS (ESI) $(M+H)^+$: 517.00.

**Example 58**

***N*-[4-({ethyl[2-(1-methoxy-1-methylethyl)-1-(tetrahydro-2*H*-pyran-4-ylmethyl)-1*H*-benzimidazol-5-yl]amino} sulfonyl)phenyl]-2,2-dimethylpropanamide**

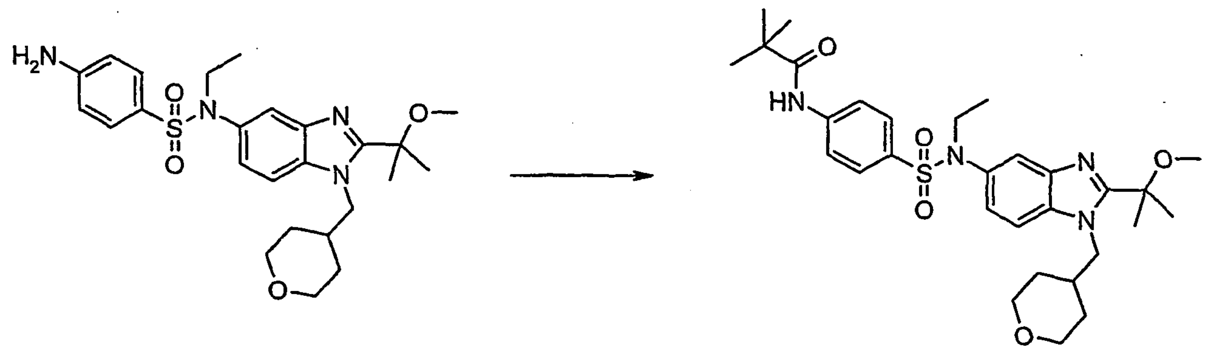

Following the procedure for Example 50, using 4-amino-*N*-ethyl-*N*-[2-(1-methoxy-1-methylethyl)-1-(tetrahydro-2*H*-pyran-4-ylmethyl)-1*H*-benzimidazol-5-yl]benzenesulfonamide (51.5 mg, 0.095 mmol) (for preparation, see the step A in Example 57), DMAP (50.0 mg, 0.409 mmol) and trimethylacetyl chloride (24.6 mg, 0.20 mmol) in MeCN (5 mL), the crude product was purified by reversed-phase HPLC using 20-60% $CH_3CN/H_2O$ and then lyophilized affording the title compound as the corresponding TFA salt. Yield: 45.8 mg (85%); $^1$HNMR (400 MHz, $CD_3OD$): δ 1.06 (t, J=7.13 Hz, 3 H), 1.30 (s, 9 H), 1.49 - 1.60 (m, 4 H), 1.80 (s, 6 H), 2.32 - 2.46 (m, 1 H), 3.34 (s, 3 H), 3.35 - 3.42 (m, 2 H), 3.71 (q, J=7.03 Hz, 2 H), 3.90 - 4.00 (m, 2 H), 4.53 (d, J=7.42 Hz, 2 H), 7.20 (dd, J=8.98,1.95 Hz, 1 H), 7.42 (d, J=1.76 Hz, 1 H), 7.48 - 7.55 (m, 2 H), 7.75 - 7.81 (m, 2 H), 7.82 (s, 1 H); MS (ESI) $(M+H)^+$: 571.0 0; Anal. Calcd for $C_{30}H_{42}N_4O_5S$+ 1.20 TFA+0.80 $H_2O$ (722.00): C, 53.90; H, 6.25; N, 7.76; Found: C, 53.93; H, 6.25; N, 7.67.

**Example 59**

**2-{[4-({ethyl[2-(1-methoxy-1-methylethyl)-1-(tetrahydro-2*H*-pyran4-ylmethyl)-1*H*-benzimidazol-5-yl]amino}sulfonyl)phenyl]amino}-2-oxoethyl acetate**

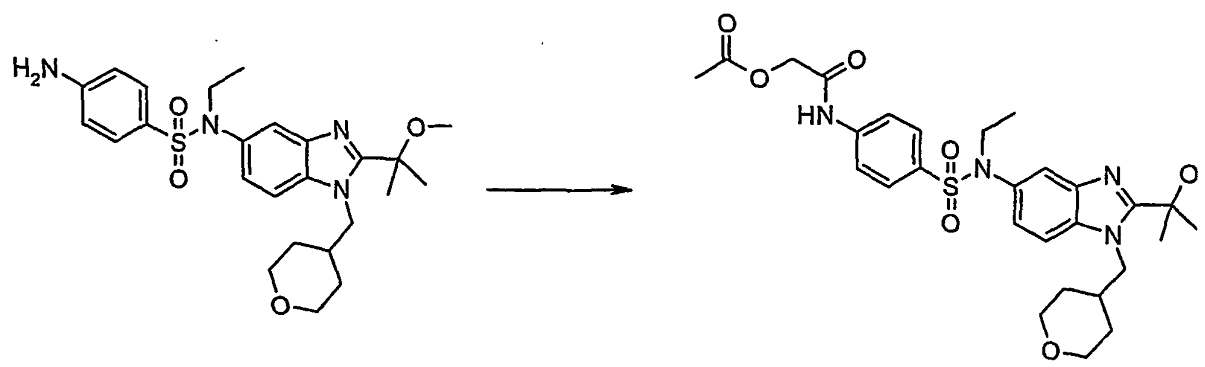

Following the procedure for Example 50, using 4-amino-*N*-ethyl-*N*-[2-(1-methoxy-1-methylethyl)-1-(tetrahydro-2*H*-pyran-4-ylmethyl)-1*H*-benzimidazol-5-yl]benzenesulfonamide (100.0 mg, 0.185 mmol) (for preparation, see the step

A in Example 57), DMAP (97.7 mg, 0.80 mmol) and 2-chloro-2-oxoethyl acetate (54.6 mg, 0.40 mmol) in MeCN (10 mL), the crude product was purified by MPLC using Hex/EtOAc on silica gel to give 68.7 mg (63%) of a light yellow solid as the title compound. $^1$HNMR (400 MHz, $CD_3OD$): δ 1.06 (t, J=7.13 Hz, 3 H), 1.48 - 1.56 (m, 4 H), 1.76 (s, 6 H), 2.17 (s, 3 H), 2.30 - 2.46 (m, 1 H), 3.28 (s, 3 H), 3.32 - 3.42 (m, 2 H), 3.70 (q, J=7.23 Hz, 2 H), 3.85 - 4.04 (m, 2 H), 4.47 (d, J=7.62 Hz, 2 H), 4.70 (s, 2 H), 7.11 (dd, J=8.79, 1.95 Hz, 1 H), 7.33 (d, J=1.95 Hz, 1 H), 7.50 - 7.58 (m, 2 H), 7.68 (d, J=8.79 Hz, 1 H), 7.72 - 7.79 (m, 2 H); MS (ESI) $(M+H)^+$: 587.0; Anal. Calcd for $C_{29}H_{38}N_4O_7S$+ 0.70 TFA+3.10 $H_2O$ +0.90 $CH_3CN$ (759.32): C, 50.93; H, 6.32; N, 9.04; Found: C, 50.90 ; H, 6.26; N, 9.05.

**Example 60**

***N*-[4-({ethyl[2-(1-methoxy-1-methylethyl)-1-(tetrahydro-2*H*-pyran-4-ylmethyl)-1*H*-benzimidazol-5-yl]amino} sulfonyl)phenyl]-2-hydroxyacetamide**

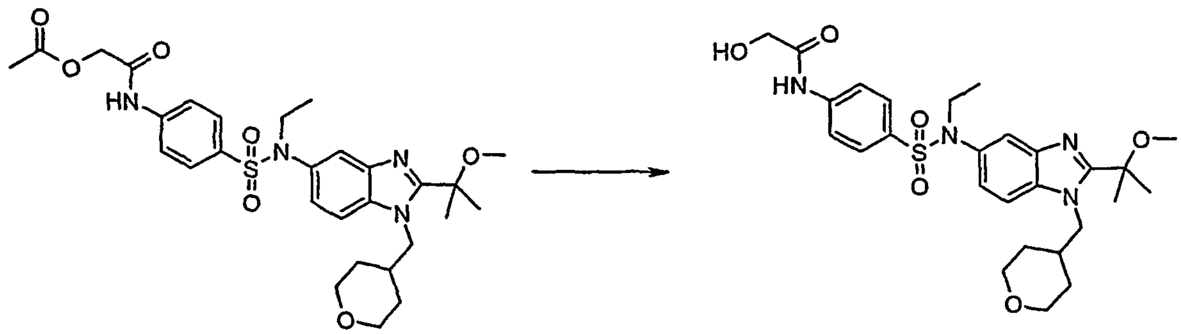

Following the procedure for Example 52, using 2-{[4-({ethyl[2-(1-methoxy-1-methylethyl)-1-(tetrahydro-2*H*-pyran-4-ylmethyl)-1*H*-benzimidazol-5-yl]amino}sulfonyl)phenyl]amino}-2-oxoethyl acetate (46.3 mg, 0.0789 mmol) (for preparation, see the Example 59) and a drop of sodium methoxide (25% in MeOH) in MeOH (5 mL), the crude product was purified by reversed-phase HPLC using 10-45% $CH_3CN/H_2O$ and then lyophilized affording the title compound as the corresponding TFA salt. Yield: 27.4 mg (64%); $^1$HNMR (400 MHz, $CD_3OD$): δ 1.07 (t, J=7.13 Hz, 3 H), 1.50 - 1.56 (m, 4 H), 1.80 (s, 6 H), 2.32 - 2.46 (m, 1 H), 3.34 (s, 3 H), 3.35 - 3.41 (m, 2 H), 3.72 (q, J=7.03 Hz, 2 H), 3.91 - 3.99 (m, 2 H), 4.13 (s, 2 H), 4.53 (d, J=7.42 Hz, 2 H), 7.20 (dd, J=8.98, 1.95 Hz, 1 H), 7.44 (d, J=1.76 Hz, 1 H), 7.51 - 7.58 (m, 2 H), 7.79 - 7.85 (m, 3 H); MS (ESI) $(M+H)^+$: 545.0; Anal. Calcd for $C_{27}H_{36}N_4O_6S$+ 1.30 TFA+0.50 $H_2O$ (701.91): C, 50.65; H, 5.50; N, 7.98; Found: C, 50.61; H, 5.50; N, 8.12.

**Example 61**

***N*-ethyl-4-{[(isopropylamino)carbonyl]amino}-*N*-[2-(1-methoxy-1-methylethyl)-1-(tetrahydro-2*H*-pyran-4-ylmethyl)-1*H*-benzimidazol-5-yl]benzenesulfonamide**

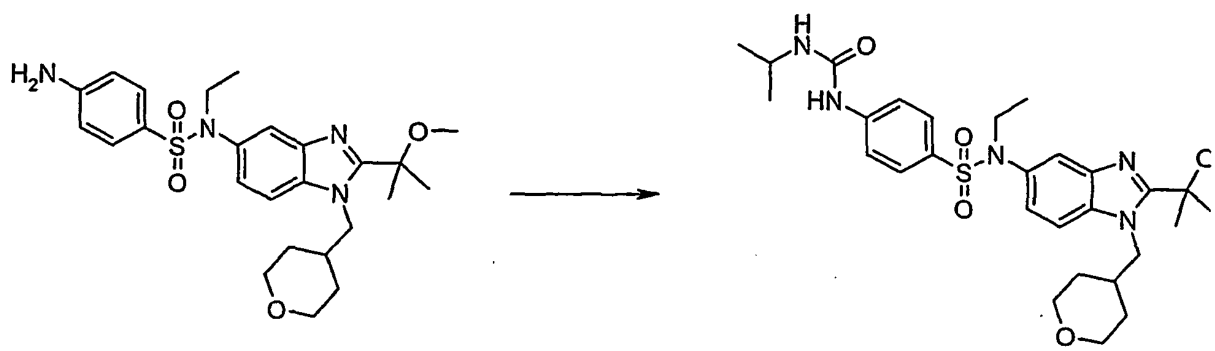

Following the procedure for Example 53, using 4-amino-*N*-ethyl-*N*-[2-(1-methoxy-1-methylethyl)-1-(tetrahydro-2*H*-pyran-4-ylmethyl)-1*H*-benzimidazol-5-yl]benzenesulfonamide (45.7 mg, 0.0845 mmol) (for preparation, see the step A in Example 57) and 2-isocyanatopropane (0.2 mL) in DCE (5 mL), the crude product was purified by reversed-phase HPLC using 20-50% $CH_3CN/H_2O$ and then lyophilized affording the title compound as the corresponding TFA salt. Yield: 17.1 mg (35%); $^1$HNMR (400 MHz, $CD_3OD$): δ 1.05 (t, J=7.13 Hz, 3 H), 1.18 (d, J=6.44 Hz, 6 H), 1.50 - 1.57 (m, 4 H), 1.79 (s, 6 H), 2.31 - 2.46 (m, 1 H), 3.33 (s, 3 H), 3.35 - 3.43 (m, 2 H), 3.69 (q, J=7.03 Hz, 2 H), 3.84 - 3.92 (m, 1 H), 3.92 - 3.99 (m, 2 H), 4.52 (dd, J=7.42 Hz, 2 H), 7.18 (dd, J=8.98, 1.95 Hz, 1 H), 7.41 (d, J=1.76 Hz, 1 H), 7.42 - 7.48 (m, 2 H), 7.48 - 7.56 (m, 2 H), 7.79 (d, J=8.79 Hz, 1 H); MS (ESI) $(M+H)^+$: 572.0; Anal. Calcd for $C_{29}H_{41}N_5O_5S$+ 1.60 TFA+0.40 $H_2O$ (761.39): C, 50.80; H, 5.75; N, 9.20; Found: C, 50.83; H, 5.77; N, 9.01.

**Example 62**

***N*-(4-{[[2-(1-methoxy-1-methylethyl)-1-(tetrahydro-2*H*-pyran-4-ylmethyl)-1*H*-benzimidazol-5-yl](methyl)amino] sulfonyl}phenyl)acetamide**

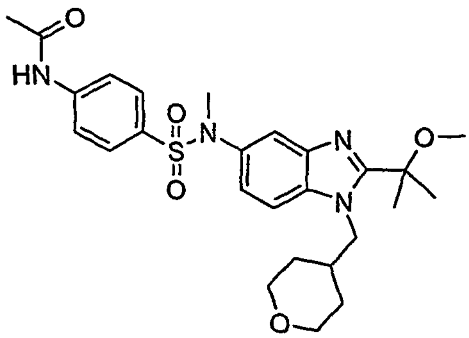

**Step A. *N*-(4-{[[2-(1-methoxy-1-methylethyl)-1-(tetrahydro-2*H*-pyran-4-ylmethyl)-1*H*-benzimidazol-5-yl](methyl) amino]sulfonyl}phenyl)acetamide**

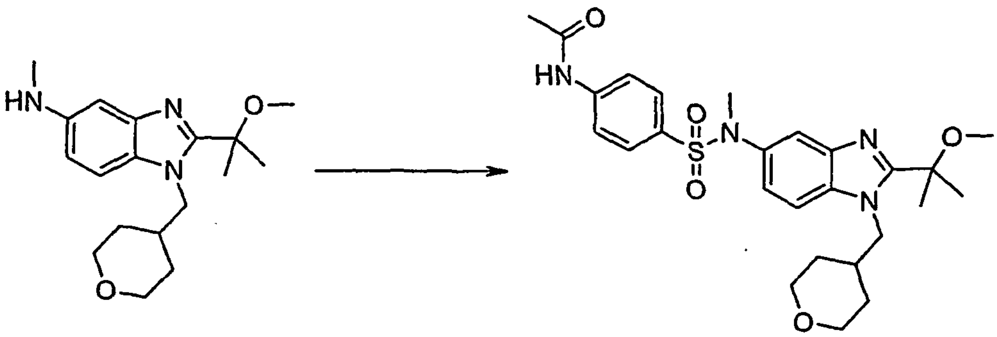

Following the procedure for the step A in Example 46, using 2-(1-methoxy-1-methylethyl)-*N*-methyl-1-(tetrahydro-2*H*-pyran-4-ylmethyl)-1*H*-benzimidazol-5-amine hydrochloride (77.8 mg, 0.15 mmol) (for preparation, see the following steps B, C and D), DMAP (73.3 mg, 0.60 mmol) and 4-(acetylamino)benzenesulfonyl chloride (68.9 mg, 0.30 mmol) in MeCN (10 mL), the crude product was purified by MPLC using EtOAc/MeOH (20:1) on silica gel to give 55.7 mg (72%) of a white solid as the title compound. $^1$HNMR (400 MHz, $CD_3OD$): δ 1.49 - 1.62 (m, 4 H), 1.83 (s, 6 H), 2.14 (s, 3 H), 2.32 - 2.46 (m, 1 H), 3.26 (s, 3 H), 3.32 - 3.37 (m, 2 H), 3.38 (s, 3 H), 3.90 - 4.00 (m, 2 H), 4.57 (d, J=7.42 Hz, 2 H), 7.33 (dd, J=8.98, 1.95 Hz, 1 H), 7.43 - 7.50 (m, 2 H), 7.54 (d, J=1.76 Hz, 1 H), 7.68 - 7.76 (m, 2 H), 7.89 (d, J=8.98 Hz, 1 H); MS (ESI) $(M+H)^+$: 515.0; Anal. Calcd for $C_{26}H_{34}N_4O_5S$+ 1.3 HCl +0.4 $CH_3OH$ (574.86): C, 55.16, H, 6.47, N, 9.75; Found: C, 55.25; H, 6.38; N, 9.58.

**Step B. *N*-[2-(1-hydroxy-1-methylethyl)-1-(tetrahydro-2*H*-pyran-4-ylmethyl)-1*H*-benzimidazol-5-yl]-*N*-methylacetamide**

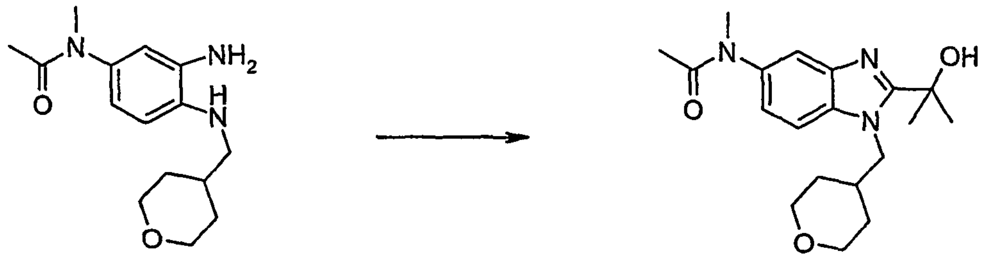

Following the procedure for the step B in Example 54, using *N*-{3-amino-4-[(tetrahydro-2*H*-pyran-4-ylmethyl) amino]phenyl}-*N*-methylacetamide (1.14 g, 4.11 mmol) (for preparation, see the steps B, C and D in Example 45), 2-hydroxy-2-methylpropanoic acid (0.470 g, 4.52 mmol), diisopropylethylamine (0.800 g, 6.17 mmol) and HATU (1.88 g, 4.93 mmol) in DMF (40 mL) and then in acetic acid (50 mL), the crude product was purified by MPLC using EtOAc/ MeOH (20:1) on silica gel to give 0.475 g (33%) of a brown solid as the title compound. MS (ESI) $(M+H)^+$: 346.03.

**Step C. *N*-[2-(1-methoxy-1-methylethyl)-1-(tetrahydro-2*H*-pyran-4-ylmethyl)-1*H*-benzimidazol-5-yl]-*N*-methylacetamide**

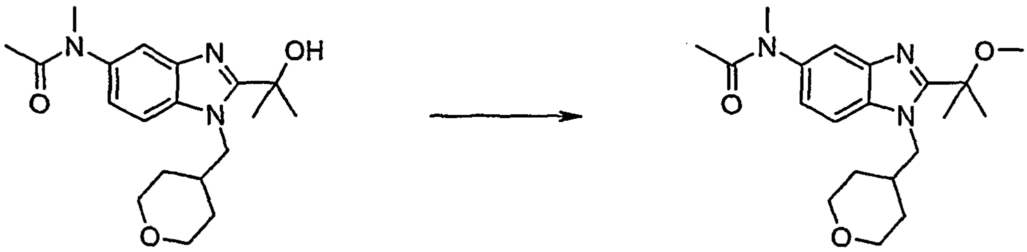

Following the procedure for the step C in Example 54, using *N*-[2-(1-hydroxy-1-methylethyl)-1-(tetrahydro-2*H*-pyran-4-ylmethyl)-1*H*-benzimidazol-5-yl]-*N*-methylacetamide (103.1, 0.299 mmol), iodomethane (93.6 mg, 0.66 mmol) and sodium hydride (26.4 mg, 60%, 0.66 mmol) in THF (10 mL, 110 mg 100%) of the title compound was obtained as a colorless syrup. MS (ESI) $(M+H)^+$: 360.05.

**Step D. 2-(1-methoxy-1-methylethyl)-*N*-methyl-1-(tetrahydro-2*H*-pyran-4-ylmethyl)-1*H*-benzimidazol-5-amine**

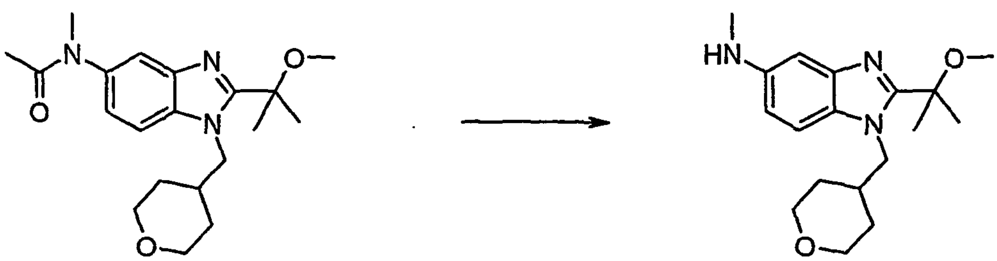

Following the procedure for the step D in Example 54, using *N*-[2-(1-methoxy-1-methylethyl)-1-(tetrahydro-2*H*-pyran-4-ylmethyl)-1*H*-benzimidazol-5-yl]-*N*-methylacetamide (110 mg, 0.299 mmol) in 5 mL of EtOH-2*N* HCl (3:2), 121.6 mg (100%) of a grey white solid was obtained as the title product. MS (ESI) $(M+H)^+$: 318.57.

**Example 63**

**4-[(aminocarbonyl)amino]-*N*-[2-(1-methoxy-1-methylethyl)-1-(tetrahydro-2*H*-pyran-4-ylmethyl)-1*H*-benzimidazol-5-yl]-*N*-methylbenzenesulfonamide**

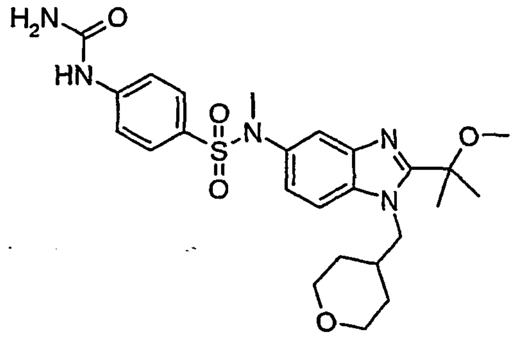

Following the procedure for the step A in Example 62, using 2-(1-methoxy-1-methylethyl)-*N*-methyl-1-(tetrahydro-2*H*-pyran-4-ylmethyl)-1*H*-benzimidazol-5-amine hydrochloride (48.6 mg, 0.120 mmol) (for preparation, see the steps B, C and D in example 62), DMAP (58.6 mg, 0.48 mmol) and 4-[(aminocarbonyl)amino]benzenesulfonyl chloride (42.1 mg, 0.178 mmol) in MeCN (5 mL), the crude product was purified by reversed-phase HPLC using 10-45% $CH_3CN/H_2O$ and then lyophilized affording the title compound as the corresponding TFA salt. Yield: 34.7 mg (56%); $^1$HNMR (400 MHz, $CD_3OD$): δ 1.47 - 1.59 (m, 4 H), 1.80 (s, 6 H), 2.31 - 2.46 (m, 1 H), 3.24 (s, 3 H), 3.34 (s, 3 H), 3.35 - 3.41 (m, 2 H), 3.87 - 4.04 (m, 2 H), 4.53 (d, J=7.42 Hz, 2 H), 7.28 (dd, J=8.79, 1.95 Hz, 1 H), 7.37 - 7.44 (m, 2 H), 7.47 (d, J=1.56 Hz, 1 H), 7.50 - 7.58 (m, 2 H), 7.81 (d, J=8.98 Hz, 1 H); MS (ESI) $(M+H)^+$: 516.0; Anal. Calcd for $C_{25}H_{33}N_5O_5S$+ 1.3TFA (663.87): C, 49.94, H, 5.21, N, 10.55; Found: C, 50.07; H, 5.16; N, 10.44.

**Example 64**

**2-Hydroxy-*N*-(4-{[[2-(1-methoxy-1-methylethyl)-1-(tetrahydro-2*H*-pyran-4-ylmethyl)-1*H*-benzimidazol-5-yl](methyl)amino]sulfonyl}phenyl)acetamide**

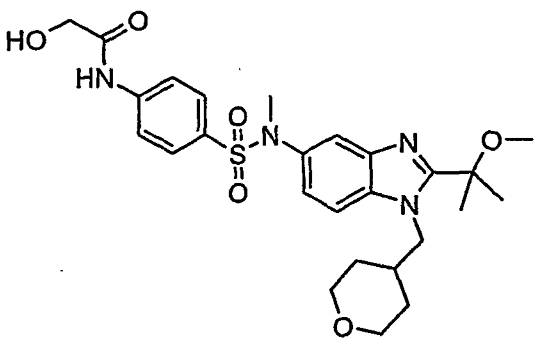

**Step A. 2-Hydroxy-*N*-(4-{[[2-(1-methoxy-1-methylethyl)-1-(tetrahydro-2*H*-pyran-4-ylmethyl)-1*H*-benzimidazol-5-yl](methyl)amino]sulfonyl}phenyl)acetamide**

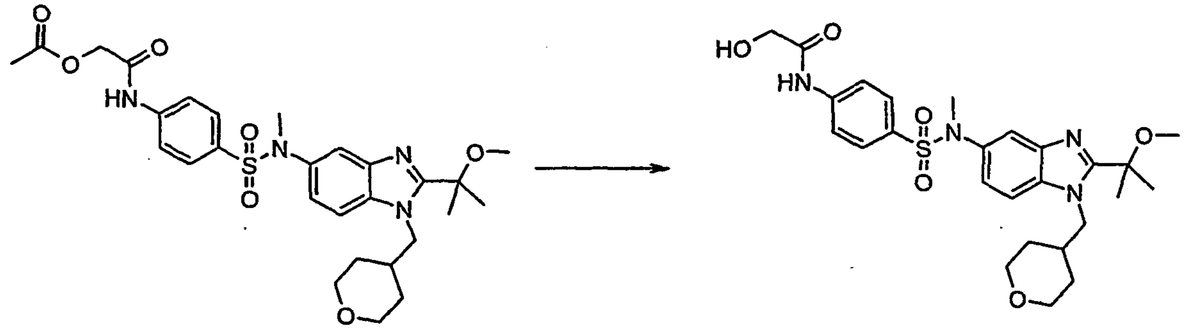

Following the procedure for Example 52, using 2-[(4-{[[2-(1-methoxy-1-methylethyl)-1-(tetrahydro-2*H*-pyran-4-ylmethyl)-1*H*-benzimidazol-5-yl](methyl)amino]sulfonyl}phenyl)amino]-2-oxoethyl acetate (100 mg, crude, 0.097 mmol) (for preparation, see the following steps B, C and D) and a drop of sodium methoxide (25% in MeOH) in MeOH (10 mL), the crude product was purified by reversed-phase HPLC using 10-50% $CH_3CN/H_2O$ and then lyophilized affording the title compound as the corresponding TFA salt. Yield: 16.7 mg (32%); $^1$HNMR (400 MHz, $CD_3OD$): δ 1.47 - 1.60 (m, 4 H), 1.80 (s, 6 H), 2.29 - 2.46 (m, 1 H), 3.25 (s, 3 H), 3.34 (s, 3 H), 3.35 - 3.40 (m, 2 H), 3.90 - 4.01 (m, 2 H), 4.13 (s, 2 H), 4.53 (d, J=7.42 Hz, 2 H), 7.27 (dd, J=8.88, 2.05 Hz, 1 H), 7.47 (d, J=2.15 Hz, 1 H), 7.48 - 7.53 (m, 2 H), 7.79 (s, 1 H), 7.79 - 7.84 (m, 2 H); MS (ESI) $(M+H)^+$: 531.0; Anal. Calcd for $C_{26}H_{34}N_4O_6S$+ 1.40 TFA+0.2 $H_2O$ (693.88): C, 49.85, H, 5.20, N, 8.07; Found: C, 49.78; H, 5.18; N, 8.20.

**Step B. *N*-[2-(1-methoxy-1-methylethyl)-1-(tetrahydro-2*H*-pyran-4-ylmethyl)-1*H*-benzimidazol-5-yl]-*N*-methyl-4-nitrobenzenesulfonamide**

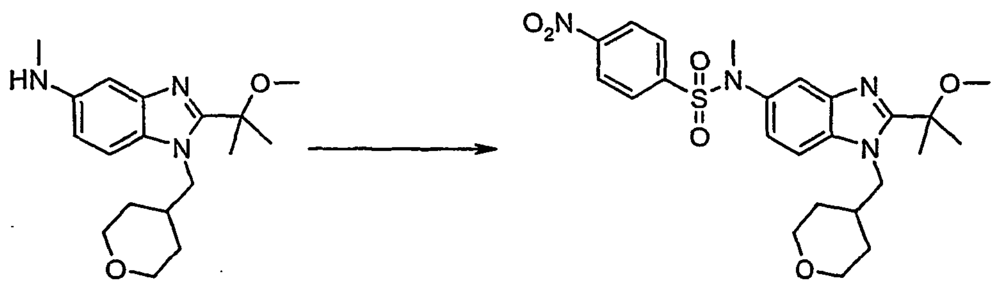

Following the procedure for the step A in Example 46, using 2-(1-methoxy-1-methylethyl)-*N*-methyl-1-(tetrahydro-2*H*-pyran-4-ylmethyl)-1*H*-benzimidazol-5-amine hydrochloride (72.9 mg, 0.179 mmol) (for preparation, see the steps B, C and D in example 62), DMAP (87.5 mg, 0.716 mmol) and 4-nitrobenzenesulfonyl chloride (59.8 mg, 0.269 mmol) in MeCN (6 mL), the crude product was purified by MPLC using Hex/EtOAc (1:1) on silica gel to give 49.8 mg (55%) of a yellow solid as the title compound. MS (ESI) $(M+H)^+$: 502.98.

**Step C. 4-Amino-*N*-[2-(1-methoxy-1-methylethyl)-1-(tetrahydro-2*H*-pyran-4-ylmethyl)-1*H*-benzimidazol-5-yl]-*N*-methylbenzenesulfonamide**

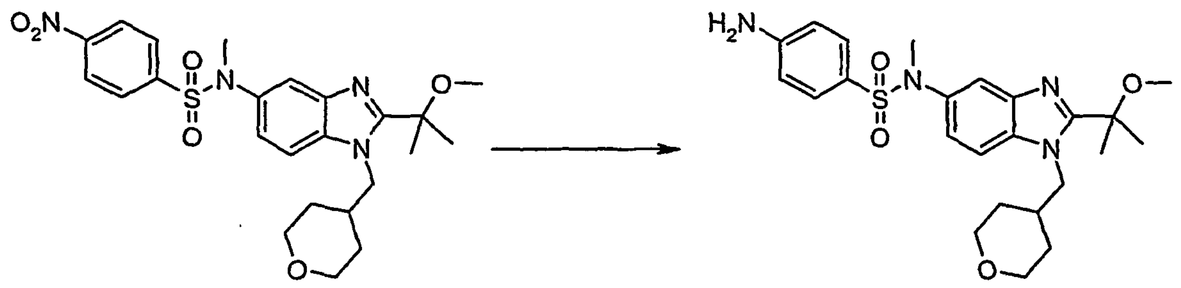

Following the procedure for the step A in Example 49, using *N*-[2-(1-methoxy-1-methylethyl)-1-(tetrahydro-2*H*-pyran-4-ylmethyl)-1*H*-benzimidazol-5-yl]-*N*-methyl-4-nitrobenzenesulfonamide (48.9 mg, 0.097 mmol) and 10% Pd/C (20 mg) in ethyl acetate (20 mL), 70.1 mg (100%) of a grey solid was obtained. MS (ESI) $(M+H)^+$: 474.06.

**Step D. 2-[(4-{[[2-(1-methoxy-1-methylethyl)-1-(tetrahydro-2*H*-pyran-4-ylmethyl)-1*H*-benzimidazol-5-yl](methyl)amino]sulfonyl}phenyl)amino]-2-oxoethyl acetate**

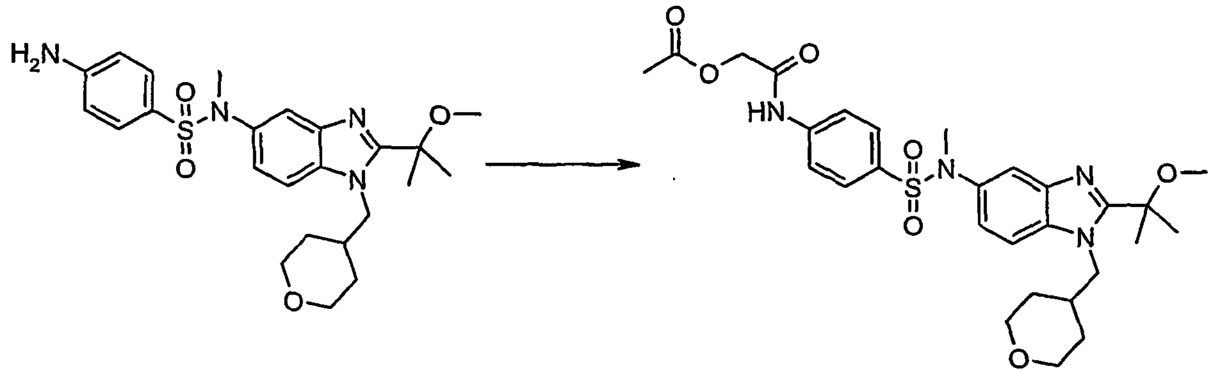

Following the procedure for Example 50, using 4-amino-*N*-[2-(1-methoxy-1-methylethyl)-1-(tetrahydro-2*H*-pyran-4-ylmethyl)-1*H*-benzimidazol-5-yl]-*N*-methylbenzenesulfonamide (70.1 mg, 0.097 mmol), DMAP (35.6 mg, 0.291 mmol) and 2-chloro-2-oxoethyl acetate (26.5 mg, 0.194mmol) in MeCN (6 mL), 100 mg of the crude title product was obtained, which was used directly for next step.

**Example 65**

***N*-(4-{[[2-(1-ethoxy-1-methylethyl)-1-(tetrahydro-2*H*-pyran-4-ylmethyl)-1*H*-benzimidazol-5-yl](methyl)amino] sulfonyl}phenyl)acetanide**

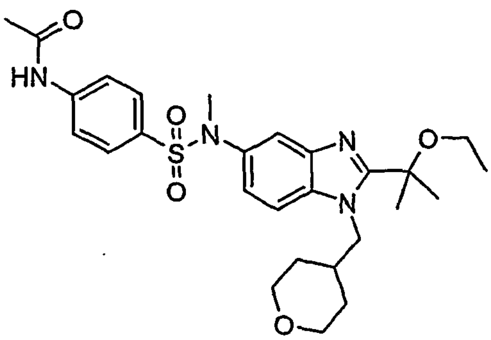

**Step A. *N*-(4-{[[2-(1-ethoxy-1-methylethyl)-1-(tetrahydro-2*H*-pyran-4-ylmethyl)-1*H*-benzimidazol-5-yl](methyl) amino]sulfonyl}phenyl)acetamide**

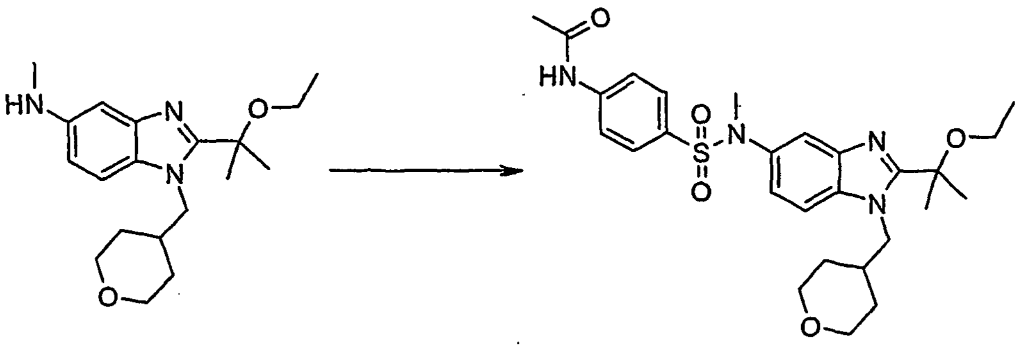

Following the procedure for the step A in Example 46, using 2-(1-ethoxy-1-methylethyl)-*N*-methyl-1-(tetrahydro-2*H*-pyran-4-ylmethyl)-1*H*-benzimidazol-5-amine hydrochloride (96.2 mg, 0.15 mmol) (for preparation, see the following steps B and C), DMAP (73.3 mg, 0.60 mmol) and 4-(acetylamino)benzenesulfonyl chloride (70.0 mg, 0.30 mmol) in MeCN (10 mL), the crude product was purified MPLC using EtOAc/MeOH (20:1) on silica gel to give 46.2 mg (55%) of a white solid as the title compound. $^1$HNMR (400 MHz, $CD_3OD$): δ 1.25 - 1.33 (m, 3 H), 1.43 - 1.64 (m, 4 H), 1.84 (s, 6 H), 2.14 (s, 3 H), 2.35 - 2.51 (m, 1 H,) 3.25 (s, 3 H), 3.31 - 3.41 (m, 2 H), 3.60 (q, J=6.90 Hz, 2 H), 3.95 (m, 2 H), 4.62 (d, J=7.62 Hz, 2 H), 7.31 (dd, J=8.98, 2.15 Hz, 1 H), 7.43 - 7.50 (m, 2 H), 7.52 (d, J=1.76 Hz, 1 H), 7.67 - 7.76 (m, 2 H), 7.87 (d, J=8.79 Hz, 1 H); MS (ESI) $(M+H)^+$: 529.0; Anal. Calcd for $C_{27}H_{36}N_4O_5S$+ 0.7 HCl ++0.7 $H_2O$+0.6 $CH_3OH$ (586.03): C, 56.57, H, 6.97, N, 9.56; Found: C, 56.60; H, 6.96; N, 9.59.

**Step B. *N*-[2-(1-ethoxy-1-methylethyl)-1-(tetrahydro-2*H*-pyran-4-ylmethyl)-1*H*-benzimidazol-5-yl]-*N*-methylacetamide**

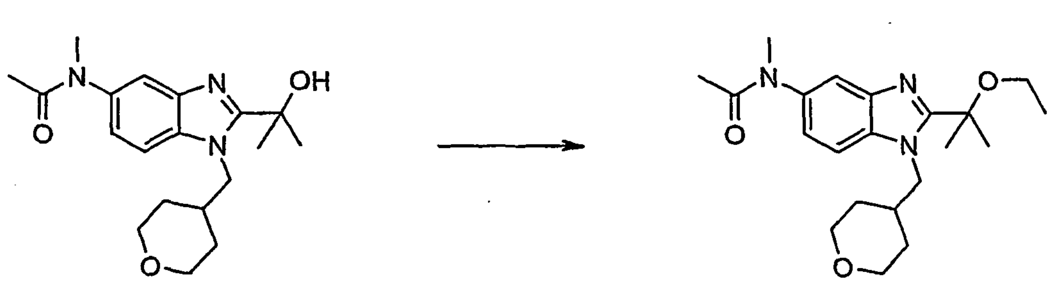

Following the procedure for step C in Example 62, using of *N*-[2-(1-hydroxy-1-methylethyl)-1-(tetrahydro-2*H*-pyran-4-ylmethyl)-1*H*-benzimidazol-5-yl]-*N*-methylacetamide (51.8 mg, 0.15 mmol) (for preparation see the step B in Example 62), sodium hydride (13.5 mg, 60%, 0.33 mmol) and iodoethane (51.5 mg, 0.33 mmol) in THE (5 mL), 73.5 mg (100%) of the title compound was obtained as a colorless syrup. MS (ESI) (M+H)+: 374.04.

**Step C. 2-(1-ethoxy-1-methylethyl)-*N*-methyl-1-(tetrahydro-2*H*-pyran-4-ylmethyl)-1*H*-benzimidazol-5-amine**

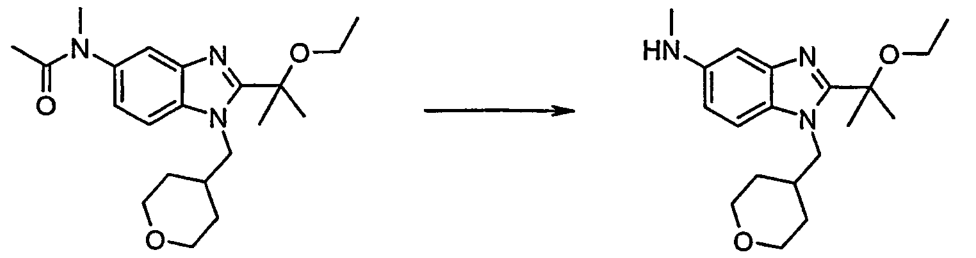

Following the procedure for step D in Example 62, using *N*-[2-(1-ethoxy-1-methylethyl)-1-(tetrahydro-2*H*-pyran-4-ylmethyl)-1*H*-benzimidazol-5-yl]-*N*-methylacetamide of (73.5 mg, 0.15 mmol) in 5 mL of EtOH-2*N* HCl (3:2), 96.32 mg (100%) of a grey solid was obtained as the title product. MS (ESI) (M+H)+: 332.02.

**Example 66**

***N*-[4-({[1-(2-azetidin-1-ylethyl)-2-*tert*-butyl-1*H*-benzimidazol-5-yl]amino}sulfonyl)phenyl]acetamide**

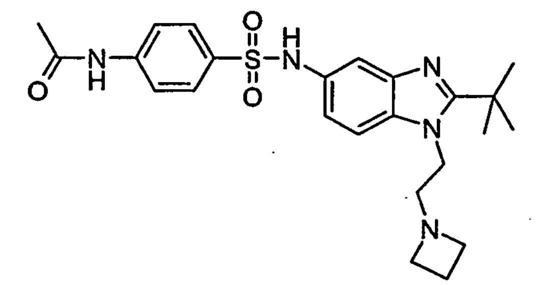

**Step A. *N*-[4-({[1-(2-azetidin-1-ylethyl)-2-*tert*-butyl-1*H*-benzimidazol-5-yl]amino}sulfonyl)phenyl]acetamide**

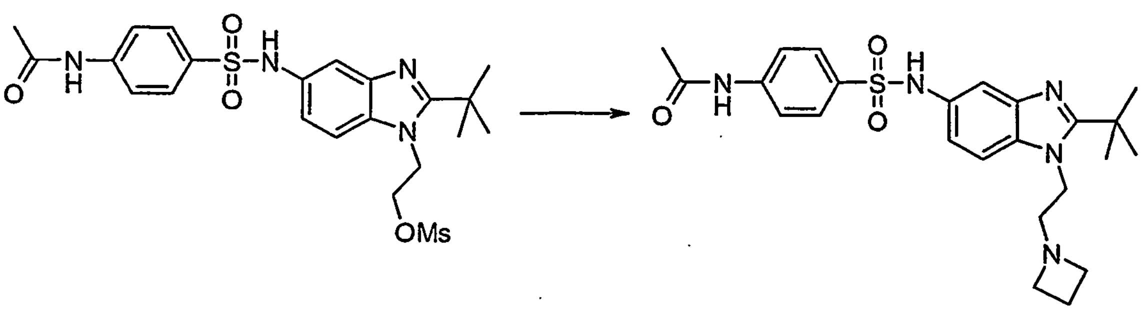

2-[5-({[4-(acetylamino)phenyl]sulfonyl}amino)-2-*tert*-butyl-1*H*-benzimidazol-1-yl]ethyl methanesulfonate (0.10

g, 0.196 mmol), azetidine (0.20 g, 3.93 mmol) and KI (0.65 g, 0.393 mmol) were mixed together and heated to 80°C in DMF (3 mL). The solvent was concentrated. The crude product was purified by preparative reverse-phase HPLC using 10-90% $CH_3CN/H_2O$ as eluent and then lyophilized to give the title compound as the corresponding TFA salt. Yield: 30 mg (26%); MS (ESI) $(M+H)^+$: 470.0.

**Step B. *N*-(4-{[(4-fluoro-3-nitrophenyl)amino]sulfonyl}phenyl)acetamide**

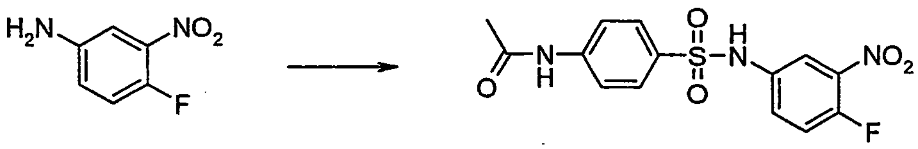

4-Fluoro-3-nitroaniline (6.22 g, 39.8 mmol) and 4-(acetylamino)benzenesulfonyl chloride (10.2 g, 43.8 mmol) were heated to 50°C for 20hrs. in pyridine (70 mL). The solvent was concentrated. The crude product was recovered in DCM and washed with water, 2N HCl, saturated $NaHCO_3$ solution, water and brine. The organic layer was dried over anhydrous $MgS0_4$. The solvent was concentrated giving the title compound that was used for the next step without further purification. Yield: 10 g (70%); MS (ESI) $(M+H)^+$: 354.0.

**Step C. *N*-{4-[({4-[(2-hydroxyethyl)amino]-3-nitrophenyl}amino)sulfonyl]phenyl}acetamide**

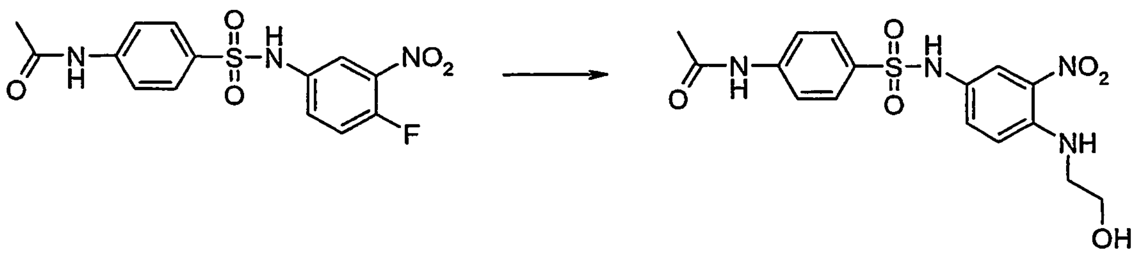

*N*-(4-{[(4-fluoro-3-nitrophenyl)amino]sulfonyl}phenyl)acetamide (2.91g, 8.23 mmol), 2-aminoethanol (3.00 mL, 49.4 mmol) and pyridine (1.33 mL, 16.4 mmol) in DMSO (30 mL) were heated at 90°C for 40 min. The reaction mixture was cooled down to room temperature, and was poured into water (250 mL) at 0°C. The dark-purple mixture was acidified with concentrated HCl until red color appears. The compound was extracted with EtOAc (3X). The combined organic layers were washed with brine and dried over anhydrous $MgSO_4$. The solvent was concentrated giving the title compound that was used for the next step without further purification. Yield: 3.25 g (99%); MS (ESI) $(M+H)^+$: 395.2.

**Step D. *N*-{4-[({4-[(2-{[*tert*-butyl(dimethyl)siyl]oxy}ethyl)amino]-3-nitrophenyl}amino)sulfonyl]phenyl}acetamide**

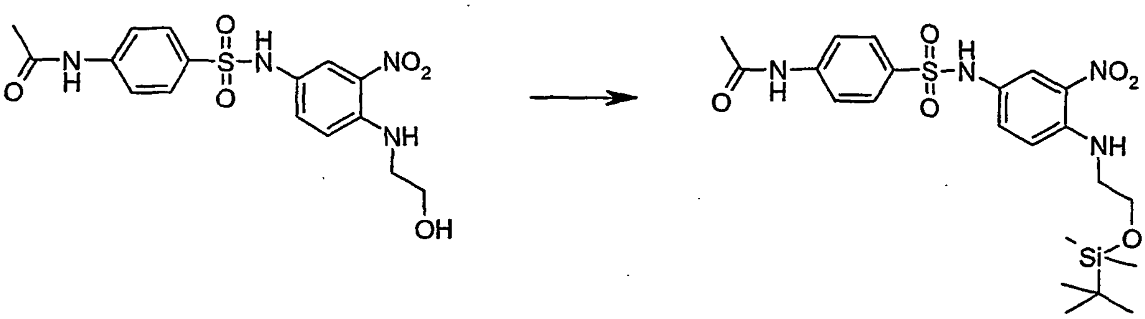

A solution of TBDMSCl (1.36g, 9.05 mmol) in EtOAc (100 mL) was slowly added to a solution of *N*-{4-[({4-[(2-hydroxyethyl)amino]-3-nitrophenyl}amino)sulfonyl]phenyl} acetamide (3.25 g, 8.23 mmol) and imidazole (0.67 g, 9.88 mmol) in EtOAc (250 mL) at room temperature. The reaction mixture was stirred overnight. The reaction was quenched with water and washed with saturated $NH_4Cl$ solution, water and brine. The organic layer was dried over anhydrous $MgSO_4$. The solvent was concentrated. The crude product was purified by flash chromatography on silica gel using EtOAc/Hex (3:1) as eluent to give the title compound. Yield: 3.39 g (81%); MS (ESI) $(M+H)^+$: 509.1.

**Step E. *N*-{4-[({3-amino-4-[(2-{[*tert*-butyl(dimethyl)silyl]oxy}ethyl)amino]phenyl}amino)sulfonyl]phenyl}acetamide**

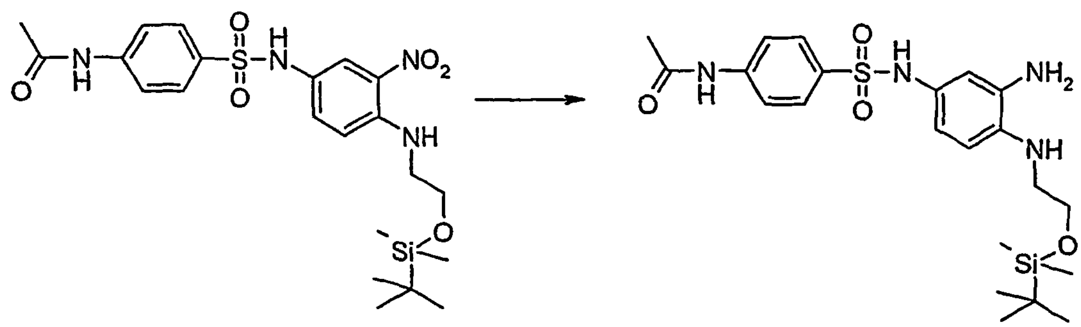

*N*-{4-[({4-[(2-{[*tert*-butyl(dimethyl)silyl]oxy}ethyl)amino]-3-nitrophenyl}amino)sulfonyl]phenyl}acetamide (3.27 g, 6.42 mmol) was hydrogenated in EtOAc (200 mL) catalyzed by 10% Pd/C at 50 psi $H_2$ in Parr shaker overnight at room temperature. The mixture was filtered through a celite pad. The solvent was concentrated giving the title compound that was used for the next step without further purification. Yield: 3.05 g (99%); MS (ESI) $(M+H)^+$: 479.1.

**Step F. *N*-{5-({[4-(acetylamino)phenyl]sulfonyl}amino)-2-[(2-{[*tert*-butyl(dimethyl)silyl]oxy}ethyl)amino]phenyl}-2,2-dimethylpropanamide**

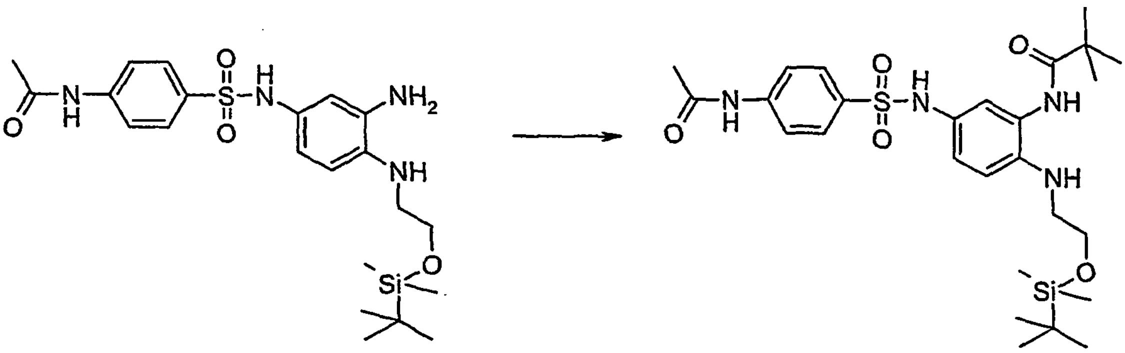

A solution of tBuCOCl (0.85 mL, 7.20 mmol) in DCM (50 mL) was added dropwise to a solution of *N*-{4-[({3-amino-4-[(2-{[*tert*-butyl(dimethyl)silyl]oxy}ethyl)amino]phenyl}amino)sulfonyl]phenyl}acetamide (3.05 g, 6.37 mmol) and $Et_3N$ (1.20 mL, 8.60 mmol) in DCM (250 mL) at 0°C. The reaction mixture was stirred for 1.5 hours and washed with water (3X), saturated $NaHCO_3$ solution, water and brine. The organic layer was dried over anhydrous $MgSO_4$. The solvent was concentrated giving the title compound that was used for the next step without further purification. Yield: 3.59 g (99%); MS (ESI) $(M+H)^+$: 563.0.

**Step G. 2-[5-({[4-(acetylamino)phenyl]sulfonyl}amino)-2-*tert*-butyl-1*H*-benzimidazol-1-yl]ethyl acetate**

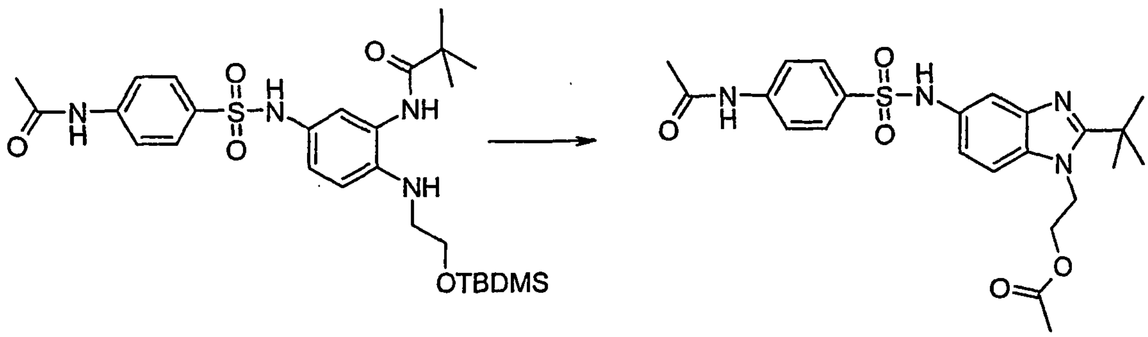

*N*-{5-({[4-(acetylamino)phenyl]sulfonyl}amino)-2-[(2-{[*tert*-butyl(dimethyl)silyl] oxy]ethyl)amino]phenyl}-2,2-dimethylpropanamide (2.87g, 5.10 mmol) was dissolved in glacial acetic acid (40 mL) and heated to 170°C in a microwave oven for 15 min. The solvent was concentrated. The crude compound was purified on silica gel by flash chromatography using EtOAc as eluent. The fractions containing the desired material were purified by preparative reverse-phase HPLC using 10-90% $CH_3CN/H_2O$ as eluent and then lyophilized to give the title compound as the corresponding TFA salt. Yield: 260 mg (8%); $^1H$ NMR (400 MHz, $CD_3OD$): δ 1.64 (s, 9 H), 1.83 (s, 3 H), 2.10 (s, 3 H), 4.53 (t, J=5.66 Hz, 2 H), 4.83 - 4.90 (m, 2 H), 7.27 (dd, J=9.08, 2.05 Hz, 1 H), 7.60 (d, J=1.95 Hz, 1 H), 7.62 - 7.73 (m, 4 H), 7.79 (d, J=8.98 Hz, 1 H); MS (ESI) $(M+H)^+$: 473.0; Anal. Calcd. for $C_{23}H_{28}N_4O_5S$ + 1.30 TFA: C, 49.53; H, 4.76; N, 9.02. Found: C, 49.51; H, 4.43; N, 9.10.

**Step H. *N*-[4-({[2-*tert*-butyl-1-(2-hydroxyethyl)-1*H*-benzimidazol-5-yl]amino}sulfonyl)phenyl]acetamide**

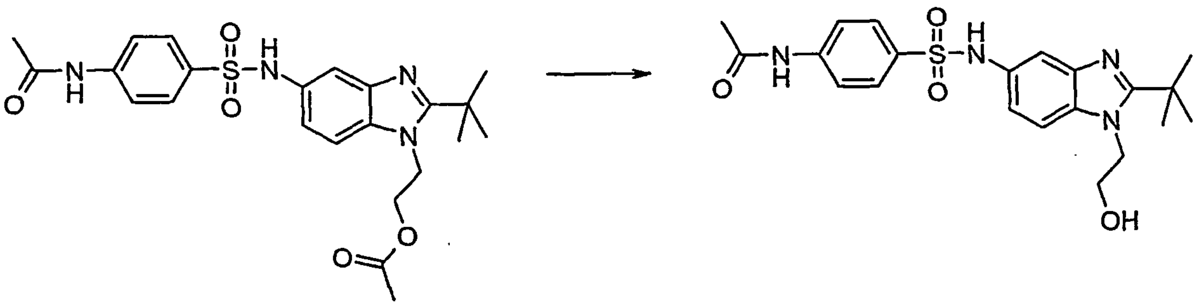

To 2-[5-({[4-(acetylamino)phenyl]sulfonyl}amino)-2-*tert*-butyl-1*H*-benzimidazol-1-yl]ethyl acetate (0.20 g, 0.423 mmol) in water (50 mL) was added 2N NaOH (5 mL) at 0°C. The reaction mixture was allowed to warm to room temperature and stirred for 2hrs. The reaction mixture was neutralized with concentrated HCl at 0°C until precipitation occurs. The product was extracted with EtOAc (3X). The combined organic layers were dried over anhydrous $Na_2SO_4$ and filtered. The solvent was concentrated giving the pure title compound. Yield: 184 mg (99%), MS (ESI) $(M+H)^+$: 431.0.

**Step I. 2-[5-({[4-(acetylamino)phenyl]sulfonyl}amino)-2-*tert*-butyl-1*H*-benzimidazol-1-yl]ethyl methanesulfonate**

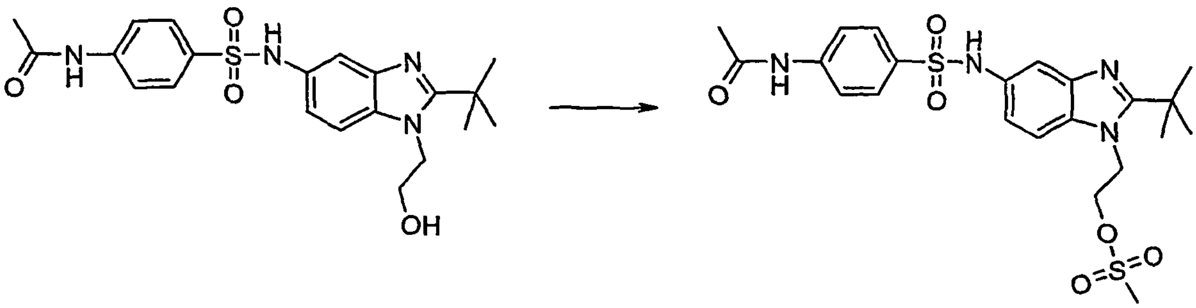

Methane sulfonyl chloride (0.36 mL, 0.47 mmol) was added to a solution of *N*-[4-({[2-*tert*-butyl-1-(2-hydroxyethyl)-1*H*-benzimidazol-5-yl]amino}sulfonyl)phenyl]acetamide (0.18 g, 0.42 mmol) and $Et_3N$ (0.90 mL, 0.64 mmol) in a 1:1 mixture of EtOAc:DCM (120 mL) at 0°C. The reaction mixture was allowed to warm to room temperature and stirred for 3hrs. The solvent was concentrated and the crude product was recovered in EtOAc (150 mL). The organic phase was washed with water, saturated $NaHCO_3$ solution, water and brine. The solution was dried over anhydrous $MgSO_4$ and filtered. The solvent was concentrated giving the pure title compound. Yield: 205 mg (94%); MS (ESI) $(M+H)^+$: 509.1.

**Example 67**

**3-[5-({[4-(acetylamino)phenyl]sulfonyl}amino)-2-*tert*-butyl-1*H*-benzimidazol-1-yl]propyl acetate**

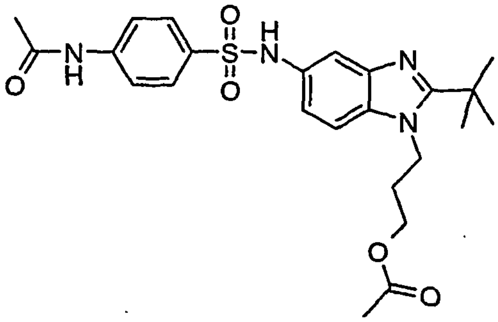

**Step A. 3-[5-({[4-(acetylamino)phenyl]sulfonyl}amino)-2-*tert*-butyl-1*H*-benzimidazol-1-yl]propyl acetate**

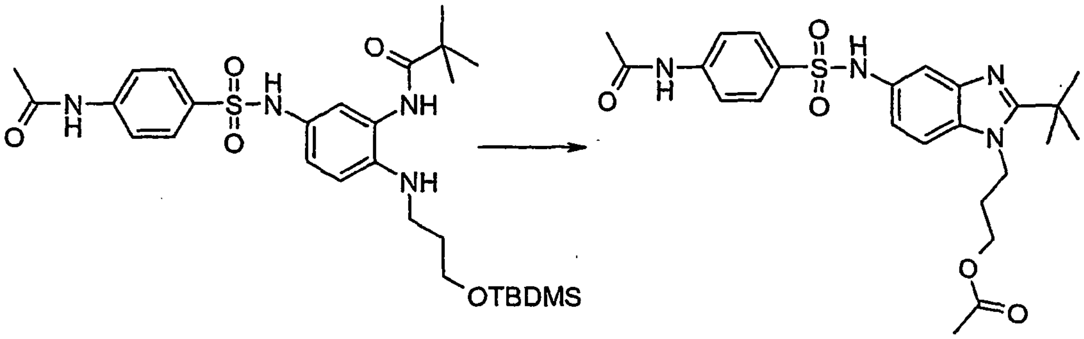

*N*-{5-({[4-(acetylamino)phenyl]sulfonyl}amino)-2-[(3-{[*tert*-butyl(dimethyl)silyl] oxy}propyl)amino]phenyl}-2,2-dimethylpropanamide (2.56 g, 4.44 mmol) (for preparation, see Example 66, Steps B to F) was dissolved in acetic acid (150 mL) and heated to 80°C overnight. The solvent was concentrated. The residue was recovered in EtOAc and washed with saturated $NaHCO_3$ solution, water and brine. The organic layer was dried with anhydrous $MgSO_4$, filtered and concentrated. The crude compound was purified on silica gel by flash chromatography using MeOH 1% to 5% in EtOAc as eluent. The fractions containing the desired material were purified by preparative reverse-phase HPLC using 10-90% $CH_3GN/H_2O$ as eluent and then lyophilized to give the title compound as the corresponding TFA salt. Yield: 60 mg (2%); $^1H$ NMR (400 MHz, $CD_3OD$): δ 1.63 (s, 9 H), 2.04 (s, 3 H), 2.10 (s, 3 H), 2.20 - 2.31 (m, 2 H), 4.25 (t, J=5.86 Hz, 2 H), 4.59 - 4.69 (m, 2 H), 7.28 (dd, J=8.88, 2.05 Hz, 1 H), 7.58 - 7.67 (m, 3 H), 7.67 - 7.75 (m, 3 H); MS (ESI) $(M+H)^+$: 487.0; Anal. Calcd. for $C_{24}H_{30}N_4O_5S$ + 1.30 TFA: C, 50.33; H, 4.97; N, 8.83. Found: C, 50.42; H, 5.10; N, 8.66.

**Step B. *N*-{4-[({4-[(3-hydroxypropyl)amino]-3-nitrophenyl}amino)sulfonyl]phenyl}acetamide**

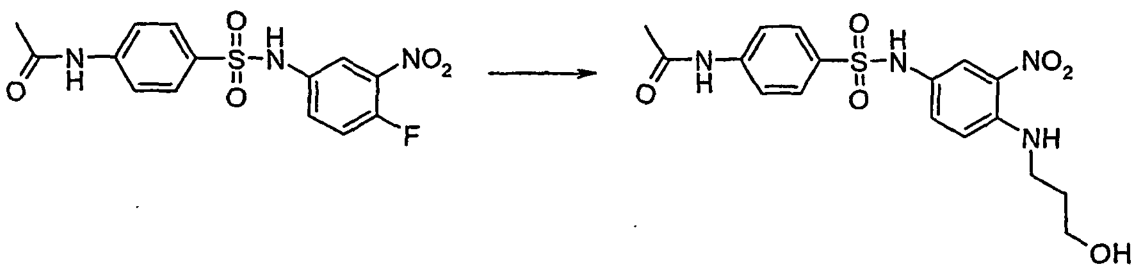

*N*-(4-{[(4-fluoro-3-nitrophenyl)amino]sulfonyl}phenyl)acetamide (4.00 g, 11.3 mmol) (for preparation, see Example 66, Step B), 3-aminopropanol (4.25 g, 56.6 mmol) and pyridine (1.83 mL, 22.6 mmol) in DMSO (50 mL) were

heated to 80°C overnight. The room temperature cooled down reaction mixture was poured in water (400 mL) at 0°C. The dark-purple mixture was acidified with concentrated HCl until red color appears. The compound was extracted with EtOAc (3X). The combined organic layers were washed with brine and dried over anhydrous $MgSO_4$. The solvent was concentrated. The crude product was purified by flash chromatography on silica gel, using EtOAc as eluent, giving the title compound. Yield: 1.67 g (36%); MS (ESI) $(M+H)^+$: 409.4.

**Step C. *N*-{4-[({4-[(3-{[*tert*-butyl(dimethyl)silyl]oxy}propyl)amino]-3-nitrophenyl}amino)sulfonyl]phenyl}acetamide**

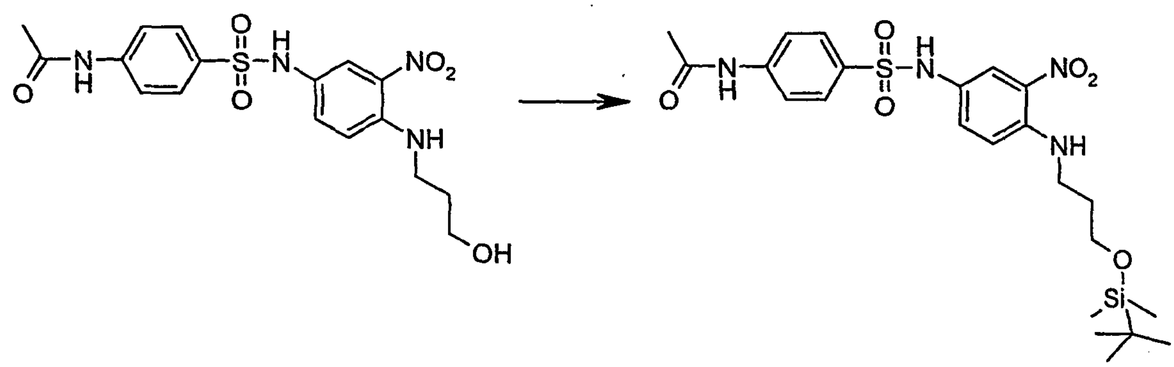

A solution of TBDMSCl (0.74 g, 4.90 mmol) in EtOAc (50 mL) was slowly added to a solution of *N*-{4-[({4-[(3-hydroxypropyl)amino]-3-nitrophenyl}amino)sulfonyl]phenyl}acetamide (1.67 g, 4.08 mmol) and imidazole (0.36 g, 5.31 mmol) in EtOAc (200 mL) at room temperature. The reaction mixture was stirred overnight. The reaction was quenched with water and washed with $NH_4Cl$ saturated solution, water and brine. The organic layer was dried over anhydrous $MgS0_4$. The solvent was concentrated. The crude product was purified by flash chromatography on silica gel, using EtOAc/Hex (3:1) as eluent, giving the title compound. Yield: 2.06 g (99%); MS (ESI) $(M+H)^+$: 523.8.

**Step E. *N*-{4-[({3-amino-4-[(3-{[*tert*-butyl(dimethyl)silyl]oxy}propyl)amino]phenyl}amino)sulfonyl]phenyl} acetamide**

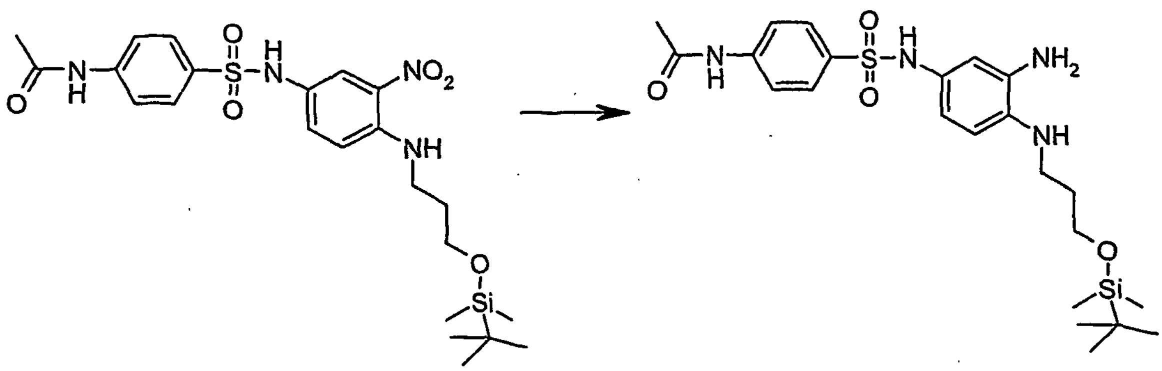

*N*-{4-[({4-[(3-{[*tert*-butyl(dimethyl)silyl]oxy}propyl)amino]-3-nitrophenyl}amino)sulfonyl]phenyl}acetamide (2.06 g, 3.94 mmol) was hydrogenated in EtOAc (200 mL) catalyzed by 10% Pd/C at 50 psi $H_2$ in Parr shaker overnight at room temperature. The mixture was filtered over a celite pad. The solvent was concentrated giving the title compound that was used for the next step without further purification. Yield: 1.78 g (91%); MS (ESI) $(M+H)^+$: 493.6.

**Step F. *N*-{5-({[4-(acetylamino)phenyl]sulfonyl}amino)-2-[(3-{[*tert*-butyl(dimethyl)silyl]oxy}propyl)amino]phenyl}-2,2-dimethylpropanamide**

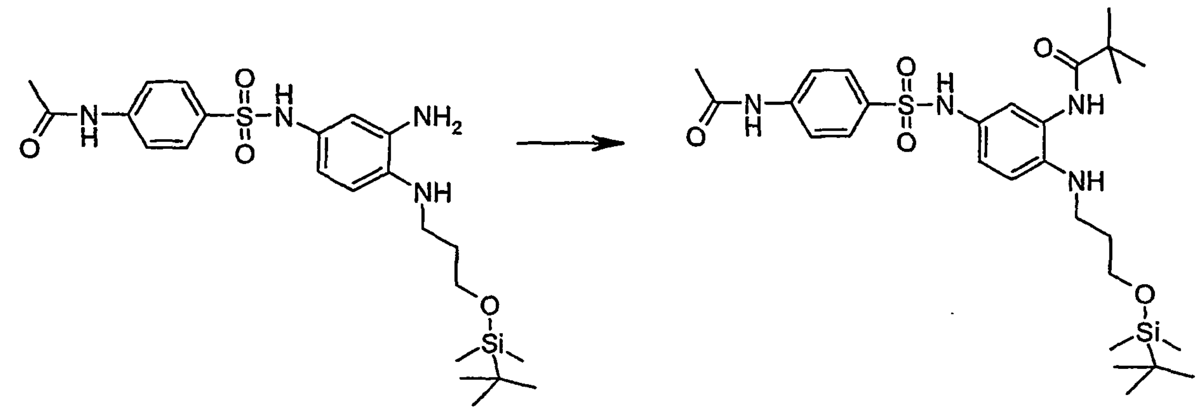

tBuCOCl (0.44 mL, 3.61 mmol) was added to a solution of *N*-{4-[({3-amino-4-[(3-{[*tert*-butyl(dimethyl)silyl]oxy} propyl)amino]phenyl}amino)sulfonyl]phenyl} acetamide (1.78 g, 3.61 mmol) and $Et_3N$ in DCM at 0°C. The reaction mixture was stirred for 3hrs. at 0°C. The reaction was quenched with saturated $NaHCO_3$ solution. The organic layer was washed with water, brine and dried over anhydrous $MgSO_4$. The solvent was concentrated giving the title compound that was used for the next step without further purification. Yield: 2.06 g (98%); MS (ESI) $(M+H)^+$: 577.9.

**Example 68**

***N*-{4-[({1-[(1*S*,4*S*)-bicyclo[2.2.1]hept-5-en-2-ylmethyl]-2-*tert*-butyl-1*H*-benzimidazol-5-yl}amino)sulfonyl]phenyl}acetamide**

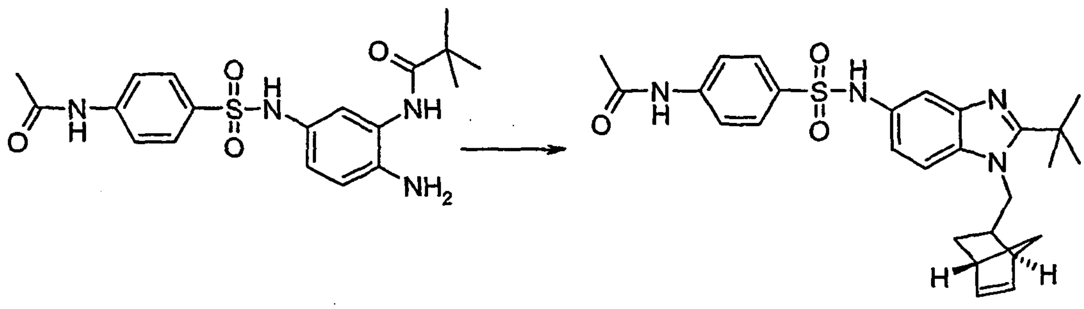

*N*-[5-({[4-(acetylamino)phenyl]sulfonyl}amino)-2-amino phenyl]-2,2-dimethyl propanamide (200 mg, 0.494 mmol) and (1*S*, 4S)-bicyclo[2.2.1]hept-5-ene-2-carbaldehyde (60 mg, 0.494 mmol) were mixed together in acetic acid (15 mL). The reaction mixture was heated to 50°C and stirred for 1 hr. $Na(BH_3)CN$ (31 mg, 0.494 mmol) was added to the warm solution and stirred for one extra hour (50°C). The reaction mixture was heated to 100°C for 3 days (typically overnight). The solvent was concentrated. The crude product was purified by preparative reverse-phase HPLC using 10-90% $CH_3CN/H_2O$ as eluent and then lyophilized to give the title compound as the corresponding TFA salt. Yield: 30 mg (10%); $^1H$ NMR (400 MHz, $CD_3OD$): δ 0.80 - 0.90 (m, 1 H), 1.26 - 1.36 (m, 1 H), 1.37 - 1.45 (m, 1 H), 1.45 - 1.53 (m, 1 H), 1.63 (d, J=6.83 Hz, 9 H), 1.79 - 1.90 (m, 1 H), 2.10 (s, 3 H), 2.11 - 2.20 (m, 1 H), 2.57 (s, 1 H), 2.85 (s, 1 H), 2.93 (s, 1 H), 4.13 - 4.44 (m, 1 H), 4.45 - 4.73 (m, 1 H), 5.91 - 6.15 (m, 1 H), 6.24 - 6.46 (m, , 1 H), 7.26 (td, J=8.98, 2.15 Hz, 1 H), 7.62 (dd, J=4.78, 2.05 Hz, 1 H), 7.63 - 7.76 (m, 5 H); MS (ESI) $(M+H)^+$: 493.0; Anal. Calcd. for $C_{27}H_{32}N_4O_3S$ + 1.70 TFA + 1.00 $H_2O$ + 0.50 MeCN: C, 52.02; H, 5.17; N, 8.69. Found: C, 52.01; H, 5.13; N, 8.66.

**Step B. *N*-(2-amino-5-nitrophenyl)-2,2-dimethylpropanamide**

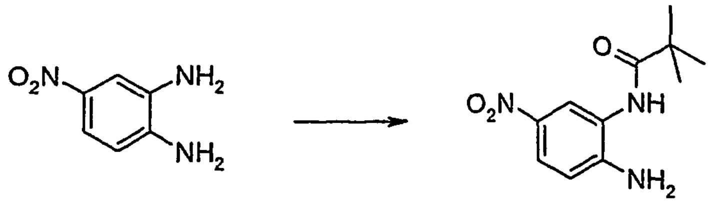

tBuCOCl (10.9 mL, 88.5 mmol) was added to a mixture of 2-amino-4-nitroaniline (13.5 g, 88.5 mmol) and pyridine (7.50 mL, 92.9 mmol) in DCM (600 mL) at 0°C. The reaction mixture was slowly allowed to warm to room temperature and stirred for 4hrs. The reaction mixture was washed with water, 0.1 N HCl, brine and dried over anhydrous $MgSO_4$. The volume of the organic layer was reduced to 200 mL. The resulting precipitate was filtered off and washed with small amount of cold DCM. The operation was repeated 3 times, giving the title compound as a pale-yellow solid. Yield: 9.35 g (44%); MS (ESI) $(M+H)^+$: 238.2.

**Step C. *N*-(2,5-diaminophenyl)-2,2-dimethylpropanamide**

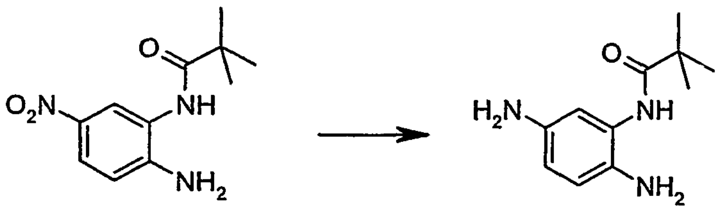

*N*-(2-amino-5-nitrophenyl)-2,2-dimethylpropanamide (9.35 g, 39.4 mmol) was hydrogenated in EtOAc (400 mL) catalyzed by 10% Pd/C at 40 psi $H_2$ in Parr shaker overnight at room temperature. The mixture was filtered through a celite pad. The solvent was concentrated giving the title compound that was used for the next step without further purification. Yield: 8.12 g (99%); MS (ESI) $(M+H)^+$: 208.2.

**Step D. *N*-[5-({[4-(acetylamino)phenyl]sulfonyl}amino)-2-aminophenyl]-2,2-dimethylpropanamide**

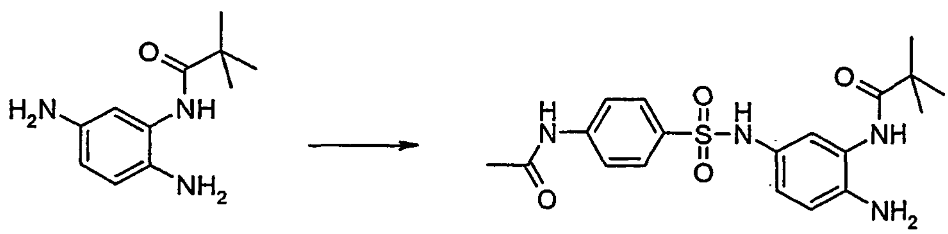

4-(Acetylamino)benzenesulfonyl chloride (4.89 g, 20.9 mmol) was added by portion (over 3hrs) to a solution of *N*-(2,5-diaminophenyl)-2,2-dimethylpropanamide (4.34 g, 20.9 mmol) and pyridine (20 mL) in MeCN (600 mL). During the addition, the reaction temperature was maintained between -30 and -40°C. The reaction mixture was allowed to warm to room temperature and stirred for 1hr. The solvent was concentrated. The residue was triturated in $Et_2O$ and filtered. The resulting solid was stirred in water (200 mL), filtered and air-dried giving the title compound that was used for the next step without further purification. Yield: 6.2 g (73%); MS (ESI) $(M+H)^+$: 405.1.

**Example 69**

***N*-[4-({[2-*tert*-butyl-1-(tetrahydro-2*H*-pyran-3-ylmethyl)-1*H*-benzimidazol-5-yl]amino}sulfonyl)phenyl]acetamide**

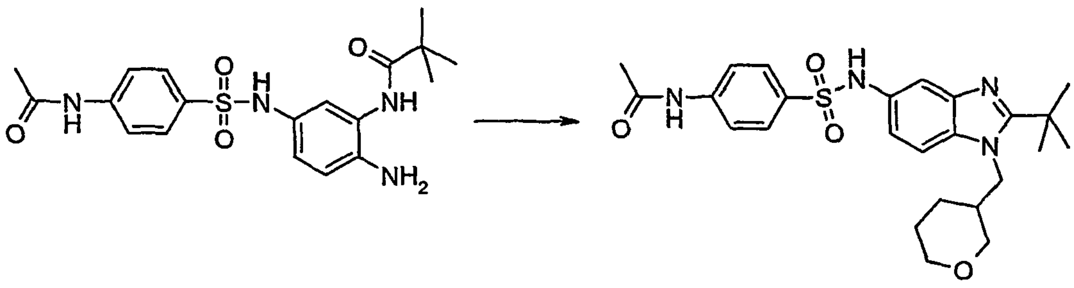

*N*-[5-({[4-(acetylamino) phenyl] sulfonyl} amino) -2-amino phenyl] -2,2- dimethyl propanamide (73 mg, 0.18 mmol) and tetrahydro-2*H*-pyran-3-carbaldehyde (31 mg, 0.27 mmol) were stirred together at room temperature for 1hr. in a 2:1 mixture of DCE and acetic acid (3 mL). Borane-pyridine complex (45 μL, 0.36 mmol) and concentrated HCl (5 drops) were added to the reaction mixture. The reaction mixture was heated to 95°C overnight. The solvent was concentrated. The crude product was purified by preparative reverse-phase HPLC using 10-90% $CH_3CN/H_2O$ as eluent and then lyophilized to give the title compound as the corresponding TFA salt. Yield: 25 mg (23%); $^1H$ NMR (400 MHz, $CD_3OD$): δ 1.26 -1.31 (m, 1 H), 1.50 - 1.61 (m, 1 H), 1.63 (s, 9 H), 1.67 - 1.77 (m, 2 H), 1.77 - 1.87 (m, 1 H), 2.10 (s, 3 H), 2.24 - 2.34 (m, 1 H), 3.39 (dd, J=11.62, 7.71 Hz, 1 H), 3.55 - 3.62 (m, 1 H), 3.66 (dd, J=11.52, 2.93 Hz, 1 H), 3.71 - 3.80 (m, 1 H), 4.38 (dd, J=15.04, 6.05 Hz, 1 H), 4.59 (dd, J=15.04, 8.98 Hz, 1 H), 7.24 (dd, J=8.98, 1.95 Hz, 1 H) ,7.61 (d, J=2.15 Hz, 1 H), 7.63 - 7.68 (m, 2 H), 7.69 - 7.74 (m, 2 H), 7.76 (d, J=8.98 Hz, 1 H); MS (ESI) $(M+H)^+$: 485.0; Anal. Calcd. for $C_{25}H_{32}N_4O_4S$ + 1.50 TFA: C, 51.29; H, 5.15; N, 8.55. Found: C, 51.43; H, 5.22; N, 8.11.

**Example 70**

***N*-[4-({[2-*tert*-butyl-1-(tetrahydrofuran-3-ylmethyl)-1*H*-benzimidazol-5-yl]amino}sulfonyl)phenyl]acetamide**

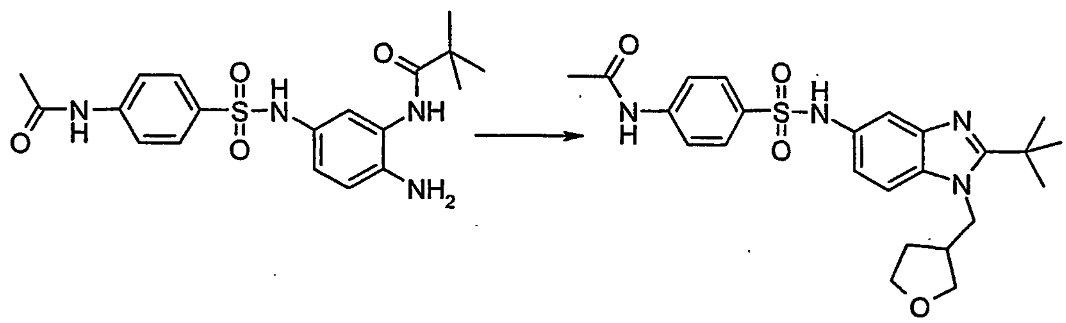

*N*-[5-({[4-(acetylamino) phenyl] sulfonyl} amino) -2-amino phenyl] -2,2- dimethyl propanamide (113 mg, 0.27 mmol), 50% tetrahydrofuran-3-carbaldehyde aqueous solution (60 mg, 0.27 mmol) and concentrated HCl (4 drops) were stirred together at room temperature in a 1:1 mixture of water and MeCN (15 mL). Borane-pyridine complex (69 μL, 0.55 mmol) was added and the reaction mixture was heated to 95°C overnight. The solvent was concentrated. The crude product was purified by preparative reverse-phase HPLC using 10-90% $CH_3CN/H_2O$ as eluent and then lyophilized to give the title compound as the corresponding TFA salt. Yield: 25 mg (15%); $^1H$ NMR (400 MHz, $CD_3OD$): δ 1.63 (s, 9 H), 1.74 -1.85 (m, 1 H), 2.04 - 2.16 (m, 4 H), 2.98 (dd, J=7.91, 5.37 Hz, 1 H), 3.60 (d, J=5.47 Hz, 2 H), 3.68 - 3.78 (m, 1 H), 3.95 - 4.04 (m, 1 H), 4.53 (d, J=7.81 Hz, 2 H), 7.25 (dd, J=8.98, 2.15 Hz, 1 H), 7.59 - 7.68 (m, 3 H), 7.68 - 7.73 (m, 2 H), 7.76 (d, J=8.98 Hz, 1 H); MS (ESI) $(M+H)^+$: 471.0; Anal. Calcd. for $C_{24}H_{30}N_4O_4S$ + 1.40 TFA + 0.10 $H_2O$ + 0.10

MeCN: C, 50.98; H, 5.05; N, 9.03. Found: C, 51.01; H, 4.79; N, 9.01.

**Example 71**

***N*-{4-[({2-*tert*-butyl-1-[2-(tetrahydro-2*H*-pyran-4-yl)ethyl]-1*H*-benzimidazol-5-yl}amino)sulfonyl]phenyl}acetamide**

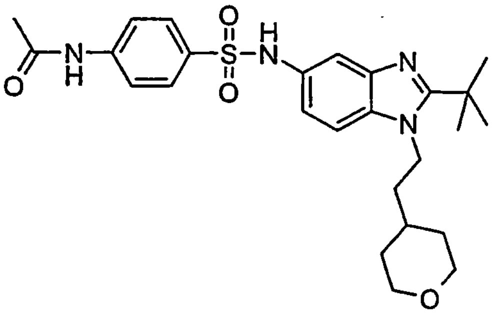

**Step A. *N*-{4-[({2-*tert*-butyl-1-[2-(tetrahydro-2*H*-pyran-4-yl)ethyl]-1*H*-benzimidazol-5-yl}amino)sulfonyl]phenyl} acetamide**

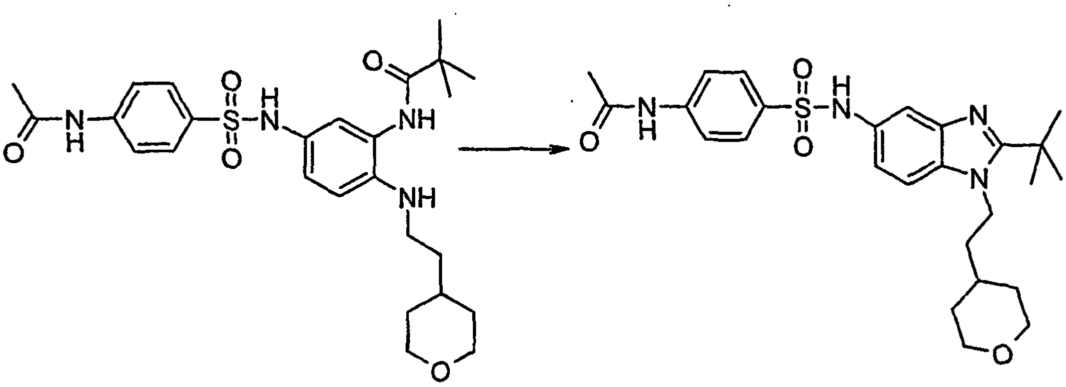

*N*-(5-({[4-(acetylamino) phenyl] sulfonyl} amino)-2-{[2-(tetrahydro-2*H*-pyran-4-yl) ethyl] amino} phenyl)-2,2-dimethylpropanamide (95 mg, 0.183 mmol) in acetic acid was heated to 100°C (10 mL) overnight. The solvent was concentrated. The crude product was purified by preparative reverse-phase HPLC using 10-90% $CH_3CN/H_2O$ as eluent and then lyophilized to give the title compound as the corresponding TFA salt. Yield: 50 mg (44%); $^1H$ NMR (400 MHz, $CD_3OD$): δ 1.32 - 1.48 (m, 1 H),1.62 (s, 9 H), 1.75 (d, J=12.89 Hz, 2 H), 1.79 - 1.89 (m, 2 H), 2.10 (s, 3 H), 3.40 - 3.51 (m, 4 H), 3.95 (dd, J=11.81, 4.00 Hz, 2 H), 4.52 - 4.60 (m, 2 H), 7.28 (dd, J=8.98, 2.15 Hz, 1 H), 7.60 - 7.65 (m, 2 H), 7.65 - 7.68 (m, 2 H),7.68 - 7.74 (m, 2 H); MS (ESI) $(M+H)^+$: 499.0; Anal. Calcd. for $C_{26}H_{34}N_4O_4S$ + 1.60 TFA + 0.20 $H_2O$: C, 51.22; H, 5.30; N, 8.18. Found: C, 51.29; H, 5.26; N, 8.13.

**Step B. *N*-(5-({[4-(acetylamino)phenyl]sulfonyl}amino)-2-{[2-(tetrahydro-2*H*-pyran-4-yl)ethyl]amino}phenyl)-2,2-dimethylpropanamide**

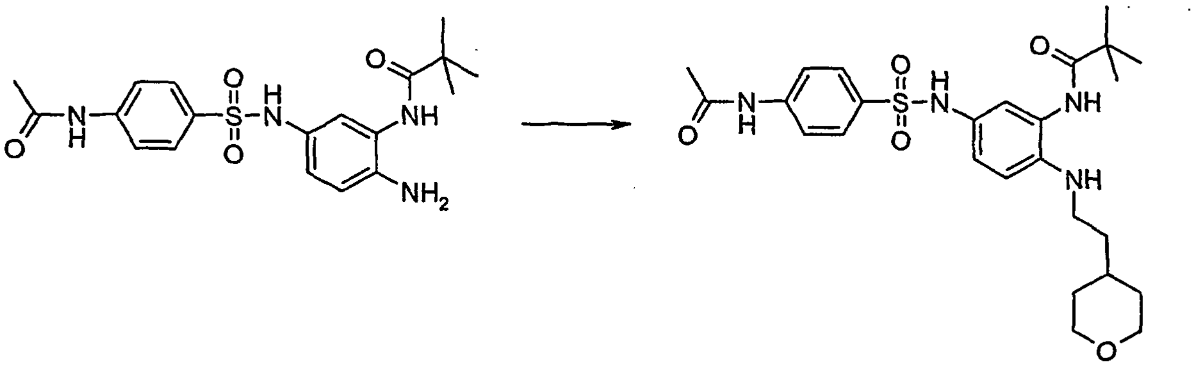

$N$-[5-({[4-(acetylamino) phenyl] sulfonyl} amino) -2-amino phenyl] -2,2- dimethyl propanamide (90 mg, 0.22 mmol) (for preparation, see Example 68, steps B to D) and tetrahydro-2*H*-pyran-4-ylacetaldehyde (85 mg, 0.66 mmol) were stirred together in acetic acid (8 mL) at 70°C for 1hr. The reaction mixture was cooled to room temperature and $Na(BH)_3CN$ (30 mg, 0.44 mmol) was added. The reaction was stirred overnight at room temperature. The solvent was concentrated. The crude product was purified by preparative reverse-phase HPLC using 10-90% $CH_3CN/H_2O$ as eluent and then lyophilized to give the title compound as the corresponding TFA salt. Yield: 95 mg (69%); MS (ESI) $(M+H)^+$: 517.6.

**Example 72**

***N*-(4-{[[2-*tert*-butyl-1-(cyclobutylmethyl)-1*H*-benzimidazol-5-yl](methyl)amino]sulfonyl}phenyl)acetamide**

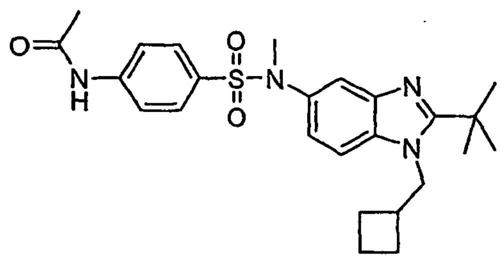

**Step A. *N*-(4-{[[2-*tert*-butyl-1-(cyclobutylmethyl)-1*H*-benzimidazol-5-yl](methyl)amino]sulfonyl}phenyl)acetamide**

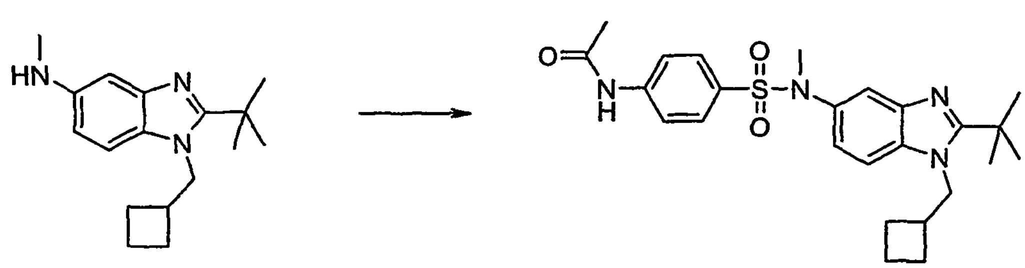

4-(acetylamino)benzenesulfonyl chloride (55 mg, 0.2 mmol) was added to a solution of 2-*tert*-butyl-1-(cyclobutylmethyl)-*N*-methyl-1*H*-benzimidazol-5-amine (53 mg, 0.19 mmol) and DMAP (48 mg, 0.39 mmol) in MeCN (5 mL) at 70°C. The reaction mixture was stirred for 1 hr. and allowed to cool to room temperature. The solvent was concentrated. The crude product was purified by preparative reverse-phase HPLC using 10-90% $CH_3CN/H_2O$ as eluent and then lyophilized to give the title compound as the corresponding TFA salt. Yield: 82 mg (71%); $^1H$ NMR (400 MHz, $CD_3OD$):

δ 1.65 (s, 9 H), 1.83 - 1.97 (m, 3 H), 2.04 - 2.13 (m, 3 H), 2.14 (s, 3 H), 2.79 - 2.93 (m, 1 H), 3.25 (s, 3 H), 4.64 (d, J=6.64 Hz, 2 H), 7.31 (dd, J=9.08, 2.05 Hz, 1 H), 7.41 - 7.49 (m, 2 H), 7.52 (d, J=1.95 Hz, 1 H), 7.71 (d, J=8.79 Hz, 2 H), 7.80 (d, J=8.98 Hz, 1 H); MS (ESI) $(M+H)^+$: 469.0.

**Step B. *N*-(4-fluoro-3-nitrophenyl)acetamide**

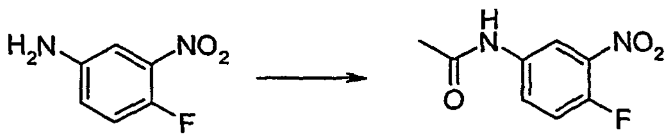

Acetyl Chloride (2.39 mL, 33.6 mmol) was slowly added to a solution of 3-nitro-4-fluoroaniline (5.00 g, 32.0 mmol) and $Et_3N$ in DCM (500 mL) at 0°C. The reaction mixture was allowed to warm to room temperature and stirred overnight. The reaction mixture was washed with water, saturated $NaHCO_3$ solution, brine, dried over anhydrous $MgSO_4$ and filtered. The solvent was concentrated giving the title compound that was used for the next step without further purification. Yield: 5.65 g (87%); MS (ESI) $(M+H)^+$: 199.1.

**Step C. *N*-(4-fluoro-3-nitrophenyl)-*N*-methylacetamide**

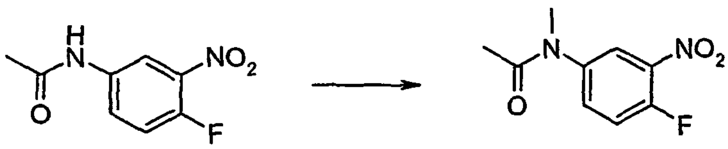

NaH 60% suspension in oil (1.25 g, 31.3 mmol) was added to a solution of *N*-(4-fluoro-3-nitrophenyl)acetamide (5.65 g, 28.0 mmol) in THF (600 mL) at 0°C. The reaction mixture was stirred for 1 hr. MeI (2.13 mL, 34.2 mmol) was added. The reaction mixture was allowed to warm to room temperature, stirred overnight and quenched at 0°C with saturated $NH_4Cl$ solution. The aqueous layer was extracted with EtOAc (3X). The combined organic layers were washed with brine and dried over anhydrous $MgSO_4$. The solvent was concentrated giving the title compound that was used for the next step without further purification. Yield: 3.10 g (51 %); MS (ESI) $(M+H)^+$: 213.1.

**Step D. *N*-{4-[(cyclobutylmethyl)amino]-3-nitrophenyl}-*N*-methylacetamide**

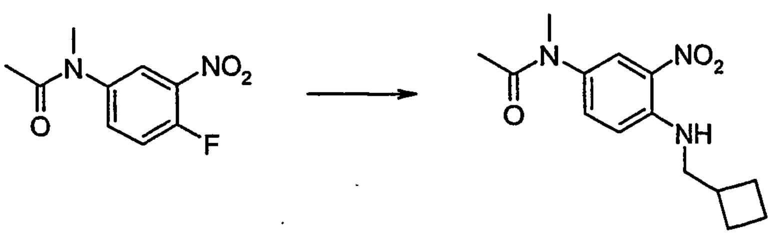

A solution of (clobutylmethyl)amine in $Et_2O$ (excess) was added to a solution of *N*-(4-fluoro-3-nitrophenyl)-*N*-methylacetamide (1.00 g, 4.71 mmol) and DIPEA (1.00 mL, 5.65 mmol) in DMF (50 mL) at 0°C. The reaction mixture was allowed to warm to room temperature and stirred overnight. The solvent was concentrated. The crude product was purified by flash chromatography on silica gel, using EtOAc/Hep (30 to 90%) as eluent, giving the title compound. Yield: 1.01 g (77%); MS (ESI) $(M+H)^+$: 278.3.

**Step E. *N*-{3-amino-4-[(cyclobutylmethyl)amino]phenyl}-*N*-methylacetamide**

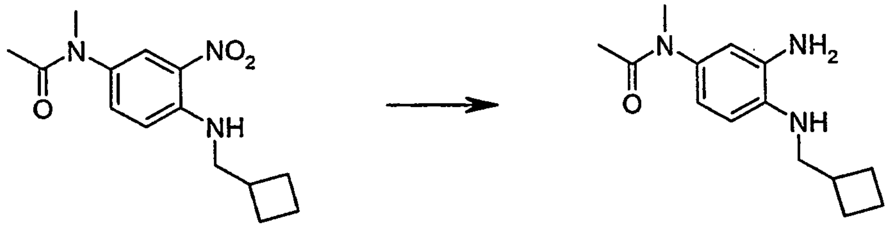

*N*-{4-[(cyclobutylmethyl)amino]-3-nitrophenyl}-*N*-methylacetamide (1.01 g, 3.64 mmol) was hydrogenated in EtOAc (150 mL) catalyzed by 10% Pd/C at 50 psi $H_2$ in Parr shaker overnight at room temperature. The mixture was filtered through a celite pad. The solvent was concentrated giving the title compound that was used for the next step without further purification. Yield: 833 mg (92%); MS (ESI) $(M+H)^+$: 248.3.

**Step F. *N*-[2-*tert*-butyl-1-(cyclobutylmethyl)-1*H*-benzimidazol-5-yl]-*N*-methylacetamide**

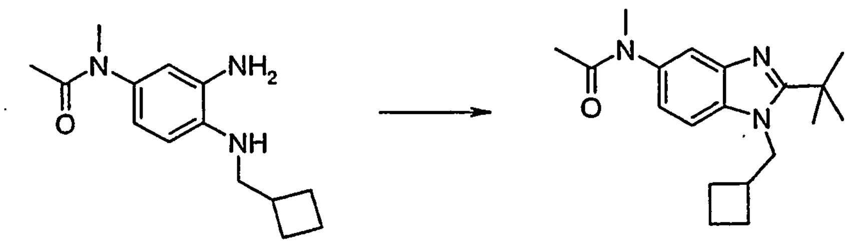

tBuCOCl (0.41 mL, 3.36 mmol) was added to a solutin of *N*-{3-amino-4-[(cyclobutylmethyl)amino]phenyl}-*N*-methylacetamide (0.83 mg, 3.36 mmol) and $Et_3N$ (0.50 mL, 3.53 mmol) in DCM (125 mL) at 0°C. The reaction mixture was allowed to warm to room temperature and stirred for 3 hrs. The solvent was concentrated and the crude compound was recovered in acetic acid (100 mL). The solution was heated to 100°C overnight. The solvent was concentrated. The crude product was purified by flash chromatography on silica gel, using EtOAc/Hep (30 to 90%) as eluent, giving the title compound. Yield: 529 mg (50%); MS (ESI) $(M+H)^+$: 314.4.

**Step G. 2-*tert*-butyl-1-(cyclobutylmethyl)-*N*-methyl-1*H*-benzimidazol-5-amine**

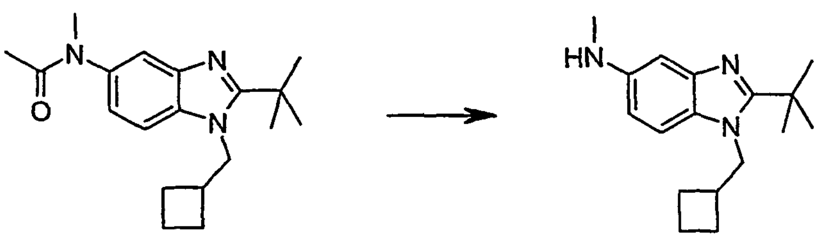

*N*-[2-*tert*-butyl-1-(cyclobutylmethyl)-1*H*-benzimidazol-5-yl]-*N*-methylacetamide (0.53 g, 1.68 mmol) was heated to 80°C overnight in concentrated HCl (10 mL). The room temperature cooled down reaction mixture was poured in water (100 mL). The resulting mixture was brought to slightly basic pH using NaOH solution at 0°C. The compound was

extracted with EtOAc (3X) and the combined organic layers were washed with brine, dried over anhydrous $Na_2SO_4$ and filtered. The solvent was concentrated giving the title compound that was used for the next step without further purification. Yield: 343 mg (75%); MS (ESI) $(M+H)^+$: 272.4.

**Example 73**

**4-[(aminocarbonyl)amino]-*N*-[2-*tert*-butyl-1-(cyclobutylmethyl)-1*H*-benzimidazol-5-yl]-*N*-methylbenzenesulfonamide**

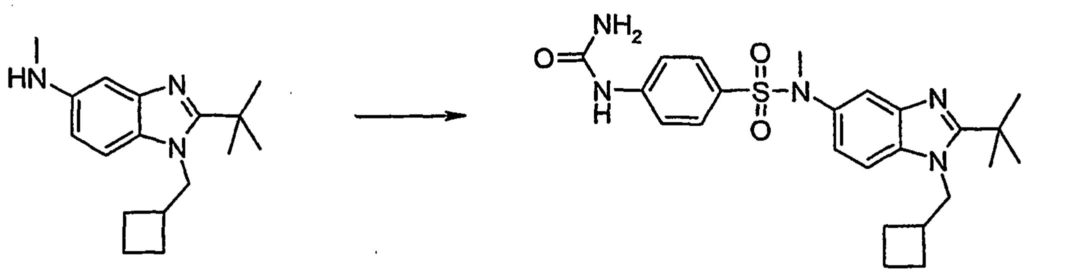

Following the procedure for Step A in Example 72, , using the 4-[(aminocarbonyl) amino] benzenesulfonyl chloride (57 mg, 0.24 mmol) and 2-*tert*-butyl-1-(cyclobutylmethyl)-*N*-methyl-1*H*-benzimidazol-5-amine (55 mg, 0.20 mmol), the title compound was obtained as the corresponding TFA salt. Yield: 60 mg (50%); $^1H$ NMR (400 MHz, $CD_3OD$): δ 1.65 (s, 9 H), 1.81 - 1.99 (m, 3 H), 2.04 - 2.15 (m, 3 H), 2.80 - 2.92 (m, 1 H), 3.23 (s, 3 H), 4.64 (d, J=6.44 Hz, 2 H), 7.33 (dd, J=9.08, 2.05 Hz, 1 H), 7.36 - 7.42 (m, 2 H), 7.50 (d, J=1.95 Hz, 1 H), 7.51 - 7.56 (m, 2 H), 7.81 (d, J=9.18 Hz, 1 H); MS (ESI) $(M+H)^+$: 470.0.

**Example 74**

***N*-(4-{[[2-*tert*-butyl-1-(cyclobutylmethyl)-1*H*-benzimidazol-5-yl](methyl)amino]sulfonyl}phenyl)-2,2-dimethylpropanamide**

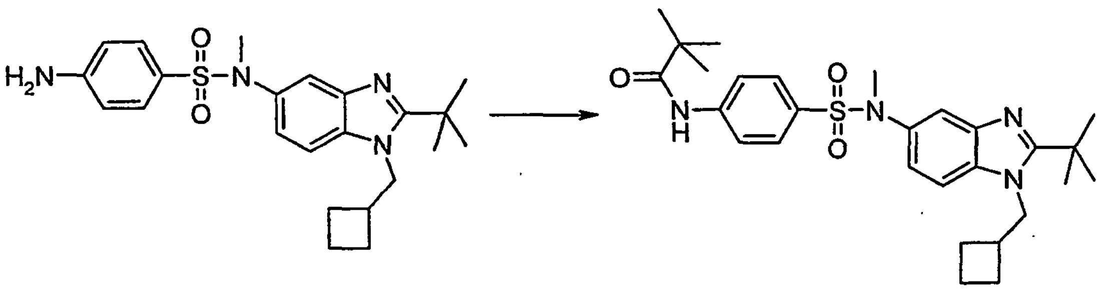

2,2-Dimethylpropanoyl chloride (31 mg, 0.25 mol) was added to a solution of 4-amino-*N*-[2-*tert*-butyl-1-(cyclobutylmethyl)-1*H*-benzimidazol-5-yl]-*N*-methylbenzenesulfonamide (100 mg, 0.23 mmol) and $Et_3N$ (40 μL, 0.28 mmol) in DCM (15 mL) at 0°C. The reaction mixture was allowed to warm to room temperature and stirred for 4 hours. The solvent was concentrated. The crude product was purified by preparative reverse-phase HPLC using 10-90% $CH_3CN/H_2O$ as eluent and then lyophilized to give the title compound as the corresponding TFA salt. Yield: 95 mg (64%); 1H NMR (400 MHz, $CD_3OD$): δ 1.29 (s, 9 H), 1.65 (s, 9 H), 1.80 - 1.98 (m, 2 H), 2.04 - 2.15 (m, 4 H), 2.80 - 2.93 (m, 1 H), 3.25 (s, 3 H), 4.64 (d, J=6.44 Hz, 2 H), 7.31 (dd, J=8.98, 2.15 Hz, 1 H), 7.41 - 7.48 (m, 2 H), 7.51 (d, J=1.56 Hz, 1 H), 7.73 - 7.79 (m, 2 H), 7.81 (d, J=8.98 Hz, 1 H); MS (ESI) $(M+H)^+$: 511.0.

**Example 75**

***N*-(4-{[[2-(1,1-difluoroethyl)-1-(tetrahydro-2*H*-pyran4-ylmethyl)-1*H*-benzimidazol-5-yl](methyl)amino]sulfonyl} phenyl)-2-hydroxyacetamide**

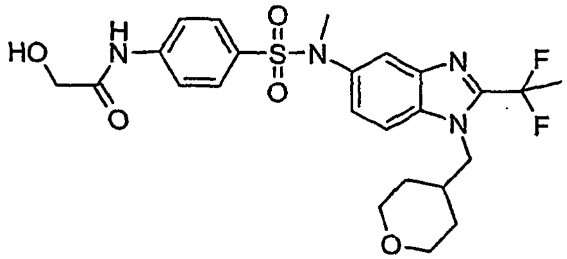

**Step A. *N*-(4-{[[2-(1,1-difluoroethyl)-1-(tetrahydro-2*H*-pyran-4-ylmethyl)-1*H*-benzimidazol-5-yl](methyl)amino] sulfonyl}phenyl)-2-hydroxyacetamide**

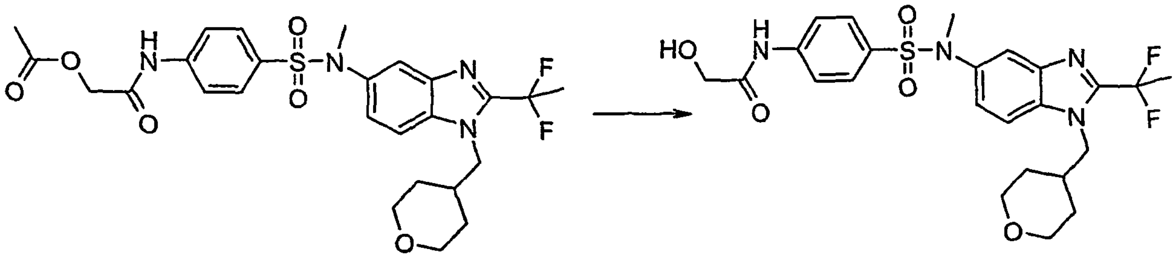

4NNaOH (10 drops) was added to a solution of 2-[(4-{[[2-(1,1-difluoroethyl)-1-(tetrahydro-2*H*-pyran-4-ylmethyl)-1*H*-benzimidazol-5-yl](methyl)amino]sulfonyl}phenyl)amino]-2-oxoethyl acetate (74 mg, 0.13 mmol) in MeOH (5 mL). The reaction mixture was stirred for 1hr. at room temperature and poured in water (100 mL). The mixture was acidified with concentrated HCl and the compound was extracted with EtOAc (3X). The combined organic layers were dried over anhydrous $Na_2SO_4$. The solvent was concentrated. The crude product was purified by preparative reverse-phase HPLC using 10-90% $CH_3CN/H_2O$ as eluent and then lyophilized to give the title compound as the corresponding TFA salt. Yield: 48 mg (57%); $^1H$ NMR (400 MHz, $CDCl_3$): δ 1.44 - 1.58 (m, 4 H), 2.25 (t, J=19.23 Hz, 4 H), 3.21 (s, 3 H), 3.29 - 3.44 (m, 2 H), 4.01 (d, J=11.33 Hz, 2 H), 4.24 (s, 2 H), 4.28 (d, J=7.42 Hz, 2 H), 7.30 (s, 1 H), 7.36 - 7.52 (m, 4 H), 7.66 (d, J=8.79 Hz, 2 H), 8.75 (s, 1 H); MS (ESI) $(M+H)^+$: 523.0; Anal. Calcd. for $C_{24}H_{28}F_2N_4O_5S$ + 0.90 TFA + 0.40 $H_2O$ + 0.10 MeCN: C, 49.06; H, 4.75; N, 9.02. Found: C, 49.07; H, 4.76; N, 9.04.

**Step B. *N*-{5-[acetyl(methyl)amino]-2-[(tetrahydro-2*H*-pyran-4-ylmethyl)amino]phenyl}-2,2-difluoropropanamide**

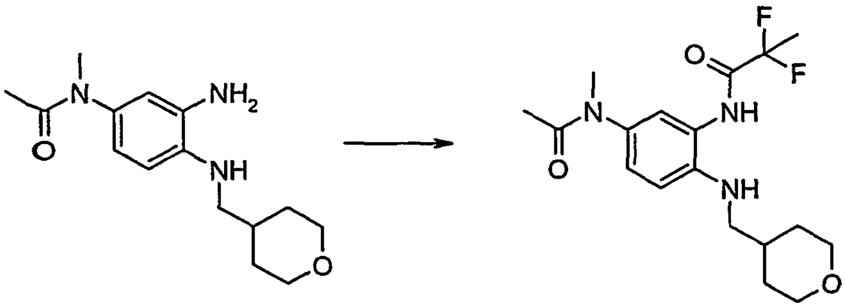

HATU (1.44 g, 3.78 mmol) and *N*-{3-amino-4-[(tetrahydro-2*H*-pyran-4-ylmethyl)amino]phenyl}-*N*-methylacetamide (1.00 g, 3.60 mmol) (for preparation, see Example 39, steps B to E) were added to a solution of 2,2-difluoropropanoic acid (0.40 g, 3.60 mmol) and DIPEA (0.75 mL, 4.32 mmol) in DMF (100 mL) at room temperature. The reaction mixture was stirred overnight. The solvent was concentrated and the crude product was recovered in EtOAc. The organic was washed with water, saturated $NaHCO_3$ solution and brine. The organic layer was dried over anhydrous $Na_2SO_4$ and filtered. The solvent was concentrated giving the title compound that was used for the next step without further purification. Yield: 1.00 g (75%); MS (ESI) $(M+H)^+$: 370.2.

**Step C. *N*-[2-(1,1-difluoroethyl)-1-(tetrahydro-2*H*-pyran-4-ylmethyl)-1*H*-benzimidazol-5-yl]-*N*-methylacetamide**

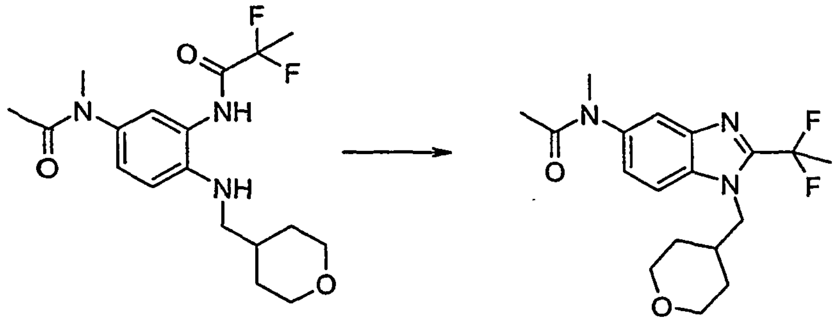

*N*-{5-[acetyl(methyl)amino]-2-[(tetrahydro-2*H*-pyran-4-ylmethyl)amino]phenyl}-2,2-difluoropropanamide (1.00 g, 2.70 mmol) was heated to 90°C overnight in acetic acid (20 mL). The solvent was concentrated. The crude product was purified by flash chromatography on silica gel, using MeOH 3.5% and acetone 8% in DCM as eluent, giving the title compound. Yield: 0.48 g (50%); MS (ESI) $(M+H)^+$: 352.0.

**Step D. 2-(1,1-difluoroethyl)-*N*-methyl-1-(tetrahydro-2*H*-pyran-4-ylmethyl)-1*H*-benzimidazol-5-amine**

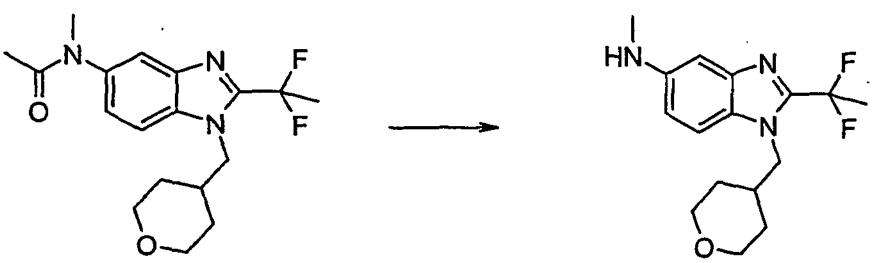

*N*-[2-(1,1-difluoroethyl)-1-(tetrahydro-2*H*-pyran-4-ylmethyl)-1*H*-benzimidazol-5-yl]-*N*-methylacetamide (0.48 g, 1.37 mmol) was heated to 80°C overnight in concentrated HCl (80 mL). The reaction mixture was cool to 0°C and

brought to slightly basic pH using NaOH solution. The compound was extracted with EtOAc (3X) and the combined organic layers were washed with brine, dried over anhydrous $Na_2SO_4$ and filtered. The solvent was concentrated giving the title compound that was used for the next step without further purification. Yield: 0.42 g (98%); MS (ESI) $(M+H)^+$: 310.2.

**Step E. *N*-[2-(1,1-difluoroethyl)-1-(tetrahydro-2*H*-pyran-4-ylmethyl)-1*H*-benzimidazol-5-yl]-*N*-methyl-4-nitrobenzenesulfonamide**

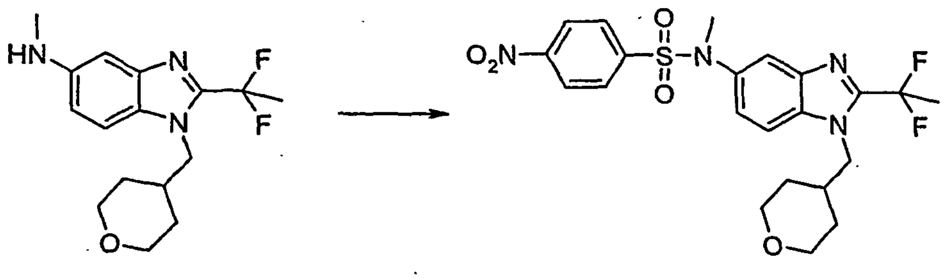

4-Nitrobenzenesulfonyl chloride (0.28 g, 1.27 mmol) was added to a solution of 2-(1,1-difluoroethyl)-*N*-methyl-1-(tetrahydro-2*H*-pyran-4-ylmethyl)-1*H*-benzimidazol-5-amine (0.26 g, 0.84 mmol) and DMAP (0.21 g, 1.69 mmol) in DCE (50 mL). The reaction mixture was heated to 70°C for 1 hr. The solvent was concentrated. The crude product was purified by preparative reverse-phase HPLC using 10-90% $CH_3CN/H_2O$ as eluent and then lyophilized to give the title compound. Yield: 415 mg (99%); MS (ESI) $(M+H)^+$: 495.3.

**Step F. 4-amino-*N*-[2-(1,1-difluoroethyl)-1-(tetrahydro-2*H*-pyran-4-ylmethyl)-1*H*-benzimidazol-5-yl]-*N*-methylbenzenesulfonamide**

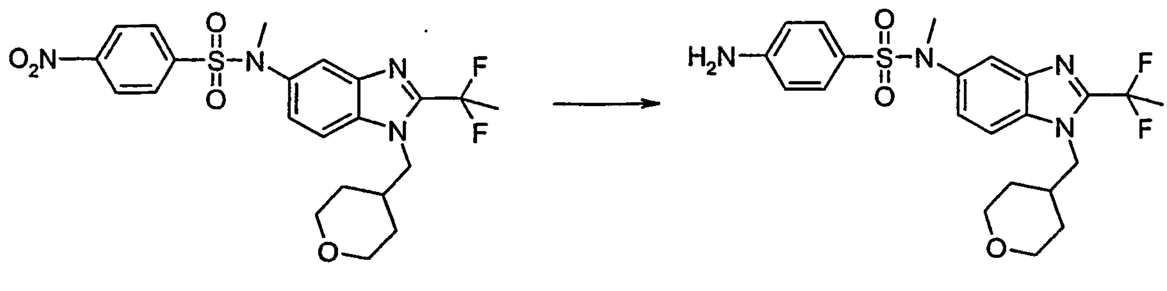

*N*-[2-(1,1-difluoroethyl)-1-(tetrahydro-2*H*-pyran-4-ylmethyl)-1*H*-benzimidazol-5-yl]-*N*-methyl-4-nitrobenzenesulfonamide (415 mg, 0.84 mmol) was hydrogenated in EtOAc (100 mL) catalyzed by 10% Pd/C at 40 psi $H_2$ in Parr shaker overnight at room temperature. The mixture was filtered over a celite pad. The solvent was concentrated giving the title compound that was used for the next step without further purification. Yield: 386 g (99%); MS (ESI) $(M+H)^+$: 465.5.

**Step G. 2-[(4-{[[2-(1,1-difluoroethyl)-1-(tetrahydro-2*H*-pyran-4-ylmethyl)-1*H*-benzimidazol-5-yl](methyl)amino] sulfonyl}phenyl)amino]-2-oxoethyl acetate**

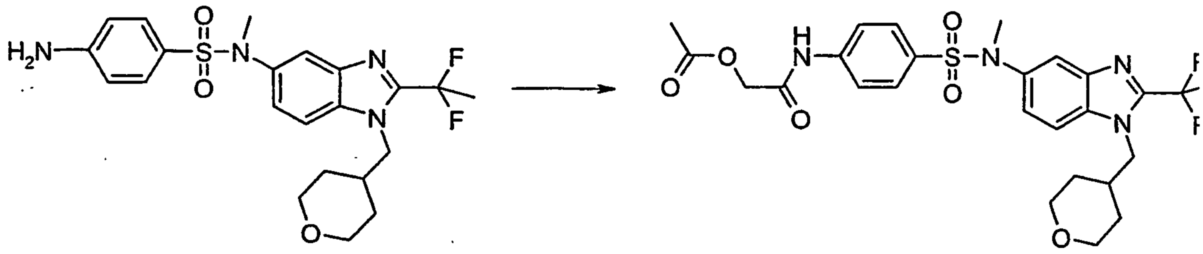

2-Chloro-2-oxoethyl acetate (36 mg, 0.26 mmol) was added to a solution of 4-amino-*N*-[2-(1,1-difluoroethyl)-1-(tetrahydro-2*H*-pyran-4-ylmethyl)-1*H*-benzimidazol-5-yl]-*N*-methylbenzenesulfonamide (100 mg, 0.21 mmol) and $Et_3N$ (44 μL, 0.32 mmol) in DCM (20 mL) at 0°C. The reaction mixture was allowed to warm to room temperature and stirred for 4 hrs. The solvent was concentrated. The crude product was purified by preparative reverse-phase HPLC using 10-90% $CH_3CN/H_2O$ as eluent and then lyophilized to give the title compound. Yield: 74 mg (62%); MS (ESI) $(M+H)^+$: 565.6.

**Example 76**

***N*-(4-{[[2-(1,1-difluoroethyl)-1-(tetrahydro-2*H*-pyran-4-ylmethyl)-1*H*-benzimidazol-5-yl](methyl)amino]sulfonyl} phenyl)acetamide**

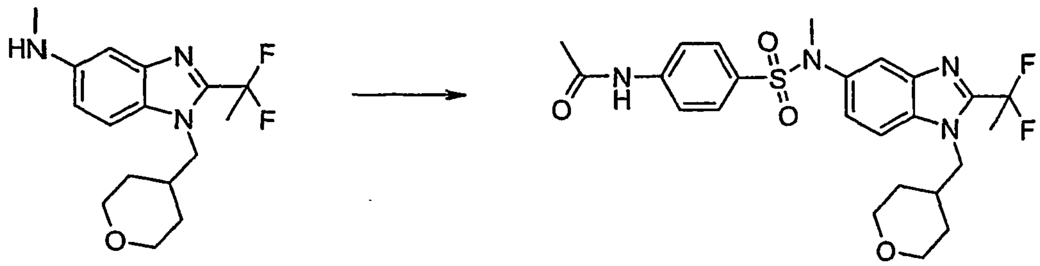

4-(Acetylamino)benzenesulfonyl chloride (45 mg, 0.19 mmol) was added to a solution of 2-(1,1-difluoroethyl)-*N*-methyl-1-(tetrahydro-2*H*-pyran-ylmethyl)-1*H*-benzimidazol-5-amine (50 mg, 0.16 mmol) and DMAP (39 mg, 0.32 mmol) in MeCN (5 mL) at 70°C. The reaction mixture was stirred for 1 hr. and allowed to cool to room temperature. The solvent was concentrated. The crude product was purified by preparative reverse-phase HPLC using 10-90% $CH_3CN/H_2O$ as eluent and then lyophilized to give the title compound as the corresponding TFA salt. Yield: 87 mg (86%); $^1H$ NMR (400 MHz, $CD_3OD$): δ 1.39 - 1.55 (m, 5 H), 2.14 (s, 3 H), 2.21 (t, J=19.33 Hz, 3 H), 3.24 (s, 3 H) 3.32 - 3.38 (m, 2 H), 3.88 - 3.97 (m, 2 H), 4.34 (d, J=7.62 Hz, 2 H), 7.23 (dd, J=8.88, 2.05 Hz, 1 H), 7.35 (d, J=1.95 Hz, 1 H), 7.43 - 7.48 (m, 2 H), 7.64 (d, J=8.79 Hz, 1 H), 7.67 - 7.73 (m, 2 H); MS (ESI) $(M+H)^+$: 507.0; Anal. Calcd. for $C_{24}H_{28}F_2N_4O_4S$ + 1.20 TFA + 0.20 $H_2O$ + 0.10 MeCN: C, 49.07; H, 4.63; N, 8.82. Found: C, 49.04; H, 4.59; N, 8.84.

**Example 77**

***N*-(4-{[[2-(1,1-dilluoroethyl)-1-(tetrahydro-2*H*-pyran-4-ylmethyl)-1*H*-benzimidazol-5-yl](methyl)amino]sulfonyl} phenyl)-3-methylbutanamide**

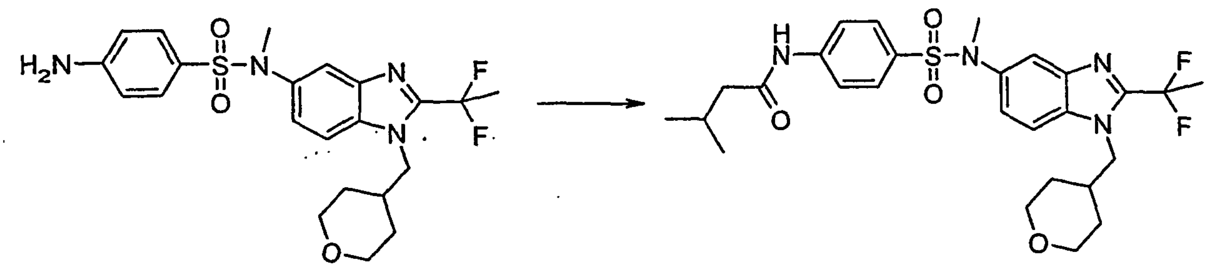

3-Methylbutanoyl chloride (15 mg, 0.12 mmol) was added to a solution of 4-amino-*N*-[2-(1,1-difluoroethyl)-1-(-tetrahydro-2*H*-pyran-4-ylmethyl)-1*H*-benzimidazol-5-yl]-*N*-methylbenzenesulfonamide (50 mg, 0.10 mmol) and $Et_3N$ (22 μL, 0.16 mmol) in DCM (10 mL) at 0°C. The reaction mixture was allowed to warm to room temperature and stirred overnight. The solvent was concentrated. The crude product was purified by preparative reverse-phase HPLC using 10-90% $CH_3CN/H_2O$ as eluent and then lyophilized to give the title compound as the corresponding TFA salt. Yield: 25 mg (35%); $^1$H NMR (400 MHz, $CDCl_3$): δ 1.03 (d, J=6.44 Hz, 6 H), 1.43 - 1.56 (m, 4 H), 2.16 - 2.35 (m, 6 H), 3.13 (s, 1 H), 3.21 (s, 3 H), 3.29 - 3.41 (m, 2 H), 3.94 - 4.05 (m, 2 H), 4.27 (d, J=7.42 Hz, 2 H), 7.33 (s, 1 H), 7.37 - 7.43 (m, 2 H), 7.45 - 7.53 (m, 3 H), 7.63 (d, J=8.79 Hz, 2 H); MS (ESI) $(M+H)^+$: 549.0; Anal. Calcd. for $C_{27}H_{34}F_2N_4O_4S$ + 1.40 TFA + 0.30 MeCN: C, 50.67; H, 5.08; N, 8.36. Found: C, 50.68; H, 5.07; N, 8.32.

**Example 78**

***N*-(4-{[[2-(1,1-difluoroethyl)-1-(tetrahydro-2*H*-pyran-4-ylmethyl)-1*H*-benzimidazol-5-yl](methyl)amino]sulfonyl} phenyl)-2,2-dimethylpropanamide**

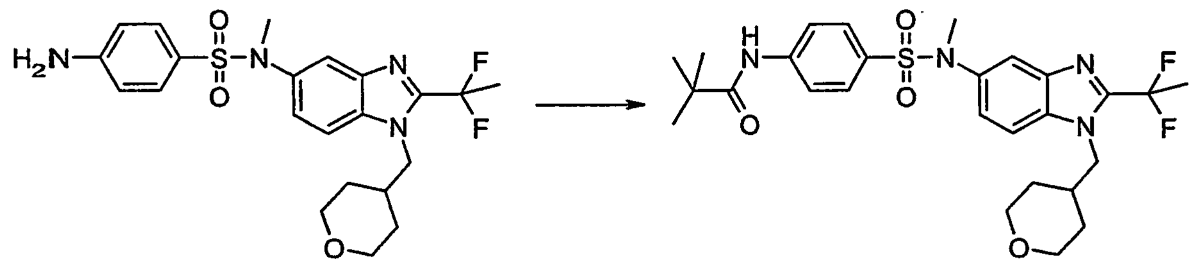

2,2-Dimethylpropanoyl chloride (15 mg, 0.12 mmol) was added to a solution of 4-amino-*N*-[2-(1,1-difluoroethyl)-1-(tetrahydro-2*H*-pyran-4-ylmethyl)-1*H*-benzimidazol-5-yl]-*N*-methylbenzenesulfonamide (50 mg, 0.10 mmol) and $Et_3N$ (22 μL, 0.16 mmol) in DCM (10 mL) at 0°C. The reaction mixture was allowed to warm to room temperature and stirred overnight. The solvent was concentrated. The crude product was purified by preparative reverse-phase HPLC using 10-90% $CH_3CN/H_2O$ as eluent and then lyophilized to give the title compound. Yield: 31 mg (43%); $^1$H NMR (400 MHz, $CDCl_3$): δ 1.34 (s, 9 H), 1.46 - 1.56 (m, 3 H), 1.69 (s, 2 H), 2.17 (s, 3 H), 2.26 (t, J=19.23 Hz, 3 H), 3.29 - 3.40 (m, 2 H), 3.98 (dt, J=11.23, 2.98 Hz, 2 H), 4.26 (d, J=7.42 Hz, 2 H), 5.30 (s, 1 H), 7.31 (s, 1 H), 7.32 - 7.42 (m, 2 H), 7.47 - 7.53 (m, 1 H), 7.59 (s, 1 H), 7.62 - 7.69 (m, 2 H); MS (ESI) $(M+H)^+$: 549.0; Anal. Calcd. for $C_{27}H_{34}F_2N_4O_4S$: C, 59.11; H, 6.25; N, 10.21. Found: C, 59.54; H, 6.16; N, 10.05.

**Example 79**

***N*-[2-(1,1-difluoroethyl)-1-(tetrahydro-2*H*-pyran-4-ylmethyl)-1*H*-benzimidazol-5-yl]-4-{[(isopropylamino)carbonyl]amino}-*N*-methylbenzenesulfonamide**

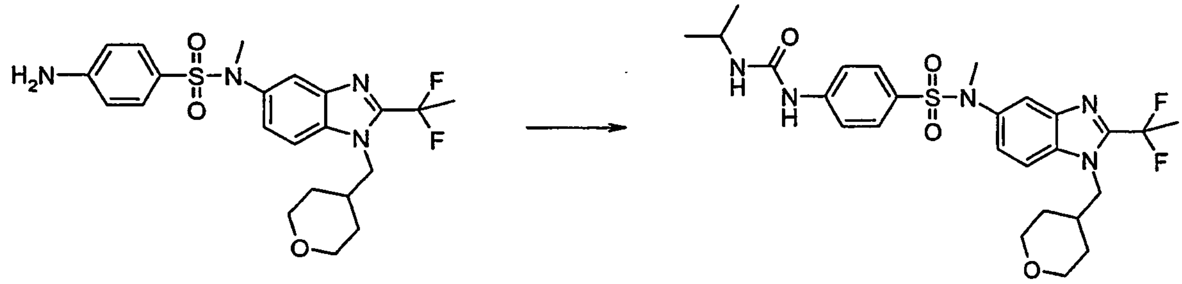

2-isocyanatopropane (excess) was added to a solution of 4-amino-*N*-[2-(1,1-difluoroethyl)-1-(tetrahydro-2*H*-pyran-4-ylmethyl)-1*H*-benzimidazol-5-yl]-*N*-methylbenzenesulfonamide (50 mg, 0.10 mmol) in DCE (5 mL). The reaction mixture was stirred at 90°C overnight. The solvent was concentrated. The crude product was purified by preparative reverse-phase HPLC using 10-90% $CH_3CN/H_2O$ as eluent and then lyophilized to give the title compound as the corresponding TFA salt. Yield: 12 mg (16%); $^1H$ NMR (400 MHz, $CDCl_3$): δ 1.20 (d, J=6.44 Hz, 6 H), 1.23 (d, J=6.64 Hz, 1 H), 1.44 - 1.57 (m, 4 H), 1.80 (s, 1 H), 2.26 (t, J=19.14 Hz, 3 H), 3.19 (s, 3 H), 3.28 - 3.41 (m, 2 H), 3.91 - 4.05 (m, 3 H), 4.27 (d, J=7.42 Hz, 2 H), 7.22 (s, 1 H), 7.31 - 7.48 (m, 6 H); MS (ESI) $(M+H)^+$: 550.0.

**Example 80**

**4-{Bis[(isopropylamino)carbonyl]amino}-*N*-[2-(1,1-difluoroethyl)-1-(tetrahydro-2*H*-pyran-4-ylmethyl)-1*H*-benzimidazol-5-yl]-*N*-methylbenzenesulfonamide**

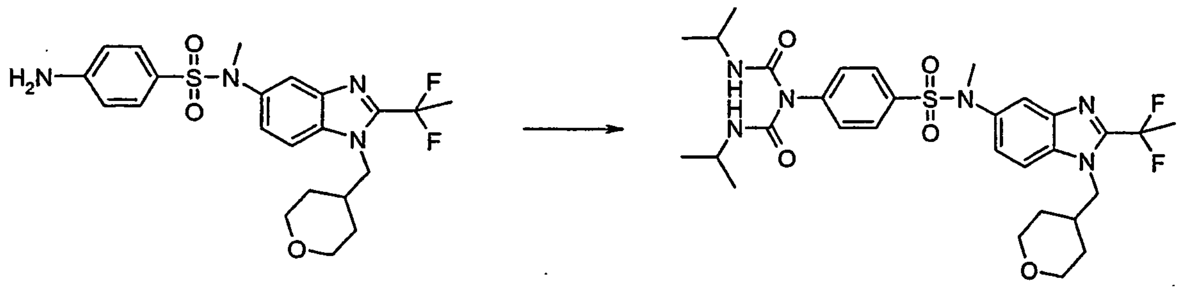

The title compound was obtained as a by-product in Example 79. The material was purified by preparative reverse-phase HPLC using 10-90% $CH_3CN/H_2O$ as eluent and then lyophilized to give the corresponding TFA salt. Yield: 18 mg (22%); $^1H$ NMR (400 MHz, $CDCl_3$): δ 1.15 (d, J=6.64 Hz, 12 H), 1.44 - 1.55 (m, 4 H), 1.82 (s, 2 H), 2.27 (t, J=19.23 Hz, 3 H), 3.28 (s, 3 H), 3.29 - 3.39 (m, 2 H), 3.88 - 4.03 (m, 3 H), 4.25 (d, J=7.42 Hz, 2 H), 6.55 (s, 1 H), 7.24 (dd, J=8.79, 1.95 Hz, 1 H), 7.39 (d, J=8.59 Hz, 2 H), 7.53 (d, J=1.76 Hz, 1 H), 7.73 (d, J=8.59 Hz, 2 H); MS (ESI) $(M+H)^+$: 635.0.

**Example 81**

***N*-[4-({methyl[1-(tetrahydro-2*H*-pyran-4-ylmethyl)-2-(trifluoromethyl)-1*H*-benzimidazol-5-yl]amino}sulfonyl) phenyl]acetamide**

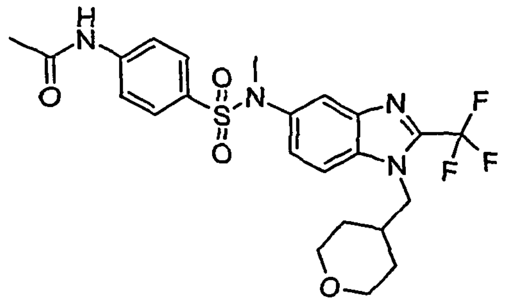

**Step A. *N*-[4-({methyl[1-(tetrahydro-2*H*-pyran-4-ylmethyl)-2-(trifluoromethyl)-1*H*-benzimidazol-5-yl]amino}sulfonyl)phenyl]acetamide**

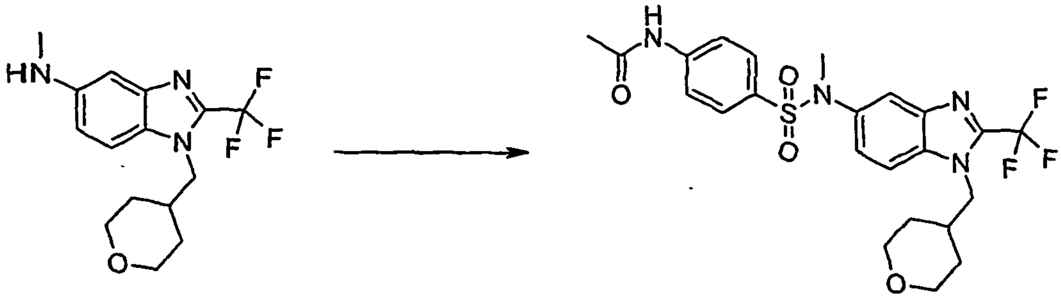

*N*-methyl-1-(tetrahydro-2*H*-pyran-4-ylmethyl)-2-(trifluoromethyl)-1*H*-benzimidazol-5-amine hydrochloride (76.1 mg, 0.2 mmol) (for preparation, see the following steps B, C, D, E, F and G), DMAP (97.7 mg, 0.8 mmol) and 4-(acetylamino)benzenesulfonyl chloride (93.5 mg, 0.4 mmol) in MeCN (5 mL) were stirred overnight at room temperature. The reaction mixture was quenched with $H_2O$ (6 mL). Upon evaporation, the crude product was purified by reversed-phase HPLC using 20-70% $CH_3CN/H_2O$ and then lyophilized affording the title compound as the corresponding TFA salt. Yield: 49.1 mg (48%). $^1$HNMR (400 MHz, $CD_3OD$): 1.39 - 1.56 (m, 4 H), 2.14 (s, 3 H), 2.19 - 2.32 (m, 1 H), 3.24 (s, 3 H), 3.31 - 3.39 (m, 2 H), 3.85 - 4.01 (m, 2 H), 4.32 (d, J=7.42 Hz, 2 H), 7.32 (dd, J=8.88, 2.05 Hz, 1 H), 7.40 (d, J=1.95 Hz, 1 H), 7.43 - 7.49 (m, 2 H), 7.67 - 7.75 (m, 3 H). MS (ESI) $(M+H)^+$ = 511.0. Anal. Calcd for $C_{23}H_{25}F_3N_4O_4S$+ 0.4 TFA+0.2 $H_2O$ (559.75): C, 51.07, H, 4.65, N, 10.01; Found: C, 51.16; H, 4.74; N, 9.65.

**Step B. *N*-(4-fluoro-3-nitrophenyl)acetamide**

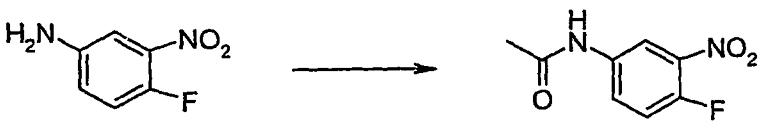

4-Fluoro-3-nitro-aniline (45.0 g, 0.288 mol) was added in portions to acetic anhydride (150 mL) at room temperature. The reaction mixture was stirred at room temperature for 2 h. The white solid was collected and dried *in vacuo* to give the title compound (42.0 g, 70%). $^1$H NMR (400 MHz, $CDCl_3$): δ 2.23 (s, 3 H), 7.26 (m, 1 H), 7.50 (s broad, 1 H), 7.87 (m, 1 H), 8.23 (dd, *J*=6.44, 2.73 Hz, 1 H).

**Step C. *N*-(4-fluoro-3-nitrophenyl)-*N*-methylacetamide**

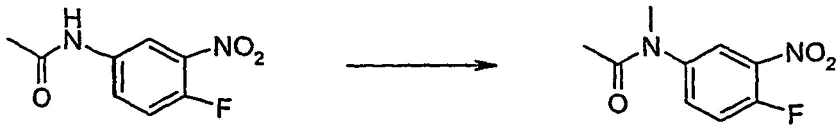

Sodium hydride (2.40 g, 60 mmol) was added in portions to a solution of *N*-(4-fluoro-3-nitrophenyl)acetamide (7.93 g, 40 mmol) in THF (120 mL) at 0°C. Stirring for 20 min, iodomethane (17.0 g, 120 mmol) was added. The reaction mixture was stirred at room temperature for 2 h, quenched with saturaed $NaHCO_3$ (30 mL) and extracted with EtOAc (3x100 mL). The combined organic phases were washed with saturated NaCl (2x30 mL). After filtration and concentration, 8.73 g (100%) of the title compound was obtained as a brown solid. $^1H$ NMR (400 MHz, $CDCl_3$): δ 1.92 (s, 3 H), 3.30 (s, 3 H), 7.38 (s, 1 H), 7.52 (s, 1 H), 7.95 (s, 1 H).

**Step D. *N*-methyl-*N*-{3-nitro-4-[(tetrahydro-2*H*-pyran-4-ylmethyl)amino]phenyl}acetamide**

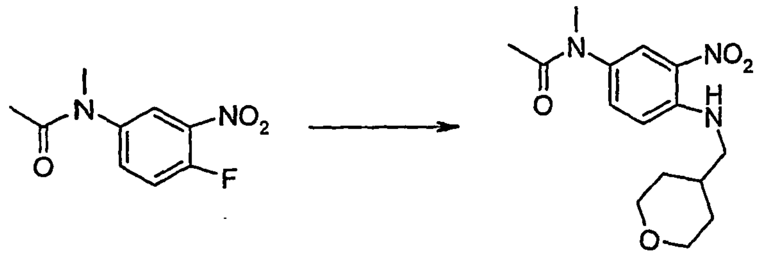

4-Aminomethylpyran (2.50 g, 21.7 mmol ) was added to a mixture of *N*-(4-fluoro-3-nitrophenyl)-*N*-methylacetamide (4.61 g, 21.27 mmol) and sodium carbonate (5.10 g, 47.7 mmol) in EtOH (120 mL) at room temperature. The reaction mixture was heated for 3 days at 60 °C. Upon evaporation of ethanol, the residue was dissolved in EtOAc (400 mL), washed with $H_2O$ (3x50 mL), saturated NaCl (3x50 mL), and dried over $Na_2SO_4$. After filtation and concentration, 6.62 g (100%) of the title compound was obtained as an orange-red solid. $^1H$ NMR (400 MHz, $CDCl_3$): δ 1.38 - 1.52 (m, 2 H), 1.72 - 1.81 (m, 2 H), 1.90 (s, 3 H), 1.93 - 2.02 (m, 1 H), 3.23 (s, 3 H), 3.23 - 3.27 (m, 2 H), 3.36 - 3.49 (m, 2 H), 4.01 - 4.07 (m, 2 H), 6.91 (d, *J*=9.18 Hz, 1 H), 7.29 (dd, *J*=9.08, 2.64 Hz, 1 H), 8.05 (d, *J*=2.34 Hz, 1 H), 8.22 (t, *J*=5.37 Hz, 1 H). MS (ESI) $(M+H)^+$ = 309.12.

**Step E. *N*-{3-amino-4-[(tetrahydro-2*H*-pyran-4-ylmethyl)amino]phenyl}-*N*-methylacetamide**

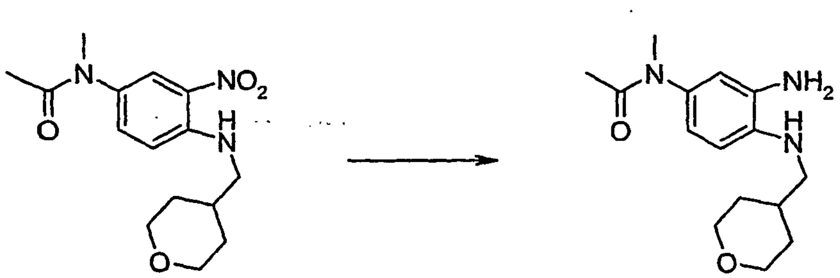

*N*-methyl-*N*-{3-nitro-4-[(tetrahydro-2*H*-pyran-4-ylmethyl)amino]phenyl}acetamide (5.39 g, 16.7 mmol) was hydrogenated in ethyl acetate (200 mL) catalyzed by 10% Pd/C (0.2 g) at 30-40 psi $H_2$ in Parr shaker for 18 h at room temperature. After filtration through celite and concentration, 6.0 g (100%) of a purple solid was obtained as HCl salt, which was used in the next step without purification. $^1H$ NMR (400 MHz, $CD_3OD$): δ 1.32 - 1.46 (m, 2 H), 1.78 - 1.84 (m, 2 H), 1.85 (s, 3 H), 1.91 - 2.06 (m, 1 H), 3.16 (d, *J*=6.83 Hz, 2 H), 3.20 (s, 3 H), 3.39 - 3.51 (m, 2 H), 3.94 - 4.03 (m, 2

H), 7.01 (d, *J*=8.59 Hz, 1 H), 7.12 (d, *J*=2.15 Hz, 1 H), 7.17 (dd, *J*=8.49, 4.39 Hz, 1 H). MS (ESI) $(M+H)^+$ = 278.7

**Step F. *N*-methyl-*N*-[1-(tetrahydro-2*H*-pyran-4-ylmethyl)-2-(trifluoromethyl)-1*H*-benzimidazol-5-yl]acetamide**

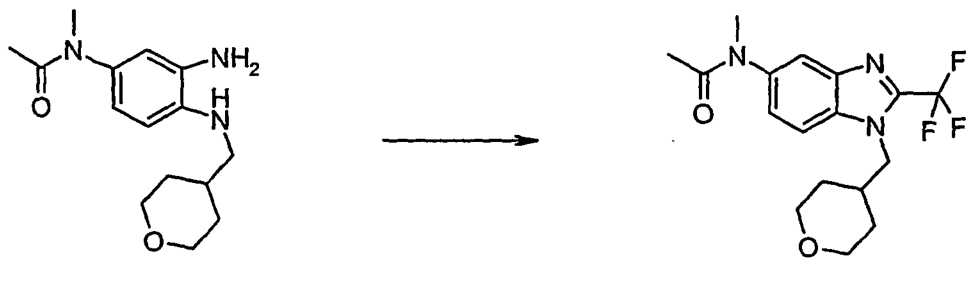

A solution of *N*-{3-amino-4-[(tetrahydro-2*H*-pyran-4-ylmethyl)amino]phenyl}-*N*-methylacetamide hydrochoride (395.1 mg, 1.42 mmol) in trifluoroacetic acid (10 mL) was heated to reflux for 20 h. After evaporation of the solvent, the crude product was used directly for next step without purification. MS (ESI) $(M+H)^+$: 356.02.

**Step G. *N*-methyl-1-(tetrahydro-2*H*-pyran-4-ylmethyl)-2-(trifluoromethyl)-1*H*-benzimidazol-5-amine**

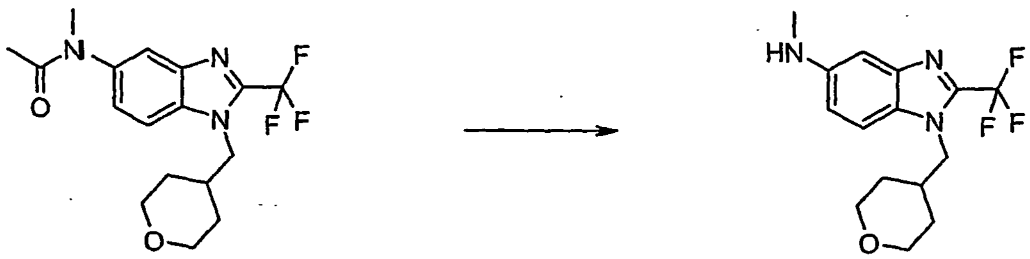

The crude *N*-methyl-*N*-[1-(tetrahydro-2*H*-pyran-4-ylmethyl)-2-(trifluoromethyl)-1*H*-benzimidazol-5-yl]acetamide (~500 mg, 1.42 mmol) was dissolved in 10 mL of EtOH-2*N* HCl (3:2), and then heated at 120°C in a Personal Chemistry SmithSynthesizer microwave instrument for 4 h. After concentration and dried *in vacuo*, 539 mg (100%) of a grey white solid was obtained as the title product, which was used directly for Step A. MS (ESI) $(M+H)^+$ = 314.20.

**Example 82**

**4-[(aminocarbonyl)aminol-*N*-methyl-*N*-[1-(tetrahydro-2*H*-pyran-4-ylmethyl)-2-(trifluoromethyl)-1*H*-benzimidazol-5-yl]benzenesulfonamide**

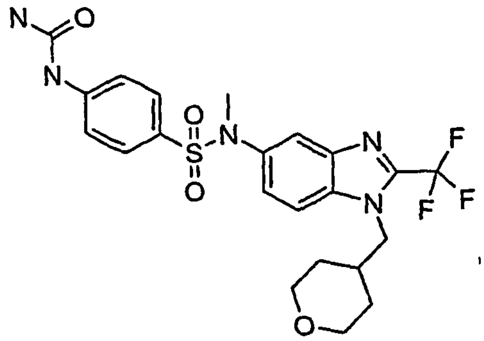

Following the procedure for Example 81, using *N*-methyl-1-(tetrahydro-2*H*-pyran-4-ylmethyl)-2-(trifluoromethyl)-1*H*-benzimidazol-5-amine hydrochloride (76.1 mg, 0.2 mmol) (for preparation, see the steps B, C, D, E, F and G in Example 81), DMAP (97.7 mg, 0.8 mmol) and 4-[(aminocarbonyl)amino]benzenesulfonyl chloride (94.0 mg, 0.4 mmol) in MeCN (6 mL), the crude product was purified by reversed-phase HPLC using 20-50% $CH_3CN/H_2O$ and then lyophilized affording the title compound as the corresponding TFA salt. Yield: 42.9 mg (42%). $^1$HNMR (400 MHz, $CD_3OD$): δ 1.40 - 1.52 (m, 4 H), 2.15 - 2.34 (m, 1 H), 3.23 (s, 3 H), 3.31 - 3.40 (m, 2 H), 3.87 - 3.98 (m, 2 H), 4.32 (d, J=7.81 Hz, 2 H), 7.32 (dd, J=8.88, 2.05 Hz, 1 H), 7.37 - 7.43 (m, 3 H), 7.48 - 7.56 (m, 2 H), 7.72 (d, J=8.79 Hz, 1 H). MS (ESI) $(M+H)^+$ = 512.0. Anal. Calcd for $C_{22}H_{24}F_3N_5O_4S$+ 0.3 TFA (545.73): C, 49.74, H, 4.49, N, 12.83; Found: C, 49.84; H, 4.55; N, 12.78.

**Example 83**

***N*-methyl-4-nitro-*N*-[1-(tetrahydro-2*H*-pyran-4-ylmethyl)-2-(trifluoromethyl)-1*H*-benzimidazol-5-yl]benzenesulfonamide**

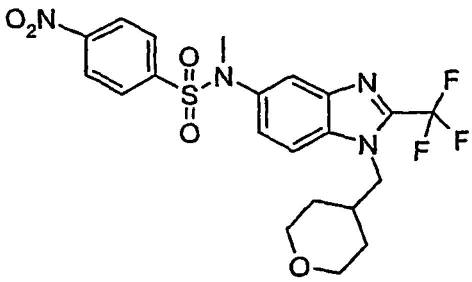

Following the procedure for Example 81, using *N*-methyl-1-(tetahydro-2*H*-pyran-4-ylmethyl)-2-(trifluoromethyl)-1*H*-benzimidazol-5-amine hydrochloride (387.0 mg, 1.0 mmol) (for preparation, see the steps B, C, D, E, F and G in Example 81), DMAP (488.7 mg, 4.0 mmol) and 4-nitrobenzenesulfonyl chloride (443.2 mg, 2.0 mmol) in MeCN (10 mL), the crude product was purified by MPLC using Hex/EtOAc (1:1) on silica gel to give 295.0 mg (59%) of a yellow solid as the title compound. $^1$HNMR (400 MHz, $CD_3OD$): δ 1.39 - 1.54 (m, 4 H), 2.14 - 2.34 (m, 1 H), 3.32 (s, 3 H), 3.33 - 3.40 (m, 2 H), 3.86 - 4.01 (m, 2 H), 4.32 (d, J=7.42 Hz, 2 H), 7.31 (dd, J=8.88, 2.05 Hz, 1 H), 7.45 (d, J=2.15 Hz, 1 H), 7.74 (d, J=8.98 Hz, 1 H), 7.76 - 7.82 (m, 2 H), 8.27 - 8.42 (m, 2 H). MS (ESI) $(M+H)^+$ = 499.0. Anal. Calcd for $C_{21}H_{21}F_3N_4O_5S$+ 0.50 TFA+0.20 $H_2O$ (559.10): C, 47.26; H, 3.95; N, 10.02; Found: C, 47.24; H, 3.80; N, 10.20.

**Example 84**

**4-amino-*N*-methyl-*N*-[1-(tetrahydro-2*H*-pyran-4-ylmethyl)-2-(trifluoromethyl)-1*H*-benzimidazol-5-yl]benzenesulfonamide**

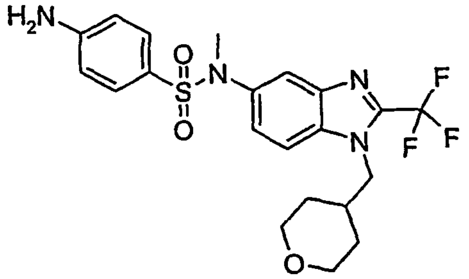

*N*-methyl-4-nitro-*N*-[1-(tetrahydro-2*H*-pyran-4-ylmethyl)-2-(trifluoromethyl)-1*H*-benzimidazol-5-yl]benzenesulfonamide (235.6 mg, 0.47 mmol) (for preparation, see the Example 83) was hydrogenated in ethyl acetate (20 mL) catalyzed by 10% Pd/C (90 mg) at 30-40 psi $H_2$ in Parr shaker for 5 h at room temperature. After filtration through celite and concentration, 229.8 mg (100%) of a white solid was obtained. Small amounts of the crude product was purified by reversed-phase HPLC using 20-70% $CH_3CN/H_2O$ and then lyophilized affording the title compound as the corresponding TFA salt. $^1$HNMR (400 MHz, $CD_3OD$): δ 1.38 - 1.55 (m, 4 H), 2.15 - 2.35 (m, 1 H), 3.18 (s, 3 H), 3.33 - 3.40 (m, 2 H), 3.82 - 4.02 (m, 2 H), 4.32 (d, J=7.62 Hz, 2 H), 6.58 - 6.69 (m, 2 H), 7.15 - 7.23 (m, 2 H), 7.35 (dd, J=8.98, 1.95 Hz, 1 H), 7.40 (d, J=1.56 Hz, 1 H), 7.71 (d, J=8.79 Hz, 1 H). MS (ESI) $(M+H)^+$ = 469.0. Anal. Calcd for $C_{21}H_{23}F_3N_4O_3S$+ 0.40 TFA (514.11): C, 50.93; H, 4.59; N, 10.90; Found: C, 51.00; H, 4.72; N, 10.54.

**Example 85**

**2,2-dimethyl-*N*-[4-({methyl[1-(tetrahydro-2*H*-pyran-4-ylmethyl)-2-(trifluoromethyl)-1*H*-benzimidazol-5-yl]amino}sulfonyl)phenyl]propanamide**

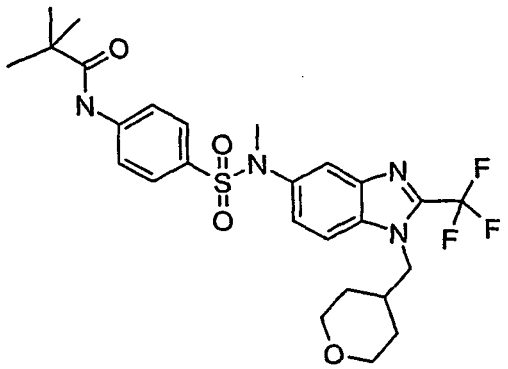

4-Amino-*N*-methyl-*N*-[1-(tetrahydro-2*H*-pyran-4-ylmethyl)-2-(trifluoromethyl)-1*H*-benzimidazol-5-yl]benzenesulfonamide (50.3 mg, 0.107 mmol) (for preparation, see the Example 84), DMAP (59.0 mg, 0.483 mmol) and trimethylacetyl chloride (14.7 mg, 0.122 mmol) in MeCN (5 mL) were stirred for 6 h at room temperature. The reaction mixture was diluted with EtOAc (100 mL), washed with saturated $NaHCO_3$ (10 mL) and saturated NaCl (10 mL), and dried over $Na_2SO_4$. Upon evaporation, the residue was purified by by MPLC using Hex/EtOAc (1:1) on silica gel to give 30.5 mg (52%) of a white solid as the title compound. $^1$HNMR (400 MHz, $CD_3OD$): δ 1.29 (s, 9 H), 1.39 - 1.58 (m, 4 H), 2.15 - 2.37 (m, 1 H), 3.24 (s, 3 H), 3.31 - 3.40 (m, 2 H), 3.87 - 3.99 (m, 2 H), 4.32 (d, J=7.62 Hz, 2 H), 7.32 (dd, J=8.88, 2.05 Hz, 1 H), 7.40 (d, J=1.95 Hz, 1 H), 7.42 - 7.49 (m, 2 H), 7.72 (d, J=8.98 Hz, 1 H), 7.74 - 7.79 (m, 2 H), 9.35 (s, 1 H). MS (ESI) $(M+H)^+$ = 553.0. Anal. Calcd for $C_{26}H_{31}F_3N_4O_4S$+ 0.1 TFA+0.50 $H_2O$ (573.03): C, 54.92; H, 5.65; N, 9.78; Found: C, 54.77; H, 5.54; N, 10.09.

**Example 86**

**2-{[4-({methyl[1-(tetrahydro-2*H*-pyran-4-ylmethyl)-2-(trifluoromethyl)-1*H*-benzimidazol-5-yl]amino}sulfonyl) phenyl]amino}-2-oxoethyl acetate**

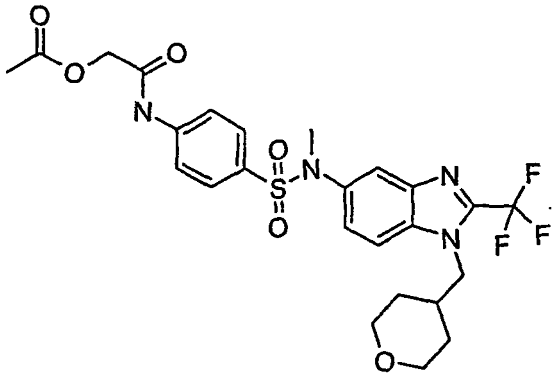

Following the procedure for Example 85, using 4-Amino-*N*-methyl-*N*-[1-(tetrahydro-2*H*-pyran-4-ylmethyl)-2-(trifluoromethyl)-1*H*-benzimidazol-5-yl]benzenesulfonamide (113.0 mg, 0.241 mmol) (for preparation, see the Example 84), DMAP (109.0 mg, 0.892 mmol) and 2-chloro-2-oxoethyl acetate chloride (38.1 mg, 0.279 mmol) in MeCN (10 mL), the crude product was purified by MPLC using Hex/EtOAc (1:1) on silica gel to give 90.0 mg (66%) of a white solid as the title compound. $^1$HNMR (400 MHz, $CD_3OD$): δ 1.41 - 1.54 (m, 4 H), 2.16 (s, 3 H), 2.20 - 2.33 (m, 1 H), 3.25 (s, 3 H), 3.31 - 3.40 (m, 2 H), 3.87 - 3.98 (m, 2 H), 4.32 (d, J=7.62 Hz, 2 H), 4.69 (s, 2 H), 7.32 (dd, J=8.88, 2.45 Hz, 1 H), 7.41 (d, J=1.56 Hz, 1 H), 7.44 - 7.50 (m, 2 H), 7.69 - 7.76 (m, 3 H), 10.24 (s, 1 H). MS (ESI) $(M+H)^+$ = 569.1. Anal. Calcd for $C_{25}H_{27}F_3N_4O_6S$+ 0.1 TFA+0.40 $H_2O$ (587.18): C, 51.55; H, 4.79; N, 9.54; Found: C, 51.60; H, 4.74; N, 9.56.

**Example 87**

**4-{[(isopropylamino)carbonyl]amino}-*N*-methyl-*N*-[1-(tetrahydro-2*H*-pyran-4-ylmethyl)-2-(trifluoromethyl)-1*H*-benzimidazol-5-yl]benzenesulfonamide**

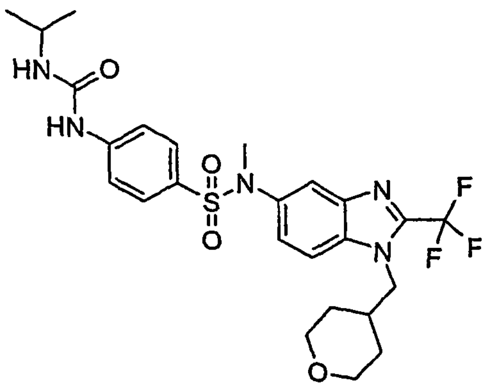

4-Amino-*N*-methyl-*N*-[1-(tetrahydro-2*H*-pyran-4-ylmethyl)-2-(trifluoromethyl)-1*H*-benzimidazol-5-yl]benzenesulfonamide (31.3 mg, 0.067 mmol) (for preparation, see the Example 84) and 2-isocyanatopropane (0.5 mL) in DCE (5 mL) was heated overnight at 80 °C. After evaporation, the crude product was purified by MPLC using Hex/EtOAc (1:1) on silica gel to give 17.2 mg (46%) of a white solid as the title compound. $^1$HNMR (400 MHz, $CD_3OD$): δ 1.17 (d, J=6.44 Hz, 6 H), 1.41 - 1.53 (m, 4 H), 2.18 - 2.33 (m, 1 H), 3.23 (s, 3 H), 3.31 - 3.39 (m, 2 H), 3.83 - 3.90 (m, 1 H), 3.90 - 3.96 (m, 2 H), 4.32 (d, J=7.42 Hz, 2 H), 7.32 (dd, J=8.88, 2.05 Hz, 1 H), 7.36 - 7.40 (m, 2 H), 7.40 (d, J=1.56 Hz, 1 H),

7.46 - 7.52 (m, 2 H), 7.72 (d, J=8.59 Hz, 1 H). MS (ESI) $(M+H)^+$ = 554.0. Anal. Calcd for $C_{25}H_{30}F_3N_5O_4S$+ 0.70 TFA+0.20 $H_2O$ +0.5$CH_3OH$ (653.06): C, 49.48; H, 5.11; N, 10.72; Found: C, 49.50; H, 5.16; N, 10.71.

**Example 88**

**2-Hydroxy-*N*-[4-({methyl[1-(tetrahydro-2*H*-pyran-4-ylmethyl)-2-(trifluoromethyl)-1*H*-benzimidazol-5-yl]amino} sulfonyl)phenyl]acetamide**

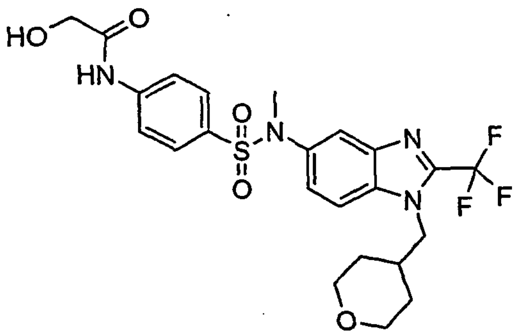

2-{[4-({Methyl[1-(tetrahydro-2*H*-pyran-4-ylmethyl)-2-(trifluoromethyl)-1*H*-benzimidazol-5-yl]amino}sulfonyl) phenyl]amino}-2-oxoethyl acetate (56.3 mg, 0.107 mmol) (for preparation, see the Example 86) and a drop of sodium methoxide (25% in MeOH) in MeOH (10 mL) was stirred overnight at room temperature. After evaporation, the crude product was purified by reversed-phase HPLC using 15-65% $CH_3CN/H_2O$ and then lyophilized affording the title compound as the corresponding TFA salt. Yield: 36.6 mg (65%). $^1$HNMR (400 MHz, $CD_3OD$): δ 1.39 - 1.56 (m, 4 H), 2.15 - 2.35 (m, 1 H), 3.25 (s, 3 H), 3.32 - 3.41 (m, 2 H), 3.86 - 4.00 (m, 2 H), 4.13 (s, 2 H), 4.32 (d, J=7.62 Hz, 2 H), 7.33 (dd, J=8.88, 2.05 Hz, 1 H), 7.41 (d, J=1.56 Hz, 1 H), 7.45 - 7.53 (m, 2 H), 7.72 (d, J=8.40 Hz, 1 H), 7.76 - 7.85 (m, 2 H). MS (ESI) $(M+H)^+$ = 527.0. Anal. Calcd for $C_{23}H_{25}F_3N_4O_5S$+0.80 $H_2O$ (540.95): C, 51.07; H, 4.96; N, 10.36; Found: C, 51.22; H, 5.11; N, 10.14.

## Claims

**1.** A compound of Formula I or a pharmaceutically acceptable salt thereof:

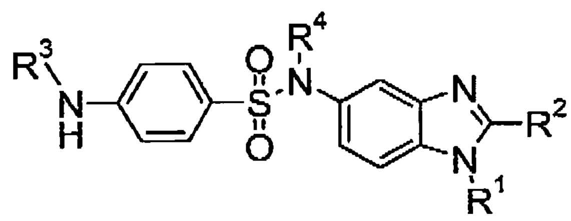

I

wherein
$R^1$ is selected from $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cyclalkenyl, and $C_{3-6}$heterocycloalkyl, wherein said $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloakenyl, and $C_{3-6}$teterocycloalkyl used in defining $R^1$ is optionally substituted by one or more groups selected from halogen, cyano, nitro, methoxy, ethoxy, methyl, ethyl, hydroxy, amino, $C_{1-6}$alkylamino and di$C_{1-6}$alkylamino;
$R^2$ is selected from $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl, wherein said $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl used in defining $R^2$ is optionally substituted by one or more groups selected from halogen, methoxy, ethoxy,

methyl, ethyl, hydroxy, amino, $C_{1-6}$akylamino and di$C_{1-6}$alkylamino;
$R^3$ is selected from -H, $C_{1-6}$alkyl, $C_{2-6}$alkenyl, and $C_{1-6}$acyl, wherein said $C_{1-6}$alkyl, $C_{2-6}$alkenyl, and $C_{1-6}$acyl used in defining $R^3$ is optionally substituted with one or more groups selected from $CH_3C(=O)$-O-, halogen, cyano, methoxy, ethoxy, hydroxy, amino, alkylamino, dialkylamino, and $C_{3-6}$heterocycloalkyl; and
$R^4$ is selected from -H, $C_{1-6}$alkyl, $C_{2-6}$alkenyl, $C_{3-6}$cycloalkyl, and $C_{3-6}$cycloalkyl-$C_{1-4}$alkyl.

**2.** A compound as claimed in claim 1, wherein
$R^1$ is selected from $C_{1-6}$alkyl, $C_{2-6}$alkenyl, $C_{3-6}$cycloalkyl-$C_{1-4}$alkyl, $C_{4-6}$cycloalkenyl-$C_{1-4}$alkyl and $C_{3-6}$heterocycloalkyl-$C_{1-4}$alkyl, wherein said $C_{1-6}$alkyl, $C_{2-6}$alkenyl, $C_{3-6}$cycloalkyl-$C_{1-4}$alkyl, $C_{4-6}$cycloalkenyl-$C_{1-4}$alkyl and $C_{3-6}$heterocycloalkyl-$C_{1-4}$alkyl used in defining $R^1$ is optionally substituted by one or more groups selected from halogen, methoxy, ethoxy, methyl, hydroxy and amino;
$R^2$ is selected from $C_{1-6}$alkyl, $C_{2-6}$alkenyl, $C_{3-6}$cycloalkyl, $C_{3-6}$cycloalkyl-$C_{1-4}$alkyl, and $C_{4-6}$cycloalkenyl-$C_{1-4}$alkyl, wherein said $C_{1-6}$alkyl, $C_{2-6}$alkenyl, $C_{3-6}$cycloalkyl, $C_{3-6}$cycloalkyl-$C_{1-4}$alkyl, and $C_{4-6}$cycloalkenyl-$C_{1-4}$alkyl used in defining $R^2$ is optionally substituted by one or more groups selected from halogen, methoxy, ethoxy and hydroxy;
$R^3$ is selected from -H, $C_{1-6}$alkyl, $C_{2-6}$alkenyl, and $C_{1-6}$acyl, wherein said $C_{1-6}$alkyl, $C_{2-6}$alkenyl, and $C_{1-6}$acyl used in defining $R^3$ is optionally substituted with one or more groups selected from $CH_3C(=O)$-O-, halogen, methoxy, ethoxy, hydroxy, amino, methylamino, dimethylamino, and $C_{3-6}$heterocycloalkyl; and
$R^4$ is selected from -H and $C_{1-3}$alkyl.

**3.** A compound as claimed in claim 1,
$R^1$ is selected from cyclopentyl-methyl, cyclohexyl-methyl, cyclobutyl-methyl, cyclopropyl-methyl, 4,4-difluorocyclohexyl-methyl, bicyclo[2.2.1]hept-5-en-2-ylmethyl, tetrahydropyranyl-methyl, tetrahydropyranyl-ethyl, tetrahydrofuranyl-methyl, morpholinyl-methyl, piperdinylethyl, N-methyl-piperdinylmethyl, and piperdinyl-methyl;
$R^2$ is selected from t-butyl, n-butyl, 2-methyl-2-butyl, isopentyl, 2-methoxy-2-propyl, 2-hydroxy-propyl, trifluoromethyl, 1,1-difluoroethyl, 2,2,2-trifluoroethyl,1-methyl-propyl, 1,1-dimethyl-propyl, 1,1-dimethyl-3-buten-1-yl, ethyl, and 2-propyl;
$R^3$ is selected from -H, $C_{1-6}$alkyl, and $C_{1-6}$acyl, wherein said $C_{1-6}$alkyl, and $C_{1-6}$acyl used in defining $R^3$ is optionally substituted with one or more groups selected from $CH_3C(=O)$-O-, halogen, methoxy, hydroxy, amino, methylamino, dimethylamino, pyrrolidinyl, and morpholinyl; and
$R^4$ is selected from -H and methyl.

**4.** A compound as claimed in claim 1, wherein
$R^1$ is selected from cyclohexyl-methyl, cyclopentyl-methyl, cyclobutyl-methyl, cyclopropyl-methyl, 4,4-difluorocyclohexy-methyl and tetrahydropyranyl-methyl;
$R^2$ is t-butyl and 1,1-difluoroethyl;
$R^3$ is selected from -H, methyl, ethyl, propyl, 2-propyl, 2-hydroxyethyl, 2-methoxyethyl, formyl, acetyl, ethylcarbonyl, 2-propylcarbonyl, t-butylcarbonyl, uriedo, N-isopropyl-ureido, 2-amino-acetyl, 2-methylamino-acetyl, 2-dimethylaminoacetyl, 2-acetyloxy-acetyl, 2-hydroxy-acetyl, 2-bromo-acetyl, 2-(morpholin-1-yl)-acetyl, and 2-(pyrrolindin-1-yl)-acetyl; and
$R^4$ is selected from -H and methyl.

**5.** A compound selected from:

*N*-(4-{[[2-*tert*-butyl-1-(cyclohexylmethyl)-1*H*-benzimidazol-5 yl](methyl)amino]sulfonyl}phenyl) acetamide;
*N*-[2-*tert*-Butyl-1-(cyclohexylmethyl)-1*H*-benzimidazol-5-yl]-*N*-methyl-4-nitrobenzenesulfonamide;
4-Amino-*N*-[2-*tert*-butyl-1-(cyclohexylinethyl)-1*H*-benzimidazol-5-yl]-*N*-methylbenzenesulfonamide;
*N*-(4-{[[2-*tert*-Butyl-1-(cyclohexylmethyl)-1*H*-benzimidazol-5-yl](methyl)amino]sulfonyl}phenyl)propanamide;
*N*-(4-{[[2-*tert*-Butyl-1-(cyclohexylmethyl)-1*H*-benzimidazol-5-yl](methyl)amino]sulfonyl}phenyl)-2-methylpropanmade;
*N*-(4-{[[2-*tert*-Butyl-1-(cylohexylmethyl)-1*H*-benzimidazol-5-yl](methyl)amino]sulfonyl}phenyl)-2,2-dimethylpropanamide;
*N*-[2-*tert*-Butyl-1-(cyclohexylmethyl)-1*H*-benzimidazol-5-yl]-4-(ethylamino)-*N*-methylbenzenesulfonamide;
*N*-[2-*tert*-Butyl-1-(cyclohexylmethyl)-1*H*-benzimidazol-5-yl]-4-(formylamino)-*N*-methylbenzenesulfonamide;
*N*-(4-{[[2-*tert*-Butyl-1-(cyclohexylmethyl)-1*H*-benzimidazol-5-yl](methyl)amino]sulfonyl}phenyl)-2-pyrrolidin-1-ylacetamide;
*N*[1]-(4-{[[2-*tert*-Butyl-1-(cyclohexylmethyl)-1*H*-benzimidazol-5-yl](methyl)amino]sulfonyl}phenyl)-*N*[2],*N*[2]-dimethylglycinamide;
*N*-(4-{[[2-*tert*-Butyl-1-(cyclohexylmethyl)-1*H*-benzimidazol-5-yl](methyl)armno]sulfonyl}phenyl)-2-morpholin-4-

ylacetamide;
$N^1$-(4-{[[2-*tert*-Butyl-1-(cyclohexylmethyl)-1*H*-benzimidazol-5-yl](methyl)amino]sulfonyl}phenyl)glycinamide;
2-[(4-{[[2-*tert*-Butyl-1-(cyclohexylmethyl)-1*H*-benzimidazol-5-yl](methyl)amino]sulfonyl}phenyl)amino]-2-oxoethyl acetate;
*N*-(4-{[[2-*tert*-Butyl-1-(cyclohexylmethyl)-1*H*-benzimidazol-5-yl](methyl)amino]sulfonyl}phenyl)-2-hydroxyacetamide;
5-Bromo-*N*-[2-*tert*-butyl-1-(cyclohexylmethyl)-1*H*-benzimidazol-5-yl]-6-chloro-*N*-methylpyridine-3-sulfonamide;
5-Bromo-*N*-[2-*tert*-butyl-1-(cyclohexylmethyl)-1*H*-benzimidazol-5-yl]-6-[(2-hydroxyethyl)amino]-*N*-methylpyridine-3-sulfonamide;
*N*-[2-*tert*-Butyl-1-(cyclohexylmethyl)-1*H*-benzimidazol-5-yl]-6-[(2-hydroxyethyl)amino]-*N*-methylpyridine-3-sulfonamide;
*N*-(5-{[[2-*tert*-Butyl-1-(cyclohexylmethyl)-1*H*-benzimidazol-5-yl](methyl)amino]sulfonyl}pyridin-2-yl)acetamide;
*N*-(3-{[[2-*tert*-Butyl-1-(cyclohexylmethyl)-1*H*-benzimidazol-5-yl](methyl)amino]sulfonyl}phenyl)acetamide;
$N^1$-(4-{[[2-*tert*-Butyl-1-(cyclohexylmethyl)-1*H*-benzimidazol-5-yl](methyl)amino]sulfonyl}phenyl)-$N^2$-(2-hydroxyethyl)glycinamide;
4-[(Aminocarbonyl)amimo]-*N*-[2-*tert*-butyl-1-(tetrahydro-2*H*-pyran-4-ylmethyl)-1*H*-benzimidazol-5-yl]-*N*-methylbenzenesulfonamide;
*N*-(4-{[(2-*tert*-Butyl-1-(tetrahydro-2*H*-pyran-4-ylmethyl)-1*H*-benzimidazol-5-yl)(methyl)amino]sulfonyl}phenyl) acetamide;
*N*-(4-{[[2-*tert*-Butyl-1-(tetrahydro-2*H*-pyran-4-ylmethyl)-1*H*-benzimidazol-5-yl](methyl)amino]sulfonyl}phenyl)-*N*-methylacetamide;
*N*-(4-{[[2-*tert*-Butyl-1-(tetrahydro-2*H*-pyran-4-ylmethyl)-1*H*-benzimidazol-5-yl](methyl)amino]sulfonyl}phenyl)-2,2-dimethylpropanamide;
*N*-(4-{[[2-*tert*-Butyl-1-(tetrahydro-2*H*-pyran-4-ylmethyl)-1*H*-benzimidazol-5-yl](methyl)amino]sulfonyl}phenyl)-2-hydroxyacetamide;
$N^1$-(4-{[[2-*tert*-Butyl-1-(tetrahydro-2*H*-pyran-4-ylmethyl)-1*H*-benzimidazol-5-yl](methyl)amino]sulfonyl}phenyl)-$N^2$,$N^2$-dimethylglycinamide;
$N^1$-(4-{[[2-*tert*-Butyl-1-(tetrahydro-2*H*-pyran-4-ylmethyl)-1*H*-benzimidazol-5 yl](methyl)amino]sulfonyl}phenyl) glycinamide;
$N^1$-(4-{[[2-*tert*-Butyl-1-(tetrahydro-2*H*-pyran-4-ylmethyl)-1*H*-benzimidazol-5-yl](methyl)amino]sulfonyl}phenyl)-$N^2$-methylglycinamide;
*N*-[2-*tert*-Butyl-1-(tetrahydro-2*H*-pyran-4-ylmethyl)-1*H*-benzimidazol-5-yl]-6-[(2-hydroxyethyl)amino]-*N*-methylpyridine-3-sulfonamide;
*N*-[2-*tert*-Butyl-1-(tetrahydro-2*H*-pyran-4-ylmethyl)-1*H*-benzimidazol-5-yl]-6-[(2-methoxyethyl)amino]-*N*-methylpyridine-3-sulfonamide;
*N*-[2-*tert*-Butyl-1-(tetrahydro-2*H*-pyran-4-ylmethyl)-1*H*-benzimidazol-5-yl]-6-(foromylamino)-*N*-methylpyridine-3-sulfonamide;
*N*-(5-{[[2-*tert*-Butyl-1-(tetrahydro-2*H*-pyran-4-ylmethyl)-1*H*-benzimidazol-5-yl](methyl)amino]sulfonyl}pyridin-2-yl)acetamide;
*N*-[4-({[2-*tert*-Butyl-1-(tetrahydro-2*H*-pyran-4-ylmethyl)-1*H*-benzimidazol-5-yl]amino}sulfonyl)phenyl]acetamide;
*N*-[4-({(2-*tert*-Butyl-1-(cyclohexylmethyl)-1*H*-benzimidazol-5-yl]amino}sulfonyl)phenyl]acetamide;
*N*-(4-{[[2-*tert*-Butyl-1-(2-piperidin-1-ylethyl)-1*H*-benzimidazol-5-yl](methyl)amino]sulfonyl}phenyl)acetamide;
*N*-(4-{[[2-*tert*-Butyl-1-(1,4-dioxan-2-ylmethyl)-1*H*-benzimidazol-5-yl](methyl)amino]sulfonyl}phenyl)acetamide;
*N*-(4-{[{2-*tert*-Butyl-1-[(1-methylpiperidin-2-yl)methyl]-1*H*-benzimidazol-5-yl}(methyl)amino]sulfonyl}phenyl) acetamide;
*N*-(4-{[(2-*tert*-Butyl-1-{[(2*R*)-1-methylpiperidin-2-yl]methyl}-1*H*-benzimidazol-5-yl)(methyl)amino]sulfonyl}phenyl)acetamide;
*N*-[4-({methyl[1-(tetrahydro-2*H*-pyran-4-ylmethyl)-2-(trifluoromethyl)-1*H*-benzimidazol-5-yl]amino}sulfonyl) phenyl]acetamide;
4-Bromo-*N*-[1-(cyclohexylmethyl)-2-(1,1-dimethylethyl)-1*H*-benzimidazol-5-yl]-*N*-methyl-benzenesulfonamide;
*N*-[2-*tert*-butyl-1-(cyclohexylmethyl)-1*H*-benzimidazol-5-yl]-4-[(2-hydroxyethyl)amino]-*N*-methylbenzenesulfonamide;
*N*-[2-*tert*-butyl-1-(cyclohexylmethyl)-1*H*-benzimidazol-5-yl]-4-(dimethylamino)-*N*-methylbenzenesulfonamide;
4-[bis(2-hydroxyethyl)amino]-*N*-[2-*tert*-butyl-1-(cyclohexylmethyl)-1*H*-benimidazol-5-yl]-*N*-methylbenzenesulfonamide;

*N*-[2-*tert*-butyl-1-(cyclohexylmethyl)-1*H*-benzimidazol-5-yl]-*N*,4-dimethyl-3,4-dihydro-2*H*-1,4-benzoxazine-7-sulfonamide;
*N*-[4-({methyl[2-(1-methyl-1-pyridin-2-ylethyl)-1-(tetrahydro-2*H*-pyran-4-ylmethyl)-1*H*-benzimidazol-5-yl]amino}sulfonyl)phenyl]acetamide;
*N*-(4-{[[2-*tert*-butyl-1-(tetrahydro-2*H*-pyran-4-ylmethyl)-1*H*-benzimidazol-5-yl](ethyl)amino]sulfonyl}phenyl) acetamide;
4-[(aminocarbonyl)amino]-*N*-[2-*tert*-butyl-1-(tetrahydro-2*H*-pyran-4-ylmethyl)-1*H*-benzimidazol-5-yl]-*N*-ethylbenzenesulfonamide;
*N*-[2-*tert*-butyl-1-(tetrahydro-2*H*-pyran-4-ylmethyl)-1*H*-benzimidazol-5-yl]-*N*-ethyl-4-{[(methylamino)carbonyl] amino}benzenesulfonamide;
4-amino-*N*-[2-*tert*-butyl-1-(tetrahydro-2*H*-pyran-4-ylmethyl)-1*H*-benzimidazol-5-yl]-*N*-ethylbenzenesulfonamide;
*N*-(4-{[[2-*tert*-butyl-1-(tetrahydro-2*H*-pyran-4-ylmethyl)-1*H*-benzimidazol-5-yl](ethyl)amino]sulfonyl}phenyl)-2,2-dimethylpropanamide;
2-[(4-{[[2-*tert*-butyl-1-(tetrahydro-2*H*-pyran-4-ylmethyl)-1*H*-benzimidazol-5-yl](ethyl)amino]sulfonyl}phenyl) amino]-2-oxoethyl acetate;
*N*-(4-{[[2-*tert*-butyl-1-(tetrahydro-2*H*-pyran-4-ylmethyl)-1*H*-benzimidazol-5-yl](ethyl)amino]sulfonyl}phenyl)-2-hydroxyacetamide;
*N*-[2-*tert*-butyl-1-(tetrahydro-2*H*-pyran-4-ylmethyl)-1*H*-benzimidazol-5-yl]-*N*-ethyl-4-{[(isopropylamino)carbonyl]amino}benzenesulfonamide;
*N*-[4-({ethyl[2-(1-methy-1-methylethyl)-1-(tetrahyhro-2*H*-pyran-4-ylmethyl)-1*H*-benzimidazol-5-yl]amino}sulfonyl)phenyl]acetamide;
4-[(aminocarbonyl)amino]-*N*-ethyl-*N*-[2-(1-methoxy-1-methylethyl)-1-(tetrahydro-2*H*-pyran-ylmethyl)-1*H*-benzimidazo]-5-yl]benzenesulfonamide;
*N*-ethyl-*N*-[2-(1-methoxy-1-methylethyl)-1-(tetrahydro-2*H*-pyran-4-ylmethyl)-1*H*-benzimidazol-5-yl]-4-{[(methylamino)carbonyl]amino}benzenesulfonamide;
4-amino-*N*-ethyl-*N*-[2-(1-methoxy-1-methylethyl)-1-(tetrahydro-2*H*-pyran-4-ylmethyl)-1*H*-benzimidazol-5-yl] benzenesulfonamide;
*N*-[4-( {ethyl [2-(1- methoxy- 1- methylethyl)- 1-(tetrahydro- 2*H*-pyran- 4- ylmethyl)- 1*H*-benzimidazol- 5- yl] amino}-sulfonyl)phenyl]-2,2-dimethylpropanamide;
2-{[4-({ethyl[2-(1-methoxy-1-methylethyl)-1-(tetrahydro-2*H*-pyran-4-ylmethyl)-1*H*-benzimidazol-5-yl]amino} sulfonyl)phenyl]amino}-2-oxoethyl acetate;
*N*-[4-({ethyl[2-(1-methoxy-1-methylethyl)-1-(tetrahydro-2*H*-pyran-4-ylmethyl)-1*H*-benzimidazol-5-yl]amino}sulfonyl)phenyl]-2-hydroxyacetamide;
*N*-ethyl-4-{[(isopropylamino)carbonyl]amino}-*N*-[2-(1-methoxy-1-methylethyl)-1-(tetrahydro-2*H*-pyran-4-ylmethyl)-1*H*-benzimidazol-5-yl]benzenesulfonamide;
*N*-(4-{[[2-(1-methoxy-1-methylethyl)-1-(tetrahydro-2*H*-pyran-4-ylmethyl)-1*H*-benzimidazol-5-yl](methyl)amino] sulfonyl}phenyl)acetamide;
4-[(aminocarbonyl)amino]-*N*-[2-(1-methoxy-1-methylethyl)-1-(tetrahydro-2*H*-pyran-4-ylmethyl)-1*H*-benzimidazol-5-yl]-*N*-methylbenzenesulfonamide;
2-Hydroxy-*N*-(4-{[[2-(1-methoxy-1-methylethyl)-1-(tetrahydro-2*H*-pyran-4-ylmethyl)-1*H*-benzimidazol-5-yl] (methyl)amino]sulfonyl}phenyl)acetamide;
*N*-(4-{[[2-(1-ethoxy-1-methylethyl)-1-(tetrahydro-2*H*-pyran-4-ylmethyl)-1*H*-benzimidazol-5-yl](methyl)amino] sulfonyl}phenyl)acetamide;
*N*-[4-({[1-(2-azetidin-1-ylethyl)-2-*tert*-butyl-1*H*-benzimidazol-5-yl]amino}sulfonyl)phenyl]acetamide;
3-[5-({[4-(acetylamino)phenyl]sulfonyl}amino)-2-*tert*-butyl-1*H*-benzimidazol-1-yl]propyl acetate;
*N*-{4-[({1-[(1*S*,4*S*)-bicyclo[2.2,1]hept-5-en-2-ylmethyl]-2-*tert*-butyl-1*H*-benzimidazol-5-yl}amino)sulfonyl]phenyl}acetamide;
*N*-[4-({[2-*tert*-butyl-1-(tetrahydro-2*H*-pyran-3-ylmethyl)-1*H*-benzimidazol-5-yl]amino}sulfonyl)phenyl]acetamide;
*N*-{4-[({2-*tert*-butyl-1-[2-(tetrahydro-2*H*-pyran-4-yl)ethyl]-1*H*-benzimidazol-5-yl}amino)sulfonyl]phenyl}acetamide;
*N*-(4-{[[2-*tert*-butyl-1-(cyclobutylmethyl)-1*H*-benzimidazol-5-yl](methyl)amino]sulfonyl}phenyl)acetamide;
4-[(aminocarbonyl) amino]-*N*-[2-*tert*-butyl- 1-(cyclobutylmethyl)- 1*H*- benzimidazol- 5- yl]-*N*-methylbenzenesulfonamide;
*N*-(4-{[[2-*tert*-butyl-1-(cylobutylmethyl)-1*H*-benzimidazol-5-yl](methyl)amino]sulfonyl}phenyl)-2,2-dimethylpropanamide;
*N*-(4-{[[2-(1,1-difluoroethyl)-1-(tetrahydro-2*H*-pyran-4-ylmethyl)-1*H*-benzimidazol-5-yl](methyl)amino]sulfonyl}phenyl)-2-hydroxyacetamide;
*N*-(4-{[[2-(1,1-difluoroethyl)-1-(tetrahydro-2*H*-pyran-4-ylmethyl)-1*H*-benzimidazol-5-yl](methyl)amino]sulfonyl}phenyl)acetamide;
*N*-(4-{[[2-(1,1-difluoroethyl)-1-(tetrahydro-2*H*-pyran-4-ylmethyl)-1*H*-benzimidazol-5-yl](methyl)amino]sulfonyl}phenyl)-3-methylbutanamide;
*N*-(4-{[[2-(1,1-difluoroethyl)-1-(tetrahydro-2*H*-pyran-4-ylmethyl)-1*H*-benzimidazol-5-yl](methyl)amino]sulfonyl}phenyl)-2,2-dimethylpropanamide;
*N*-[2-(1,1-difluoroethyl)-1-(tetrahydro-2*H*-pyran-4-ylmethyl)-1*H*-benzimidazol-5-yl]-4-{[(isopropylamino)carbonyl]amino}-*N*-methylbenzenesulfonamide;
4-{Bis[(isopropylamino)carbonyl]amino}-*N*-[2-(1,1-difluoroethyl)-1-(tetrahydro-2*H*-pyran-4-ylmethyl)-1*H*-benzimidazol-5-yl]-*N*-methylbenzenesulfonamide;
*N*-[4-({methyl[1-(tetrahydro-2*H*-pyran-4-ylmethyl)-2-(trifluoromethyl)-1*H*-benzimidazol-5-yl]amino}sulfonyl)phenyl]acetamide;
4-[(aminocarbonyl)amino]-*N*-methyl-*N*-[1-(tetrahydro-2*H*-pyran-4-ylmethyl)-2-(trifluoromethyl)-1*H*-benzimidazol-5-yl]benzenesulfonamide;
*N*-methyl-4-nitro-*N*-[1-(tetrahydro-2*H*-pyran-4-ylmethyl)-2-(trifluoromethyl)-1*H*-benzimidazol-5-yl]benzenesulfonamide;
4-amino-*N*-methyl-*N*-[1-(tetrahydro-2*H*-pyran-4-ylmethyl)-2-(trifluoromethyl)-1*H*-benzimidazol-5-yl]benzenesulfonamide;
2,2-dimethyl-*N*-[4-({methyl[1-(tetrahydro-2*H*-pyran-4-ylmethyl)-2-(trifluoromethyl)-1*H*-benzimidazol-5-yl]amino}sulfonyl)phenyl]propanamide;
2-{[4-({methyl[1-(tetrahydro-2*H*-pyran-4-ylmethyl)-2-(trifluoromethyl)-1*H*-benzimidazol-5-yl]amino}sulfonyl)phenyl]amino}-2-oxoethyl acetate;
4-{[(isopropylamino)carbonyl]amino}-*N*-methyl-*N*-[1-(tetrahydro-2*H*-pyran-4-ylmethyl)-2-(trifluoromethyl)-1*H*-benzimidazol-5-yl]benzenesulfonamide;
2-Hydroxy-*N*-[4-({methyl[1-(tetrahydro-2*H*-pyran-4-ylmethyl)-2-(trifluoromethyl)-1*H*-benzimidazol-5-yl]amino}sulfonyl)phenyl]acetamide

and pharmaceutically acceptable salts thereof.

**6.** A compound of Formula IA, a pharmaceutically acceptable salt thereof, diastereomers, enantiomers, or mixtures thereof:

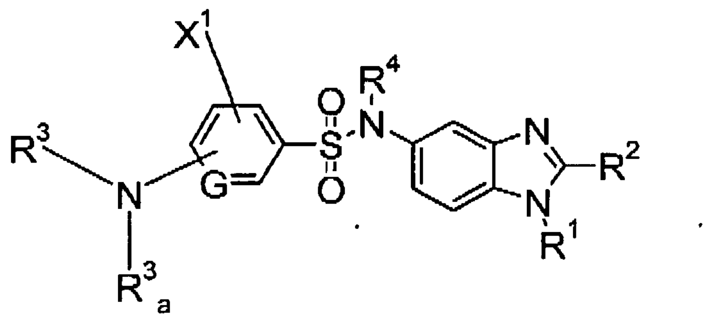

**IA**

wherein
G is CH or N;
$X^1$ is halogen;
$R^1$ is selected from $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloalkyl, wherein said $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloalkyl used in defining $R^1$ is optionally substituted by one or more groups selected from halogen, cyano, nitro, methoxy, ethoxy, methyl, ethyl, hydroxy, $CH_3C(=O)$-O-, amino, $C_{1-6}$alkylamino and di$C_{1-6}$alkylamino;
$R^2$ is selected from $C_{1-10}$alkyl, $C_{2-10}$alkenyl, $C_{3-10}$cycloalkyl, $C_{2-10}$cycloalkyl-$C_{1-4}$alkyl, and $C_{4-8}$cycloalkenyl-

$C_{1-4}$alkyl, wherein said $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl used in defining $R^2$ is optionally substituted by one or more groups selected from halogen, $C_{3-5}$heteroaryl, methoxy, ethoxy, methyl, ethyl, hydroxy, amino, $C_{1-6}$alkylamino and di$C_{1-6}$alkylamino;
$R^3$ and $R^3{}_a$ are independently selected from -H, $C_{1-6}$alkyl, $C_{2-6}$alkenyl, $C_{1-3}$alkyl-O-C(=O)-, $C_{1-6}$alkyl-HN-C(=O)-, $H_2$N-C(=O)-, and $C_{1-6}$acyl, wherein said $C_{1-6}$alkyl, $C_{2-6}$alkenyl, and $C_{1-6}$acyl used in defining $R^3$ is optionally substituted with one or more groups selected from $CH_3C(=O)$-O-, halogen, cyano, methoxy, ethoxy, hydroxy, amino, $C_{1-6}$alkylamino, di$C_{1-6}$alkylamino, and $C_{3-6}$heterocycloalkyl; and
$R^4$ is selected from -H, $C_{1-6}$alkyl, $C_{2-6}$alkenyl, $C_{3-6}$cycloalkyl, and $C_{3-6}$cycloalkyl-$C_{1-4}$alkyl.

**7.** A compound as claimed in claim 6 wherein
G is CH or N;
$R^1$ is selected from $C_{1-6}$alkyl, $C_{2-6}$alkenyl, $C_{3-6}$cycloalkyl-$C_{1-4}$alkyl, $C_{4-6}$cycloalkenyl-$C_{1-4}$alkyl and $C_{3-6}$heterocycloalkyl-$C_{1-4}$alkyl, wherein said $C_{1-6}$alkyl, $C_{2-6}$alkenyl, $C_{3-6}$cycloalkyl-$C_{1-4}$alkyl, $C_{4-6}$cycloalkenyl-$C_{1-4}$alkyl and $C_{3-6}$heterocycloalkyl-$C_{1-4}$alkyl used in defining $R^1$ is optionally substituted by one or more groups selected from halogen, methoxy, ethoxy, methyl, hydroxy, $CH_3C(=O)$-O-, amino, $C_{1-6}$alkylamino and di$C_{1-6}$alkylamino;
$R^2$ is selected from $C_{1-6}$alkyl, $C_{2-6}$alkenyl, $C_{3-6}$cycloalkyl, $C_{3-6}$cycloalkyl-$C_{1-4}$alkyl, and $C_{4-6}$cycloalkenyl-$C_{1-4}$alkyl, wherein said $C_{1-6}$alkyl, $C_{2-6}$alkenyl, $C_{3-6}$cycloalkyl, $C_{3-6}$cycloalkyl-$C_{1-4}$alkyl, and $C_{4-6}$cycloalkenyl-$C_{1-4}$alkyl used in defining $R^2$ is optionally substituted by one or more groups selected from halogen, $C_{3-5}$heteroaryl, methoxy, ethoxy and hydroxy;
$R^3$ is selected from -H, $C_{1-6}$alkyl, $C_{2-6}$alkenyl, $C_{1-3}$alkyl-O-C(-O)-, $C_{1-3}$alkyl-HN-C(=O)-, $H_2$N-C(=O)-, and $C_{1-6}$acyl, wherein said $C_{1-6}$alkyl, $C_{2-6}$alkenyl, and $C_{1-6}$acyl used in defining $R^3$ is optionally substituted with one or more groups selected from $CH_3C(=O)$-O-, halogen, methoxy, ethoxy, hydroxy, amino, methylamino, dimethylamino, and $C_{3-6}$heterocycloalkyl; and
$R^4$ is selected from -H and $C_{1-3}$alkyl.

**8.** A compound as claimed in claim 6 wherein
G is CH or N;
$R^1$ is selected from cyclopentyl-methyl, cyclohexyl-methyl, cyclobutyl-methyl, cyclopropyl-methyl, 4,4-difluorocyclohexanemethyl, bicyclo[2.2.1]hept-5-en-2-ylmethyl, tetrahydropyranyl-methyl, tetrahydropyranyl-ethyl, tetrahydrofuranyl-methyl, morpholinyl-methyl, piperdinylethyl, N-methyl-piperdinylmethyl, and piperdinyl-methyl;
$R^2$ is selected from t-butyl, n-butyl, 2-methyl-2-butyl, isopentyl, 2-methoxy-2-propyl, 2-hydroxy-propyl, trifluoromethyl, 1,1-difluoroethyl, 2,2,2-trifluoroethyl,1-methyl-propyl, 1,1-dimethyl-propyl, 1,1-dimethyl-3-buten-1-yl, ethyl, and 2-propyl;
$R^3$ is selected from -H, $C_{1-6}$alkyl, and $C_{1-6}$acyl, wherein said $C_{1-6}$alkyl, and $C_{1-6}$acyl used in defining $R^3$ is optionally substituted with one or more groups selected from $CH_3C(=O)$-O-, halogen, methoxy, hydroxy, amino, methylamino, dimethylamino, pyrrolidinyl, piperidinyl and morpholinyl; and
$R^4$ is selected from -H and methyl.

**9.** A compound as claimed in claim 6 wherein
GisCHorN;
$X^1$ is bromo;
$R^1$ is cyclohexyl-methyl, cyclobutyl-methyl, 4,4-difluorocyclohexanemethyl, N-methylpiperidine-2-yl methyl, and tetrahydropyranyl-methyl;
$R^2$ is t-butyl and 1,1-difluoroethyl;
$R^3$ is selected from -H, methyl, ethyl, propyl, 2-propyl, 2-hydroxyethyl, 2-methoxyethyl, formyl, acetyl, uriedo, N-isopropyl-ureido, ethylcarbonyl, 2-propylcarbonyl, t-butylcarbonyl, 2-amino-acetyl, 2-methylamino-acetyl, 2-dimethylamino-acetyl, 2-acetyloxy-acetyl, 2-hydroxy-acetyl, 2-bromo-acetyl, 2-(morpholin-1-yl)-acetyl, and 2-(pyrrolindin-1-yl)-acetyl; and
$R^4$ is selected from -H and methyl.

**10.** A compound of Formula IB, a pharmaceutically acceptable salt thereof, diastereomers, enantiomers, or mixtures thereof:

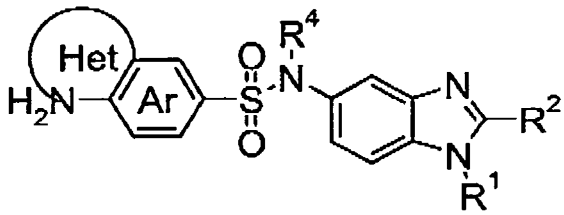

IB

wherein

$R^1$ is selected from $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloalkyl, wherein said $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloalkyl used in defining $R^1$ is optionally substituted by one or more groups selected from halogen, cyano, nitro, methoxy, ethoxy, methyl, ethyl, hydroxy, amino, $C_{1-6}$alkylamino and di$C_{1-6}$alkylamino;

$R^2$ is selected from $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl, wherein said $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl used in defining $R^2$ is optionally substituted by one or more groups selected from halogen, methoxy, ethoxy, methyl, ethyl, hydroxy, amino, $C_{1-6}$alkylamino and di$C_{1-6}$alkylamino;

"Het" is a nitrogen (as shown in Formula IB) containing heterocycle ring that is fused with phenyl ring "Ar," wherein "Het" is optionally substituted with one or more groups selected from $C_{1-3}$alkyl, halogen, cyano, methoxy, ethoxy, hydroxy, and amino; and

$R^4$ is selected from -H, $C_{1-6}$alkyl, $C_{2-6}$alkenyl, $C_{3-6}$cycloalkyl, and $C_{3-6}$cycloalkyl-$C_{1-4}$alkyl.

**11.** A compound as claimed in claim 10 wherein

$R^1$ is selected from $C_{1-6}$alkyl, $C_{2-6}$alkenyl, $C_{3-6}$cycloalkyl-$C_{1-4}$alkyl, $C_{4-6}$cycloalkenyl-$C_{1-4}$alkyl and $C_{3-6}$heterocycloalkyl-$C_{1-4}$alkyl, wherein said $C_{1-6}$alkyl, $C_{2-6}$alkenyl, $C_{3-6}$cycloalkyl-$C_{1-4}$alkyl, $C_{4-6}$cycloalkenyl-$C_{1-4}$alkyl and $C_{3-6}$heterocycloalkyl-$C_{1-4}$alkyl used in defining $R^1$ is optionally substituted by one or more groups selected from halogen, methoxy, ethoxy, methyl, hydroxy, amino, $C_{1-6}$alkylamino and di$C_{1-6}$alkylamino;

$R^2$ is selected from $C_{1-6}$alkyl, $C_{2-6}$alkenyl, $C_{3-6}$cycloalkyl, $C_{3-6}$cycloalkyl-$C_{1-4}$alkyl, and $C_{4-6}$cycloalkenyl-$C_{1-4}$alkyl, wherein said $C_{1-6}$alkyl, $C_{2-6}$alkenyl, $C_{3-6}$cycloalkyl, $C_{3-6}$cycloalkyl-$C_{1-4}$alkyl, and $C_{4-6}$cycloalkenyl-$C_{1-4}$alkyl used in defining $R^2$ is optionally substituted by one or more groups selected from halogen, methoxy, ethoxy and hydroxy;

"Het" is morpholinyl, wherein said morpholinyl is optionally substituted with one or more groups selected from $C_{1-3}$alkyl, halogen, cyano, methoxy, ethoxy, hydroxy, and amino; and

$R^4$ is selected from -H and $C_{1-3}$alkyl.

**12.** A compound as claimed in claim 10 wherein

$R^1$ is selected from cyclopentyl-methyl, cyclohexyl-methyl, cyclobutyl-methyl, cyclopropyl-methyl, 4,4-difluorocyclohexyl-methyl, bicyclo[2.2.1]hept-5-en-2-ylmethyl, tetrahydropyranyl-methyl, tetrahydropyranyl-ethyl, tetrahydrofuranyl-methyl; morpholinyl-methyl, piperdinylethyl, N-methyl-piperdinylmethyl, and piperdinyl-methyl;

$R^2$ is selected from t-butyl, n-butyl, 2-methyl-2-butyl, isopentyl, 2-methoxy-2-propyl, 2-hydroxy-propyl, trifluoromethyl, 1,1-difluoroethyl, 2,2,2-trifluoroethyl,1-methyl-propyl, 1,1-dimethyl-propyl, 1,1-dimethyl-3-buten-1-yl, ethyl, and 2-propyl;

"Het" is morpholinyl, wherein said morpholinyl is optionally substituted with one or more $C_{1-3}$alkyl; and

$R^4$ is selected from -H and methyl.

**13.** A compound as claimed in claim 10 wherein

$R^1$ is cyclohexyl-methyl, cyclobutyl-methyl, cyclopropyl-methyl, 4,4-difluorocyclohexanemethyl, N-methylpiperidine-2-yl methyl, and tetrahydropyranyl-methyl;

$R^2$ is t-butyl and 1,1-difluoroethyl;

"Het" is morpholinyl, wherein said morpholinyl is optionally substituted with one or more $C_{1-3}$alkyl; and

$R^4$ is selected from -H and methyl.

**14.** A compound according to any one of claims 1-13 for use as a medicament.

**15.** The use of a compound according to any one of claims 1-13 in the manufacture of a medicament for the therapy of pain.

**16.** The use of a compound according to any one of claims 1-13 in the manufacture of a medicament for the treatment of anxiety disorders.

**17.** The use of a compound according to any one of claims 1-13 in the manufacture of a medicament for the treatment of cancer, multiple sclerosis, Parkinson's disease, cancer, Huntington's chorea, Alzheimer's disease, gastrointestinal disorders and cardiovascular disorders.

**18.** A pharmaceutical composition comprising a compound according to any one of claims 1-13 and a pharmaceutically acceptable carrier.

**19.** A method for preparing a compound of Formula I,

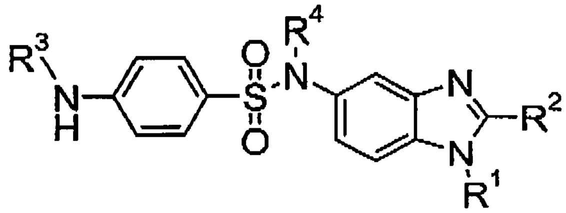

I

comprising:

reacting a compound of Formula II,

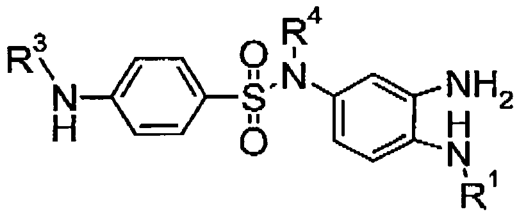

II

with a compound of $R^2COX$, in the presence of a base, such as an alkylamine, and optionally a coupling reagent, followed by treatment with an acid;

wherein
X is selected from Cl, Br, F and OH;
$R^1$ is selected from $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloakyl, wherein said $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocyclalkyl-$C_{1-4}$alkyl, $C_{3-10}$cycloalkyl, $C_{4-8}$cycloalkenyl, and $C_{3-6}$heterocycloalkyl used in defining $R^1$ is optionally substituted by one or more groups selected from halogen, cyano, nitro, methoxy, ethoxy, methyl, ethyl, hydroxy, amino, $C_{1-6}$alkylamino and di$C_{1-6}$alkylamino;
$R^2$ is selected from $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$akyl, wherein said $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl used in defining $R^2$ is optionally substituted by one or more groups selected from halogen, methoxy, methoxy, methyl, ethyl, hydroxy, amino, $C_{1-6}$alkylamino and di$C_{1-6}$alkylamino;
$R^3$ is selected from -H, $C_{1-6}$alkyl and $C_{1-6}$acyl optionally substituted with one or more groups selected from $CH_3C(=O)$-O-, halogen, cyano, methoxy, ethoxy, hydroxy, amino, alkylamino, dialkylamino, and $C_{3-6}$heterocycloalkyl; and

$R^4$ is selected from -H, $C_{1-6}$alkyl, $C_{2-6}$alkenyl, $C_{3-6}$cycloalkyl, and $C_{3-6}$cycloalkyl-$C_{1-4}$alkyl.

**20.** A compound of 2-Bromo-*N*-(4-{[[2-tert-butyl-1-(cyclohexylmethyl)-1*H*-benzimdazol-5-yl](methyl)amino]sulfonyl} phenyl)acetamide.

**21.** A method for preparing a compound of Formula IA,

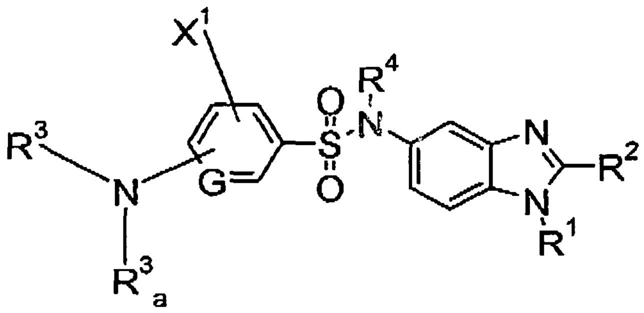

**IA**

comprising:

reacting a compound of Formula ILA,

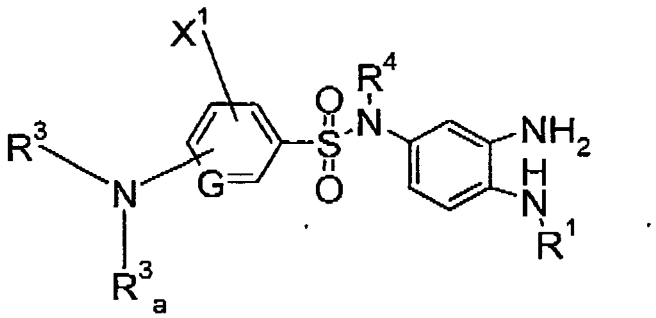

**IIA**

with a compound of $R^2COX$, in the presence of a base, such as an alkylamine, and optionally a coupling reagent, followed by treatment with an acid;

wherein
X and $X^1$ are independently selected from Cl, Br, F and OH;
G is CH or N;
$R^1$ is selected from $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloalkyl, wherein said $C_{1-10}$alkyl, $C_{2-10}$alkenyl, $C_{3-10}$cycloalkyl-$C_{1-4}$alkyl, $C_{4-8}$cycloalkenyl-$C_{1-4}$alkyl, $C_{3-6}$heterocycloalkyl-$C_{1-4}$alkyl, $C_{3-10}$cycloalkyl, $C_{4-8}$cycloalkenyl, and $C_{3-6}$heterocycloalkyl used in defining $R^1$ is optionally substituted by one or more groups selected from halogen, cyano, nitro, methoxy, ethoxy, methyl, ethyl, hydroxy, $CH_3C(=O)-O-$, amino, $C_{1-6}$alkylamino and di$C_{1-6}$alkylamino;
$R^2$ is selected from $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl, wherein said $C_{1-10}$alkyl, $C_{2-10}$alkenyl, $C_{3-10}$cycloalkyl, $C_{3-10}$cycloalkyl-$C_{1-4}$alkyl, and $C_{4-8}$cycloalkenyl-$C_{1-4}$alkyl used in defining $R^2$ is optionally substituted by one or more groups selected from halogen, $C_{3-5}$heteroaryl, methoxy, ethoxy, methyl, ethyl, hydroxy, amino, $C_{1-6}$alkylamino and di$C_{1-6}$alkylamino;
$R^3$ and $R^3_a$ are independently selected from -H, $C_{1-6}$alkyl, $C_{2-6}$alkenyl, $C_{1-3}$alkyl-O-C(=O)-, $C_{1-6}$alkyl-HN-C(=O)-,

$H_2N-C(=O)-$, and $C_{1-6}$acyl, wherein said $C_{1-6}$alkyl, $C_{2-6}$alkenyl, and $C_{1-6}$acyl used in defining $R^3$ is optionally substituted with one or more groups selected from $CH_3C(=O)-O-$, halogen, cyano, methoxy, ethoxy, hydroxy, amino, $C_{1-6}$alkylamino, di$C_{1-6}$alkylamino, and $C_{3-6}$heterocycloalkyl; and
$R^4$ is selected from -H, $C_{1-6}$alkyl, $C_{2-6}$alkenyl, $C_{3-6}$cycloalkyl, and $C_{3-6}$cycloalkyl-$C_{1-4}$alkyl.

**Patentansprüche**

1. Verbindungen der Formel I und deren pharmazeutisch annehmbare Salze:

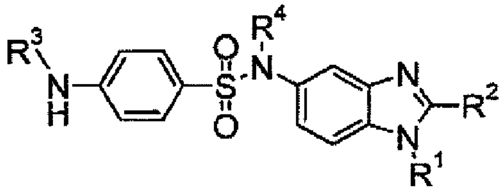

I

wobei
$R^1$ ausgewählt ist aus $C_{1-10}$-Alkyl, $C_{2-10}$-Alkenyl, $C_{3-10}$-Cycloalkyl-$C_{1-4}$-alkyl, $C_{4-8}$-Cycloalkenyl-$C_{1-4}$-alkyl, $C_{3-6}$-Heterocycloalkyl-$C_{1-4}$-alkyl, $C_{3-10}$-Cycloalkyl, $C_{4-8}$-Cycloalkenyl und $C_{3-6}$-Heterocycloalkyl, wobei die bei der Definition von $R^1$ verwendeten $C_{1-10}$-Alkyl-, $C_{2-10}$-Alkenyl-, $C_{3-10}$-Cycloalkyl-$C_{1-4}$-alkyl-, $C_{4-8}$-Cycloalkenyl-$C_{1-4}$-alkyl-, $C_{3-6}$-Heterocycloalkyl-$C_{1-4}$-alkyl-, $C_{3-10}$-Cycloalkyl-, $C_{4-8}$-Cycloalkenyl- und $C_{3-6}$-Heterocycloalkylreste gegebenenfalls durch eine oder mehrere Gruppen ausgewählt aus Halogen, Cyano, Nitro, Methoxy, Ethoxy, Methyl, Ethyl, Hydroxy, Amino, $C_{1-6}$-Alkylamino und Di-$C_{1-6}$-alkylamino substituiert sind;
$R^2$ ausgewählt ist aus $C_{1-10}$-Alkyl, $C_{2-10}$-Alkenyl, $C_{3-10}$-Cycloalkyl, $C_{3-10}$-Cycloalkyl-$C_{1-4}$-alkyl und $C_{4-8}$-Cycloalkenyl-$C_{1-4}$-alkyl, wobei die bei der Definition von $R^2$ verwendeten $C_{1-10}$-Alkyl-, $C_{2-10}$-Alkenyl-, $C_{3-10}$-Cycloalkyl-, $C_{3-10}$-Cycloalkyl-$C_{1-4}$-alkyl- und $C_{4-8}$-Cycloalkenyl-$C_{1-4}$-alkylreste gegebenenfalls durch eine oder mehrere Gruppen ausgewählt aus Halogen, Methoxy, Ethoxy, Methyl, Ethyl, Hydroxy, Amino, $C_{1-6}$-Alkylamino und Di-$C_{1-6}$-alkylamino substituiert sind;
$R^3$ ausgewählt ist aus -H, $C_{1-6}$-Alkyl, $C_{2-6}$-Alkenyl und $C_{1-6}$-Acyl, wobei die bei der Definition von $R^3$ verwendeten $C_{1-6}$-Alkyl-, $C_{2-6}$-Alkenyl- und $C_{1-6}$-Acylreste gegebenenfalls durch eine oder mehrere Gruppen ausgewählt aus $CH_3C(=O)-O-$, Halogen, Cyano, Methoxy, Ethoxy, Hydroxy, Amino, Alkylamino, Dialkylamino und $C_{3-6}$-Heterocycloalkyl substituiert sind; und
$R^4$ ausgewählt ist aus -H, $C_{1-6}$-Alkyl, $C_{2-6}$-Alkenyl, $C_{3-6}$-Cycloalkyl und $C_{3-6}$-Cycloalkyl-$C_{1-4}$-alkyl.

2. Verbindungen nach Anspruch 1, wobei
$R^1$ ausgewählt ist aus $C_{1-6}$-Alkyl, $C_{2-6}$-Alkenyl, $C_{3-6}$-Cycloalkyl-$C_{1-4}$-alkyl, $C_{4-6}$-Cycloalkenyl-$C_{1-4}$-alkyl und $C_{3-6}$-Heterocycloalkyl-$C_{1-4}$-alkyl, wobei die bei der Definition von $R^1$ verwendeten $C_{1-6}$-Alkyl-, $C_{2-6}$-Alkenyl-, $C_{3-6}$-Cycloalkyl-$C_{1-4}$-alkyl-, $C_{4-6}$-Cycloalkenyl-$C_{1-4}$-alkyl- und $C_{3-6}$-Heterocycloalkyl-$C_{1-4}$-alkylreste gegebenenfalls durch eine oder mehrere Gruppen ausgewählt aus Halogen, Methoxy, Ethoxy, Methyl, Hydroxy und Amino substituiert sind;
$R^2$ ausgewählt ist aus $C_{1-6}$-Alkyl, $C_{2-6}$-Alkenyl, $C_{3-6}$-Cycloalkyl, $C_{3-6}$-Cycloalkyl-$C_{1-4}$-alkyl und $C_{4-6}$-Cycloalkenyl-$C_{1-4}$-alkyl, wobei die bei der Definition von $R^2$ verwendeten $C_{1-6}$-Alkyl-, $C_{2-6}$-Alkenyl-, $C_{3-6}$-Cycloalkyl-, $C_{3-6}$-Cycloalkyl-$C_{1-4}$-alkyl- und $C_{4-6}$-Cycloalkenyl-$C_{1-4}$-alkylreste gegebenenfalls durch eine oder mehrere Gruppen ausgewählt aus Halogen, Methoxy, Ethoxy und Hydroxy substituiert sind;
$R^3$ ausgewählt ist aus -H, $C_{1-6}$-Alkyl, $C_{2-6}$-Alkenyl und $C_{1-6}$-Acyl, wobei die bei der Definition von $R^3$ verwendeten $C_{1-6}$-Alkyl-, $C_{2-6}$-Alkenyl- und $C_{1-6}$-Acylgruppen gegebenenfalls durch eine oder mehrere Gruppen ausgewählt aus $CH_3C(=O)-O-$, Halogen, Methoxy, Ethoxy, Hydroxy, Amino, Methylamino, Dimethylamino und $C_{3-6}$-Heterocycloalkyl substituiert sind; und $R^4$ ausgewählt ist aus -H und $C_{1-3}$-Alkyl.

3. Verbindungen nach Anspruch 1, wobei
$R^1$ ausgewählt ist aus Cyclopentylmethyl, Cyclohexylmethyl, Cyclobutylmethyl, Cyclopropylmethyl, 4,4-Difluorcyclohexylmethyl, Bicyclo[2.2.1]hept-5-en-2-ylmethyl, Tetrahydropyranylmethyl, Tetrahydropyranylethyl, Tetrahydrofuranylmethyl, Morpholinylmethyl, Piperdinylethyl, N-Methylpiperdinylmethyl und Piperdinylmethyl;
$R^2$ ausgewählt ist aus t-Butyl, n-Butyl, 2-Methyl-2-butyl, Isopentyl, 2-Methoxy-2-propyl, 2-Hydroxypropyl, Trifluormethyl, 1,1-Difluorethyl, 2,2,2-Trifluorethyl, 1-Methylpropyl, 1,1-Dimethylpropyl, 1,1-Dimethyl-3-buten-1-yl, Ethyl

und 2-Propyl;
$R^3$ ausgewählt ist aus -H, $C_{1-6}$-Alkyl und $C_{1-6}$-Acyl, wobei die bei der Definition von $R^3$ verwendeten $C_{1-6}$-Alkyl- und $C_{1-6}$-Acylreste gegebenenfalls durch eine oder mehrere Gruppen ausgewählt aus $CH_3C(=O)$-0-, Halogen, Methoxy, Hydroxy, Amino, Methylamino, Dimethylamino, Pyrrolidinyl und Morpholinyl substituiert sind; und
$R^4$ ausgewählt ist aus -H und Methyl.

**4.** Verbindungen nach Anspruch 1, wobei
$R^1$ ausgewählt ist aus Cyclohexylmethyl, Cyclopentylmethyl, Cyclobutylmethyl, Cyclopropylmethyl, 4,4-Difluorcyclohexylmethyl und Tetrahydropyranylmethyl;
$R^2$ für t-Butyl oder 1,1-Difluorethyl steht;
$R^3$ ausgewählt ist aus -H, Methyl, Ethyl, Propyl, 2-Propyl, 2-Hydroxyethyl, 2-Methoxyethyl, Formyl, Acetyl, Ethylcarbonyl, 2-Propylcarbonyl, t-Butylcarbonyl, Ureido, N-Isopropylureido, 2-Aminoacetyl, 2-Methylaminoacetyl, 2-Dimethylaminoacetyl, 2-Acetyloxyacetyl, 2-Hydroxyacetyl, 2-Bromacetyl, 2-(Morpholin-1-yl)acetyl und 2-(Pyrrolidin-1-yl)acetyl; und $R^4$ ausgewählt ist aus -H und Methyl.

**5.** Verbindungen, ausgewählt aus:

N-(4-{[[2-tert.-Butyl-1-(cyclohexylmethyl)-1H-benzimidazol-5-yl](methyl)amino]sulfonyl}phenyl)acetamid;
N-[2-tert.-Butyl-1-(cyclohexylmethyl)-1H-benzimidazol-5-yl]-N-methyl-4-nitrobenzolsulfonsäureamid;
4-Amino-N-[2-tert.-butyl-1-(cyclohexylmethyl)-1H-benzimidazol-5-yl]-N-methylbenzolsulfonsäureamid;
N-(4-{[[2-tert.-Butyl-1-(cyclohexylmethyl)-1H-benzimidazol-5-yl](methyl)amino]sulfonyl}phenyl)propanamid;
N-(4-{[[2-tert.-Butyl-1-(cyclohexylmethyl)-1H-benzimidazol-5-yl](methyl)amino]sulfonyl}phenyl)-2-methylpropanamid;
N-(4-{[[2-tert.-Butyl-1-(cyclohexylmethyl)-1H-benzimidazol-5-yl](methyl)amino]sulfonyl}phenyl)-2,2-dimethylpropanamid;
N-[2-tert.-Butyl-1-(cyclohexylmethyl)-1H-benzimidazol-5-yl]-4-(ethylamino)-N-methylbenzolsulfonsäureamid;
N-[2-tert.-Butyl-1-(cyclohexylmethyl)-1H-benzimidazol-5-yl]-4-(formylamino)-N-methylbenzolsulfonsäureamid;
N-(4-{[[2-tert.-Butyl-1-(cyclohexylmethyl)-1H-benzimidazol-5-yl](methyl)amino]sulfonyl}phenyl)-2-pyrrolidin-1-ylacetamid;
$N^1$-(4-{[[2-tert.-Butyl-1-(cyclohexylmethyl)-1H-benzimidazol-5-yl](methyl)amino]sulfonyl}phenyl)-$N^2$,$N^2$-dimethylglycinamid;
N-(4-{[[2-tert.-Butyl-1-(cyclohexylmethyl)-1H-benzimidazol-5-yl](methyl)amino]sulfonyl}phenyl)-2-morpholin-4-ylacetamid;
$N^1$-(4-{[[2-tert.-Butyl-1-(cyclohexylmethyl)-1H-benzimidazol-5-yl](methyl)amino]sulfonyl}phenyl)glycinamid;
Essigsäure-2-[(4-{[[2-tert.-butyl-1-(cyclohexylmethyl)-1H-benzimidazol-5-yl](methyl)amino]sulfonyl}phenyl)amino]-2-oxoethylester;
N-(4-{[[2-tert.-Butyl-1-(cyclohexylmethyl)-1H-benzimidazol-5-yl](methyl)amino]sulfonyl}phenyl)-2-hydroxyacetamid;
5-Brom-N-[2-tert.-butyl-1-(cyclohexylmethyl)-1H-benzimidazol-5-yl]-6-chlor-N-methylpyridin-3-sulfonsäureamid;
5-Brom-N-[2-tert.-butyl-1-(cyclohexylmethyl)-1H-benzimidazol-5-yl]-6-[(2-hydroxyethyl)amino]-N-methylpyridin-3-sulfonsäureamid;
N-[2-tert.-Butyl-1-(cyclohexylmethyl)-1H-benzimidazol-5-yl]-6-[(2-hydroxyethyl)amino]-N-methylpyridin-3-sulfonsäureamid;
N-(5-{[[2-tert.-Butyl-1-(cyclohexylmethyl)-1H-benzimidazol-5-yl](methyl)amino]sulfonyl}pyridin-2-yl)acetamid;
N-(3-{[[2-tert.-Butyl-1-(cyclohexylmethyl)-1H-benzimidazol-5-yl](methyl)amino]sulfonyl}phenyl)acetamid;
$N^1$-(4-{[[2-tert.-Butyl-1-(cyclohexylmethyl)-1H-benzimidazol-5-yl](methyl)amino]sulfonyl}phenyl)-$N^2$-(2-hydroxyethyl)glycinamid;
4-[(Aminocarbonyl)amino]-N-[2-tert.-butyl-1-(tetrahydro-2H-pyran-4-ylmethyl)-1H-benzimidazol-5-yl]-N-methylbenzolsulfonsäureamid;
N-(4-{[[2-tert.-Butyl-1-(tetrahydro-2H-pyran-4-ylmethyl)-1H-benzimidazol-5-yl](methyl)amino]sulfonyl}phenyl)acetamid;
N-(4-{[[2-tert.-Butyl-1-(tetrahydro-2H-pyran-4-ylmethyl)-1H-benzimidazol-5-yl](methyl)amino]sulfonyl}phenyl)-N-methylacetamid;
N-(4-{[[2-tert.-Butyl-1-(tetrahydro-2H-pyran-4-ylmethyl)-1H-benzimidazol-5-yl](methyl)amino]sulfonyl}phenyl)-2,2-dimethylpropanamid;
N-(4-{[[2-tert.-Butyl-1-(tetrahydro-2H-pyran-4-ylmethyl)-1H-benzimidazol-5-yl](methyl)amino]sulfonyl}phenyl)-2-hydroxyacetamid;

$N^1$-(4-{[[2-tert.-Butyl-1-(tetrahydro-2H-pyran-4-ylmethyl)-1H-benzimidazol-5-yl](methyl)amino]sulfonyl}phenyl)-$N^2$,$N^2$-dimethylglycinamid;
$N^1$-(4-{[[2-tert.-Butyl-1-(tetrahydro-2H-pyran-4-ylmethyl)-1H-benzimidazol-5-yl](methyl)amino]sulfonyl}phenyl) glycinamid;
$N^1$-(4-{[[2-tert.-Butyl-1-(tetrahydro-2H-pyran-4-ylmethyl)-1H-benzimidazol-5-yl](methyl)amino]sulfonyl}phenyl)-$N^2$-methylglycinamid;
N-[2-tert.-Butyl-1-(tetrahydro-2H-pyran-4-ylmethyl)-1H-benzimidazol-5-yl]-6-[(2-hydroxyethyl)amino]-N-methylpyridin-3-sulfonsäureamid;
N-[2-tert.-Butyl-1-(tetrahydro-2H-pyran-4-ylmethyl)-1H-benzimidazol-5-yl]-6-[(2-methoxyethyl)amino]-N-methylpyridin-3-sulfonsäureamid;
N-[2-tert.-Butyl-1-(tetrahydro-2H-pyran-4-ylmethyl)-1H-benzimidazol-5-yl]-6-(formylamino)-N-methylpyridin-3-sulfonsäureamid;
N-(5-{[[2-tert.-Butyl-1-(tetrahydro-2H-pyran-4-ylmethyl)-1H-benzimidazol-5-yl](methyl)amino]sulfonyl}pyridin-2-yl)acetamid;
N-[4-({[2-tert.-Butyl-1-(tetrahydro-2H-pyran-4-ylmethyl)-1H-benzimidazol-5-yl]amino}sulfonyl)phenyl]acetamid;
N-[4-({[2-tert.-Butyl-1-(cyclohexylmethyl)-1H-benzimidazol-5-yl]amino}sulfonyl)phenyl]acetamid;
N-(4-{[[2-tert.-Butyl-1-(2-piperidin-1-ylethyl)-1H-benzimidazol-5-yl(methyl)amino]sulfonyl}phenyl)acetamid;
N-(4-{[[2-tert.-Butyl-1-(1,4-dioxan-2-ylmethyl)-1H-benzimidazol-5-yl](methyl)amino]sulfonyl}phenyl)acetamid;
N-(4-{[{2-tert.-Butyl-1-[(1-methylpiperidin-2-yl)methyl]-1H-benzimidazol-5-yl}(methyl)amino]sulfonyl}phenyl) acetamid;
N-(4-{[(2-tert.-Butyl-1-{[(2R)-1-methylpiperidin-2-yl]methyl}-1H-benzimidazol-5-yl)(methyl)amino]sulfonyl}phenyl)acetamid;
N-[4-({Methyl[1-(tetrahydro-2H-pyran-4-ylmethyl)-2-(trifluormethyl)-1H-benzimidazol-5-yl]amino}sulfonyl)phenyl]acetamid;
4-Brom-N-[1-(cyclohexylmethyl)-2-(1,1-dimethylethyl)-1H-benzimidazol-5-yl]-N-methylbenzolsulfonsäureamid;
N-[2-tert.-Butyl-1-(cyclohexylmethyl)-1H-benzimidazol-5-yl]-4-[(2-hydroxyethyl)amino]-N-methylbenzolsulfonsäureamid;
N-[2-tert.-Butyl-1-(cyclohexylmethyl)-1H-benzimidazol-5-yl]-4-(dimethylamino)-N-methylbenzolsulfonsäureamid;
4-[Bis(2-hydroxyethyl)amino]-N-[2-tert.-butyl-1-(cyclohexylmethyl)-1H-benzimidazol-5-yl]-N-methylbenzolsulfonsäureamid;
N-[2-tert.-Butyl-1-(cyclohexylmethyl)-1H-benzimidazol-5-yl]-N,4-dimethyl-3,4-dihydro-2H-1,4-benzoxazin-7-sulfonsäureamid;
N-[4-({Methyl[2-(1-methyl-1-pyridin-2-ylethyl)-1-(tetrahydro-2H-pyran-4-ylmethyl)-1H-benzimidazol-5-yl]amino}sulfonyl)phenyl]acetamid;
N-(4-{[[2-tert.-Butyl-1-(tetrahydro-2H-pyran-4-ylmethyl)-1H-benzimidazol-5-yl](ethyl)amino]sulfonyl}phenyl) acetamid;
4-[(Aminocarbonyl)amino]-N-[2-tert.-butyl-1-(tetrahydro-2H-pyran-4-ylmethyl)-1H-benzimidazol-5-yl]-N-ethylbenzolsulfonsäureamid;
N-[2-tert.-Butyl-1-(tetrahydro-2H-pyran-4-ylmethyl)-1H-benzimidazol-5-yl]-N-ethyl-4-{[(methylamino)carbonyl] amino}benzolsulfonsäureamid;
4-Amino-N-[2-tert.-butyl-1-(tetrahydro-2H-pyran-4-ylmethyl)-1H-benzimidazol-5-yl]-N-ethylbenzolsulfonsäureamid;
N-(4-{[[2-tert.-Butyl-1-(tetrahydro-2H-pyran-4-ylmethyl)-1H-benzimidazol-5-yl](ethyl)amino]sulfonyl}phenyl)-2,2-dimethylpropanamid;
2-[(4-{[[2-tert.-Butyl-1-(tetrahydro-2H-pyran-4-ylmethyl)-1H-benzimidazol-5-yl](ethyl)amino]sulfonyl}phenyl) amino]-2-oxoethylacetat;
N-(4-{[[2-tert.-Butyl-1-(tetrahydro-2H-pyran-4-ylmethyl)-1H-benzimidazol-5-yl](ethyl)amino]sulfonyl}phenyl)-2-hydroxyacetamid;
N-[2-tert.-Butyl-1-(tetrahydro-2H-pyran-4-ylmethyl)-1H-benzimidazol-5-yl]-N-ethyl-4-{[(isopropylamino)carbonyl]amino}benzolsulfonsäureamid;
N-[4-({Ethyl[2-(1-methoxy-1-methylethyl)-1-(tetrahydro-2H-pyran-4-ylmethyl)-1H-benzimidazol-5-yl]amino} sulfonyl)phenyl]acetamid;
4-[(Aminocarbonyl)amino]-N-ethyl-N-[2-(1-methoxy-1-methylethyl)-1-(tetrahydro-2H-pyran-4-ylmethyl)-1H-benzimidazol-5-yl]benzolsulfonsäureamid;
N-Ethyl-N-[2-(1-methoxy-1-methylethyl)-1-(tetrahydro-2H-pyran-4-ylmethyl)-1H-benzimidazol-5-yl]-4-{[(methylamino)carbonyl]amino}benzolsulfonsäureamid;
4-Amino-N-ethyl-N-[2-(1-methoxy-1-methylethyl)-1-(tetrahydro-2H-pyran-4-ylmethyl)-1H-benzimidazol-5-yl]benzolsulfonsäureamid;
N-[4-({Ethyl[2-(1-methoxy-1-methylethyl)-1-(tetrahydro-2H-pyran-4-ylmethyl)-1H-benzimidazol-5-yl]amino}sulfonyl)phenyl]-2,2-dimethylpropanamid;
Essigsäure-2-{[[4-({ethyl[2-(1-methoxy-1-methylethyl)-1-(tetrahydro-2H-pyran-4-ylmethyl)-1H-benzimidazol-5-yl]amino}sulfonyl)phenyl]amino}-2-oxoethylester;
N-[4-({Ethyl[2-(1-methoxy-1-methylethyl)-1-(tetrahydro-2H-pyran-4-ylmethyl)-1H-benzimidazol-5-yl]amino}sulfonyl)phenyl]-2-hydroxyacetamid;
N-Ethyl-4-{[(isopropylamino)carbonyl]amino}-N-[2-(1-methoxy-1-methylethyl)-1-(tetrahydro-2H-pyran-4-ylmethyl)-1H-benzimidazol-5-yl]benzolsulfonsäureamid;
N-(4-{[[2-(1-Methoxy-1-methylethyl)-1-(tetrahydro-2H-pyran-4-ylmethyl)-1H-benzimidazol-5-yl](methyl)amino]sulfonyl}phenyl)acetamid;
4-[(Aminocarbonyl)amino]-N-[2-(1-methoxy-1-methylethyl)-1-(tetrahydro-2H-pyran-4-ylmethyl)-1H-benzimidazol-5-yl]-N-methylbenzolsulfonsäureamid;
2-Hydroxy-N-(4-{[[2-(1-methoxy-1-methylethyl)-1-(tetrahydro-2H-pyran-4-ylmethyl)-1H-benzimidazol-5-yl](methyl)amino]sulfonyl}phenyl)acetamid;
N-(4-{[[2-(1-Ethoxy-1-methylethyl)-1-(tetrahydro-2H-pyran-4-ylmethyl)-1H-benzimidazol-5-yl](methyl)amino]sulfonyl}phenyl)acetamid;
N-[4-({[1-(2-Azetidin-1-ylethyl)-2-tert.-butyl-1H-benzimidazol-5-yl]amino}sulfonyl)phenyl]acetamid; Essigsäure-3-[5-({[4-(acetylamino)phenyl]sulfonyl}amino)-2-tert.-butyl-1H-benzimidazol-1-yl]propylester;
N-{4-[({1-[(1S,4S)-Bicyclo[2.2.1]hept-5-en-2-ylmethyl]-2-tert.-butyl-1H-benzimidazol-5-yl}amino)sulfonyl]phenyl}acetamid;
N-[4-({[2-tert.-Butyl-1-(tetrahydro-2H-pyran-3-ylmethyl)-1H-benzimidazol-5-yl]amino}sulfonyl)phenyl]acetamid;
N-{4-[({2-tert.-Butyl-1-[2-(tetrahydro-2H-pyran-4-yl)ethyl]-1H-benzimidazol-5-yl}amino)sulfonyl]phenyl}acetamid;
N-(4-{[[2-tert.-Butyl-1-(cyclobutylmethyl)-1H-benzimidazol-5-yl](methyl)amino]sulfonyl}phenyl)acetamid;
4-[(Aminocarbonyl)amino]-N-[2-tert.-butyl-1-(cyclobutylmethyl)-1H-benzimidazol-5-yl]-N-methylbenzolsulfonsäureamid;
N-(4-{[[2-tert.-Butyl-1-(cyclobutylmethyl)-1H-benzimidazol-5-yl](methyl)amino]sulfonyl}phenyl)-2,2-dimethylpropanamid;
N-(4-{[[2-(1,1-Difluormethyl)-1-(tetrahydro-2H-pyran-4-ylmethyl)-1H-benzimidazol-5-yl](methyl)amino]sulfonyl}phenyl)-2-hydroxyacetamid;
N-(4-{[[2-(1,1-Difluormethyl)-1-(tetrahydro-2H-pyran-4-ylmethyl)-1H-benzimidazol-5-yl](methyl)amino]sulfonyl}phenyl)acetamid;
N-(4-{[[2-(1,1-Difluorethyl)-1-(tetrahydro-2H-pyran-4-ylmethyl)-1H-benzimidazol-5-yl](methyl)amino]sulfonyl}phenyl)-3-methylbutanamide;
N-(4-{[[2-(1,1-Difluormethyl)-1-(tetrahydro-2H-pyran-4-ylmethyl)-1H-benzimidazol-5-yl](methyl)amino]sulfonyl}phenyl)-2,2-dimethylpropanamid;
N-[2-(1,1-Difluorethyl)-1-(tetrahydro-2H-pyran-4-ylmethyl)-1H-benzimidazol-5-yl]-4-{[(isopropylamino)carbonyl]amino}-N-methylbenzolsulfonsäureamid;
4-{Bis[(isopropylamino)carbonyl]amino-N-[2-(1,1-difluorethyl)-1-(tetrahydro-2H-pyran-4-ylmethyl)-1H-benzimidazol-5-yl]-N-methylbenzolsulfonsäureamid;
N-[4-({Methyl[1-(tetrahydro-2H-pyran-4-ylmethyl)-2-(trifluormethyl)-1H-benzimidazol-5-yl]amino}sulfonyl)phenyl]acetamid;
4-[(Aminocarbonyl)amino]-N-methyl-N-[1-(tetrahydro-2H-pyran-4-ylmethyl)-2-(trifluormethyl)-1H-benzimidazol-5-yl]benzolsulfonsäureamid;
N-Methyl-4-nitro-N-[1-(tetrahydro-2H-pyran-4-ylmethyl)-2-(trifluormethyl)-1H-benzimidazol-5-yl]benzolsulfonsäureamid;
4-Amino-N-methyl-N-[1-(tetrahydro-2H-pyran-4-ylmethyl)-2-(trifluormethyl)-1H-benzimidazol-5-yl]benzolsulfonsäureamid;
2,2-Dimethyl-N-[4-({methyl[1-(tetrahydro-2H-pyran-4-ylmethyl)-2-(trifluormethyl)-1H-benzimidazol-5-yl]amino}sulfonyl)phenyl]propanamid;
Essigsäure-2-{[4-[1-(tetrahydro-2H-pyran-4-ylmethyl)-2-(trifluormethyl)-1H-benzimidazol-5-yl]amino}sulfonyl)phenyl]amino}-2-oxoethylester;
4-{[(Isopropylamino)carbonyl]amino}-N-methyl-N-[1-(tetrahydro-2H-pyran-4-ylmethyl)-2-(trifluormethyl)-1H-benzimidazol-5-yl]benzolsulfonsäureamid;

2-Hydroxy-N-[4-({methyl[1-(tetrahydro-2H-pyran-4-ylmethyl)-2-(trifluormethyl)-1H-benzimidazol-5-yl]amino}sulfonyl)phenyl]acetamid und deren pharmazeutisch annehmbaren Salzen.

**6.** Verbindungen der Formel IA und deren pharmazeutisch annehmbare Salze, Diastereomere, Enantiomere und Mischungen davon:

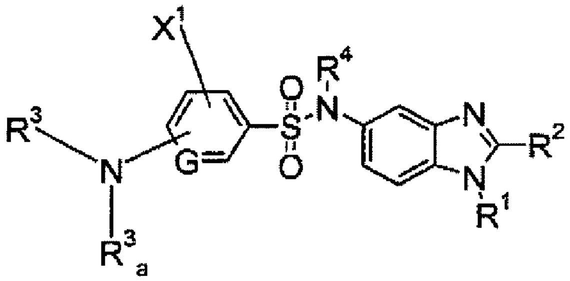

IA

wobei
G für CH oder N steht;
$X^1$ für Halogen steht;
$R^1$ ausgewählt ist aus $C_{1-10}$-Alkyl, $C_{2-10}$-Alkenyl, $C_{3-10}$-Cycloalkyl-$C_{1-4}$-alkyl, $C_{4-8}$-Cycloalkenyl-$C_{1-4}$-alkyl, $C_{3-6}$-Heterocycloalkyl-$C_{1-4}$-alkyl, $C_{3-10}$-Cycloalkyl, $C_{4-8}$-Cycloalkenyl und $C_{3-6}$-Heterocycloalkyl, wobei die bei der Definition von $R^1$ verwendeten $C_{1-10}$-Alkyl-, $C_{2-10}$-Alkenyl-, $C_{3-10}$-Cycloalkyl-$C_{1-4}$-alkyl-, $C_{4-8}$-Cycloalkenyl-$C_{1-4}$-alkyl-, $C_{3-6}$-Heterocycloalkyl-$C_{1-4}$-alkyl-, $C_{3-10}$-Cycloalkyl-, $C_{4-8}$-Cycloalkenyl- und $C_{3-6}$-Heterocycloalkylreste gegebenenfalls durch eine oder mehrere Gruppen ausgewählt aus Halogen, Cyano, Nitro, Methoxy, Ethoxy, Methyl, Ethyl, Hydroxy, $CH_3C(=O)$-O-, Amino, $C_{1-6}$-Alkylamino und Di-$C_{1-6}$-alkylamino substituiert sind;
$R^2$ ausgewählt ist aus $C_{1-10}$-Alkyl, $C_{2-10}$-Alkenyl, $C_{3-10}$-Cycloalkyl, $C_{3-10}$-Cycloalkyl-$C_{1-4}$-alkyl und $C_{4-8}$-Cycloalkenyl-$C_{1-4}$-alkyl, wobei die bei der Definition von $R^2$ verwendeten $C_{1-10}$-Alkyl-, $C_{2-10}$-Alkenyl-, $C_{3-10}$-Cycloalkyl-, $C_{3-10}$-Cycloalkyl-$C_{1-4}$-alkyl- und $C_{4-8}$-Cycloalkenyl-$C_{1-4}$-alkylreste gegebenenfalls durch eine oder mehrere Gruppen ausgewählt aus Halogen, $C_{3-5}$-Heteroaryl, Methoxy, Ethoxy, Methyl, Ethyl, Hydroxy, Amino, $C_{1-6}$-Alkylamino und Di-$C_{1-6}$-alkylamino substituiert sind;
$R^3$ und $R^{3a}$ unabhängig voneinander ausgewählt sind aus -H, $C_{1-6}$-Alkyl, $C_{2-6}$-Alkenyl, $C_{1-3}$-Alkyl-O-C (=0) -, $C_{1-6}$-Alkyl-HN-C(=O)-, $H_2N$-C(=O)- und $C_{1-6}$-Acyl, wobei die bei der Definition von $R^3$ verwendeten $C_{1-6}$-Alkyl-, $C_{2-6}$-Alkenyl- und $C_{1-6}$-Acylreste gegebenenfalls durch eine oder mehrere Gruppen ausgewählt aus $CH_3C(=O)$-O-, Halogen, Cyano, Methoxy, Ethoxy, Hydroxy, Amino, $C_{1-6}$-Alkylamino, Di-$C_{1-6}$-alkylamino und $C_{3-6}$-Heterocycloalkyl substituiert sind; und
$R^4$ ausgewählt ist aus -H, $C_{1-6}$-Alkyl, $C_{2-6}$-Alkenyl, $C_{3-6}$-Cycloalkyl und $C_{3-6}$-Cycloalkyl-$C_{1-4}$-alkyl.

**7.** Verbindungen nach Anspruch 6, wobei
G für CH oder N steht;
$R^1$ ausgewählt ist aus $C_{1-6}$-Alkyl, $C_{2-6}$-Alkenyl, $C_{3-6}$-Cycloalkyl-$C_{1-4}$-alkyl, $C_{4-6}$-Cycloalkenyl-$C_{1-4}$-alkyl und $C_{3-6}$-Heterocycloalkyl-$C_{1-4}$-alkyl, wobei die bei der Definition von $R^1$ verwendeten $C_{1-6}$-Alkyl-, $C_{2-6}$-Alkenyl-, $C_{3-6}$-Cycloalkyl-$C_{1-4}$-alkyl-, $C_{4-6}$-Cycloalkenyl-$C_{1-4}$-alkyl- und $C_{3-6}$-Heterocycloalkyl-$C_{1-4}$-alkylreste gegebenenfalls durch eine oder mehrere Gruppen ausgewählt aus Halogen, Methoxy, Ethoxy, Methyl, Hydroxy $CH_3C(=O)$-O-, Amino, $C_{1-6}$-Alkylamino und Di-$C_{1-6}$-alkylamino substituiert sind;
$R^2$ ausgewählt ist aus $C_{1-6}$-Alkyl, $C_{2-6}$-Alkenyl, $C_{3-6}$-Cycloalkyl, $C_{3-6}$-Cycloalkyl-$C_{1-4}$-alkyl und $C_{4-6}$-Cycloalkenyl-$C_{1-4}$-alkyl, wobei die bei der Definition von $R^2$ verwendeten $C_{1-6}$-Alkyl-, $C_{2-6}$-Alkenyl-, $C_{3-6}$-Cycloalkyl-, $C_{3-6}$-Cycloalkyl-$C_{1-4}$-alkyl- und $C_{4-6}$-Cycloalkenyl-$C_{1-4}$-alkylreste gegebenenfalls durch eine oder mehrere Gruppen ausgewählt aus Halogen, $C_{3-5}$-Heteroaryl, Methoxy, Ethoxy und Hydroxy substituiert sind;
$R^3$ ausgewählt ist aus -H, $C_{1-6}$-Alkyl, $C_{2-6}$-Alkenyl, $C_{1-3}$-Alkyl-O-C(=O)-, $C_{1-3}$-Alkyl-HN-C(=O)-, $H_2N$-C(=O)-und $C_{1-6}$-Acyl, wobei die bei der Definition von $R^3$ verwendeten $C_{1-6}$-Alkyl-, $C_{2-6}$-Alkenyl- und $C_{1-6}$-Acylgruppen gegebenenfalls durch eine oder mehrere Gruppen ausgewählt aus $CH_3C(=O)$-O-, Halogen, Methoxy, Ethoxy, Hydroxy, Amino, Methylamino, Dimethylamino und $C_{3-6}$-Heterocycloalkyl substituiert sind; und $R^4$ ausgewählt ist aus -H und $C_{1-3}$-Alkyl.

**8.** Verbindungen nach Anspruch 6, wobei

G für CH oder N steht;
$R^1$ ausgewählt ist aus Cyclopentylmethyl, Cyclohexylmethyl, Cyclobutylmethyl, Cyclopropylmethyl, 4,4-Difluorcyclohexanmethyl, Bicyclo[2.2.1]hept-5-en-2-ylmethyl, Tetrahydropyranylmethyl, Tetrahydropyranylethyl, Tetrahydrofuranylmethyl, Morpholinylmethyl, Piperdinylethyl, N-Methylpiperdinylmethyl und Piperdinylmethyl;
$R^2$ ausgewählt ist aus t-Butyl, n-Butyl, 2-Methyl-2-butyl, Isopentyl, 2-Methoxy-2-propyl, 2-Hydroxypropyl, Trifluormethyl, 1,1-Difluorethyl, 2,2,2-Trifluorethyl, 1-Methylpropyl, 1,1-Dimethylpropyl, 1,1-Dimethyl-3-buten-1-yl, Ethyl und 2-Propyl;
$R^3$ ausgewählt ist aus -H, $C_{1-6}$-Alkyl und $C_{1-6}$-Acyl, wobei die bei der Definition von $R^3$ verwendeten $C_{1-6}$-Alkyl- und $C_{1-6}$-Acylreste gegebenenfalls durch eine oder mehrere Gruppen ausgewählt aus $CH_3C(=O)$-0-, Halogen, Methoxy, Hydroxy, Amino, Methylamino, Dimethylamino, Pyrrolidinyl und Morpholinyl substituiert sind; und
$R^4$ ausgewählt ist aus -H und Methyl.

**9.** Verbindungen nach Anspruch 6, wobei
G für CH oder N steht;
$X^1$ für Brom steht;
$R^1$ für Cyclohexylmethyl, Cyclobutylmethyl, 4,4-Difluorcyclohexanemethyl, N-Methylpiperidin-2-ylmethyl oder Tetrahydropyranylmethyl steht;
$R^2$ für t-Butyl oder 1,1-Difluorethyl steht;
$R^3$ ausgewählt ist aus -H, Methyl, Ethyl, Propyl, 2-Propyl, 2-Hydroxyethyl, 2-Methoxyethyl, Formyl, Acetyl, Ureido, N-Isopropylureido, Ethylcarbonyl, 2-Propylcarbonyl, t-Butylcarbonyl, 2-Aminoacetyl, 2-Methylaminoacetyl, 2-Dimethylaminoacetyl, 2-Acetyloxyacetyl, 2-Hydroxyacetyl, 2-Bromacetyl, 2-(Morpholin-1-yl)-acetyl und 2-(Pyrrolidin-1-yl)-acetyl; und
$R^4$ ausgewählt ist aus -H und Methyl.

**10.** Verbindungen der Formel IB und deren pharmazeutisch annehmbare Salze, Diastereomere, Enantiomere und Mischungen:

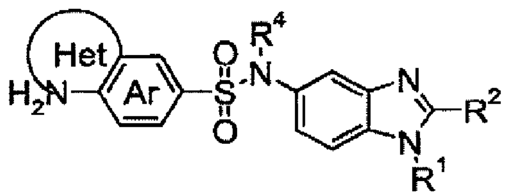

IB

wobei
$R^1$ ausgewählt ist aus $C_{1-10}$-Alkyl, $C_{2-10}$-Alkenyl, $C_{3-10}$-Cycloalkyl-$C_{1-4}$-alkyl, $C_{4-8}$-Cycloalkenyl-$C_{1-4}$-alkyl, $C_{3-6}$-Heterocycloalkyl-$C_{1-4}$-alkyl, $C_{3-10}$-Cycloalkyl, $C_{4-8}$-Cycloalkenyl und $C_{3-6}$-Heterocycloalkyl, wobei die bei der Definition von $R^1$ verwendeten $C_{1-10}$-Alkyl-, $C_{2-10}$-Alkenyl-, $C_{3-10}$-Cycloalkyl-$C_{1-4}$-alkyl-, $C_{4-8}$-Cycloalkenyl-$C_{1-4}$-alkyl-, $C_{3-6}$-Heterocycloalkyl-$C_{1-4}$-alkyl-, $C_{3-10}$-Cycloalkyl-, $C_{4-8}$-Cycloalkenyl- und $C_{3-6}$-Heterocycloalkylreste gegebenenfalls durch eine oder mehrere Gruppen ausgewählt aus Halogen, Cyano, Nitro, Methoxy, Ethoxy, Methyl, Ethyl, Hydroxy, Amino, $C_{1-6}$-Alkylamino und Di-$C_{1-6}$-alkylamino substituiert sind;
$R^2$ ausgewählt ist aus $C_{1-10}$-Alkyl, $C_{2-10}$-Alkenyl, $C_{3-10}$-Cycloalkyl, $C_{3-10}$-Cycloalkyl-$C_{1-4}$-alkyl und $C_{4-8}$-Cycloalkenyl-$C_{1-4}$-alkyl, wobei die bei der Definition von $R^2$ verwendeten $C_{1-10}$-Alkyl-, $C_{2-10}$-Alkenyl-, $C_{3-10}$-Cycloalkyl-, $C_{3-10}$-Cycloalkyl-$C_{1-4}$-alkyl- und $C_{4-8}$-Cycloalkenyl-$C_{1-4}$-alkylreste gegebenenfalls durch eine oder mehrere Gruppen ausgewählt aus Halogen, Methoxy, Ethoxy, Methyl, Ethyl, Hydroxy, Amino, $C_{1-6}$-Alkylamino und Di-$C_{1-6}$-alkylamino substituiert sind;
"Het" für einen (wie in Formel IB gezeigten) stickstoffhaltigen heterocyclischen Ring steht, der mit dem Phenylring "Ar" kondensiert ist, wobei "Het" gegebenenfalls durch eine oder mehrere Gruppen ausgewählt aus $C_{1-3}$-Alkyl, Halogen, Cyano, Methoxy, Ethoxy, Hydroxy und Amino substituiert ist; und
$R^4$ ausgewählt ist aus -H, $C_{1-6}$-Alkyl, $C_{2-6}$-Alkenyl, $C_{3-6}$-Cycloalkyl und $C_{3-6}$-Cycloalkyl-$C_{1-4}$-alkyl.

**11.** Verbindungen nach Anspruch 10, wobei
$R^1$ ausgewählt ist aus $C_{1-6}$-Alkyl, $C_{2-6}$-Alkenyl, $C_{3-6}$-Cycloalkyl-$C_{1-4}$-alkyl, $C_{4-6}$-Cycloalkenyl-$C_{1-4}$-alkyl und $C_{3-6}$-Heterocycloalkyl-$C_{1-4}$-alkyl, wobei die bei der Definition von $R^1$ verwendeten $C_{1-6}$-Alkyl-, $C_{2-6}$-Alkenyl-, $C_{3-6}$-Cycloalkyl-

$C_{1-4}$-alkyl-, $C_{4-6}$-Cycloalkenyl-$C_{1-4}$-alkyl- und $C_{3-6}$-Heterocycloalkyl-$C_{1-4}$-alkylreste gegebenenfalls durch eine oder mehrere Gruppen ausgewählt aus Halogen, Methoxy, Ethoxy, Methyl, Hydroxy, Amino, $C_{1-6}$-Alkylamino und Di-$C_{1-6}$-alkylamino substituiert sind;
$R^2$ ausgewählt ist aus $C_{1-6}$-Alkyl, $C_{2-6}$-Alkenyl, $C_{3-6}$-Cycloalkyl, $C_{3-6}$-Cycloalkyl-$C_{1-4}$-alkyl und $C_{4-6}$-Cycloalkenyl-$C_{1-4}$-alkyl, wobei die bei der Definition von $R^2$ verwendeten $C_{1-6}$-Alkyl-, $C_{2-6}$-Alkenyl-, $C_{3-6}$-Cycloalkyl-, $C_{3-6}$-Cycloalkyl-$C_{1-4}$-alkyl- und $C_{4-6}$-Cycloalkenyl-$C_{1-4}$-alkylreste gegebenenfalls durch eine oder mehrere Gruppen ausgewählt aus Halogen, Methoxy, Ethoxy und Hydroxy substituiert sind;
"Het" für Morpholinyl steht, wobei dieses Morpholinyl gegebenenfalls durch eine oder mehrere Gruppen ausgewählt aus $C_{1-3}$-Alkyl, Halogen, Cyano, Methoxy, Ethoxy, Hydroxy und Amino substituiert ist; und
$R^4$ ausgewählt ist aus -H und $C_{1-3}$-Alkyl.

**12.** Verbindungen nach Anspruch 10, wobei
$R^1$ ausgewählt ist aus Cyclopentylmethyl, Cyclohexylmethyl, Cyclobutylmethyl, Cyclopropylmethyl, 4,4-Difluorcyclohexylmethyl, Bicyclo[2.2.1]hept-5-en-2-ylmethyl, Tetrahydropyranylmethyl, Tetrahydropyranylethyl, Tetrahydrofuranylmethyl, Morpholinylmethyl, Piperdinylethyl, N-Methylpiperdinylmethyl und Piperdinylmethyl;
$R^2$ ausgewählt ist aus t-Butyl, n-Butyl, 2-Methyl-2-butyl, Isopentyl, 2-Methoxy-2-propyl, 2-Hydroxypropyl, Trifluormethyl, 1,1-Difluorethyl, 2,2,2-Trifluorethyl, 1-Methylpropyl, 1,1-Dimethylpropyl, 1,1-Dimethyl-3-buten-1-yl, Ethyl und 2-Propyl;
"Het" für Morpholinyl steht, wobei dieses Morpholinyl gegebenenfalls durch einen oder mehrere $C_{1-3}$-Alkylreste substituiert ist; und
$R^4$ ausgewählt ist aus -H und Methyl.

**13.** Verbindungen nach Anspruch 10, wobei
$R^1$ ausgewählt ist aus Cyclohexylmethyl, Cyclobutylmethyl, Cyclopropylmethyl, 4,4-Difluorcyclohexanmethyl, N-Methylpiperidin-2-ylmethyl und Tetrahydropyranylmethyl;
$R^2$ für t-Butyl oder 1,1-Difluorethyl steht;
"Het" für Morpholinyl steht, wobei dieses Morpholinyl gegebenenfalls durch einen oder mehrere $C_{1-3}$-Alkylreste substituiert ist; und
$R^4$ ausgewählt ist aus -H und Methyl.

**14.** Verbindungen nach einem der Anasprüche 1-13 zur Verwendung als Medikament.

**15.** Verwendung einer Verbindung nach einem der Ansprüche 1-13 bei der Herstellung eines Medikaments zur Therapie von Schmerzen.

**16.** Verwendung einer Verbindung nach einem der Ansprüche 1-13 bei der Herstellung eines Medikaments zur Behandlung von Angststörungen.

**17.** Verwendung einer Verbindung nach einem der Ansprüche 1-13 bei der Herstellung eines Medikaments zur Behandlung von Krebs, Multipler Sklerose, Parkinson-Krankheit, Krebs, Chorea Huntington, Alzheimer-Krankheit, Erkrankungen des Magen-Darm-Trakts und Herzkreislaufkrankheiten.

**18.** Pharmazeutische Zusammensetzung, enthaltend eine Verbindung nach einem der Ansprüche 1-13 und einen pharmazeutisch annehmbaren Träger.

**19.** Verfahren zur Herstellung einer Verbindung der Formel I

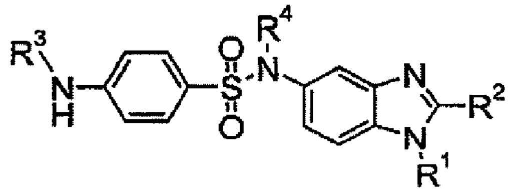

**I**

bei dem man:

eine Verbindung der Formel II,

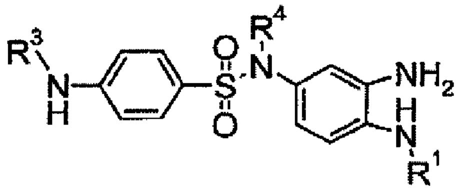

II

in Gegenwart einer Base wie einem Alkylamin und gegebenenfalls einem Kupplungsmittel mit einer Verbindung der Formel $R^2COX$ umsetzt und anschließend mit einer Säure behandelt; wobei
X ausgewählt ist aus Cl, Br, F und OH;
$R^1$ ausgewählt ist aus $C_{1-10}$-Alkyl, $C_{2-10}$-Alkenyl, $C_{3-10}$-Cycloalkyl-$C_{1-4}$-alkyl, $C_{4-8}$-Cycloalkenyl-$C_{1-4}$-alkyl, $C_{3-6}$-Heterocycloalkyl-$C_{1-4}$-alkyl, $C_{3-10}$-Cycloalkyl, $C_{4-8}$-Cycloalkenyl und $C_{3-6}$-Heterocycloalkyl, wobei die bei der Definition von $R^1$ verwendeten $C_{1-10}$-Alkyl-, $C_{2-10}$-Alkenyl-, $C_{3-10}$-Cycloalkyl-$C_{1-4}$-alkyl-, $C_{4-8}$-Cycloalkenyl-$C_{1-4}$-alkyl-, $C_{3-6}$-Heterocycloalkyl-$C_{1-4}$-alkyl-, $C_{3-10}$-Cycloalkyl-, $C_{4-8}$-Cycloalkenyl- und $C_{3-6}$-Heterocycloalkylreste gegebenenfalls durch eine oder mehrere Gruppen ausgewählt aus Halogen, Cyano, Nitro, Methoxy, Ethoxy, Methyl, Ethyl, Hydroxy, Amino, $C_{1-6}$-Alkylamino und Di-$C_{1-6}$-alkylamino substituiert sind;
$R^2$ ausgewählt ist aus $C_{1-10}$-Alkyl, $C_{2-10}$-Alkenyl, $C_{3-10}$-Cycloalkyl, $C_{3-10}$-Cycloalkyl-$C_{1-4}$-alkyl und $C_{4-8}$-Cycloalkenyl-$C_{1-4}$-alkyl, wobei die bei der Definition von $R^2$ verwendeten $C_{1-10}$-Alkyl-, $C_{2-10}$-Alkenyl-, $C_{3-10}$-Cycloalkyl-, $C_{3-10}$-Cycloalkyl-$C_{1-4}$-alkyl- und $C_{4-8}$-Cycloalkenyl-$C_{1-4}$-alkylreste gegebenenfalls durch eine oder mehrere Gruppen ausgewählt aus Halogen, Methoxy, Ethoxy, Methyl, Ethyl, Hydroxy, Amino, $C_{1-6}$-Alkylamino und Di-$C_{1-6}$-alkylamino substituiert sind;
$R^3$ ausgewählt ist aus -H, $C_{1-6}$-Alkyl und $C_{1-6}$-Acyl, gegebenenfalls substituiert durch eine oder mehrere Gruppen ausgewählt aus $CH_3C(=O)$-O-, Halogen, Cyano, Methoxy, Ethoxy, Hydroxy, Amino, Alkylamino, Dialkylamino und $C_{3-6}$-Heterocycloalkyl; und
$R^4$ ausgewählt ist aus -H, $C_{1-6}$-Alkyl, $C_{2-6}$-Alkenyl, $C_{3-6}$-Cycloalkyl und $C_{3-6}$-Cycloalkyl-$C_{1-4}$-alkyl.

**20.** Verbindungen von 2-Brom-N-(4-{[[2-tert.-butyl-1-(cyclohexylmethyl)-1H-benzimidazol-5-yl](methyl)amino]sulfonyl}phenyl)acetamid.

**21.** Verfahren zur Herstellung einer Verbindung der Formel IA,

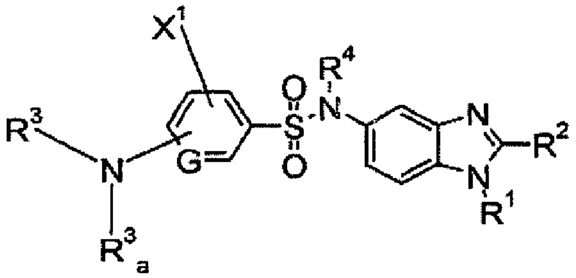

IA

bei dem man:

eine Verbindung der Formel IIA,

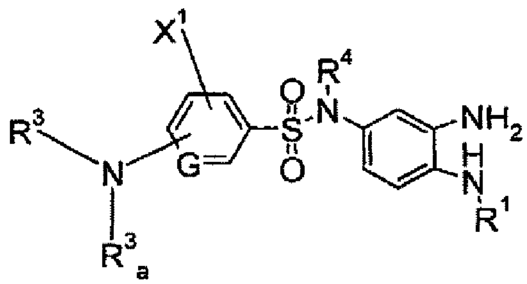

IIA

in Gegenwart einer Base wie einem Alkylamin und gegebenenfalls einem Kupplungsmittel mit einer Verbindung der Formel $R^2COX$ umsetzt und anschließend mit einer Säure behandelt; wobei
X und $X^1$ unabhängig voneinander ausgewählt sind aus Cl, Br, F und OH;
G für CH oder N steht;
$R^1$ ausgewählt ist aus $C_{1-10}$-Alkyl, $C_{2-10}$-Alkenyl, $C_{3-10}$-Cycloalkyl-$C_{1-4}$-alkyl, $C_{4-8}$-Cycloalkenyl-$C_{1-4}$-alkyl, $C_{3-6}$-Heterocycloalkyl-$C_{1-4}$-alkyl, $C_{3-10}$-Cycloalkyl, $C_{4-8}$-Cycloalkenyl und $C_{3-6}$-Heterocycloalkyl, wobei die bei der Definition von $R^1$ verwendeten $C_{1-10}$-Alkyl-, $C_{2-10}$-Alkenyl-, $C_{3-10}$-Cycloalkyl-$C_{1-4}$-alkyl-, $C_{4-8}$-Cycloalkenyl-$C_{1-4}$-alkyl-, $C_{3-6}$-Heterocycloalkyl-$C_{1-4}$-alkyl-, $C_{3-10}$-Cycloalkyl-, $C_{4-8}$-Cycloalkenyl- und $C_{3-6}$-Heterocycloalkylreste gegebenenfalls durch eine oder mehrere Gruppen ausgewählt aus Halogen, Cyano, Nitro, Methoxy, Ethoxy, Methyl, Ethyl, Hydroxy, $CH_3C(=O)$-O-, Amino, $C_{1-6}$-Alkylamino und Di-$C_{1-6}$-alkylamino substituiert sind;
$R^2$ ausgewählt ist aus $C_{1-10}$-Alkyl, $C_{2-10}$-Alkenyl, $C_{3-10}$-Cycloalkyl, $C_{3-10}$-Cycloalkyl-$C_{1-4}$-alkyl und $C_{4-8}$-Cycloalkenyl-$C_{1-4}$-alkyl, wobei die bei der Definition von $R^2$ verwendeten $C_{1-10}$-Alkyl-, $C_{2-10}$-Alkenyl-, $C_{3-10}$-Cycloalkyl-, $C_{3-10}$-Cycloalkyl-$C_{1-4}$-alkyl- und $C_{4-8}$-Cycloalkenyl-$C_{1-4}$-alkylreste gegebenenfalls durch eine oder mehrere Gruppen ausgewählt aus Halogen, $C_{3-5}$-Heteroaryl, Methoxy, Ethoxy, Methyl, Ethyl, Hydroxy, Amino, $C_{1-6}$-Alkylamino und Di-$C_{1-6}$-alkylamino substituiert sind;
$R^3$ und $R^{3a}$ unabhängig voneinander ausgewählt sind aus -H, $C_{1-6}$-Alkyl, $C_{2-6}$-Alkenyl, $C_{1-3}$-Alkyl-O-C (=0) -, $C_{1-6}$-Alkyl-HN-C(=O)-, $H_2N$-C(=O)- und $C_{1-6}$-Acyl, wobei die bei der Definition von $R^3$ verwendeten $C_{1-6}$-Alkyl-, $C_{2-6}$-Alkenyl- und $C_{1-6}$-Acylreste gegebenenfalls durch eine oder mehrere Gruppen ausgewählt aus $CH_3C$ (=O)-O-, Halogen, Cyano, Methoxy, Ethoxy, Hydroxy, Amino, $C_{1-6}$-Alkylamino, Di-$C_{1-6}$-alkylamino und $C_{3-6}$-Heterocycloalkyl substituiert sind; und
$R^4$ ausgewählt ist aus -H, $C_{1-6}$-Alkyl, $C_{2-6}$-Alkenyl, $C_{3-6}$-Cycloalkyl und $C_{3-6}$-Cycloalkyl-$C_{1-4}$-alkyl.

**Revendications**

**1.** Composé de formule I ou un sel pharmaceutiquement acceptable de celui-ci :

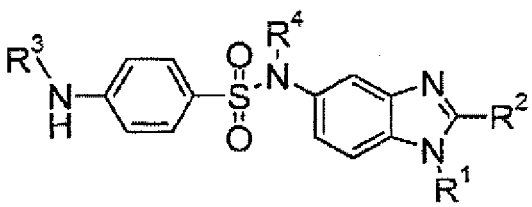

I

dans laquelle
$R^1$ est choisi parmi $C_{1-10}$alkyle, $C_{2-10}$alcényle, $C_{3-10}$cycloalkyl-$C_{1-4}$alkyle, $C_{4-8}$cycloalcényl-$C_{1-4}$alkyle, $C_{3-6}$hétérocycloalkyl-$C_{1-4}$alkyle, $C_{3-10}$cycloalkyle, $C_{4-8}$cycloalcényle et $C_{3-6}$hétérocycloalkyle où lesdits $C_{1-10}$alkyle, $C_{2-10}$alcényle, $C_{3-10}$cycloalkyl-$C_{1-4}$alkyle, $C_{4-8}$cycloalcényl-$C_{1-4}$alkyle, $C_{3-6}$hétérocycloalkyl-$C_{1-4}$alkyle, $C_{3-10}$cycloalkyle, $C_{4-8}$cycloalcényle et $C_{3-6}$hétérocycloalkyle, utilisés pour définir $R^1$ sont éventuellement substitués par un ou plusieurs groupements choisis parmi halogène, cyano, nitro, méthoxy, éthoxy, méthyle, éthyle, hydroxy,

amino, $C_{1-6}$alkylamino et di$C_{1-6}$alkylamino ;
$R^2$ est choisi parmi $C_{1-10}$alkyle, $C_{2-10}$alcényle, $C_{3-10}$cycloalkyle, $C_{3-10}$cycloalkyl-$C_{1-4}$alkyle et $C_{4-8}$cycloalcényl-$C_{1-4}$alkyle où lesdits $C_{1-10}$alkyle, $C_{2-10}$alcényle, $C_{3-10}$cycloalkyle, $C_{3-10}$cycloalkyl-$C_{1-4}$alkyle et $C_{4-8}$cycloalcényl-$C_{1-4}$alkyle utilisés pour définir $R^2$ sont éventuellement substitués par un ou plusieurs groupements choisis parmi halogène, méthoxy, éthoxy, méthyle, éthyle, hydroxy, amino, $C_{1-6}$alkylamino et di$C_{1-6}$alkylamino ;
$R^3$ est choisi parmi -H, $C_{1-6}$alkyle, $C_{2-6}$alcényle et $C_{1-6}$acyle où lesdits $C_{1-6}$alkyle, $C_{2-6}$alcényle et $C_{1-6}$acyle utilisés pour définir $R^3$ sont éventuellement substitués par un ou plusieurs groupements choisis parmi $CH_3C(=O)$-O-, halogène, cyano, méthoxy, éthoxy, hydroxy, amino, alkylamino, dialkylamino et $C_{3-6}$hétérocycloalkyle ; et
$R^4$ est choisi parmi -H, $C_{1-6}$alkyle, $C_{2-6}$alcényle, $C_{3-6}$cycloalkyle et $C_{3-6}$cycloalkyl-$C_{1-4}$alkyle.

2. Composé selon la revendication 1, **caractérisé en ce que**
$R^1$ est choisi parmi $C_{1-6}$alkyle, $C_{2-6}$alcényle, $C_{3-6}$cycloalkyl-$C_{1-4}$alkyle, $C_{4-6}$cycloalcényl-$C_{1-4}$alkyle et $C_{3-6}$hétérocycloalkyl-$C_{1-4}$alkyle où lesdits $C_{1-6}$alkyle, $C_{2-6}$alcényle, $C_{3-6}$cycloalkyl-$C_{1-4}$alkyle, $C_{4-6}$cycloalcényl-$C_{1-4}$alkyle et $C_{3-6}$hétérocycloalkyl-$C_{1-4}$alkyle utilisés pour définir $R^1$ sont éventuellement substitués par un ou plusieurs groupements choisis parmi halogène, méthoxy, éthoxy, méthyle, hydroxy et amino ;
$R^2$ est choisi parmi $C_{1-6}$alkyle, $C_{2-6}$alcényle, $C_{3-6}$cycloalkyle, $C_{3-6}$cycloalkyl-$C_{1-4}$alkyle et $C_{4-6}$cycloalcényl-$C_{1-4}$alkyle, où lesdits $C_{1-6}$alkyle, $C_{2-6}$alcényle, $C_{3-6}$cycloalkyle, $C_{3-6}$cycloalkyl-$C_{1-4}$alkyle et $C_{4-6}$cycloalcényl-$C_{1-4}$alkyle utilisés pour définir $R^2$ sont éventuellement substitués par un ou plusieurs groupements choisis parmi halogène, méthoxy, éthoxy et hydroxy ;
$R^3$ est choisi parmi -H, $C_{1-6}$alkyle, $C_{2-6}$alcényle et $C_{1-6}$acyle, où lesdits $C_{1-6}$alkyle, $C_{2-6}$alcényle et $C_{1-6}$acyle utilisés pour définir $R^3$ sont éventuellement substitués par un ou plusieurs groupements choisis parmi $CH_3C(=O)$-O-, halogène, méthoxy, éthoxy, hydroxy, amino, méthylamino, diméthylamino et $C_{3-6}$hétérocycloalkyle ; et
$R^4$ est choisi parmi -H et $C_{1-3}$alkyle.

3. Composé selon la revendication 1,
$R^1$ est choisi parmi cyclopentylméthyle, cyclohexylméthyle, cyclobutylméthyle, cyclopropylméthyle, 4,4-difluorocyclohexylméthyle, bicyclo[2,2,1]hept-5-én-2-ylméthyle, tétrahydropyranylméthyle, tétrahydropyranyléthyle, tétrahydrofuranylméthyle, morpholinylméthyle, pipéridinyléthyle, N-méthylpipéridinylméthyle et pipéridinylméthyle ;
$R^2$ est choisi parmi t-butyle, n-butyle, 2-méthyl-2-butyle, isopentyle, 2-méthoxy-2-propyle, 2-hydroxypropyle, trifluorométhyle, 1,1-difluoroéthyle, 2,2,2-trifluoroéthyl,1-méthylpropyle, 1,1-diméthylpropyle, 1,1-diméthyl-3-butén-1-yle, éthyle et 2-propyle ;
$R^3$ est choisi parmi -H, $C_{1-6}$alkyle et $C_{1-6}$acyle, où lesdits $C_{1-6}$alkyle et $C_{1-6}$acyle utilisés pour définir $R^3$ sont éventuellement substitués par un ou plusieurs groupements choisis parmi $CH_3C(=O)$-O-, halogène, méthoxy, hydroxy, amino, méthylamino, diméthylamino, pyrrolidinyle et morpholinyle ; et
$R^4$ est choisi parmi -H et méthyle ;

4. Composé selon la revendication 1, **caractérisé en ce que**
$R^1$ est choisi parmi cyclohexylméthyle, cyclopentylméthyle, cyclobutylméthyle, cyclopropylméthyle, 4,4-difluorocyclohexylméthyle et tétrahydropyranylméthyle ;
$R^2$ est t-butyle et 1,1-difluoroéthyle ;
$R^3$ est choisi parmi -H, méthyle, éthyle, propyle, 2-propyle, 2-hydroxyéthyle, 2-méthoxyéthyle, formyle, acétyle, éthylcarbonyle, 2-propylcarbonyle, t-butylcarbonyl, uréido, N-isopropyluréido, 2-aminoacétyle, 2-méthylaminoacétyle, 2-diméthylaminoacétyle, 2-acétyloxyacétyle, 2-hydroxyacétyle, 2-bromoacétyle, 2-(morpholino-1-yl)acétyle et 2-(pyrrolidin-1-yl)acétyle ; et
$R^4$ est choisi parmi -H et méthyle.

5. Composé choisi parmi :

le *N*-(4-{[[2-*tert*-butyl-1-(cyclohexylméthyl)-1*H*-benzimidazol-5-yl](méthyl)amino]sulfonyl}phényl)acétamide ;
le *N*-[2-*tert*-butyl-1-(cyclohexylméthyl)-1*H*-benzimidazol-5-yl]-*N*-méthyl-4-nitrobenzènesulfonamide ;
le 4-amino-*N*-[2-*tert*-butyl-1-(cyclohexylméthyl)-1*H*-benzimidazol-5-yl]-*N*-méthylbenzènesulfonamide ;
le *N*-(4-{[[2-*tert*-Butyl-1-(cyclohexylméthyl)-1*H*-benzimidazol-5-yl](méthyl)amino]sulfonyl}phényl)propanamide ;
le *N*-(4-{[[2-*tert*-butyl-1-(cyclohexylméthyl)-1*H*-benzimidazol-5-yl](méthyl)amino]sulfonyl}phényl)-2-méthylpropanamide ;
le *N*-(4-{[[2-*tert*-butyl-1-(cyclohexylméthyl)-1*H*-benzimidazol-5-yl](méthyl)amino]sulfonyl}phényl)-2,2-diméthylpropanamide ;
le *N*-[2-*tert*-butyl-1-(cyclohexylméthyl)-1*H*-benzimidazol-5-yl]-4-(éthylamino)-*N*-méthylbenzènesulfonamide ;
le *N*-[2-*tert*-butyl-1-(cyclohexylméthyl)-1*H*-benzimidazol-5-yl]-4-(formylamino)-*N*-méthylbenzènesulfonamide ;

le *N*-(4-{[[2-*tert*-butyl-1-(cyclohexylméthyl)-1*H*-benzimidazol-5-yl](méthyl)amino]sulfonyl}phényl)-2-pyrrolidin-1-ylacétamide ;
le *N1*-(4-{[[2-*tert*-butyl-1-(cyclohexylméthyl)-1*H*-benzimidazol-5-yl](méthyl)amino]sulfonyl}phényl)-$N^2$-$N^2$-diméthylglycinamide ;
le *N*-(4-{[[2-*tert*-butyl-1-(cyclohexylméthyl)-1*H*-benzimidazol-5-yl](méthyl)amino]sulfonyl}phényl)-2-morpholin-4-ylacétamide ;
le *N1*-(4-{[[2-*tert*-butyl-1-(cyclohexylméthyl)-1*H*-benzimidazol-5-yl](méthyl)amino]sulfonyl}phényl)-glycinamide ;
l'acétate de 2-[(4-{[[2-*tert*-butyl-1-(cyclohexylméthyl)-1*H*-benzimidazol-5-yl](méthyl)amino]sulfonyl}phényl) amino]-2-oxoéthyle
le *N*-(4-{[[2-*tert*-butyl-1-(cyclohexylméthyl)-1*H*-benzimidazol-5-yl](méthyl)amino]sulfonyl}phényl)-2-hydroxyacétamide ;
le 5-bromo-*N*-[2-*tert*-butyl-1-(cyclohexylméthyl)-1*H*-benzimidazol-5-yl]-6-chloro-*N*-méthylpyridine-3-sulfonamide ;
le 5-bromo-*N*-[2-*tert*-butyl-1-(cyclohexylméthyl)-1*H*-benzimidazol-5-yl]-6-[(2-hydroxyéthyl)amino]-*N*-méthylpyridine-3-sulfonamide ;
le *N*-[2-*tert*-butyl-1-(cyclohexylméthyl)-1*H*-benzimidazol-5-yl]-6-[(2-hydroxyéthyl)amino]-*N*-méthylpyridine-3-sulfonamide ;
le *N*-(5-{[[2-*tert*-butyl-1-(cyclohexylméthyl)-1*H*-benzimidazol-5-yl](méthyl)amino]sulfonyl}pyridin-2-yl)acétamide ;
le *N*-(3-{[[2-*tert*-butyl-1-(cyclohexylméthyl)-1*H*-benzimidazol-5-yl](méthyl)amino]sulfonyl}phényl)acétamide ;
le *N1*-(4-{[[2-*tert*-butyl-1-(cyclohexylméthyl)-1*H*-benzimidazol-5-yl](méthyl)amino]sulfonyl}phényl)-*N2*-(2-hydroxyéthyl)glycinamide ;
le 4-[(aminocarbonyl)amino]-*N*-[2-*tert*-butyl-1-(tétrahydro-2*H*-pyran-4-ylméthyl)-1*H*-benzimidazol-5-yl]-*N*-méthylbenzènesulfonamide ;
le *N*-(4-{[[2-*tert*-butyl-1-(tétrahydro-2*H*-pyran-4-ylméthyl)-1*H*-benzimidazol-5-yl](méthyl)amino]sulfonyl}phényl)acétamide ;
le *N*-(4-{[[2-*tert*-butyl-1-(tétrahydro-2*H*-pyran-4-ylméthyl)-1*H*-benzimidazol-5-yl](méthyl)amino]sulfonyl}phényl)-N-méthylacétamide ;
le *N*-(4-{[[2-*tert*-butyl-1-(tétrahydro-2*H*-pyran-4-ylméthyl)-1*H*-benzimidazol-5-yl](méthyl)amino]sulfonyl}phényl)-2,2-diméthylpropanamide ;
le *N*-(4-{[[2-*tert*-butyl-1-(tétrahydro-2*H*-pyran-4-ylméthyl)-1*H*-benzimidazol-5-yl](méthyl)amino]sulfonyl}phényl)-2-hydroxyacétamide ;
le *N1*-(4-{[[2-*tert*-butyl-1-(tétrahydro-2*H*-pyran-4-ylméthyl)-1*H*-benzimidazol-5-y1](méthyl)amino]sulfonyl}phényl)-$N^2$-$N^2$-diméthylglycinamide ;
le *N1*-(4-{[[2-*tert*-butyl-1-(tétrahydro-2*H*-pyran-4-ylméthyl)-1*H*-benzimidazol-5-yl](méthyl)amino]sulfonyl}phényl)glycinamide ;
le *N1*-(4-{[[2-*tert*-butyl-1-(tétrahydro-2*H*-pyran-4-ylméthyl)-1*H*-benzimidazol-5-yl](méthyl)amino]sulfonyl}phényl)-$N^2$-méthylglycinamide ;
le *N*-[2-*tert*-butyl-1-(tétrahydro-2*H*-pyran-4-ylméthyl)-1*H*-benzimidazol-5-yl]-6-[(2-hydroxyéthyl)amino]-*N*-méthylpyridine-3-sulfonamide ;
le *N*-[2-*tert*-butyl-1-(tétrahydro-2*H*-pyran-4-ylméthyl)-1*H*-benzimidazol-5-yl]-6-[(2-méthoxyéthyl)amino]-*N*-métylpyridine-3-sulfonamide ;
le *N*-[2-*tert*-butyl-1-(tétrahydro-2*H*-pyran-4-ylméthyl)-1*H*-benzimidazol-5-yl]-6-(formylamino)-*N*-méthylpyridine-3-sulfonamide ;
le *N*-(5-{[[2-*tert*-butyl-1-(tétrahydro-2*H*-pyran-4-ylméthyl)-1*H*-benzimidazol-5-yl](méthyl)amino]sulfonyl}pyridin-2-yl)acétamide ;
le *N*-[4-({[2-*tert*-butyl-1-(tétrahydro-2*H*-pyran-4-ylméthyl)-1*H*-benzimidazol-5-yl]amino}sulfonyl)phényl]acétamide ;
le *N*-[4-({[2-*tert*-butyl-1-(cyclohexylméthyl)-1*H*-benzimidazol-5-yl]amino}sulfonyl)phényl]acétamide ;
le *N*-(4-{[[2-*tert*-butyl-1-(2-pipéridin-1-yléthyl)-1*H*-benzimidazol-5-yl](méthyl)amino]sulfonyl}phényl)acétamide ;
le *N*-(4-{[[2-*tert*-butyl-1-(1,4-dioxan-2-ylméthyl)-1*H*-benzimidazol-5-yl](méthyl)amino]sulfonyl}phényl)acétamide ;
le *N*-(4-{[{2-*tert*-butyl-1-[(1-méthylpipéridin-2-yl)méthyl]-1*H*-benzimidazol-5-yl}(méthyl)amino]sulfonyl}phényl) acétamide ;
le *N*-(4-{[(2-*tert*-butyl-1-{[(2R)-1-méthylpipéridin-2-yl]méthyl}-1*H*-benzimidazol-5-yl)(méthyl)amino]sulfonyl} phényl)acétamide ;
le N-[4-({méthyl[1-tétrahydro-2*H*-pyran-4-ylméthyl)-2-(trifluorométhyl)-1*H*-benzimidazol-5-yl]amino}sulfonyl)phényl]acétamide ;
le 4-bromo-*N*-[1-(cyclohexylméthyl)-2-(1,1-diméthyléthyl)-1*H*-benzimidazol-5-yl]-*N*-méthylbenzènesulfonamide ;
le *N*-[2-*tert*-butyl-1-(cyclohexylméthyl)-1*H*-benzimidazol-5-yl]-4-[(2-hydroxyéthyl)amino]-*N*-méthylbenzènesulfonamide ;
le *N*-[2-*tert*-butyl-1-(cyclohexylméthyl)-1*H*-benzimidazol-5-yl]-4-(diméthylamino)-*N*-méthylbenzènesulfonamide ;
le 4-[bis-(2-hydroxyéthyl)amino]-*N*-[2-*tert*-butyl-1-(cyclohexylméthyl)-1*H*-benzimidazol-5-yl]-*N*-méthylbenzènesulfonamide ;
le *N*-[2-*tert*-butyl-1-(cyclohexylméthyl)-1*H*-benzimidazol-5-yl]-*N*,4-diméthyl-3,4-dihydro-2*H*-1,4-benzoxazine-7-sulfonamide ;
le *N*-[4-({méthyl[2-(1-méthyl-1-pyridin-2-yléthyl)-1-(tétrahydro-2H-pyran-4-ylméthyl)-1*H*-benzimidazol-5-yl]amino}sulfonyl)phényl]acétamide ;
le *N*-(4-{[[2-*tert*-butyl-1-(tétrahydro-2*H*-pyran-4-ylméthyl)-1*H*-benzimidazol-5-yl](éthyl)amino]sulfonyl}phényl)acétamide ;
le 4-[(aminocarbonyl)amino]-N-[2-*tert*-butyl-1-(tétrahydro-2*H*-pyran-4-ylméthyl)-1*H*-benzimidazol-5-yl]-*N*-éthylbenzènesulfonamide ;
le *N*-[2-*tert*-butyl-1-(tétrahydro-2*H*-pyran-4-ylméthyl)-1*H*-benzimidazol-5-yl]-*N*-éthyl-4-{[(méthylamino)carbonyl]amino}benzènesulfonamide ;
le 4-amino-*N*-[2-*tert*-butyl-1-(tétrahydro-2*H*-pyran-4-ylméthyl)-1*H*-benzimidazol-5-yl]-*N*-éthylbenzènesulfonamide ;
le *N*-(4-{[[2-*tert*-butyl-1-(tétrahydro-2*H*-pyran-4-ylméthyl)-1*H*-benzimidazol-5-yl](éthyl)amino]sulfonyl}phényl)-2,2-diméthylpropanamide ;
l'acétate de 2-[(4-{[[2-*tert*-butyl-1-(tétrahydro-2*H*-pyran-4-ylméthy)-1*H*-benzimidazol-5-yl](éthyl)amino]sulfonyl}phényl)amino]-2-oxoéthyle
le *N*-(4-{[[2-*tert*-butyl-1-(tétrahydro-2*H*-pyran-4-ylméthyl)-1*H*-benzimidazol-5-yl](éthyl)amino]sulfonyl}phényl)-2-hydroxyacétamide ;
le *N*-[2-*tert*-butyl-1-(tétrahydro-2*H*-pyran-4-ylméthyl)-1*H*-benzimidazol-5-yl]-*N*-éthyl-4-{[(isopropylamino)carbonyl]amino}benzènesulfonamide ;
le *N*-[4-({éthyl[2-(1-méthoxy-1-méthyléthyl)-1-(tétrahydro-2*H*-pyran-4-ylméthyl)-1*H*-benzimidazol-5-yl]amino}sulfonyl)phényl]acétamide ;
le 4-[(aminocarbonyl)amino]-*N*-éthyl-*N*-[2-(1-méthoxy-1-méthyléthyl)-1-(tétrahydro-2*H*-pyran-4-ylméthyl)-1*H*-benzimidazol-5-yl]benzènesulfonamide ;
le *N*-éthyl-*N*-[2-(1-méthoxy-1-méthyléthyl)-1-(tétrahydro-2*H*-pyran-4-ylméthyl)-1*H*-benzimidazol-5-yl]-4-{[(méthylamino)carbonyl]amino}benzènesulonfamide ;
le 4-amino-*N*-éthyl-*N*-[2-(1-méthoxy-1-méthyléthyl)-1-(tétrahydro-2*H*-pyran-4-ylméthyl)-1*H*-benzimidazol-5-yl]benzènesulfonamide ;
le *N*-[4-({éthyl[2-(1-méthoxy-1-méthyléthyl)-1-(tétrahydro-2*H*-pyran-4-ylméthyl)-1*H*-benzimidazol-5-yl]amino}sulfonyl)phényl]2,2-diméthylpropanamide ;
l'acétate de 2-{[4-({éthyl[2-(1-méthoxy-1-méthyléthyl)-1-(tétrahydro-2*H*-pyran-4-ylméthyl)-1*H*-benzimidazol-5-yl]amino}sulfonyl)phényl]amino}-2-oxoéthyle
le *N*-[4-({éthyl-[2-(1-méthoxy-1-méthyléthyl)-1-(tétrahydro-2*H*-pyran-4-ylméthyl)-1*H*-benzimidazol-5-yl]amino}sulfonyl)phényl]-2-hydroxyacétamide ;
le *N*-éthyl-4-{[(isopropylamino)carbonyl]amino}-*N*-[2-(1-méthoxy-1-méthyléthyl)-1-(tétrahydro-2*H*-pyran-4-ylméthyl)-1*H*-benzimidazol-5-yl]benzènesulfonamide ;
le *N*-(4-{[[2-(1-méthoxy-1-méthyléthyl)-1-(tétrahydro-2*H*-pyran-4-ylméthyl)-1*H*-benzimidazol-5-yl](méthyl)amino]sulfonyl}phényl)acétamide ;
le 4-[(aminocarbonyl)amino]-*N*-[2-(1-méthoxy-1-méthyléthyl)-1-(tétrahydro-2*H*-pyran-4-ylméthyl)-1*H*-benzimidazol-5-yl]-*N*-méthylbenzènesulfonamide ;
le 2-hydroxy-*N*-(4-{[[2-(1-méthoxy-1-méthyléthyl)-1-(tétrahydro-2*H*-pyran-4-ylméthyl)-1*H*-benzimidazol-5-yl](méthyl)amino]sulfonyl}phényl)acétamide ;
le *N*-(4-{[[2-(1-éthoxy-1-méthyléthyl)-1-(tétrahydro-2*H*-pyran-4-ylméthyl)-1*H*-benzimidazol-5-yl](méthyl)amino]sulfonyl}phényl)acétamide ;
le *N*-[4-({[1-(2-azétidin-1-yléthyl)-2-*tert*-butyl-1*H*-benzimidazol-5-yl]amino}sulfonyl)phényl]acétamide ; l'acétate de 3-[5-({[4-(acétylamino)phényl]sulfonyl}amino)-2-*tert*-butyl-1*H*-benzimidazol-1-yl]propyle ;
le *N*-{4-[({1-[(1*S*,4*S*)-bicyclo[2,2,1]hept-5-én-2-ylméthyl]-2-*tert*-butyl-1*H*-benzimidazol-5-yl]amino)sulfonyl]phényl}acétamide ;
le *N*-[4-({[2-*tert*-butyl-1-(tétrahydro-2*H*-pyran-3-ylméthyl)-1*H*-benzimidazol-5-yl]amino}sulfonyl)phényl]acétamide ;
le N-{4-[({2-*tert*-butyl-1-[2-(tétrahydro-2*H*-pyran-4-yl)éthyl]-1*H*-benzimidazol-5-yl}amino)sulfonyl]phényl} acétamide ;
le *N*-(4-{[[2-*tert*-butyl-1-(cyclobutylméthyl)-1*H*-benzimidazol-5-yl](méthyl)amino]sulfonyl}phényl)acétamide ;
le 4-[(aminocarbonyl)amino]-*N*-[2-*tert*-butyl-1-(cyclobutylméthyl)-1*H*-benzimidazol-5-yl]-*N*-méthylbenzènesulfonamide ;
le *N*-(4-{[[2-*tert*-butyl-1-(cyclobutylméthyl)-1*H*-benzimidazol-5-yl](méthyl)amino]sulfonyl}phényl)-2,2-diméthylpropanamide ;
le *N*-(4-{[[2-(1,1-difluoroéthyl)-1-(tétrahydro-2*H*-pyran-4-ylméthyl)-1*H*-benzimidazol-5-yl](méthyl)amino]sulfonyl}phényl)-2-hydroxyacétamide ;
le *N*-(4-{[[2-(1,1-difluoroéthyl)-1-(tétrahydro-2*H*-pyran-4-ylméthyl)-1*H*-benzimidazol-5-yl](méthyl)amino]sulfonyl}phényl)acétamide ;
le *N*-(4-{[[2-(1,1-difluoroéthyl)-1-(tétrahydro-2*H*-pyran-4-ylméthyl)-1*H*-benzimidazol-5-yl](méthyl)amino]sulfonyl}phényl)-3-méthylbutanamide ;
le *N*-(4-{[[2-(1,1-difluroéthyl)-1-(tétrahydro-2*H*-pyran-4-ylméthyl)-1*H*-benzimidazol-5-yl](méthyl)amino]sulfonyl}phényl)-2,2-diméthylpropanamide ;
le *N*-[2-(1,1-difluoroéthyl)-1-(tétrahydro-2*H*-pyran-4-ylméthyl)-1*H*-benzimidazol-5-yl]-4-{[(isopropylamino)carbonyl]amino}-*N*-méthylbenzènesulfonamide ;
le 4-{bis-[(isopropylamino)carbonyl]amino}-*N*-[2-(1,1-difluoroéthyl)-1-(tétrahydro-2H-pyran-4-ylméthyl)-1*H*-benzimidazol-5-yl]-*N*-méthylbenzènesulfonamide ;
le *N*-[4-({méthyl[1-(tétrahydro-2*H*-pyran-4-ylméthyl)-2-(trifluorométhyl)-1*H*-benzimidazol-5-yl]amino}sulfonyl) phényl]acétamide ;
le 4-[(aminocarbonyl)amino]-*N*-méthyl-*N*-[1-(tétrahydro-2*H*-pyran-4-ylméthyl)-2-(trifluorométhyl)-1*H*-benzimidazol-5-yl]benzènesulfonamide ;
le *N*-méthyl-4-nitro-*N*-[1-(tétrahydro-2*H*-pyran-4-ylméthyl)-2-(trifluorométhyl)-1*H*-benzimidazol-5-yl]benzènesulfonamide ;
le 4-amino-*N*-méthyl-*N*-[1-(tétrahydro-2*H*-pyran-4-ylméthyl)-2-(trifluorométhyl)-1*H*-benzimidazol-5-yl]benzènesulfonamide ;
le 2,2-diméthyl-*N*-[4-({méthyl[1-(tétrahydro-2*H*-pyran-4-ylméthyl)-2-(trifluorométhyl)-1*H*-benzimidazol-5-yl] amino}sulfonyl)phényl]propanamide ;
l'acétate de 2-{[4-({méthyl[1-(tétrahydro-2*H*-pyran-4-ylméthyl)-2-(trifluorométhyl)-1*H*-benzimidazol-5-yl]amino} sulfonyl)phényl]amino}-2-oxoéthyle ;
le 4-{[(isopropylamino)carbonyl]amino}-*N*-méthyl-*N*-[1-(tétrahydro-2*H*-pyran-4-ylméthyl)-2-(trifluorométhyl)-1*H*-benzimidazol-5-yl]benzènesulfonamide ;
le 2-hydroxy-*N*-[4-({méthyl[1-(tétrahydro-2*H*-pyran-4-ylméthyl)-2-(trifluorométhyl)-1*H*-benzimidazol- 5- yl] amino} sulfonyl) phényl] acétamide

et les sels pharmaceutiquement acceptables de ceux-ci.

**6.** Composé de formule IA, un sel pharmaceutiquement acceptable de celui-ci, des diastéréoisomères, des énantiomères ou des mélanges de ceux-ci :

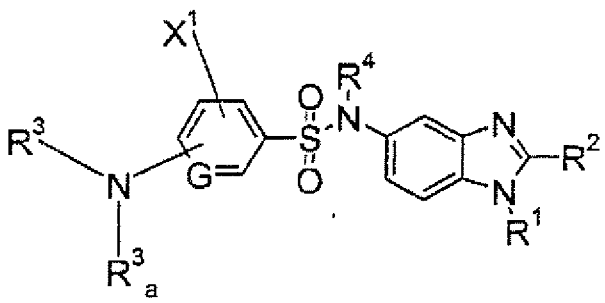

IA

dans laquelle
G est CH ou N ;

$X^1$ est halogène ;
$R^1$ est choisi parmi $C_{1-10}$alkyle, $C_{2-10}$alcényle, $C_{3-10}$cycloalkyl-$C_{1-4}$alkyle, $C_{4-8}$cycloalcényl-$C_{1-4}$alkyle, $C_{3-6}$hétérocycloalkyl-$C_{1-4}$alkyle, $C_{3-10}$cycloalkyle, $C_{4-8}$cycloalcényle et $C_{3-6}$hétérocycloalkyle où lesdits $C_{1-10}$alkyle, $C_{2-10}$alcényle, $C_{3-10}$cycloalkyl-$C_{1-4}$alkyle, $C_{4-8}$cycloalcényl-$C_{1-4}$alkyle, $C_{3-6}$hétérocycloalkyl-$C_{1-4}$alkyle, $C_{3-10}$cycloalkyle, $C_{4-8}$cycloalcényle et $C_{3-6}$hétérocycloalkyle utilisés pour définir $R^1$ sont éventuellement substitués par un ou plusieurs groupements choisis parmi halogène, cyano, nitro, méthoxy, éthoxy, méthyle, éthyle, hydroxy, $CH_3C(=O)$-O-, amino, $C_{1-6}$alkylamino et di$C_{1-6}$alkylamino ;
$R^2$ est choisi parmi $C_{1-10}$alkyle, $C_{2-10}$alcényle, $C_{3-10}$cycloalkyle, $C_{3-10}$cycloalkyl-$C_{1-4}$alkyle et $C_{4-8}$cycloalcényl-$C_{1-4}$alkyle où lesdits $C_{1-10}$alkyle, $C_{2-10}$alcényle, $C_{3-10}$cycloalkyle, $C_{3-10}$cycloalkyl-$C_{1-4}$alkyle et $C_{4-8}$cycloalcényl-$C_{1-4}$alkyle utilisés pour définir $R^2$ sont éventuellement substitués par un ou plusieurs groupements choisis parmi halogène, $C_{3-5}$hétéroaryle, méthoxy, éthoxy, méthyle, éthyle, hydroxy, amino, $C_{1-6}$alkylamino et di$C_{1-6}$alkylamino ;
$R^3$ et $R^3_a$ sont choisis indépendamment parmi -H, $C_{1-6}$alkyle, $C_{2-6}$alcényle, $C_{1-3}$alkyl-O-C(=O)-, $C_{1-6}$alkyl-HN-C(=O)-, $H_2N$-C(=O)- et $C_{1-6}$acyle où lesdits $C_{1-6}$alkyle, $C_{2-6}$alcényle et $C_{1-6}$acyle utilisés pour définir $R^3$ sont éventuellement substitués par un ou plusieurs groupements choisis parmi $CH_3C(=O)$-O-, halogène, cyano, méthoxy, éthoxy, hydroxy, amino, $C_{1-6}$alkylamino, di$C_{1-6}$alkylamino et $C_{3-6}$hétérocycloalkyle ; et
$R^4$ est choisi parmi -H, $C_{1-6}$alkyle, $C_{2-6}$alcényle, $C_{3-6}$cycloalkyle et $C_{3-6}$cycloalkyl-$C_{1-4}$alkyle.

**7.** Composé selon la revendication 6, **caractérisé en ce que**
G est CH ou N ;
$R^1$ est choisi parmi $C_{1-6}$alkyle, $C_{2-6}$alcényle, $C_{3-6}$cycloalkyl-$C_{1-4}$alkyle, $C_{4-6}$cycloalcényl-$C_{1-4}$alkyle et $C_{3-6}$hétérocycloalkyl-$C_{1-4}$alkyle où lesdits $C_{1-6}$alkyle, $C_{2-6}$alcényle, $C_{3-6}$cycloalkyl-$C_{1-4}$alkyle, $C_{4-6}$cycloalcényl-$C_{1-4}$alkyle et $C_{3-6}$hétérocycloalkyl-$C_{1-4}$alkyle utilisés pour définir $R^1$ sont éventuellement substitués par un ou plusieurs groupements choisis parmi halogène, méthoxy, éthoxy, méthyle, hydroxy, $CH_3C(=O)$-O-, amino, $C_{1-6}$alkylamino et di$C_{1-6}$alkylamino ;
$R^2$ est choisi parmi $C_{1-6}$alkyle, $C_{2-6}$alcényle, $C_{3-6}$cycloalkyle, $C_{3-6}$cycloalkyl-$C_{1-4}$alkyle et $C_{4-6}$cycloalcényl-$C_{1-4}$alkyle, où lesdits $C_{1-6}$alkyle, $C_{2-6}$alcényle, $C_{3-6}$cycloalkyle, $C_{3-6}$cycloalkyl-$C_{1-4}$alkyle et $C_{4-6}$cycloalcényl-$C_{1-4}$alkyle utilisés pour définir $R^2$ sont éventuellement substitués par un ou plusieurs groupements choisis parmi halogène, $C_{3-5}$hétéroaryle, méthoxy, éthoxy et hydroxy ;
$R^3$ est choisi parmi -H, $C_{1-6}$alkyle, $C_{2-6}$alcényle, $C_{1-3}$alkyl-O-C(=O)-, $C_{1-3}$alkyl-HN-C(=O)-, $H_2N$-C(=O)- et $C_{1-6}$acyle où lesdits $C_{1-6}$alkyle, $C_{2-6}$alcényle et $C_{1-6}$acyle utilisés pour définir $R^3$ sont éventuellement substitués par un ou plusieurs groupements choisis parmi $CH_3C(=O)$-O-, halogène, méthoxy, éthoxy, hydroxy, amino, méthylamino, diméthylamino et $C_{3-6}$hétérocycloalkyle ; et
$R^4$ est choisi parmi -H et $C_{1-3}$alkyle.

**8.** Composé selon la revendication 6, **caractérisé en ce que**
G est CH ou N ;
$R^1$ est choisi parmi cyclopentylméthyle, cyclohexylméthyle, cyclobutylméthyle, cyclopropylméthyle, 4,4-difluorocyclohexaneméthyle, bicyclo[2,2,1]hept-5-én-2-ylméthyle, tétrahydropyranylméthyle, tétrahydropyranyléthyle, tétrahydrofuranylméthyle, morpholinylméthyle, pipéridinyléthyle, N-méthylpipéridinylméthyle et pipéridinylméthyle ;
$R^2$ est choisi parmi t-butyle, n-butyle, 2-méthyl-2-butyle, isopentyle, 2-méthoxy-2-propyle, 2-hydroxypropyle, trifluorométhyle, 1,1-difluoroéthyle, 2,2,2-trifluoroéthyl-1-méthylpropyle, 1,1-diméthylpropyle, 1,1-diméthyl-3-butén-1-yle, éthyle et 2-propyle ;
$R^3$ est choisi parmi -H, $C_{1-6}$alkyle et $C_{1-6}$acyle, où lesdits $C_{1-6}$alkyle et $C_{1-6}$acyle utilisés pour définir $R^3$ sont éventuellement substitués par un ou plusieurs groupements choisis parmi $CH_3C(=O)$-O-, halogène, méthoxy, hydroxy, amino, méthylamino, diméthylamino, pyrrolidinyle, pipéridinyle et morpholinyle ; et
$R^4$ est choisi parmi -H et méthyle ;

**9.** Composé selon la revendication 6, **caractérisé en ce que**
G est CH ou N ;
$X^1$ est bromo ;
$R^1$ est cyclohexylméthyle, cyclobutylméthyle, 4,4-difluorocyclohexaneméthyle, N-méthylpipéridin-2-ylméthyle et tétrahydropyranylméthyle ;
$R^2$ est t-butyle et 1,1-difluoroéthyle ;
$R^3$ est choisi parmi -H, méthyle, éthyle, propyle, 2-propyle, 2-hydroxyéthyle, 2-méthoxyéthyle, formyle, acétyle, uréido, N-isopropyl-uréido, éthylcarbonyle, 2-propylcarbonyle, t-butylcarbonyle, 2-aminoacétyle, 2-méthylaminoacétyle, 2-diméthylaminoacétyle, 2-acétyloxyacétyle, 2-hydroxyacétyle, 2-bromoacétyle, 2-(morpholin-1-yl)acétyle et 2-(pyrrolidin-1-yl)acétyle ; et
$R^4$ est choisi parmi -H et méthyle.

**10.** Composé de formule IB, un sel pharmaceutiquement acceptable de celui-ci, des diastéréoisomères, des énantiomères ou des mélanges de ceux-ci :

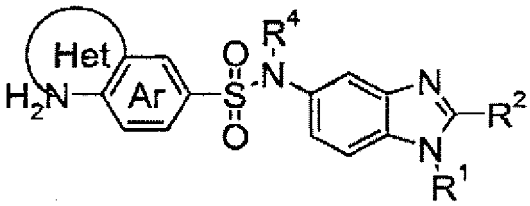

IB

dans laquelle

$R^1$ est choisi parmi $C_{1-10}$alkyle, $C_{2-10}$alcényle, $C_{3-10}$cycloalkyl-$C_{1-4}$alkyle, $C_{4-8}$cycloalcényl-$C_{1-4}$alkyle, $C_{3-6}$hétérocycloalkyl-$C_{1-4}$alkyle, $C_{3-10}$cycloalkyle, $C_{4-8}$cycloalcényle et $C_{3-6}$hétérocycloalkyle où lesdits $C_{1-10}$alkyle, $C_{2-10}$alcényle, $C_{3-10}$cycloalkyl-$C_{1-4}$alkyle, $C_{4-8}$cycloalcényl-$C_{1-4}$alkyle, $C_{3-6}$hétérocycloalkyl-$C_{1-4}$alkyle, $C_{3-10}$cycloalkyle, $C_{4-8}$cycloalcényle et $C_{3-6}$hétérocycloalkyle, utilisés pour définir $R^1$ sont éventuellement substitués par un ou plusieurs groupements choisis parmi halogène, cyano, nitro, méthoxy, éthoxy, méthyle, éthyle, hydroxy, amino, $C_{1-6}$alkylamino et di$C_{1-6}$alkylamino ;

$R^2$ est choisi parmi $C_{1-10}$alkyle, $C_{2-10}$alcényle, $C_{3-10}$cycloalkyle, $C_{3-10}$cycloalkyl-$C_{1-4}$alkyle et $C_{4-8}$cycloalcényl-$C_{1-4}$alkyle où lesdits $C_{1-10}$alkyle, $C_{2-10}$alcényle, $C_{3-10}$cycloalkyle, $C_{3-10}$cycloalkyl-$C_{1-4}$alkyle et $C_{4-8}$cycloalcényl-$C_{1-4}$alkyle utilisés pour définir $R^2$ sont éventuellement substitués par un ou plusieurs groupements choisis parmi halogène, méthoxy, éthoxy, méthyle, éthyle, hydroxy, amino, $C_{1-6}$alkylamino et di$C_{1-6}$alkylamino ;

"Het" est un hétérocycle azoté (comme le montre la formule IB) qui est condensé avec un cycle phényle "Ar", où "Het" est éventuellement substitué par un ou plusieurs groupements choisis parmi $C_{1-3}$alkyle, halogène, cyano, méthoxy, éthoxy, hydroxy et amino ; et

$R^4$ est choisi parmi -H, $C_{1-6}$alkyle, $C_{2-6}$alcényle, $C_{3-6}$cycloalkyle et $C_{3-6}$cycloalkyl-$C_{1-4}$alkyle.

**11.** Composé selon la revendication 10, **caractérisé en ce que**

$R^1$ est choisi parmi $C_{1-6}$alkyle, $C_{2-6}$alcényle, $C_{3-6}$cycloalkyl-$C_{1-4}$alkyle, $C_{4-6}$cycloalcényl-$C_{1-4}$alkyle et $C_{3-6}$hétérocycloalkyl-$C_{1-4}$alkyle où lesdits $C_{1-6}$alkyle, $C_{2-6}$alcényle, $C_{3-6}$cycloalkyl-$C_{1-4}$alkyle, $C_{4-6}$cycloalcényl-$C_{1-4}$alkyle et $C_{3-6}$hétérocycloalkyl-$C_{1-4}$alkyle utilisés pour définir $R^1$ sont éventuellement substitués par un ou plusieurs groupements choisis parmi halogène, méthoxy, éthoxy, méthyle, hydroxy, amino, $C_{1-6}$alkylamino et di$C_{1-6}$alkylamino ;

$R^2$ est choisi parmi $C_{1-6}$alkyle, $C_{2-6}$alcényle, $C_{3-6}$cycloalkyle, $C_{3-6}$cycloalkyl-$C_{1-4}$alkyle et $C_{4-6}$cycloalcényl-$C_{1-4}$alkyle, où lesdits $C_{1-6}$alkyle, $C_{2-6}$alcényle, $C_{3-6}$cycloalkyle, $C_{3-6}$cycloalkyl-$C_{1-4}$alkyle et $C_{4-6}$cycloalcényl-$C_{1-4}$alkyle utilisés pour définir $R^2$ sont éventuellement substitués par un ou plusieurs groupements choisis parmi halogène, méthoxy, éthoxy et hydroxy ;

"Het" est morpholinyle, où ledit morpholinyle est éventuellement substitué par un ou plusieurs groupements choisis parmi $C_{1-3}$alkyle, halogène, cyano, méthoxy, éthoxy, hydroxy et amino ; et

$R^4$ est choisi parmi -H et $C_{1-3}$alkyle.

**12.** Composé selon la revendication 10, **caractérisé en ce que**

$R^1$ est choisi parmi cyclopentylméthyle, cyclohexylméthyle, cyclobutylméthyle, cyclopropylméthyle, 4,4-difluorocyclohexylméthyle, bicyclo[2,2,1]hept-5-én-2-ylméthyle, tétrahydropyranylméthyle, tétrahydropyranyléthyle, tétrahydrofuranylméthyle, morpholinylméthyle, pipéridinyléthyle, N-méthylpipéridinylméthyle et pipéridinylméthyle ;

$R^2$ est choisi parmi t-butyle, n-butyle, 2-méthyl-2-butyle, isopentyle, 2-méthoxy-2-propyle, 2-hydroxypropyle, trifluorométhyle, 1,1-difluoroéthyle, 2,2,2-trifluoroéthyl, 1-méthylpropyle, 1,1-diméthylpropyle, 1,1-diméthyl-3-butén-1-yle, éthyle et 2-propyle ;

"Het" est morpholinyle, où ledit morpholinyle est éventuellement substitué par un ou plusieurs $C_{1-3}$alkyle ; et

$R^4$ est choisi parmi -H et méthyle ;

**13.** Composé selon la revendication 10, **caractérisé en ce que**

$R^1$ est cyclohexylméthyle, cyclobutylméthyle, cyclopropylméthyle, 4,4-difluorocyclohexaneméthyle, N-méthylpipéridin-2-ylméthyle et tétrahydropyranylméthyle ;

$R^2$ est t-butyle et 1,1-difluoroéthyle ;

"Het" est morpholinyle, où ledit morpholinyle est éventuellement substitué par un ou plusieurs $C_{1-3}$alkyle ; et $R^4$ est choisi parmi -H et méthyle ;

**14.** Composé selon l'une quelconque des revendications 1 à 13, destiné à être utilisé comme médicament.

**15.** Utilisation d'un composé selon l'une quelconque des revendications 1 à 13, dans la fabrication d'un médicament destiné à la thérapie de la douleur.

**16.** Utilisation d'un composé selon l'une quelconque des revendications 1 à 13, dans la fabrication d'un médicament destiné au traitement des troubles de l'anxiété.

**17.** Utilisation d'un composé selon l'une quelconque des revendications 1 à 13, dans la fabrication d'un médicament destiné au traitement du cancer, de la sclérose en plaques, de la maladie de Parkinson, du cancer, de la chorée de Huntington, de la maladie d'Alzheimer, des troubles gastro-intestinaux et des troubles cardiovasculaires.

**18.** Composition pharmaceutique comprenant un composé selon l'une quelconque des revendications 1 à 13 et un support pharmaceutiquement acceptable.

**19.** Procédé de préparation d'un composé de formule I,

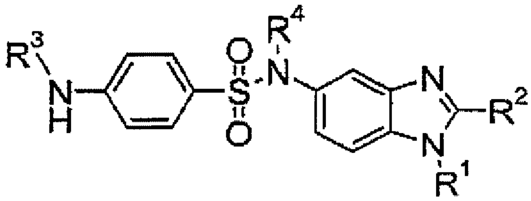

I

comprenant :

la réaction d'un composé de formule II,

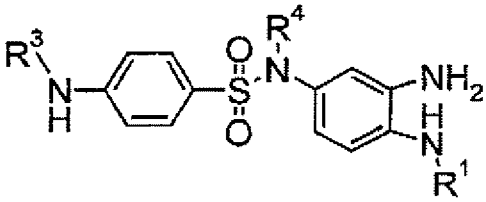

II

avec un composé de formule $R^2COX$ en présence d'une base, telle qu'une alkylamine, et éventuellement d'un réactif de couplage, suivie de traitement par un acide ;

dans lesquelles
X est choisi parmi Cl, Br, F et OH ;
$R^1$ est choisi parmi $C_{1-10}$alkyle, $C_{2-10}$alcényle, $C_{3-10}$cycloalkyl-$C_{1-4}$alkyle, $C_{4-8}$cycloalcényl-$C_{1-4}$alkyle, $C_{3-6}$hétérocycloalkyl-$C_{1-4}$alkyle, $C_{3-10}$cycloalkyle, $C_{4-8}$cycloalcényle et $C_{3-6}$hétérocycloalkyle où lesdits $C_{1-10}$alkyle, $C_{2-10}$alcényle, $C_{3-10}$cycloalkyl-$C_{1-4}$alkyle, $C_{4-8}$cycloalcényl-$C_{1-4}$alkyle, $C_{3-6}$hétérocycloalkyl-$C_{1-4}$alkyle, $C_{3-10}$cycloalkyle, $C_{4-8}$cycloalcényle et $C_{3-6}$hétérocycloalkyle, utilisés pour définir $R^1$ sont éventuellement substitués par un ou plusieurs groupements choisis parmi halogène, cyano, nitro, méthoxy, éthoxy, méthyle, éthyle, hydroxy, amino, $C_{1-6}$alkylamino et di$C_{1-6}$alkylamino ;
$R^2$ est choisi parmi $C_{1-10}$alkyle, $C_{2-10}$alcényle, $C_{3-10}$cycloalkyle, $C_{3-10}$cycloalkyl-$C_{1-4}$alkyle et $C_{4-8}$cycloalcényl-$C_{1-4}$alkyle où lesdits $C_{1-10}$alkyle, $C_{2-10}$alcényle, $C_{3-10}$cycloalkyle, $C_{3-10}$cycloalkyl-$C_{1-4}$alkyle et $C_{4-8}$cycloalcényl-

$C_{1-4}$alkyle utilisés pour définir $R^2$ sont éventuellement substitués par un ou plusieurs groupements choisis parmi halogène, méthoxy, éthoxy, méthyle, éthyle, hydroxy, amino, $C_{1-6}$alkylamino et di$C_{1-6}$alkylamino ;
$R^3$ est choisi parmi -H, $C_{1-6}$alkyle et $C_{1-6}$acyle éventuellement substitués par un ou plusieurs groupements choisis parmi $CH_3C(=O)$-O-, halogène, cyano, méthoxy, éthoxy, hydroxy, amino, alkylamino, dialkylamino et $C_{3-6}$hétérocycloalkyle ; et
$R^4$ est choisi parmi -H, $C_{1-6}$alkyle, $C_{2-6}$alcényle, $C_{3-6}$cycloalkyle et $C_{3-6}$cycloalkyl-$C_{1-4}$alkyle.

**20.** Composé de 2-bromo-*N*-(4-{[[2-tert-butyl-1-(cyclohexylméthyl)-1*H*-benzimidazol-5-yl](méthyl)amino]sulfonyl}phényl)acétamide.

**21.** Procédé de préparation d'un composé de formule Ia,

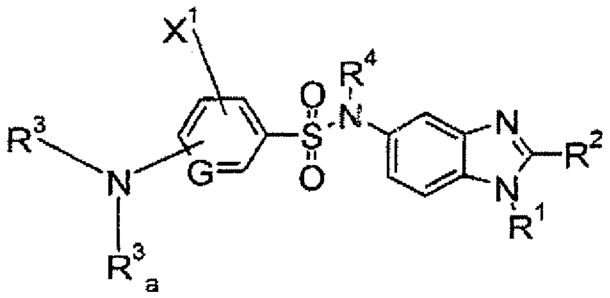

**IA**

comprenant :

la réaction d'un composé de formule IIA,

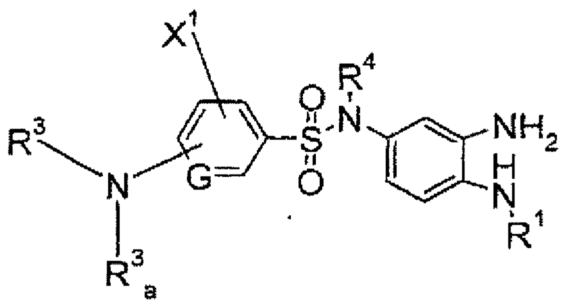

**IIA**

avec un composé de formule $R^2$COX en présence d'une base, telle qu'une alkylamine, et éventuellement d'un réactif de couplage, suivie de traitement par un acide ;
dans lesquelles
X et $X^1$ sont choisis indépendamment parmi Cl, Br, F et OH ;
G est CH ou N ;
$R^1$ est choisi parmi $C_{1-10}$alkyle, $C_{2-10}$alcényle, $C_{3-10}$cycloalkyl-$C_{1-4}$alkyle, $C_{4-8}$cycloalcényl-$C_{1-4}$alkyle, $C_{3-6}$hétérocycloalkyl-$C_{1-4}$alkyle, $C_{3-10}$cycloalkyle, $C_{4-8}$cycloalcényle et $C_{3-6}$hétérocycloalkyle où lesdits $C_{1-10}$alkyle, $C_{2-10}$alcényle, $C_{3-10}$cycloalkyl-$C_{1-4}$alkyle, $C_{4-8}$cycloalcényl-$C_{1-4}$alkyle, $C_{3-6}$hétérocycloalkyl-$C_{1-4}$alkyle, $C_{3-10}$cycloalkyle, $C_{4-8}$cycloalcényle et $C_{3-6}$hétérocycloalkyle utilisés pour définir $R^1$ sont éventuellement substitués par un ou plusieurs groupements choisis parmi halogène, cyano, nitro, méthoxy, éthoxy, méthyle, éthyle, hydroxy, $CH_3C(=O)$-O-, amino, $C_{1-6}$alkylamino et di$C_{1-6}$alkylamino ;
$R^2$ est choisi parmi $C_{1-10}$alkyle, $C_{2-10}$alcényle, $C_{3-10}$cycloalkyle, $C_{3-10}$cycloalkyl-$C_{1-4}$alkyle et $C_{4-8}$cycloalcényl-$C_{1-4}$alkyle où lesdits $C_{1-10}$alkyle, $C_{2-10}$alcényle, $C_{3-10}$cycloalkyle, $C_{3-10}$cycloalkyl-$C_{1-4}$alkyle et $C_{4-8}$cycloalcényl-$C_{1-4}$alkyle utilisés pour définir $R^2$ sont éventuellement substitués par un ou plusieurs groupements choisis parmi halogène, $C_{3-5}$hétéroaryle, méthoxy, éthoxy, méthyle, éthyle, hydroxy, amino, $C_{1-6}$alkylamino et di$C_{1-6}$alkylamino ;

$R^3$ et $R^3_a$ sont choisis indépendamment parmi -H, $C_{1-6}$alkyle, $C_{2-6}$alcényle, $C_{1-3}$alkyl-O-C(=O)-, $C_{1-6}$alkyl-HN-C(=O)-, $H_2$N-C(=O)- et $C_{1-6}$acyle où lesdits $C_{1-6}$alkyle, $C_{2-6}$alcényle et $C_{1-6}$acyle utilisés pour définir $R^3$ sont éventuellement substitués par un ou plusieurs groupements choisis parmi $CH_3C$(=O)-O-, halogène, cyano, méthoxy, éthoxy, hydroxy, amino, $C_{1-6}$alkylamino, di$C_{1-6}$alkylamino et $C_{3-6}$hétérocycloalkyle ; et

$R^4$ est choisi parmi -H, $C_{1-6}$alkyle, $C_{2-6}$alcényle, $C_{3-6}$cycloalkyle et $C_{3-6}$cycloalkyl-$C_{1-4}$alkyle.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Nomenclature of Organic Chemistry, Sections A, B, C, D, E, F, and H. Pergamon Press, 1979